(19) Europäisches Patentamt
European Patent Office
Office européen des brevets

(11) EP 4 538 276 A1

(12) EUROPEAN PATENT APPLICATION
published in accordance with Art. 153(4) EPC

(43) Date of publication:
**16.04.2025 Bulletin 2025/16**

(21) Application number: **23839929.9**

(22) Date of filing: **11.07.2023**

(51) International Patent Classification (IPC):
***C07D 491/22*** (2006.01) ***A61K 31/4745*** (2006.01)
***A61K 47/68*** (2017.01) ***A61P 35/00*** (2006.01)
***G01N 33/574*** (2006.01) ***A61K 47/65*** (2017.01)

(52) Cooperative Patent Classification (CPC):
**A61K 31/4745; A61K 47/65; A61K 47/68; A61P 35/00; C07D 491/22; G01N 33/574**

(86) International application number:
**PCT/KR2023/009854**

(87) International publication number:
**WO 2024/014837 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.07.2022 KR 20220085227**
**18.08.2022 KR 20220103591**
**08.09.2022 KR 20220114079**
**07.12.2022 KR 20220170146**
**13.03.2023 KR 20230032226**
**20.04.2023 KR 20230052142**

(71) Applicant: **Pinotbio, Inc.**
**Suwon-si, Gyeonggi-do 16506 (KR)**

(72) Inventors:
- **JUNG, Doo Young**
  **Suwon-si Gyeonggi-do 16506 (KR)**
- **LEE, Jin Soo**
  **Suwon-si Gyeonggi-do 16506 (KR)**
- **CHO, Hyun Yong**
  **Suwon-si Gyeonggi-do 16506 (KR)**
- **LEE, Byeong Sung**
  **Suwon-si Gyeonggi-do 16056 (KR)**
- **JUNG, Jin Kyo**
  **Suwon-si Gyeonggi-do 16506 (KR)**
- **SHIN, Seung Gun**
  **Suwon-si Gyeonggi-do 16506 (KR)**
- **KWEON, Sohui**
  **Suwon-si Gyeonggi-do 16505 (KR)**
- **MATHI, Gangadhar Rao**
  **Suwon-si Gyeonggi-do 16506 (KR)**

(74) Representative: **Prüfer & Partner mbB**
**Patentanwälte · Rechtsanwälte**
**Sohnckestraße 12**
**81479 München (DE)**

# (54) DDX5 PROTEIN-BINDING CAMPTOTHECIN DERIVATIVES AND PRODRUGS THEREOF

(57) **[Summary]**

The present invention relates to (a) an active camptothecin derivative represented by Chemical Formula 1, which is designed to bind to DDX5 protein and E3 ligase; (b) a prodrug thereof, preferably an antibody-drug conjugate (ADC) thereof, which is designed to release the active camptothecin derivative (a) at a target site in vivo; or (c) a complex containing a DDX5 protein-targeting ligand, in which the ability of the active camptothecin derivative (a) or an FL118 compound of Chemical Formula 2 to inhibit type 1 topoisomerase is inactivated through a linker connection.

The active camptothecin derivative designed according to the present invention can bind to DDX5 protein in cells to induce cell death through DDX5 protein degradation.

EP 4 538 276 A1

[FIG. 1]

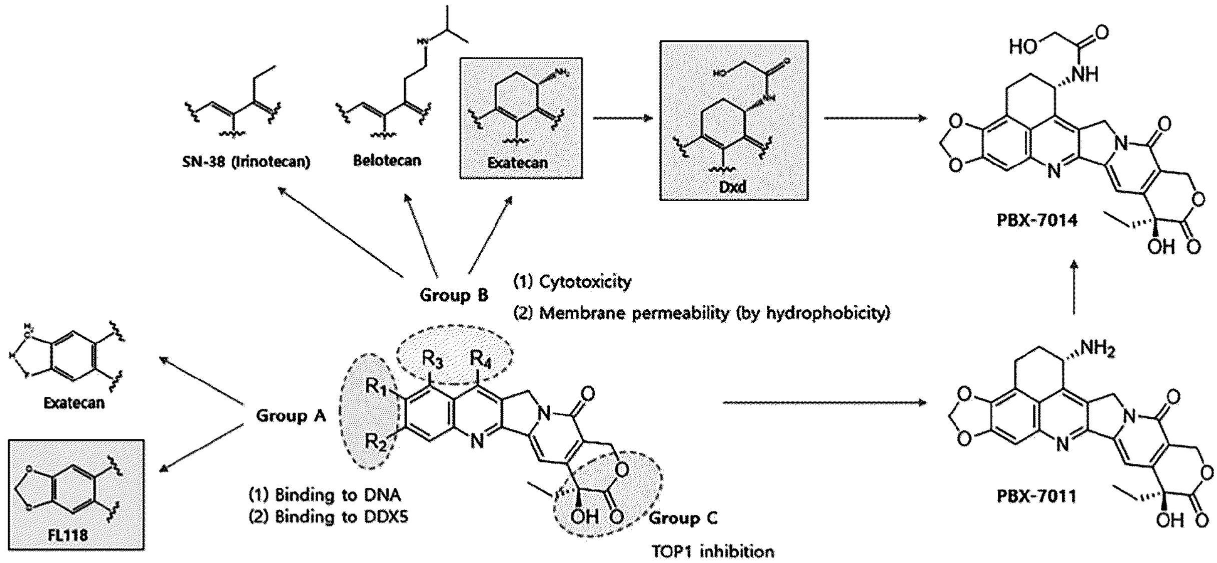


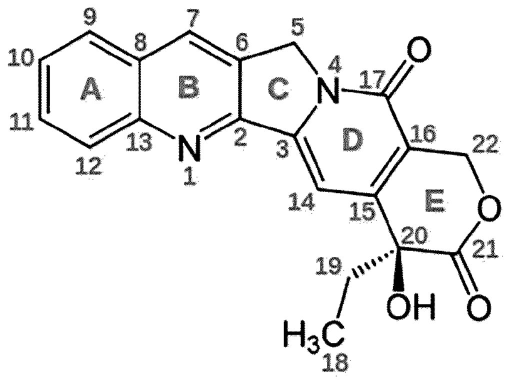

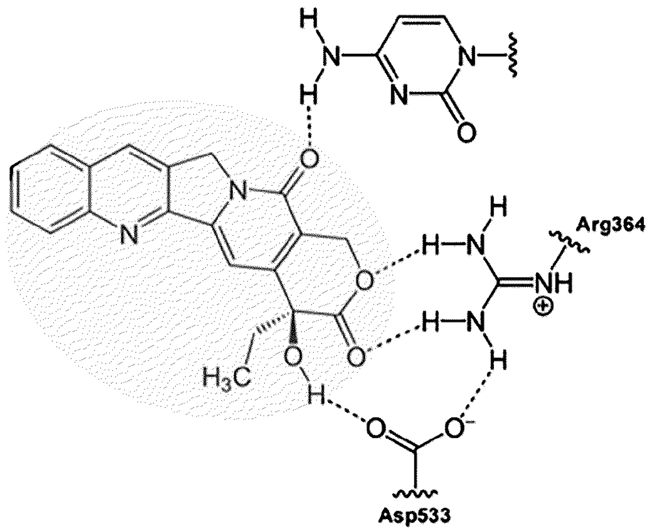

Binding of CPT to topoisomerase I

## Description

### [Technical Field]

The present invention relates to (a) an active camptothecin derivative represented by Chemical Formula 1 below, which is designed to bind to DDX5 protein and E3 ligase; (b) a prodrug thereof, preferably a carrier-drug-conjugate (CDC) thereof, particularly an antibody-drug conjugate (ADC) thereof, which is designed to release the active camptothecin derivative (a) at a target site in vivo; or (c) a complex containing a DDX5 protein-targeting ligand, in which the ability of the active camptothecin derivative (a) or an FL118 compound of Chemical Formula 2 to inhibit type 1 topoisomerase is inactivated through a linker connection.

The active camptothecin derivative designed according to the present invention can bind to DDX5 protein in cells to induce cell death through DDX5 protein degradation.

[Background Art]

Cancer genes refer to genes that originally exist to regulate the normal function of cells, but can cause cancer when they are mutated or abnormally regulated. One of the normal cell functions is the function of cell death, and in this regard, there are death promoter and death suppressor.

For example, the cancer genes include (i) oncogenes that actively promote cell division when mutations occur, and (ii) tumor suppressor genes that do not suppress cell division when mutations occur. Examples of the oncogenes include Src, EGFR, HER2, RAS, APC, BCL-2, etc., and examples of the tumor suppressor genes include p53, BRCA, Rb, PTEN, BAX, etc.

Cancer is a group of diseases in which the cell cycle of growth and division is not regulated. As shown in FIG. 30, cancer may occur when the mechanism of programmed cell death (e.g., apoptosis) is impaired.

The cancer is caused by mutations in genes in which protein products are involved in cell cycle regulation. Many cancers are associated with overexpression of specific genes or abnormal activity of mutant protein products (Oncogenes).

A single mutation does not usually cause cancer. Usually, Several genes that control cell growth are mutated before cancer occurs.

HER2-positive breast cancer, in which HER2 is abnormally expressed in breast epithelial cells, accounts for about ¼ of all breast cancer patients and has a higher recurrence rate and worse prognosis when treated with chemotherapy compared to female hormone-related breast cancer. Herceptin (component name: Trastuzumab), a HER2-targeting drug, treats breast cancer by reducing the number of HER2 on the cell surface and helping immune cells kill HER2-positive breast cancer cells.

HER2-positive breast cancer is breast cancer that has amplification or overexpression of the HER2 (ErbB2) oncogene, a member of the ErbB receptor. Trastuzumab (Herceptin) has increased the treatment effect of HER2-positive breast cancer, but HER2-positive breast cancer shows an aggressive pattern compared to other breast cancers, and more than half of the patients are resistant to existing targeted therapies or develop resistance during treatment.

Ligands of ErbB receptors attached to the cell membrane include NRG1 and HB-EGF, and activated ErbB ligands bind to their receptors (EGFR, HER3) and catalyze phosphorylation (P) by binding between receptors (EGFR-HER2, HER2-HER3), thereby inducing intracellular growth and proliferation signals. Such excessive activation of ErbB receptors causes resistance to anti-HER2 therapeutics.

Meanwhile, deregulated transcription factors may decrease the sensitivity of cancer cells to chemotherapy or radiotherapy. Drug resistance in cancer treatment may be either endogenous or acquired drug resistance. Intrinsic drug resistance in cancer treatment may be caused by abnormally expressed transcription factors that are crucial regulators of cell proliferation and cell death. Abnormal overexpression of NF-κB, STAT3, HIF-1, AR or ER and inactivation of p53 and FOXO3 a in tumor tissues protect cancer cells from death by anticancer drugs, resulting in intrinsic drug resistance.

However, chemotherapy and radiotherapy also induce acquired drug resistance through the activation of deregulated transcription factors. It has been found that ionizing radiation can stimulate the activation and induction of several different transcription factors, including STAT3, NF-κB, and HIF-1. Upregulation of NF-κB and STAT3 induces transcription of anti-apoptotic genes in cancer cells and promotes cell cycle entry and proliferation of cancer cells, thereby causing radioresistance. In addition, upregulation of transcription factors NF-κB and HIF-1 by radiation can induce genes responsible for the maintenance of EMT cells and cancer stem cells that are resistant to cancer therapy. In addition, activation of transcription factor STAT3 also promoted drug resistance to cancer treatment. Feedback activation of STAT3 by chemotherapy or radiotherapy has been found to be a molecular mechanism of cancer treatment resistance. Therefore, deregulated transcription factors and their primary targets that target together can be promising approaches for overcoming drug resistance in cancer treatment.

STAT (signal transducer and activator of transcription) transcription factor family includes STAT1, STAT2, STAT3,

STAT4, STAT5, and STAT6. STAT proteins can be activated by JAK kinases upon stimulation with growth factors, cytokines, interferons, or oncogenes to regulate interferon signalling. The DNA-binding domain of the STAT is an immunoglobin-like structure that mediates binding to specific DNA target sequences. Phosphorylation of STAT3 by JAK kinases during activation activates dimerization. The dimer then migrates to the nuclear compartment and binds to sequences in the target promoter to induce the expression of target genes. STAT3 target genes include Survivin, bcl-xl, mcl-1, cyclin D1, MMP2, MMP9, VEGF, Myc, Sox2, etc. STAT proteins regulate many biological processes, including cell growth, cell death, differentiation, and immunity. STAT3 and STAT5 within the STAT family have been involved in cancer progression.

Deregulated STAT3 and activated STAT5 are considered oncogenes to increase angiogenesis and enhance cancer cell survival. In a study using many samples from the cBioPortal database, STAT3 was found to be more frequently overexpressed in lung cancer, ovarian cancer, gastric cancer, hematopoietic cancer, and brain cancer compared to normal tissues, and STAT3 overexpression was associated with low overall survival rate. Phosphorylation of STAT3 by JAK kinase is the first step in STAT3 activation. Another study of 90 patients with glioblastoma found that high p-STAT3 levels were significantly associated with a decrease in progression-free survival rate and overall survival rate. Multivariate survival analysis showed that high p-STAT3 levels may serve as a significant prognostic indicator for poor progression-free survival and overall survival.

Inhibitors of type 1 topoisomerase (Irinotecan, Topotecan, etc.) are anticancer mechanisms that have been clinically proven to be effective/safe, and their excellent anticancer efficacy has been verified in various refractory solid cancers such as colon cancer, lung cancer, breast cancer, and ovarian cancer in clinical trials.

In this regard, a camptothecin derivative, a low-molecular-weight compound that inhibits type 1 topoisomerase (topoisomerase-1) to express anti-tumor action, is known.

The camtothecin (CPT) has low solubility in water, and in preparation for clinical trials, the National Cancer Institute (NCI) prepared a water-soluble sodium salt (NSC100880). Phase I and II clinical trials have not been completed due to high toxicity (hemorrhagic cystitis, gastrointestinal toxicity, such as nausea, vomiting, diarrhea, and bone marrow suppression, especially leukopenia and thrombocytopenia) exhibited by the compound.

Subsequently, multiple CPT analogues were synthesized to obtain compounds with lower toxicity and higher water solubility. There are two drugs, irinotecane (CPT-11) and topotecane.

Irinotecan (CPT-11), jointly developed by Dallchi and Yakult in Japan, was the world's first camptothecin-based anticancer agent, which was proven effective against lung cancer (small cell lung cancer and non-small cell lung cancer) and was released in Europe and Japan in 1994, and in 1995, was additionally proven effective against colon cancer and breast cancer. In addition, topotecan, developed by Glaxo Smith Kline, was approved for efficacy against metastatic ovarian cancer by the US FDA in April 1995 and was released. Recently, CKD-602 (belotecan), a new domestically developed camptothecin derivative, has water solubility along with a strong type 1 topoisomerase inhibitor effect, and has successfully overcome the toxicity caused by poor solubility.

All camptothecin derivatives identified so far contain a parent structure with five rings essential for cytotoxicity (FIG. 1). In terms of molecular structure, the E-ring and the A- and B-ring sites have been identified as important sites. Camptothecin includes a pentacyclic lactone structure in the E-ring essential for cytotoxicity. It has been demonstrated that the lactone group and the alpha-hydroxyl group located at carbon 20 of the E-ring are important for the stability of the type 1 topoisomerase-DNA byproduct, and modification of the A- and B-rings can increase water solubility and activity. Alteration of the first ring has been demonstrated to increase water solubility and allow greater tolerance, for example, in the case of the aforementioned drugs.

CKD-602 also attempted to substitute the B-ring site at position 7 to increase water solubility and anticancer effect. Lee et al. reported that CKD-602 had superior anticancer effect than camptothecin and topotecan in a wide range of cancer cell lines. In addition, it was confirmed that it was a relatively safe drug with a maximum tolerated dose (MTD) of 25 mg/kg in the L1210 leukemia nude mouse model. The commonly known side effects of camptothecin drugs can be largely classified into hematological side effects and non-hematological side effects. Hematological side effects include neutropenia accompanied by fever, sepsis, and bleeding, and non-hematological side effects include nausea, vomiting, skin side effects such as hair loss, and toxicity to the gastrointestinal tract, kidney, and nervous system. Kim et al. reported that in an animal study of CKD-602, no adverse drug reactions except for increased gastric secretion among the side effects of camptothecin drugs were found even when administered at a dose 10 times higher than the clinically applicable dose. **In** addition, recent domestic clinical studies have reported only reversible and controllable side effects of neutropenia and leukopenia rather than serious systemic toxicity, proving its relative safety. However, to date, it has been used in a limited manner with the main indications being the treatment of patients who have failed or cannot undergo standard chemotherapy, that is, patients with refractory or recurrent ovarian and colon cancer that have relapsed or worsened after standard treatment and are unlikely to be effective with further chemotherapy or surgery, treatment of refractory or recurrent limited disease small cell lung cancer that has failed first-line chemotherapy, and treatment of extensive disease small cell lung cancer.

SN-38 is a potent topoisomerase-I inhibitor with IC50 values in the nanomolar range in several cell lines. It is the

active form of irinotecan, a prodrug used in the treatment of colorectal cancer, and is also active in lung, breast, and brain cancers. Trop-2-SN-38 ADC is currently being successfully developed for a number of cancers, including TNBC, bladder cancer, and gastric cancer, but resistance problems still remain due to overexpression of drug efflux transporters, epigenetic silencing of Top1, and increased anti-apoptotic proteins.

Meanwhile, Daiichi Sankyo used DXD (exatecan), a cytotoxic drug that is 10 times more active in cancer cells than SN-38, in the development of Enhertu®. DXD has good solubility, is relatively safe, and has a high effect on killing surrounding cells, making it advantageous in the treatment of heterogeneous tumors. However, its half-life, which can reduce off-target effects, is short. DXD is bioconjugated to the cysteine residue of an anti-HER2 antibody with a maleimide linker and has a homogeneous DAR value of 8. Despite its high DAR value, DXD is highly stable, with only 2.1% released in plasma over 21 days (Ogitani et al., 2016). Enhertu was approved by the US FDA in 2019, and the target patients are adults with unresectable metastatic Her2-positive breast cancer who have received HER2-targeted therapy at least twice in the past.

Camptothecin derivatives such as SN-38 and Dxd (a derivative of the clinical-stage topoisomerase I inhibitor Exatecan) have recently been successfully developed as novel payloads for antibody-drug conjugates (ADCs). As a result, two ADCs using camptothecin-based payloads (Trodelvy (Sacituzumab Govitecan) including SN-38 payload and Enhertu (Trastuzumab Deruxtecan) including Dxd payload) recently received FDA approval. These camptothecin-based payloads have moderate cytotoxicity compared to existing ultra-toxic payload ADCs (DAR = 2 or 4) such as MMAE and MMAF, but their superior safety profile allows for higher drug-to-antibody ratio (DARs) such as 8. Additionally, these camptothecin-based payloads allow the use of linker systems with faster drug release profiles, such as CL-2A or GGFG linker systems. The combination of these components has led to the development of novel ADCs with a broader therapeutic range.

Antibody-drug conjugates (ADCs) that use camptothecin derivatives, such as Trodelvy and Enhertu, as payloads have been successful in delivering camptothecin compounds that exhibit strong anticancer efficacy selectively to cancer cells, thereby achieving excellent anticancer efficacy without severe systemic side effects. However, they still have several shortcomings, the most representative of which is the occurrence of resistance due to overexpression of the ABCG2 Drug Efflux Pump. Many camptothecin derivatives are rapidly released from cells by ABCG2, which results in a problem in which the therapeutic efficacy is seriously reduced in cancer cells that overexpress ABCG2 compared to cancer cells that do not express ABCG2 or express it at a normal level. This overexpression of ABCG2 is a main cause of reducing the therapeutic efficacy not only in small molecule camptothecin compounds such as Irinotecan and Topotecan, but also in ADCs, such as Trodelvy and Enhertu, that use camptothecin compounds as payloads.

## [Disclosure]

### [Technical Problem]

The present invention aims to provide a camptothecin derivative designed to bind to DDX5 protein and E3 ligase as a drug candidate that kills target cells.

The present inventors have synthesized an FL118 compound (FIG. 2), a camptothecin-based drug which has a dual MoA that degrades oncoprotein DDX5 (p68) while having excellent type 1 topoisomerase inhibition ability, and have been conducting various studies on its physicochemical properties. However, the FL118 compound had limitations in the development of formulation due to its limited physicochemical properties.

Accordingly, the present inventors intend to design and synthesize a camptothecin derivative with a novel structure that exhibits a dual mechanism of action (dual MoA) in terms of type 1 topoisomerase inhibition and DDX5 degradation, which are advantages of FL118, based on the structure of FL118 that is differentiated from SN38 drug.

Therefore, the present invention aims to newly design and provide an active camptothecin derivative having a dual mechanism of action (dual MoA) that degrades oncoprotein DDX5 together with the ability to inhibit type 1 topoisomerase, and/or a carrier-drug conjugate (CDC), particularly an antibody-drug conjugate (ADC), which is a prodrug thereof designed to release the active camptothecin derivative in vivo.

In addition, the present invention aims to provide an anticancer mechanism as a new modality that kills cancer cells and/or surrounding cells before endogenous or acquired drug resistance by causing the active camptothecin derivative to bind to DDX5 protein in cells and induce cell death through DDX5 protein degradation together with a bystander effect.

### [Technical Solution]

The first aspect of the present invention provides (a) an active camptothecin derivative represented by Chemical Formula 1 below, which is designed to bind to DDX5 protein and E3 ligase; (b) a prodrug thereof, preferably a carrier-drug-conjugate (CDC), more preferably an antibody-drug conjugate (ADC), which is designed to release the active camptothecin derivative (a) at a target site in vivo; or (c) a complex containing a DDX5 protein-targeting ligand, in which the ability of the active camptothecin derivative (a) or an FL118 compound of Chemical Formula 2 to inhibit type 1 topoisomerase is inactivated through a linker connection:

[Chemical Formula 1]

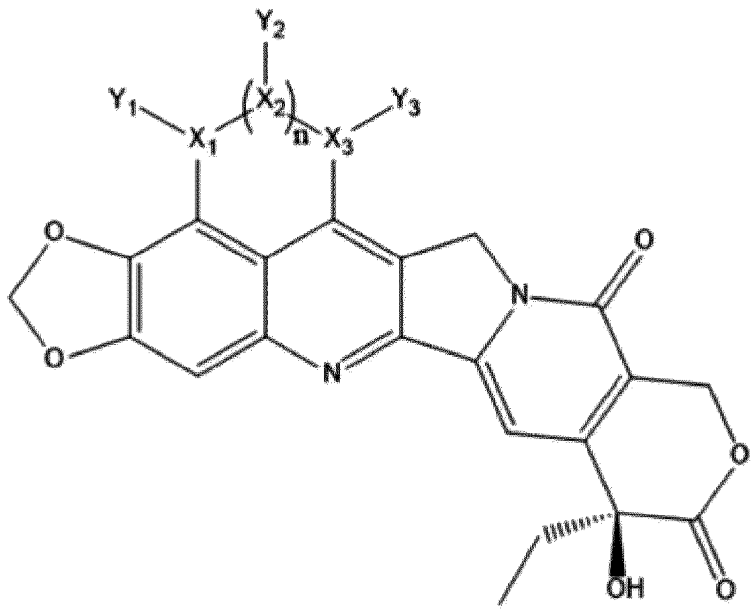

wherein $X_1$ and $X_3$ are each independently carbon, oxygen, nitrogen, or sulfur, and $X_1$ and $X_3$ may be the same or different,
$X_2$ is carbon, oxygen, nitrogen, sulfur, a single bond, or a double bond,
$X_1$, $(X_2)n$, and $X_3$ may form a six-membered or seven-membered ring (n=1 to 2),
$Y_1$, $Y_2$, and $Y_3$ may each independently be hydrogen, or a functional group containing oxygen, nitrogen, phosphorus, or sulfur.

In the complex containing a DDX5 protein-targeting ligand, the ligand targeting the DDX5 protein may be a ligand that binds to the DDX5 protein.
The active camptothecin derivative may be designed to exert a surrounding cell killing effect (bystander effect) in cancer tissue or control the degree of the effect by modifying X1, (X2)n, X3, Y1, Y2, and/or Y3 in Chemical Formula 1 to control its hydrophobicity or cell membrane permeability as desired.
Preferably, the active camptothecin derivative (a) designed to bind to DDX5 protein and E3 ligase can serve as a ligand (binder) that binds to E3 ligase as a molecular glue degrader; may kill target cells expressing DDX5 protein through a molecular glue degrader mechanism of action (MoA); and/or may have a mechanism of action (MoA) that degrades oncoprotein DDX5 together with the ability to inhibit type 1 topoisomerase.
The second aspect of the present invention provides a pharmaceutical composition for preventing or treating cancer or a composition for diagnosing cancer, including the active camptothecin derivative (a) of the first aspect, a prodrug thereof (b), preferably a carrier-drug conjugate (CDC), more preferably an antibody-drug conjugate (ADC), or a complex containing a DDX5 protein-targeting ligand (c).
For example, it may be administered to treat HER2 positive cancer, breast cancer, lung cancer, and colon cancer.
Preferably, the pharmaceutical composition for preventing or treating cancer may be used as a first-line treatment after cancer diagnosis or may be administered to an individual that (over)expresses DDX5 in cancer tissue.
The third aspect of the present invention provides a method for preparing (a) an active camptothecin derivative designed to bind to DDX5 protein and E3 ligase; (b) a prodrug thereof, preferably a carrier-drug-conjugate (CDC), more preferably an antibody-drug conjugate (ADC), which is designed to release the active camptothecin derivative (a) at a

target site in vivo; or (c) a complex containing a DDX5 protein-targeting ligand, in which the ability of the active camptothecin derivative (a) or an FL118 compound of Chemical Formula 2 to inhibit type 1 topoisomerase is inactivated through a linker connection, wherein the method is characterized by including a step of designing the active camptothecin derivative (a) to include a camptothecin-based skeleton represented by Chemical Formula 1 above as a parent nucleus, selecting a compound designed in this way, or synthesizing a compound designed in this way.

For example, the step of designing the active camptothecin derivative (a) to include a camptothecin-based skeleton represented by Chemical Formula 1 as a parent nucleus or selecting a compound designed in this way may be:

(1) a step of selecting an active camptothecin derivative designed to have a mechanism of action (MoA) for inhibiting type 1 topoisomerase and/or a mechanism of action (MoA) for degrading oncoprotein DDX5 from a compound library including a camptothecin-based skeleton represented by Chemical Formula 1 as a parent nucleus, and/or
(2) a step of confirming through in vitro experiments and/or in vivo experiments whether a compound including a camptothecin-based skeleton represented by Chemical Formula 1 as a parent nucleus inhibits type 1 topoisomerase and/or degrades oncoprotein DDX5.

The fourth aspect of the present invention provides a method for preparing (a) an active camptothecin derivative that binds to DDX5, which acts as an oncoprotein in a cell, and induces cell death through DDX5 protein degradation, (b) a prodrug thereof, preferably a carrier-drug-conjugate (CDC), more preferably an antibody-drug conjugate (ADC), which is designed to release the active camptothecin derivative (a) in vivo, or (c) a complex containing a DDX5 protein-targeting ligand, in which the ability of the active camptothecin derivative (a) to inhibit type 1 topoisomerase is inactivated through a linker connection, wherein the method is characterized by including: a first step of selecting the active camptothecin derivative (a) from the group consisting of a compound of Chemical Formula 3, a compound of Chemical Formula 4, a compound of Chemical Formula 5, a compound of Chemical Formula 6, a compound of Chemical Formula 7, a compound of Chemical Formula 8, a compound of Chemical Formula 9, and isomers thereof; and optionally, a second step of selecting a prodrug (b) thereof designed to release the active camptothecin derivative (a) selected in the first step in vivo.

[Chemical Formula 3]

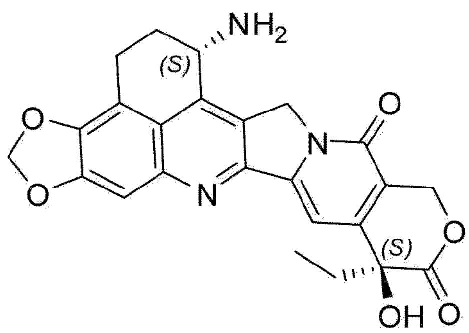

[Chemical Formula 4]

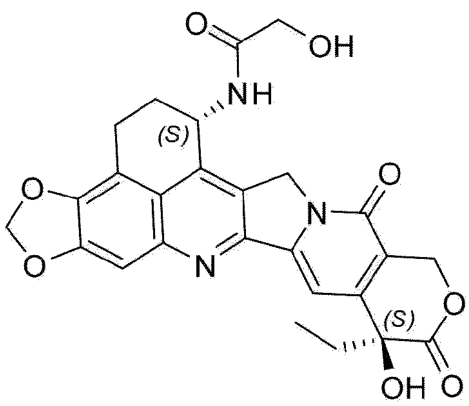

[Chemical Formula 5]

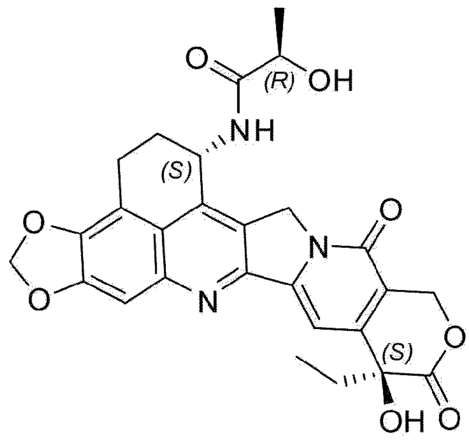

[Chemical Formula 6]

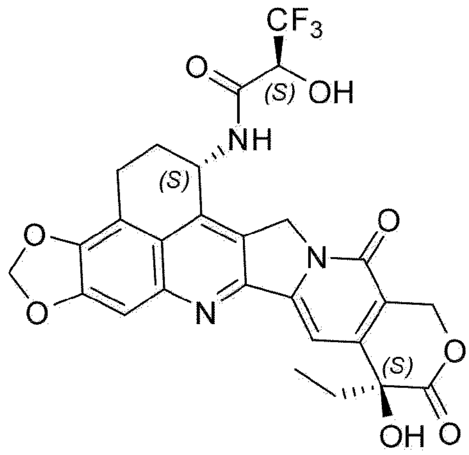

[Chemical Formula 7]

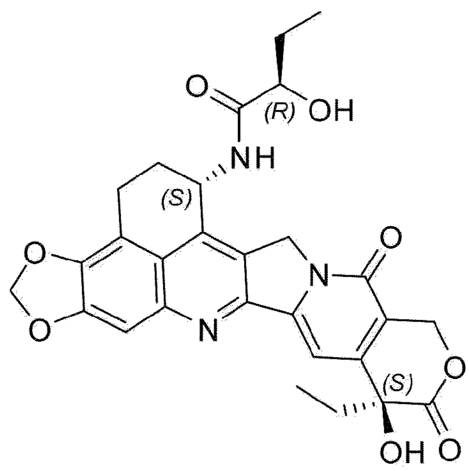

[Chemical Formula 8]

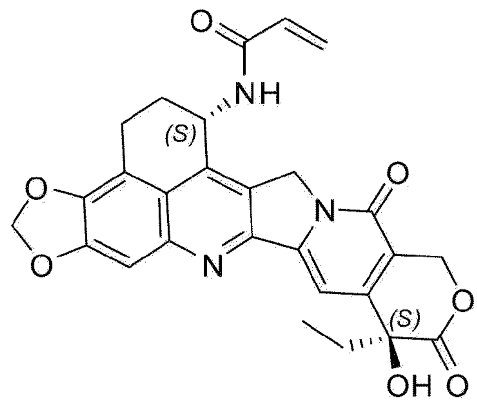

[Chemical Formula 9]

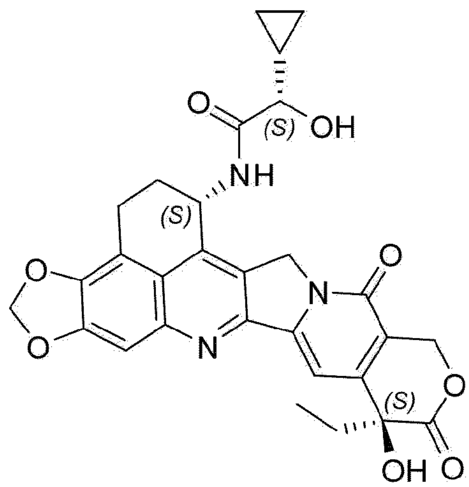

The preparing method of the third or fourth aspect may further include a step of identifying a patient group to which the active camptothecin derivative (a) or the prodrug thereof (b) is to be administered, by quantitatively or qualitatively analyzing intracellular DDX5 protein using the complex containing a DDX5 protein-targeting ligand, in which the ability of the FL118 compound of Chemical Formula 2 or the active camptothecin derivative (a) to inhibit type 1 topoisomerase is inactivated through a non-cleavable linker connection, in a tissue biopsy or liquid biopsy.

The fifth aspect of the present invention provides a compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 below, or a pharmaceutically acceptable salt thereof.

The sixth aspect of the present invention provides a pharmaceutical composition for preventing or treating cancer, including a compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, a pharmaceutically acceptable salt thereof, a solvate thereof, or a prodrug thereof, preferably a carrier-drug conjugate (CDC), more preferably an antibody-drug conjugate (ADC).

[Chemical Formula 3]

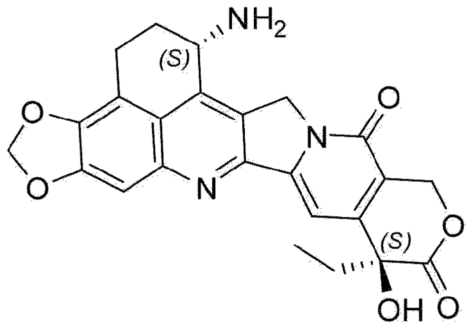

[Chemical Formula 3-1]

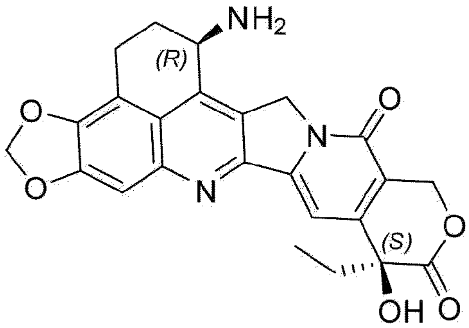

[Chemical Formula 4]

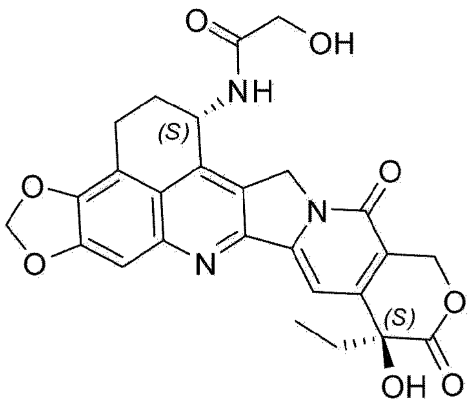

[Chemical Formula 4-1]

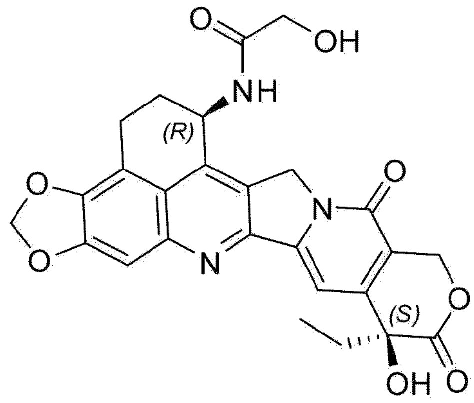

[Chemical Formula 5]

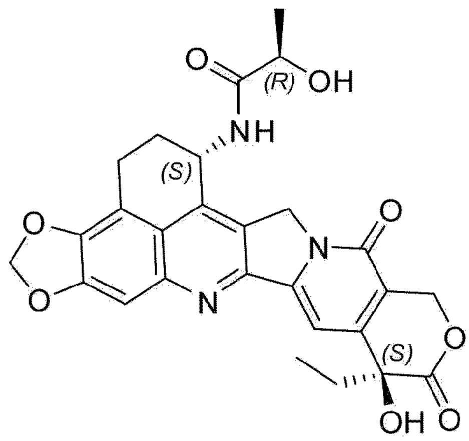

[Chemical Formula 5-1]

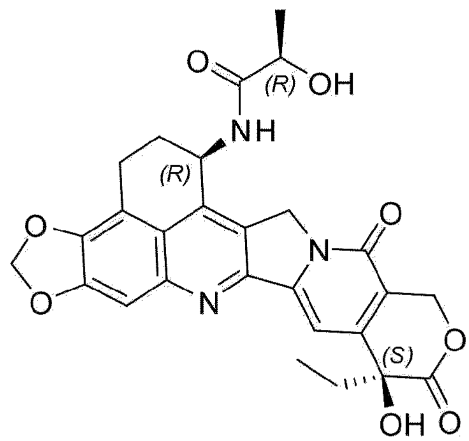

[Chemical Formula 6]

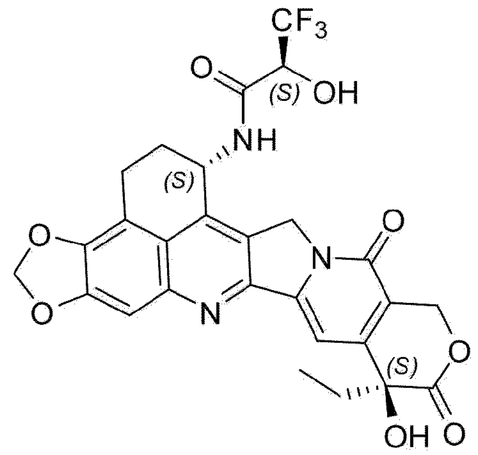

[Chemical Formula 7]

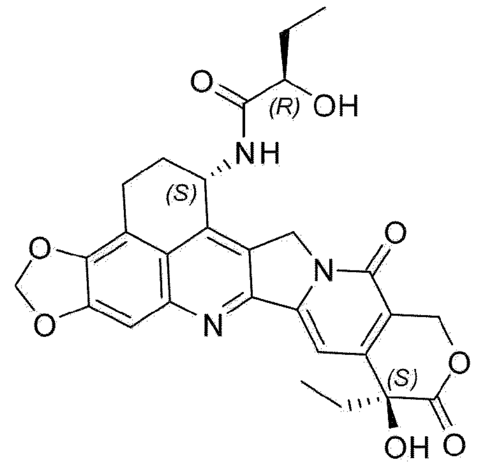

[Chemical Formula 8]

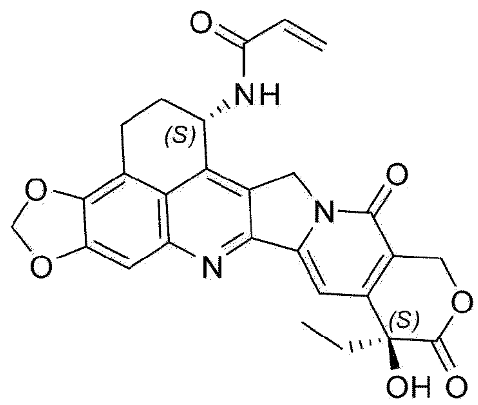

[Chemical Formula 9]

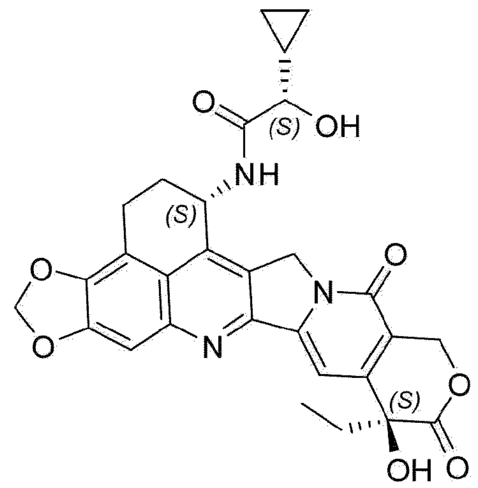

The seventh aspect of the present invention provides a pharmaceutical composition for cell death, characterized by including (a) an active camptothecin derivative represented by Chemical Formula 1 below, which is designed to bind to DDX5 protein and E3 ligase; or (b) a prodrug thereof, preferably a carrier-drug-conjugate (CDC), more preferably an antibody-drug conjugate (ADC), which is designed to release the active camptothecin derivative (a) at a target site in vivo.

The eighth aspect of the present invention provides an anticancer formulation characterized in that (a) an active camptothecin derivative represented by Chemical Formula 1 below, which is designed to bind to DDX5 protein and E3 ligase; or (b) a prodrug thereof, preferably a carrier-drug-conjugate (CDC), more preferably an antibody-drug conjugate (ADC), which is designed to release the active camptothecin derivative (a) at a target site in vivo is administered in a dosage capable of stimulating antigen-presenting cells (APCs) through cell death of cancer cells to induce an antitumor immune response.

The ninth aspect of the present invention provides a method for preparing an active camtothecin derivative (a)-based anticancer agent in which a surrounding cell killing effect (bystander effect) and/or ADME profile is controlled so that T cell exhaustion does not occur due to chronic antigen exposure, or so that it is rapidly or slowly removed via lymphatic drainage by providing a desired degree of hydrophobicity and/or controlling aggregation, wherein the active camptothecin derivative (a)-based anticancer agent is an anticancer formulation containing: (a) an active camptothecin derivative designed to bind to DDX5 protein and E3 ligase as a molecular glue degrader; or (b) a prodrug thereof, preferably a carrier-drug-conjugate (CDC), more preferably an antibody-drug conjugate (ADC), which is designed to release the active camptothecin derivative (a) at a target site in vivo, wherein the method is characterized by including a step of designing the active camptothecin derivative (a) or prodrug thereof (b) to include a camptothecin-based skeleton represented by Chemical Formula 1 below as a parent nucleus, selecting a compound designed in this way, or synthesizing a compound designed in this way.

Hereinafter, the present invention will be described.

**In** this specification, a drug is any substance used for the diagnosis, cure, alleviation, treatment or prevention of a disease (excluding food or device) or used to affect the structure or function of the body. For example, it is any chemical or biological substance that affects the body and body metabolism. Preferably, it belongs to the category of drugs as long as it binds to DDX5 protein.

For example, the drug of the present invention may be an active camptothecin derivative itself or a prodrug thereof, an active camptothecin derivative as a payload of a prodrug such as a targeted drug delivery (e.g., a carrier-drug conjugate), a camptothecin derivative or a prodrug thereof as a diagnostic agent.

**In** order for a drug to work effectively in vivo, the concentration of the drug at the target site in the body must be maintained within the therapeutic range for a certain period of time (Example 8, Table 6, FIG. 23). When the drug exists in excess in the body, it becomes toxic, and when the amount is too small at the target site, it does not exhibit a therapeutic effect.

Drug efficacy means that the drug remains in the body without being decomposed for the expected period of time showing an effect on the target indication (Example 8, Table 6, FIG. 23). A lower metabolic rate leads to a longer duration of efficacy because the time for which blood concentration is maintained is longer.

Meanwhile, in vivo efficacy and in vivo side effects of anticancer drugs are closely related to the absorption, distribution, metabolism, and excretion (ADME) characteristics of anticancer drugs. The ADME of a drug determines the

pharmacokinetics which describes how the drug moves through the body and interacts with tissues and organs.

The absorption of a drug can affect its efficacy and side effects. A drug that is poorly absorbed may not reach therapeutic concentrations in target tissues, which may result in reduced efficacy. On the other hand, a drug that is well absorbed may increase systemic exposure, which may result in non-target adverse effects.

The distribution of a drug can also affect its efficacy and side effects. A drug that is poorly distributed in target tissues may be ineffective, while a drug that is widely distributed in non-target tissues may cause toxicity in those tissues. Additionally, a drug that is highly bound to plasma proteins may have reduced distribution to target tissues, which may result in reduced efficacy.

Drug distribution in the body can be influenced by physicochemical properties, including size, charge, and lipophilicity. The ability of a drug to penetrate tumor tissue can also be influenced by factors such as the tumor microenvironment, including blood flow and cell density. Some anticancer drugs may accumulate in certain tissues and cause toxic effects.

The metabolism of a drug can also affect both efficacy and side effects. A Drug that is rapidly metabolized may have reduced efficacy due to its short half-life, while a drug that is slowly metabolized may have increased toxicity due to accumulation. In addition, some metabolites are more toxic than the parent drug and can cause side effects.

The rate and route of excretion can also affect both efficacy and side effects. A Drug that is rapidly eliminated may have reduced efficacy due to its short half-life, while a drug that is slowly eliminated may accumulate and cause toxicity. In addition, some drugs may be excreted unchanged or as active metabolites, which may result in long-term exposure or increased exposure and increased risk of side effects.

Therefore, it is essential to analyze the ADME properties of anticancer drugs to optimize their efficacy in vivo and minimize side effects. That is, evaluating and optimizing the ADME profile can improve the therapeutic index of these drugs, leading to better outcomes for cancer patients.

An anti-cancer formulation or composition is a medication or therapy designed to treat cancer or prevent the growth and spread of cancer cells in the body. The anti-cancer formulation may include a combination of drugs, chemical compounds, and/or biological agents that target cancer cells by interfering with the growth and division ability of cancer cells.

The formulation of an anti-cancer drug may reflect a complex process involving multiple stages, including drug discovery, pre-clinical development, clinical trials, and regulatory approval. The goal is to develop a safe and effective treatment that can be administered to patients with minimal side effects.

The development of an anti-cancer formulation involves designing a drug or combination of drugs capable of identifying target cancer cells and selectively targeting these cells while sparing healthy cells. This can be accomplished by targeting specific proteins or enzymes that are overexpressed in cancer cells, or by interfering with the cell cycle, DNA replication, or other cellular processes essential for cancer cell growth and survival.

Once a promising drug candidate is identified, it undergoes preclinical development, where the drug is tested in laboratory and animal models to assess safety and efficacy. If the drug shows promise in preclinical studies, it may proceed to clinical trials, where it is tested in humans to determine safety, dose, and effectiveness.

During clinical trials, drug formulations are optimized to be safely administered to patients and achieve the desired therapeutic effect. This may include adjusting the dose, route of administration, or formulation of the drug to improve efficacy and minimize side effects.

The present invention provides, as a drug candidate that kills target cells, (a) an active camptothecin derivative represented by Chemical Formula 1 below, which is designed to bind to DDX5 protein and E3 ligase; or (b) a prodrug thereof, preferably a carrier-drug-conjugate (CDC), more preferably an antibody-drug conjugate (ADC), which is designed to release the active camptothecin derivative (a) at a target site in vivo.

[Chemical Formula 1]

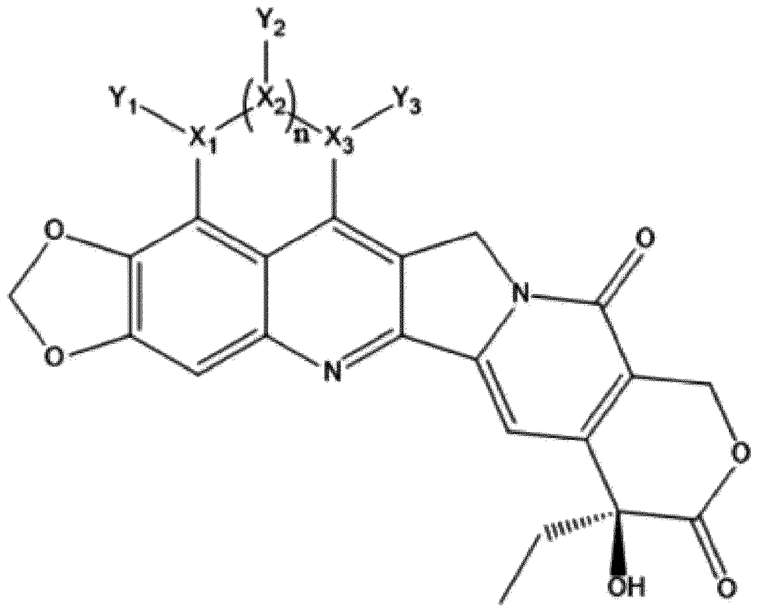

wherein $X_1$ and $X_3$ are each independently carbon, oxygen, nitrogen, or sulfur, and $X_1$ and $X_3$ may be the same or different,

$X_2$ is carbon, oxygen, nitrogen, sulfur, a single bond, or a double bond,

$X_1$, $(X_2)n$, and $X_3$ may form a six-membered or seven-membered ring (n=1 to 2),

$Y_1$, $Y_2$, and $Y_3$ may each independently be hydrogen, or a functional group containing oxygen, nitrogen, phosphorus, or sulfur.

Here, non-limiting examples of functional groups comprising oxygen, nitrogen, phosphorus or sulfur may include a functional group selected from the group consisting of -CHO, -COOH, $-NH_2$, -SH, $-CONH_2$, $-PO_3H$, $-PO_4H_2$, $-OPO_4H$, $-PO_2(OR^1)(OR^2)$($R^1$, $R^2$=$C_sH_tN_uO_wS_xP_yX_z$, X = -F, -Cl, -Br or -I, $0 \le s \le 20$, $0 \le t \le 2(s+u)+1$, $0 \le u \le 2s$, $0 \le w \le 2s$, $0 \le x \le 2s$, $0 \le y \le 2s$, $0 \le z \le 2s$), $-SO_3H$, $-OSO_3H$, $-NO_2$, $-N_3$, $-NR_3OH$(R=$C_nH_{2n+1}$, $0 \le n \le 16$), $-NR_3{}^+X^-$(R= $C_nH_m$, $0 \le n \le 16$, $0 \le m \le 34$, X = OH, Cl or Br), $NR_4{}^+X^-$(R= $C_nH_m$, $0 \le n \le 16$, $0 \le m \le 34$, X = OH, Cl or Br), - COSH, -COOCO-, -CORCO- (R = $C_lH_m$, $0 \le l \le 3$, $0 \le m \le 2l+1$), -COOR, -CN, $-N_3$, $-N_2$, -NROH(R = $C_sH_tN_uO_wS_xP_yX_z$, X = -F, -Cl, -Br or -I, $0 \le s \le 20$, $0 \le t \le 2(s+u)+1$, $0 \le u \le 2s$, $0 \le w \le 2s$, $0 \le x \le 2s$, $0 \le y \le 2s$, $0 \le z \le 2s$), $-NR^1NR^2R^3$($R^1$, $R^2$, $R^3$ = $C_sH_tN_uO_wS_xP_yX_z$, X = -F, -Cl, -Br or -I, $0 \le s \le 20$, $0 \le t \le 2(s+u)+1$, $0 \le u \le 2s$, $0 \le w \le 2s$, $0 \le x \le 2s$, $0 \le y \le 2s$, $0 \le z \le 2s$), $-CONHNR^1R^2$($R^1$, $R^2$ = $C_sH_tN_uO_wS_xP_yX_z$, X = -F, -Cl, -Br or -I, $0 \le s \le 20$, $0 \le t \le 2(s+u)+1$, $0 \le u \le 2s$, $0 \le w \le 2s$, $0 \le x \le 2s$, $0 \le y \le 2s$, $0 \le z \le 2s$), $-NR^1R^2R^3X'$($R^1$, $R^2$, $R^3$ = $C_sH_tN_uO_wS_xP_yX_z$, X = -F, -Cl, -Br or -I, X'= $F^-$,$Cl^-$,$Br^-$ ,or $I^-$, $0 \le s \le 20$, $0 \le t \le 2(s+u)+1$, $0 \le u \le 2s$, $0 \le w \le 2s$, $0 \le x \le 2s$, $0 \le y \le 2s$, $0 \le z \le 2s$), -OH, -O-, >C=O, -SS-, -SO-, $-NO_2$, -COX(X = F, Cl, Br or I), -COOCO-, -CONH-, -CN, $-SCOCH_3$, -SCN, -NCS, -NCO, -OCN, -CN, -F, -Cl, -I, -Br, epoxy group, -hydrazone, $-ONO_2$, $-PO(OH)_2$, $-C=NNH_2$, -HC=CH-, -C=C-, -C≡C-, and hydrocarbons having two or more carbon atoms.

According to the present invention, the active camptothecin derivative (a) designed to bind to DDX5 protein and E3 ligase can kill target cells expressing DDX5 protein through a molecular glue degrader mechanism of action (MoA).

The target cells may be cancer cells or senescent cells. The senescent cells also include cells that do not perform the characteristic functions of an organ.

Preferably, the active camptothecin derivative (a) designed to bind to DDX5 protein and E3 ligase according to the present invention may have a multi-mechanism of action (MoA) that degrades oncoprotein DDX5 together with the ability to inhibit type 1 topoisomerase.

For example, the present invention can induce an antitumor immune response through stimulation of antigen-presenting cells (APCs) such as dendritic cells and macrophages by exposing tumor antigens through the cell death of cancer cells in tumor tissue.

In addition, the present invention provides, as a drug candidate that kills cancer cells, (a) an active camptothecin derivative represented by Chemical Formula 1, which is designed to bind to DDX5 protein and E3 ligase; or (b) a prodrug thereof designed to release the active camptothecin derivative (a) at a target site in vivo, thereby enabling the design of a variety of active camptothecin derivative (a)-based anticancer formulations, doses, and dosages thereof to control the surrounding cell killing effect (bystander effect) and/or ADME profile so that T cell exhaustion does not occur due to chronic

antigen exposure, or so that it is rapidly or slowly removed via lymphatic drainage by providing a desired degree of hydrophobicity and/or controlling aggregation.

The active camptothecin derivative of the present invention is characterized by exhibiting an anticancer mechanism that decomposes oncoprotein DDX5 (Example 2) by being designed in the same manner as the FL118 compound of Chemical Formula 2 with respect to the modification of R1 and R2 (Group A) on the A ring, which has been proven to allow greater tolerance when designing camptothecin-based drugs as in General Formula 1, by applying a camptothecin derivative exhibiting a type 1 topoisomerase inhibitor effect (Example 1).

[General Formula 1]

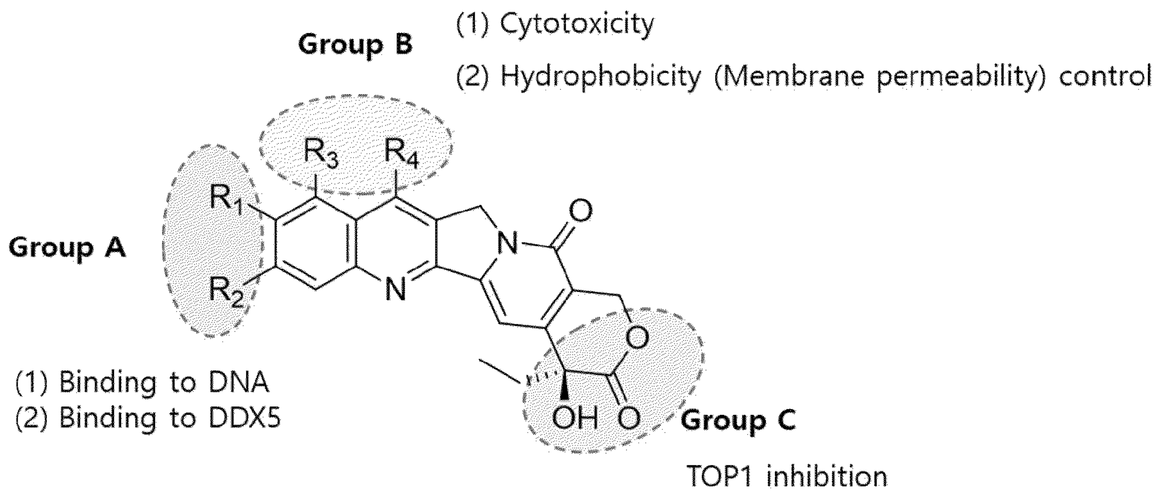


[Chemical Formula 2]

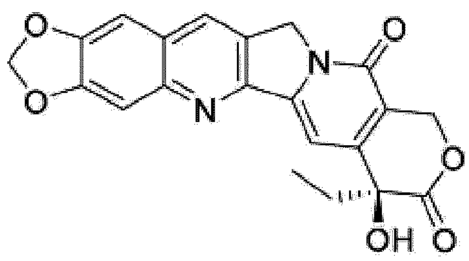

FL118

This is based on the discovery that the active camptothecin derivative of Chemical Formula 1, in which $R_1$ and $R_2$ in General Formula 1 are designed identically to the FL118 compound, such as a compound of Chemical Formula 3, Chemical Formula 4, or Chemical Formula 5 below, is also a transcription cofactor and degrades oncoprotein DDX5, thereby strongly inhibit the expression of anti-apoptotic proteins (Survivin, cIAP2, XIAP, etc.) in a concentration-dependent manner (FIGS. 11 to 14), and also killing the cells into which the compounds were introduced through cell viability experiments (FIGS. 15 and 16).

[Chemical Formula 3]

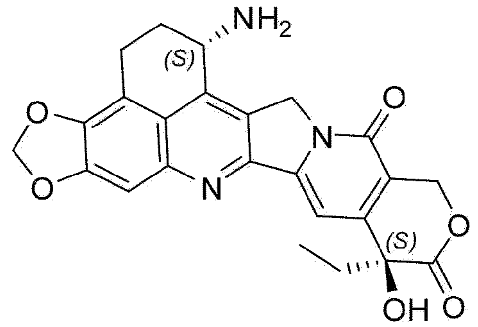

[Chemical Formula 4]

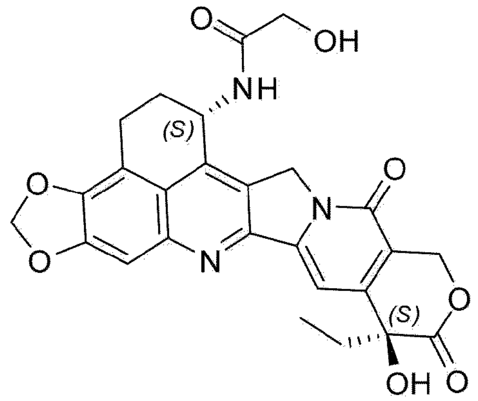

[Chemical Formula 5]

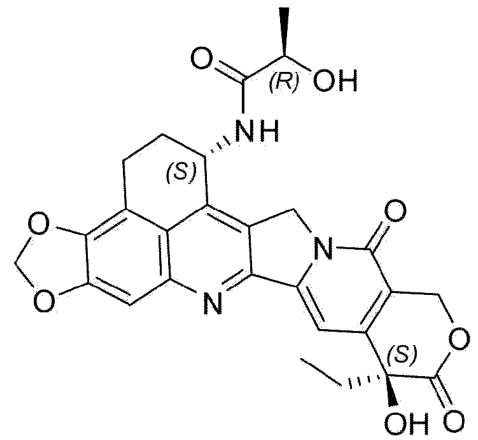

In General Formula 1 or Chemical Formula 2, the Group C site may bind to type 1 topoisomerase and the Group A site may bind to DNA to stabilize the covalent bond of the topoisomerase-DNA complex, thereby preventing the cleaved DNA fragments from being reconnected (Example 1), and/or in General Formula 1 or Chemical Formula 2, the Group A site may bind to DDX5 and the Group C site may bind to E3 ligase to induce DDX5 degradation (FIGS. 11 to 14).

Type 1 topoisomerase (Topoisomerase I) inhibitors are anticancer mechanisms with proven efficacy/safety in clinical trials, and camptothecin-based drugs such as Exatecan, Exatecan derivatives Dxd, and SN-38 have been developed as payloads for ADCs.

The Exatecan drug is a substance whose cytotoxicity is confirmed to be 5 to 10 times stronger than that of SN-38. The Exatecan is different from irinotecan and does not require activation by an enzyme. In addition, it has stronger type 1 topoisomerase inhibition activity than SN-38, which is the medicinal substance of irinotecan, or topotecan, which is used in the same clinical trial, and has stronger cytotoxic activity against various cancer cells in vitro. In particular, it also showed an effect against cancer cells that showed resistance to SN-38, etc. due to the expression of P-glycoprotein. In addition, it also showed a strong antitumor effect in a human tumor subcutaneous transplantation model of mice, and clinical trials were conducted.

The FL118 drug of Chemical Formula 2 is a hydrophobic small molecule that can penetrate cell membranes. The FL118 drug is a potent type 1 topoisomerase inhibitor, and has the same camptothecin mother nucleus structure as the existing commercialized top 1 inhibitors, such as SN-38, Topotecan, and Exatecan (FIGS. 1 and 2), but is an anticancer drug that has secured a wide therapeutic window by having differentiated anticancer efficacy and excellent safety compared to SN-38 in various cancer cells and animal models even when administered alone.

The present inventors have synthesized the FL118 drug, a camptothecin-based compound, and continued to conduct various studies on its physicochemical properties, but the FL118 had limitations in formulation development due to limitations in physicochemical properties.

The present inventors have confirmed that the FL118 drug has stronger cytotoxicity when used as a payload of antibody-drug conjugate (ADC), compared to ADC using SN-38 as a payload, and has cytotoxicity equivalent to that of Exatecan drug.

Meanwhile, the FL118 drug is a camptothecin-based compound that has excellent type 1 topoisomerase inhibition ability and dual MoA that degrades oncoprotein DDX5 (p68).

Surprisingly, it has been found that the drug Exatecan, like the drug FL118, not only strongly inhibits the expression of anti-apoptotic proteins (Survivin, cIAP2, XIAP, etc.), another major cause of anticancer drug resistance, at low concentrations, but also blocks the drug resistance mechanism (FIG. 10).

Therefore, based on this, the present invention is characterized by designing, as illustrated in FIG. 1, from the FL118 compound, (a) a novel active camptothecin derivative having a dual MoA that degrades oncoprotein DDX5 (p68) together with excellent type 1 topoisomerase inhibition ability, (b) a prodrug thereof, preferably a carrier-drug-conjugate (CDC), more preferably an antibody-drug conjugate (ADC), which is designed to release the active camptothecin derivative (a) in vivo, and (c) a complex containing a DDX5 protein-targeting ligand, in which the ability of the active camptothecin derivative (a) to inhibit type 1 topoisomerase is inactivated through a linker connection.

Specifically, the synthetic design concept of the active camptothecin derivative (a) having a dual mechanism of action (dual MoA) that degrades oncoprotein DDX5 along with the ability to inhibit type 1 topoisomerase may be designed as a structure in which as shown in FIG. 1, based on the structure activity relationship (SAR), the structure of Group C, which is a type 1 topoisomerase inhibition region, is maintained, and Group A, which is a binding site for DDX5 degradation, is also maintained to have the same structure as FL118, but the structure (R3 and R4) of Group B in General Formula 1 is adjusted as in Chemical Formula 1, thereby not only improving the physicochemical properties of the drug, but also controlling the cytotoxicity and/or cell membrane permeability of the drug to control the surrounding cell killing effect (bystander effect) (Example 9, FIGS. 25 and 26; Example 10, FIG. 27B). In addition, in some cases, it may be designed as a structure that can provide a desired degree of hydrophobicity and/or control aggregation so that the active camptothecin derivative (a) released from the killed cancer cells is removed quickly or slowly through lymphatic drainage.

When simply comparing cell viability, the newly designed and synthesized PBX-7016 (Chemical Formula 5) according to the present invention showed an IC50 value that was 1.5 to 2 times higher than that of the Dxd drug, but, surprisingly, when used as an ADC payload, the ADC containing PBX-7016 exhibited a cell viability-related cytotoxic effect that was about 5 times better than the ADC containing Dxd (Enhertu) (Example 7, FIG. 22). The compound represented by Chemical Formula 5 (PBX-7016) and the compound represented by Chemical Formula 5-1 (PBX-7017), which is an isomer thereof, are diastereomeric to each other.

Therefore, the present invention preferably designs the active camptothecin derivative to exert an appropriate surrounding cell killing effect (bystander effect) in cancer tissues by controlling cell membrane permeability as desired through modification of R3 and/or R4 in General Formula 1 (e.g., PBX-7014, PBX-7016, PBX-7018, PBX-7020, PBX-7022, PBX-7024).

As illustrated in FIG. 1, the present invention designed and synthesized a compound having a new structure, such as PBX-7011, which is similar to the structure of Exatecan, by modifying R3 and R4 of Group B in General Formula 1 (Preparation Example 1). As illustrated in FIG. 2, it was confirmed that PBX-7011 of Chemical Formula 3, which was newly designed according to the present invention, had a relatively higher hydrophilicity than FL118 of Chemical Formula 2 through the calculated LogP (or cLogP) and tPSA values.

Furthermore, compounds PBX-7014 and PBX-7016 having new structures were designed and synthesized by adjusting Group B of General Formula 1 in the newly designed PBX-7011 structure (Preparation Examples 2 and 3). The PBX-7014 and PBX-7016 were calculated to have similar hydrophilicity values to Dxd derived from Exatecan (FIGS. 1, 2, and 28).

FIG. 28 compares the solubility and topological polar surface area (tPSA) of FL118, Exatecan, SN-38, Dxd, Exatecan-Lactic acid, PBX-7011/PBX-7012, PBX-7014/PBX-7015, and PBX-7016/PBX-7017.

The relative hydrophilicity/hydrophobicity of a drug has a significant impact on its solubility, absorption, distribution, metabolism, excretion (ADME), and especially lymphatic drainage. It is important in how easily a drug pass through cell membranes and in its interactions with drug targets such as receptors.

The hydrophobicity of a drug is expressed by partition coefficient (p).

A large p value indicates hydrophobicity, while a small p value indicates hydrophilicity (polarity). **In** practice, the log P value is used as a measure to evaluate hydrophobicity. Clog P refers to the log P value of a given compound obtained using appropriate software. The smaller the cLogP value, the higher the polarity.

The polar surface area (PSA) or topological polar surface area (TPSA) of a molecule is defined as the sum of the surface area of all polar atoms or molecules, which are mainly oxygen and nitrogen, including attached hydrogen atoms. The tPSA is a medicinal chemistry measurement method commonly used to optimize the cell penetration ability of a drug. Molecules with a polar surface area greater than 140 Å2 tend not to penetrate cell membranes. A tPSA of less than 90 Å2 is generally required for a molecule to pass through the blood-brain barrier and act on receptors in the central nervous system.

PBX-7016/PBX-7017 (Chemical Formula 5 and Chemical Formula 5-1) of the present invention is obtained by introducing a methyl group, a functional group unstable in metabolism, to PBX-7014/PBX-7015 (Chemical Formula 4 and Chemical Formula 4-1) to reduce the drug lifespan. Drugs that are extremely stable in metabolism and metabolized very slowly are necessary to secure an appropriate retention time because they have concerns about toxicity and serious side effects due to accumulation.

The active camptothecin derivative (a) newly designed according to the present invention is a hydrophobic small molecule designed by modifying $R_3$ and $R_4$ of General Formula 1 so as to be able to penetrate cell membranes, and therefore, when delivered to cancer tissues, it can accumulate at a high concentration while penetrating deep into the cancer tissue, and exert a mechanism in which, after the active camptothecin derivative (a) or a prodrug thereof (b), ADC, penetrates the cell membrane, the active camptothecin derivative (a) exerts cytotoxicity inside the cell and causes cell death, and is released into the extracellular fluid to continuously penetrates the cell membrane of surrounding cells and moves into the cell, causing cell death (Example 9, FIGS. 25 and 26; Example 10, FIG. 27B). Accordingly, the active camptothecin derivative of the present invention can convert a solid cancer aggregated with high cell density into a scattered tumor with a low cell density and/or convert a cold tumor with low immune activity into a hot tumor with high immune activity. In addition, it has a high effect on killing surrounding cells, and thus, is advantageous in the treatment of heterogeneous tumors.

In summary, as illustrated in FIGS. 1, 2, 28, and 29, the present invention not only can design various camptothecin derivatives and their prodrugs (FIG. 3) with novel structures that exhibit dual MoA in terms of type 1 topoisomerase inhibition and DDX5 protein degradation, but also can control cell membrane permeability, lymphatic drainage, and/or bystander effect by controlling hydrophilicity in various ways when designing camptothecin derivatives.

**[FL118 drug]**

FL118 of Chemical Formula 2 can act as a molecular glue degrader that directly bonds DDX5 and ubiquitination regulators to each other to degrade the DDX5.

The FL118, which acts as a molecular glue, has a function of directly binding to the oncoprotein DDX5, a multifunctional master regulator, and dephosphorylates and degrades it through the proteasome degradation pathway without reducing DDX5 mRNA, and the silencing of DDX5 indicates that DDX5 is a master regulator that regulates the expression of several oncogenic proteins, including survivin, Mcl-1, XIAP, cIAP2, c-Myc, and mutant Kras.

The FL118 indirectly controls DDX5 downstream targets to inhibit cancer initiation, development, metastasis, recurrence, and treatment resistance with high efficacy as demonstrated in studies using human colorectal/pancreatic adenocarcinoma cells and tumor models.

Genetic manipulation of DDX5 in PDAC cells affects tumor growth. PDAC cells with DDX5 KO are resistant to FL118 treatment. In human tumor animal model studies, FL118 was highly efficacious in eliminating human PDAC and CRC tumors with high DDX5 expression, whereas FL118 was less effective in PDAC and CRC tumors with low DDX5 expression.

DDX5 protein is a direct target of FL118 drug and may serve as a biomarker for predicting PDAC and CRC tumor sensitivity to FL118.

Meanwhile, the FL118 drug had a Top1 inhibition efficacy equivalent to or higher than SN-38 in cancer cells, and exhibited cytotoxicity that is 5 to 20 times more potent than SN-38 in various cancer cell lines, that is, with a low $IC_{50}$ value, and in the evaluation results for 140 cell lines derived from various carcinoma, showed a very strong anticancer efficacy with an $IC_{50}$ of <100 nM for the majority of cancer cells. The FL118 drug has been proven to be excellently safe through GLP-toxicity tests in mice and beagle dogs, and has shown superior efficacy compared to SN-38 in various cancer cell line

xenograft models. Meanwhile, camptothecin-based anticancer drugs initially showed excellent anticancer responses when used in patients, but showed strong resistance to these drugs through epigenetic silencing of the Top1 gene and Top2 dependence of cancer cells. On the other hand, the FL118 drug showed strong efficacy in the xenograft model of cancer cell lines where Top1 was not expressed through epigenetic silencing or knockout.

In addition, the FL118 drug is a triple-target anticancer drug that directly targets the type 1 topoisomerase, a well-established anticancer target, while simultaneously inhibiting the Bcl family, such as Survivin, a resistance protein involved in the resistance mechanism, and inhibiting the action of the efflux pump.

Specifically, the camptothecin-based anticancer drugs, such as SN-38, show resistance in such a way that the drug is excreted out of the cell by overexpression of the ABCG2 Transporter, but the FL118 drug can overcome resistance because it is not affected by the ABCG2 Transporter. The FL118 drug can block the expression of resistance by strongly inhibiting the expression of anti-apoptotic proteins (Survivin, cIAP2, XIAP, etc.), which are another major cause of anticancer drug resistance, at low concentrations (FIG. 10).

Therefore, the FL118 drug is not excreted out of cells by the ABCG2, an efflux pump, and can block resistance caused by various anti-apoptotic proteins. As a result, the FL118 drug can overcome various mechanisms of resistance action of SN-38/Exatecan.

The FL118 drug showed strong tumor regression efficacy compared to SN-38 in colon cancer/head and neck cancer/pancreatic cancer, etc. when administered in vivo in the same amount as SN-38.

The FL118 drug also had strong anticancer efficacy when administered after inducing SN-38 resistance in tumor xenografts. The possibility of FL118 drug overcoming various resistance mechanisms to SN-38/Exatecan was confirmed in vivo.

Furthermore, the FL118 drug has an optimal PK/safety profile for targeted drug delivery (e.g., carrier-drug conjugate) application. When the FL118 drug alone is administered systemically, it is rapidly metabolized/excreted from the blood and only shows low concentrations, but it accumulates rapidly in cancer tissues immediately after administration and maintains high concentrations for a long time. For example, when ADC is applied, it ensures maximum selectivity between tumor tissues and normal tissues. This is also the case for the active camptothecin derivative (a) represented by Chemical Formula 1 (Examples 6 and 8).

In summary, the FL118 drug:

(i) selectively inhibits/degrades the DDX5, UBE2T, and USP2a, which are other anticancer targets, in addition to the Top 1, which is a well-validated anticancer target, thereby generating a strong anticancer effect;
(ii) exhibits 5-10 times stronger cellular cytotoxicity than that of SN-38, and strong cellular cytotoxicity equal to or greater than that of Exatecan;
(iii) can avoid the resistance mechanism caused by the overexpression of the transporter proteins because it is not a substrate of ABCG2 and P-GP, unlike SN-38 and Exatecan;
(iv) exhibits excellent efficacy in animal models of cancer in which the epigenetic silencing of Top 1/overexpression of Top 2, which is the main mechanisms of resistance to Top 1 inhibitors, occurs;
(v) can avoid resistance by fundamentally blocking the overexpression of anti-apoptotic proteins, which is a common resistance mechanism in most cancers; and
(vi) has already established biomarkers (DDX5, K-ras, p53) and companion diagnostic techniques that can predict anticancer responses in patients, enables personalized treatment through companion diagnosis by securing customized biomarkers, and shows strong efficacy, especially in p53/K-ras mutant cancer cells with poor prognosis.

The KRAS is one of three types of genes related to the production of RAS protein involved in cell proliferation signaling. It is known that mutations occur in about 20% or more of carcinoma overall, although it is different for each carcinoma. However, despite the fact that anticancer efficacy can be expected scientifically, it was treated as an undruggable target. This is because the surface of the RAS protein is smooth, and thus no pocket suitable for binding compounds has been found.

**[Anticancer mechanism 1 as a selective inhibitor of type 1 topoisomerase (topoisomerase-1)]**

Camtothecin is a selective inhibitor of type 1 topoisomerase (topoisomerase-1), an isomerizing enzyme involved in DNA replication, recombination, etc. (FIG. 1). Since it was proven to exhibit strong cytotoxicity in vitro, development through clinical trials was initiated by the US Cancer Research Center (NCI), but the development was halted due to various side effects such as bone marrow suppression and hemorrhagic cystitis related thereto caused by the limitation of extremely poor solubility. However, since 1990, it has been confirmed that camptothecin has a unique mechanism of action, that is, selectively inhibits DNA type 1 topoisomerase, unlike the mechanism of inhibiting DNA type 2 topoisomerase, and thus exhibits antitumor effects.

DNA topoisomerases are enzymes in the nucleus, and serve to temporarily cleave DNA or unwind the double

helix when cells require access to genetic material for replication or transcription. They also participate in a variety of intracellular activities, including chromosome enrichment and recombination, and DNA repair. The genetic code of topoisomerases is quite conservative among species.

Type 1 topoisomerase, a drug target of camptothecin, has been observed to increase in a variety of malignancies. The drug does not inhibit the free enzyme, but stabilizes the covalent bonds of the topo-DNA complex, preventing the cleaved DNA fragments from being reconnected. Therefore, the sensitivity of cells to these topoisomerase-targeting drugs is related to the levels of the enzyme present in the nucleus. The drug prevents transcription from proceeding by interfering with DNA recombination. The greater the amount of type 1 topoisomerases, the more cleavable complexes are formed, which means greater drug sensitivity. This has important clinical relevance, and type 1 topoisomerases inhibitors are used to increase the expression of type 2 topoisomerases, making it more sensitive to the type 2 topoisomerase inhibitors. This result is supported by the antagonistic relationship between type 1 and type 2 topoisomerases. It is known that, unlike the type 2 topoisomerases, the type 1 topoisomerases are not closely related to proliferation in normal tissues, exist in large quantities in solid tumors with active cell division, such as colon cancer, ovarian cancer, and esophageal cancer, including lymphoma, compared to surrounding normal tissues, and can actually cause cell death by blocking the replication and transcription of genes in tumor cells at the "S" phase of the cell cycle.

**[Anticancer mechanism 2 as a molecular glue degrader that binds to DDX5]**

The ubiquitin-proteasome system (UPS) is an important pathway for the degradation of intracellular proteins that regulates a wide range of cellular processes. The ubiquitin is a small protein that is covalently attached to lysine residues of substrate proteins by a series of enzymatic reactions involving ubiquitin activating enzymes (E1s), ubiquitin conjugating enzymes (E2s), and ubiquitin ligases (E3s). This process is called ubiquitination and is an important mechanism for regulating protein degradation, signalling, and trafficking.

Ubiquitin ligase is responsible for substrate specificity in the ubiquitination pathway.

In this regard, the active camptothecin derivative represented by Chemical Formula 1 according to the present invention is designed to bind to DDX5 protein and E3 ligase.

The DDX5 protein (also known as p68) is a multifunctional master regulator that acts in the following mechanisms: (1) biological process of co-activating transcription of many oncogenes through direct interactions with various transcription factors (e.g., c-Myc) at oncogenic gene promoters, (2) biological process of regulating miRNA and pre-RNA splicing (e.g., U1, U2, U3, ... snRNP), and (3) ribosome biogenesis (e.g., 32S rRNA, pre-ribosome).

The active camptothecin derivative represented by Chemical Formula 1 according to the present invention binds to DDX5 protein without reducing DDX5 mRNA and functionally degrades it through dephosphorylation and proteasome degradation pathways, which suggests that the active camptothecin derivative of Chemical Formula 1 can be attached to both DDX5 and ubiquitin-involved protein stability/degradation regulators, and serves as a "molecular glue degrader."

All DDX5 downstream protein targets are known to be involved in cancer initiation, development, metastasis, recurrence, and treatment resistance. Therefore, if the DDX5 downstream targets are indirectly blocked through the degradation of DDX5 protein by the active camptothecin derivative represented by Chemical Formula 1 according to the present invention, the active camptothecin derivative represented by Chemical Formula 1 may exhibit high antitumor efficacy.

**[DDX5 as a drug target and/or biomarker]**

Transcription factors are a group of proteins that bind to DNA strands called promoters or enhancers through the DNA binding domains and initiate transcription of genes with the cooperation of RNA polymerase and other cofactors. They play an important role in regulating transcription during embryogenesis and development. The physiological state of transcription factors in different cell types is also important for maintaining cellular homeostasis. Therefore, deregulation of transcription factors will lead to the development of cancer cells and tumor progression. Depending on the function in cancer cells, transcription factors may be oncogenic or tumor suppressive. In addition, transcription factors have been shown to regulate cancer stem cells, epithelial-to-mesenchymal transition (EMT), and drug response. Therefore, measuring deregulated transcription factors is thought to be able to predict the treatment outcome of cancer patients, and targeting deregulated transcription factors may be an encouraging strategy for cancer treatment. Deregulated key transcription factors may cause poor clinical outcomes in cancer patients. Therefore, predicting prognosis and drug response and targeting deregulated transcription factors will help design new drugs and therapeutic strategies for cancer treatment.

It is well known that transcription is the first step of gene expression in cells during embryogenesis, development, and homeostasis. During the transcription process, RNA transcripts with specific coding information are generated from DNA. To ensure the specificity of gene expression in different cells and tissues, a transcription factor, a group of specific proteins, select genes to be transcribed into RNA transcripts and control transcription. The transcription factor has a DNA

binding domain, and can bind to specific DNA sequences called promoters or enhancers of genes and initiate transcription of genes with the cooperation of activators, mediators, co-factors, and RNA polymerases.

The transcription factors also control the rate of transcription from DNA to mRNA. Consequently, the physiological status of transcription factors in different types of cells is important for maintaining cellular homeostasis. Changes in the physiological status of transcription factors in cells cause transcription disorders and cause translation of specific genes, resulting in pathological changes in the cells. Therefore, deregulated transcription factors can cause many human diseases. One of the common diseases with deregulated transcription factors is cancer.

Homeostatic control of human cells generally prevents inappropriate proliferation and suppresses the growth of cells that proliferate abnormally outside the normal niche. Since transcriptional control is a key process during homeostasis, if the levels or activities of transcription factors are deregulated, cells may escape homeostatic control and induce the development and progression of human cancers.

In fact, many chemotherapy can activate tumor suppressor transcription factors.

Deregulated transcription factors play an important role in tumorigenesis and tumor progression. They regulate central tumor signaling pathways such as NF-κB, Notch, Wnt/β-catenin, and JAK/STAT. Deregulated oncogenic and tumor suppressor transcription factors favor the survival of cancer cells, cancer stem cells, and EMT cells, leading to intrinsic or acquired drug resistance and tumor progression in cancer treatment.

DDX5(p68) is a well-known multifunctional DEAD-box RNA helicase and transcription cofactor. Therefore, when the transcription factor is deregulated by the physiological state of DDX5 and cancer occurs, the cancer disease can be treated by selectively degrading the DDX5(p68), which is a transcription cofactor. Similarly, the cancer disease can be prevented by selectively degrading the DDX5(p68), which is a transcription cofactor.

DDX5 is recognized as a potential biomarker and target for the treatment of various cancer types. Research over the years has greatly expanded understanding of the functional diversity of DDX5 in various cancer types and expanded knowledge of the mechanism of action (MoA). This provides a rationale for the development of novel cancer therapeutics using DDX5 as a biomarker and therapeutic target. However, although most published studies have shown that DDX5 is an oncogenic target and a biomarker of cancer treatment resistance, some studies have reported that DDX5 can act as a tumor suppressor in certain scenarios. The multiple functions of DDX5 make the DDX5 an excellent independent oncogenic biomarker and a target for targeted cancer treatment.

Transformation of normal cells into cancerous cells occurs through deregulation of different metabolic pathways involving a complex network of protein-protein interactions. Cellular enzymes and DDX5 play an important role in maintaining normal cellular metabolism, but when deregulated, they can accelerate tumor transformation. All DDX5s interact with hundreds of other cellular proteins, and depending on the specific pathway involved, both proteins can act as tumor suppressor genes or oncogenes.

The DEAD-box family of RNA helicases is involved in several metabolic pathways, including transcription and translation, as well as cell proliferation, innate immunity, and stress responses. Given their diverse roles, they are deregulated or their mutations are linked to a variety of pathological conditions, including cancer. However, in some cases, loss of function of a given DEAD-box helicase promotes tumor transformation, indicating a tumor suppressor role, while in other situations, overexpression of the same enzyme favors cancer progression, acting as a typical oncogene. The role of DDX5 as an oncogene and tumor suppressor genes has been documented in several cancer types. Understanding the interactions of different cellular contexts defined by the molecular interaction network of DDX5 in different tumors may help explain the apparently conflicting cancer-specific roles of these proteins as cancer-inducing or inhibitory factors.

Although DDX5 acts much more frequently as an oncogene, it may also have tumor suppressor functions in certain cancer types (Table 1).

Source: Secchi, M.; Lodola, C.; Garbelli, A.; Bione, S.; Maga, G. DEAD-Box RNA Helicases DDX3X and DDX5 as Oncogenes or Oncosuppressors: A Network Perspective. Cancers 2022, 14, 3820. https://doi.org/10.3390/cancers14153820

[Table 1]

| Cancer | Role [1] | **Mechanism** | **Ref.** |
|---|---|---|---|
| Colorectal | + | DDX5 overexpression promotes cancer by AKT/mTOR signaling or association with AldoA | [62-66] |
| Breast | + | upregulates a subset of miRNAs; highly correlated with Ki67; involved in β-catenin/Wnt pathway | [67-69] |
| Leukemia | + | DDX5 depletion selectively induces stress in AML cells; positive regulator of NOTCH1 signaling; DDX5 inhibition reduces tumor proliferation | [70-72] |

(continued)

| Cancer | Role [1] | **Mechanism** | **Ref.** |
|---|---|---|---|
| Non-small-cell lung/small cell lung | + | induces β-catenin to promote cell proliferation | [73,74] |
| Osteosarcoma | + | IncRNA DLEU1/miR-671-5p/DDX35 interaction to promote cancer progression | [75] |
| Prostate | + | IncRNA CCAT1/DDX5/miR-28-5p interaction to promote cancer progression; DDX5-ETV4 fusion protein | [76,77] |
| Gastric | + | high DDX5 expression activates mTOR/SK61 pathway to induce cancer progression; Inc MIAT interaction | [78,79] |
| Glioblastoma | + | NF-κB p50 subunit activation; IncRNA LINC01116 interaction; hyperactivation of ERK and downregulation of DUSP5 | [80-82] |
| Cervical | + | stimulation of the expression of TGF-β1 in CaSki cells | [83] |
| Endometrial | + | HDGF/DDX5 interaction to induce β-catenin | [84] |
| Squamous cell carcinoma (HNSCC) | + | high DDX5 expression is associated with cancer progression | [85] |
| Squamous cell carcinoma (ESCC) | + | decreased DDX5 expression is associated with inhibition of cancer progression | [86] |
| Human hepatocellular carcinoma (HCC) associated with HBV | - | inhibits cancer progression by interacting with p62/SQSTM1, by regulation of miRNAs and by associating with IncRNA HOTAIR | [87-91] |

[1] Oncogene (+), oncosuppressor (-).

The active camptothecin derivative represented by Chemical Formula 1 according to the present invention can avoid drug resistance, resistance to targeted therapy, and/or resistance during treatment, since the DDX5 protein is a drug target. In addition, the active camptothecin derivative can off the transcription induction of anti-apoptotic genes. Furthermore, the active camptothecin derivative can degrade the DDX5 protein, which is a transcription cofactor, thereby maintaining or enhancing the sensitivity of cancer cells to chemotherapy or radiotherapy.

**[Camtothecin derivatives represented by Chemical Formulae 1, 3 to 9 and isomers thereof]**

The active camptothecin derivatives represented by Chemical Formula 1, in which $R_1$ and $R_2$ in General Formula 1 are designed identically to the FL118 compound according to the present invention, such as compounds of Chemical Formula 3, Chemical Formula 4, or Chemical Formula 5, Chemical Formula 6, Chemical Formula 7, Chemical Formula 8, Chemical Formula 9, and isomers thereof, are also transcription cofactors and degrade oncoprotein DDX5, thereby strongly inhibit the expression of anti-apoptotic proteins (Survivin, cIAP2, XIAP, etc.) in a concentrationdependent manner (FIGS. 11 to 14), and also killing the cells into which the compounds were introduced through cell viability experiments (FIGS. 15 and 16).

According to the present invention, the camptothecin derivatives represented by Chemical Formula 1, for example, Chemical Formulas 3 to 9, have a pentacyclic structure having a lactone in the E ring essential for cytotoxicity, like camptothecin illustrated in FIG. 1, and are designed to maintain the lactone group and the alpha hydroxyl group located at carbon 20 of the E ring, which are important for the stability of type 1 topoisomerase-DNA byproducts, and maintain the structure of FL118 drug, $R_1$ and $R_2$ ($-OCH_2O-$ (methylenedioxo) pentagonal ring) in General Formula 1, which maintains the advantages of the above-mentioned FL118 drug, while the structural features of Exatecan drug ($R_3$ and $R_4$ in General Formula 1) are such that, compared to SN-38, in which aggregation is induced by π-π stacking of aromatic rings formed by A- and B-rings, various orientations of successive carbon-carbon single bonds of hexagonal rings extended from the A- and B-rings form a dynamic equilibrium, so that the stacking of aromatic rings formed by the A- and B-rings can be weakened or suppressed.

In addition, the compound represented by Chemical Formula 3 or Chemical Formula 3-1 is a compound designed to graft $-NH_2$, which has a large degree of freedom to rotate the molecular bond about the carbon-carbon single bonds in the hexagonal ring extended from the A- and B-rings, so that it is exposed to water ($H_2O$) to carry a (+) charge or forms a hydrogen bond with water to increase water dispersibility.

In addition, the compound represented by Chemical Formula 4 or Chemical Formula 4-1 is a compound designed to graft a functional group $CH_2(OH)CONH$- in the glycolic acid form of $-NH_2$, which has a large degree of freedom to rotate the molecular bond about the carbon-carbon single bonds in the hexagonal ring extended from the A- and B-rings, so that it is exposed to water ($H_2O$) to rotate like a propeller while forming a hydrogen bond with water to increase water dispersibility.

In addition, the compound represented by Chemical Formula 5 or Chemical Formula 5-1 is a compound designed to graft a functional group $CH_3CH(OH)CONH$- in the lactic acid form of $-NH_2$, which has a large degree of freedom to rotate the molecular bond about the carbon-carbon single bonds in the hexagonal ring extended from the A- and B-rings, so that it is exposed to water ($H_2O$) to rotate like a propeller while forming a hydrogen bond with water to increase water dispersibility.

The compound represented by Chemical Formula 5 or Chemical Formula 5-1 of the present invention is obtained by introducing a methyl group, which is a functional group that is unstable in metabolism, into the compound represented by Chemical Formula 4 or Chemical Formula 4-1 to reduce the lifespan of the drug. Drugs that are extremely stable in metabolism and metabolized very slowly are necessary to secure an appropriate retention time because they have concerns about toxicity and serious side effects due to accumulation.

Meanwhile, the DXd payload used in Enhertu was originally made from the Exatecan compound, which is hardly affected by ABCG2, but due to the presence of the glycolic acid (alphahydroxy acetic acid) functional group used to convert Exatecan to DXd, it became strongly affected by ABCG2. However, the glycolic acid functional group plays a very important role in the excellent safety/efficacy profile of Enhertu, and if this is removed, there are difficulties in manufacturing ADCs, and at the same time, the performance of ADCs in animal models and clinical trials is deteriorated (emergence of safety issues or reduction in efficacy). Therefore, there is a very high need for a new camptothecin derivative that can be easily used in ADC manufacturing while not being affected by ABCG2. The compound represented by Chemical Formula 9 (PBX-7024) is a compound derived from the compound represented by Chemical Formula 3 (PBX-7011), and is a new camptothecin compound that can be easily used in ADC manufacturing while not being affected by ABCG2.

In order to confirm the anticancer efficacy of PBX-7024, efficacy evaluation was conducted on FaDu, a cancer cell that does not express ABCG2, and A549, a cancer cell that overexpresses ABCG2. In A549, a cancer cell line overexpressing ABCG2, it can be confirmed that the camptothecin compounds including DXd show increased $IC_{50}$ values as shown in FIG. 15, whereas PBX-7016 and PBX-7024 still maintain strong efficacy.

That is, when using the PBX-7016 and PBX-7024, which are new camptothecin compounds according to the present invention, unlike ADCs using existing camptothecin compounds such as SN-38 and DXd, they have the effect of overcoming the resistance mechanism caused by ABCG2 overexpression.

FIGS. 28 and 29 compare LogP, cLogP, and tPSA (topological polar surface area) of FL118, Exatecan, SN-38, Dxd, Compound A, Compound B, Compound C, Compound D, Compound E, Compound F, Compound G, Compound H, Compound I, Compound J, Compound G', Compound H', Compound I', and Compound J'.

The compound of Chemical Formula 3 and the compound of Chemical Formula 3-1 are diastereomeric relationships with each other. Similarly, the compound of Chemical Formula 4 and the compound of Chemical Formula 4-1; and the compound of Chemical Formula 5 and the compound of Chemical Formula 5-1 are also diastereomeric relationships with each other. Therefore, compounds that are diastereomeric relationships with the compound of Chemical Formula 6, the compound of Chemical Formula 7, the compound of Chemical Formula 8, or the compound of Chemical Formula 9 also fall within the scope of the present invention.

As exemplified in Preparation Example 2, PBX-7014 of Chemical Formula 4 can be synthesized from PBX-7011 of Chemical Formula 3, and PBX-7015 of Chemical Formula 4-1 can be synthesized from PBX-7012 of Chemical Formula 3-1.

As illustrated in Preparation Example 3, the compound of Chemical Formula 5 (PBX-7016, Compound E) can be synthesized from the compound of Chemical Formula 3 (PBX-7011, Compound A), and in the same manner, the compound of Chemical Formula 5-1 (PBX-7017, Compound F) can be synthesized from the compound of Chemical Formula 3-1 (PBX-7012, Compound B).

In this specification, the camptothecin drug of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8 or Chemical Formula 9 according to the present invention includes a compound of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8 or Chemical Formula 9, as well as a pharmaceutically acceptable salt thereof, a solvate thereof, a prodrug thereof, preferably a carrier-drug conjugate (CDC), more preferably an antibody-drug conjugate (ADC).

In this specification, the pharmaceutically acceptable salts refer to salts commonly used in the pharmaceutical industry, and include, for example, salts of inorganic ions including sodium, potassium, calcium, magnesium, lithium, copper, manganese, zinc, iron, etc., salts of inorganic acids such as hydrochloric acid, phosphoric acid, and sulfuric acid, and in addition, salts of organic acids such as ascorbic acid, citric acid, tartaric acid, lactic acid, maleic acid, malonic acid,

fumaric acid, glycolic acid, succinic acid, propionic acid, acetic acid, orotate acid, and acetylsalicylic acid, and salts of amino acids such as lysine, arginine, and guanidine. In addition, there are salts of organic ions such as tetramethyl ammonium, tetraethyl ammonium, tetrapropyl ammonium, tetrabutyl ammonium, benzyl trimethyl ammonium, and benzethonium that can be used in pharmaceutical reactions, purification, and separation processes. However, the types of salts referred to in the present invention are not limited by these listed salts.

The "Solvate" refers to a compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8 or Chemical Formula 9, and a pharmaceutically acceptable salt thereof, further comprising a stoichiometric or non-stoichiometric amount of solvent bound by non-covalent intermolecular forces. When the solvent is water, the solvate is a hydrate.

**[Advantage of a drug modality called molecular glue degrader]**

Proteins in cells in the body are naturally decomposed within hours to days after performing their function. All cells in the body have a purification function called the ubiquitin proteasome system (UPS) that decomposes proteins, and in this process, the ubiquitin acts as a marker that indicates which proteins need to be decomposed, and the proteasome acts as a crusher that recognizes the ubiquitin mark and destroys the protein. In other words, several substances called ubiquitin are attached to the side of proteins that have completed their function as marks, and a substance called proteasome selects only proteins with this mark and decomposes them like a crusher. E3 ligase is an enzyme that causes the protein decomposition system in the body, and is responsible for substrate specificity in the ubiquitination pathway.

Molecular glue degrader or molecular glue is a compound that acts as an adhesive that attaches target proteins and specific enzymes (E3 ligases) in our body to each other. One of the advantages of molecular glue is that it acts as a catalyst, and after degrading a target, can be separated again to degrade another target protein.

When the E3 ligase enzyme attaches to the tumor protein through the molecular glue, the tumor protein is degraded, and other tumor proteins are degraded continuously until the target tumor protein disappears, so cancer cell proliferation can be prevented. Therefore, molecular glue for tumor proteins overcomes drug resistance, which is a problem of a target anticancer drug, and has a high therapeutic effect even with a small dosage.

In addition to the FL118 drug, the active camptothecin derivative designed in Chemical Formula 1 according to the present invention is a molecular glue degrader that binds to DDX5 protein and E3 ligase, that is, a molecular glue that activates the degradation of oncoprotein DDX5 (FIGS. 11 to 14).

The molecular glue degrader is not only a ligand (warhead) that binds to a target protein, but can also act as an E3 ligase ligand (binder).

Therefore, since the active camptothecin derivative designed in Chemical Formula 1 according to the present invention is a molecular glue that activates the degradation of oncoprotein DDX5, it can be used as a ligand that targets DDX5 protein or a ligand that binds to DDX5 protein.

Unlike kinase inhibitors, the molecular glue degrader is 'proximity-driven' and the ability to induce degradation is 'event-driven', depending on the formation of a temporary 'target proteinmolecular glue-E3 ligase' triple complex. The molecular glue separated after degradation can form additional triple complexes with the target protein, allowing multiple degradation processes to occur until the target protein disappears.

Most of drug-targeted proteins evolve resistance by adapting to the drug. However, molecular glue, which is a small molecule compound that acts as an adhesive to attach the oncoprotein and E3 ligase to each other, is a modality suitable for cancer treatment because it is highly resistant.

The shape of the drug-targeted protein changes slightly each time a genetic mutation occurs. It would be nice to block the activity of all variants with one drug, but this is not possible due to the problem of selectivity. Thus, in order for one drug to block the activity of all variants, the drug-targeted protein is degraded and completely removed.

Therefore, the active camptothecin derivative represented by Chemical Formula 1 according to the present invention is a drug that can precisely bind to the DDX5 oncoprotein, and can avoid the resistance problem of targeted therapeutic agents through a molecular glue approach that directly and selectively degrades the protein.

The active camptothecin derivative represented by Chemical Formula 1 according to the present invention has a binding strength determined for the DDX5 oncoprotein, a target protein, according to the physicochemical properties thereof, and it is preferably an irreversible drug that binds too strongly and cannot return to its previous state.

Therefore, the active camptothecin derivative represented by Chemical Formula 1 according to the present invention can control the binding strength for the DDX5 oncoprotein, a target protein, through modification of $X_1$, $(X_2)_n$, $X_3$, $Y_1$, $Y_2$, and/or $Y_3$ in Chemical Formula 1.

The active camptothecin derivative designed from Chemical Formula 1 according to the present invention has a high affinity with DDX5 protein, unlike the existing SN38 drug, and thus does not easily fall off, and acts as a molecular glue to degrade DDX5 protein, thereby irreversibly inhibiting signal transmission of cancer cells related to DDX5. Further, the active camptothecin derivative controls the cell membrane permeability of the drug as desired according to the

physicochemical properties thereof to exert a bystander effect or control the degree of its effect, thereby increasing the bystander effect to be advantageous in the treatment of heterogeneous tumors, as well as suppressing long-term cancer progression and reducing the risk of resistance development to increase the treatment response rate.

**[Cell death and drug resistance of anticancer drugs]**

The active camptothecin derivative represented by Chemical Formula 1 according to the present invention can bind to DDX5, which acts as an oncoprotein in cells, and induce cell death through DDX5 protein decomposition (FIG. 30).

There are two factors that determine tumor growth: the first is cell proliferation, and the other is cell death. When the cell cycle of tumor cells is stopped by cytotoxic substances, the tumor cells die due to apoptosis.

Cell death plays a very important role in maintaining tissue homeostasis and morphology, regulating cell numbers, removing damaged or abnormal cells, and as a defense mechanism against infection, and living organisms maintain their lives normally by precisely controlling cell growth, differentiation, and death processes.

Apoptosis is described as a complex process in which the expression of various genes and the activation of proteins, which are expression products, are accompanied to induce cell death. This apoptosis is an intracellular physiological and active suicide mechanism, and plays an essential role in maintaining the development, differentiation, and homeostasis of multicellular organisms. The apoptosis occurs in various types of cells by many factors. Unlike necrosis, there is no release of lysosomal enzymes, apoptotic bodies are found, and cell shrinkage, nuclear enrichment, and characteristic ladder-shaped DNA fragmentation are found.

The apoptosis is a part of the normal developmental program of animals and is important in cancer prevention. Caspases, a family of proteases, are involved in cell death and cleave many target proteins. When cell death is impaired, cells that should be killed can survive and proliferate to form clones that can potentially become cancerous.

Intrinsic drug resistance in cancer treatment may be caused by abnormally expressed transcription factors that are crucial regulators of cell proliferation and cell death. Therefore, the active camptothecin derivative of the present invention can induce DDX5 protein degradation and thus cell death through a mechanism of action as a molecular glue degrader that binds to DDX5, which is a transcription factor cofactor and an oncoprotein. Here, the DDX5 protein degradation can downregulate the transcription of anti-apoptotic genes. Therefore, unlike other targeted therapeutics, drug resistance caused by abnormally expressed cell proliferation and/or cell deathrelated transcription factors is unlikely to occur, and/or acquired drug resistance induced through activation of transcription factors that are deregulated during chemotherapy and/or radiotherapy can be suppressed.

That is, since the anticancer mechanism of the active camptothecin derivative according to the present invention can bypass the molecular mechanism of cancer treatment resistance, it can be free from the problem of drug resistance. Since the treatment effect is lower than before when resistance occurs, the active camptothecin derivative of the present invention can be preferred as a standard treatment or a first-line treatment after cancer diagnosis.

In summary, the active camptothecin derivative represented by Chemical Formula 1 according to the present invention can be a targeted anticancer agent that acts on the DDX5 protein, which plays an important role in the growth, survival, proliferation, metastasis, and/or metabolism of cancer cells.

**[Clinical Results and Side Effects of ADCs with camptothecin payloads)]**

Trastuzumab deruxtecan (Enhertu) is a humanized anti-HER2 antibody linked to deruxtecan, a camptothecin derivative, via a stable linker. In a first phase 1 dose-escalation study, 22 patients with HER-2-positive advanced or metastatic breast cancer, gastric cancer, or other HER-2-expressing solid tumors were treated with trastuzumab deruxtecan at 0.8 mg/kg to 8.0 mg/kg once every 3 weeks. No dose-limiting toxicity was observed and the MTD was not reached. Target drug exposure was achieved at a dose of 6.4 mg/kg selected as the recommended phase 2 dose.

The DESTINY-Breast01 clinical trial, a pivotal single-arm phase 2 clinical trial, composed of two parts. In the first part, patients with advanced/metastatic breast cancer who had received two or more prior anti-HER2 therapies were randomized to receive trastuzumab deruxtecan at doses of 5.4 mg/kg (n = 50), 6.4 mg/kg (n = 48), or 7.4 mg/kg (n = 21) once every 3 weeks. In the second part, 134 patients were administered at the 5.4 mg/kg dose, based on efficacy and toxicity data obtained in the first part. Among 184 patients treated with 5.4 mg/kg of the trastuzumab deruxtecan, the most common adverse reactions in all grades (≥20%) were nausea (77.5%), fatigue (49.8%), alopecia (49.8%), vomiting (44.3%), neutropenia (40.3%), constipation (37.5%), anemia (33.6%), decreased appetite (33.2%), diarrhea (29.2%), leukopenia (26.9%), and thrombocytopenia (24.9%). Adverse reactions of grade 3 or higher occurred in 57.1% of patients, with neutropenia (20.7%), anemia (8.7%), nausea (7.6%), leukopenia (6.5%), lymphopenia (6.5%), and fatigue (6.0%) being the most common. Dosage discontinuation, dose reduction, and treatment discontinuation due to adverse reactions occurred in 35.3%, 23.4%, and 15.2% of patients, respectively, with pneumonia (11 patients) and interstitial lung disease (5 patients) being the most common reasons. The black box warning for patients treated with trastuzumab deruxtecan includes interstitial lung disease (ILD) and pneumonia. Treatment-related interstitial lung disease and fatal outcome

occurred in 9% and 2.6% of patients treated with trastuzumab deruxtecan, respectively. As with other HER-2 targeted ADCs, patients treated with trastuzumab deruxtecan were also at increased risk of embryo-fetal toxicity and left ventricular dysfunction.

Meanwhile, sacituzumab govitecan (Trodelvy) is a humanized anti-TOP-2 IgG linked to the active metabolite of irinotecan (SN-38) via a pH-sensitive linker. In a first-in-human dose-escalation phase 1/2 clinical trial, 25 patients with various metastatic solid tumors were treated with sacituzumab govitecan at doses of 8 to 18 mg/kg on days 1 and 8 of a 21-day cycle. The MTD for the first cycle was determined to be 12 mg/kg, with neutropenia being the dose-limiting toxicity. However, this dose level was too toxic for subsequent cycles, so the doses of 8 and 10 mg/kg were chosen for the phase 2 clinical trial. In this phase 2 clinical trial, patients with a variety of metastatic epithelial cancers who had received multiple prior treatments were treated with sacituzumab govitecan at doses of 8 mg/kg (n = 81) or 10 mg/kg (n = 97). The most common adverse reactions of all grades (≥25%) reported in the 8 mg/kg and 10 mg/kg cohorts were nausea (59% vs. 63%), diarrhea (53% vs. 62%), neutropenia (42% vs. 58%), fatigue (61% vs. 52%), vomiting (36% vs. 43%), anemia (38% vs. 42%), alopecia (46% vs. 37%), and constipation (33% vs. 37%). The most common adverse reactions of all grades (≥10%) reported in the 8 mg/kg and 10 mg/kg cohorts were neutropenia (30% vs. 36%), anemia (13% vs. 12%), diarrhea (4% vs. 10%), and leukopenia (6% vs. 12%). Dose reductions occurred in 19% and 28% of patients in the 8 mg/kg and 10 mg/kg cohorts, respectively. Neutropenia was the most common adverse reaction leading to dose delays or reductions. Patients who experienced Grade ≥3 neutropenia after the first dose were significantly more in the 10 mg/kg cohort than in the 8 mg/kg cohort (47% vs. 21%).

A black box warning for severe or life-threatening neutropenia and severe diarrhea was added to the sacituzumab govitecan label. These adverse events are likely mediated by released ("free") SN-38 because they are associated with the same toxicities as the ionotecan, an SN-38 prodrug. Of the total patients treated with sacituzumab govitecan, all-grade and Grade ≥3 neutropenia occurred in 61% and 47%, respectively. Febrile neutropenia occurred in 7% of patients. All-grade and Grade ≥3 diarrhea occurred in 65% and 12% of the total patients treated with sacituzumab govitecan, respectively. Neutropenic colitis occurred in 0.5% of patients.

Meanwhile, if a patient treated with trastuzumab deruxtecan (Enhertu) experiences ILD/pneumonitis, corticosteroid treatment is applied (https://www.enhertuhcp.com/en/gastric/manage-potential-risks/ild-and-pneumonitis).

Corticosteroids play an important role in immunosuppression by modulating various aspects of the immune response and reducing inflammation. The mechanism of action of corticosteroids is to suppress excessive immune activity and to alleviate organ damage in conditions where immune dysregulation occurs.

Immunosuppressants, especially corticosteroids, are widely used to suppress excessive immune responses and alleviate organ damage in various clinical scenarios, including autoimmune diseases, transplant rejection, and immune-related adverse events (irAEs) associated with checkpoint blockade therapy.

One of the main mechanisms of action of corticosteroids is the ability to suppress the transcription of proinflammatory genes. Within immune cells, corticosteroids bind to specific receptors in the cytoplasm, forming a corticosteroid-receptor complex. This complex migrates to the cell nucleus and binds to specific DNA sequences known as glucocorticoid response elements (GREs) located in the promoters of target genes. By doing so, the corticosteroids block the recruitment of transcription factors and cofactors, ultimately blocking the transcriptional activation of proinflammatory genes. This results in decreased synthesis of inflammatory mediators such as cytokines (e.g., interleukins and tumor necrosis factor-alpha) and prostaglandins, which contribute to tissue damage.

The corticosteroids can also directly modulate immune cell function. The corticosteroids reduce the expression of adhesion molecules in endothelial cells and downregulate chemokine receptors in immune cells, thereby inhibiting the migration of immune cells to sites of inflammation. The corticosteroids also inhibit the activation and proliferation of T cells, which are important mediators of the immune response. They can interfere with the antigen presentation process and the production of co-stimulatory molecules, thereby attenuating T cell activation and reducing effector function. The corticosteroids also suppress the function of B cells, thereby reducing antibody production.

Therefore, ADCs with Camptothecin Payloads may also be drugs that induce immune responses (in some cases, pneumonia/ILD side effects).

**[ADME profile of antibody-drug conjugates (ADCs)]**

The in vivo efficacy and in vivo side effects of anticancer drugs can be influenced by the absorption, distribution, metabolism, and excretion (ADME) characteristics of the ADC and the payload released therefrom. The ADME profile of a drug can affect its ability to reach and act on cancer cells. In other words, the ADME characteristics of the ADC and the payload released therefrom can significantly affect the in vivo efficacy and in vivo side effects.

The ability of the ADC and the payload released therefrom to penetrate tumor tissue can also be influenced by factors such as the tumor microenvironment, including blood flow and cell density.

Therefore, understanding the ADME properties of the ADCs and the payloads released therefrom and optimizing drug/drug modality selection and dose/dosage can improve the therapeutic index of these ADCs and the payloads

released therefrom, leading to better outcomes for cancer patients.

Antibodies (Abs) and antibody-drug conjugates (ADCs) have different life times and ADME profiles due to their different structures and mechanisms of action.

The antibodies are large proteins naturally produced by the immune system in response to foreign substances (antigens). Their size and complex structure give them a long circulating half-life (weeks to months) and protect them from degradation and removal. The antibodies are distributed throughout the body, including tissues, and can interact with target antigens with high specificity and affinity. The antibodies are eliminated primarily by catabolism in the reticuloendothelial system (RES), which includes the liver and spleen, and in the kidneys and other organs.

The ADCs are composed of an antibody (usually a monoclonal antibody) conjugated to a cytotoxic drug molecule. The antibody component provides specificity and targeting for the tumor or diseased tissue, while the drug component (the payload released from the ADC) provides the cytotoxic activity that kills target cells. The ADCs have a shorter half-life than antibodies, typically ranging from several days to a week. This is because ADC is internalized into target cells, resulting in lysosomal degradation of ADC and release of drug payload. The ADC is mainly eliminated by RES, but drug payload released from ADC may also undergo lymphatic drainage and subsequent metabolism and excretion in the liver and kidney via the circulation (Example 4).

As expected from the pharmacokinetics and biodistribution of monoclonal antibodies, high-affinity mAb binding to cell membrane proteins in ADC can localize a significant portion of mAb to target cell populations, and chemical conjugation of payload to anticancer mAb increases the selectivity of payload delivery to cancer cells, thereby increasing the therapeutic index of payload.

The antibodies are usually administered by subcutaneous or intravenous injection and are absorbed into the bloodstream. They can be distributed throughout the body, including tissues, but are usually limited to the extracellular space due to their size. The ADCs are also administered by injection and absorbed into the bloodstream, but the drug payload released from the ADC after being targeted to tumors or diseased tissues and, in some cases, receptor-mediated endocytosis due to the antibody component can penetrate into cells and tissues depending on the physicochemical properties thereof (e.g., hydrophobicity, size, aggregation or not), and can exert bystander effects through non-selective uptake (FIG. 31). Furthermore, the metabolism and excretion of ADCs vary depending on the specific structure and the specific drug or antibody used.

The drug-to-antibody ratio (DAR) is a very important characteristic that determines the pharmacokinetic properties and biodistribution in ADC development.

The higher the DAR, the higher the efficacy in in vitro tests. However, contrary to expectations, ADCs with high DARs show lower efficacy in vivo, which is estimated to be due to the fact that ADCs with more drugs bound thereto have higher plasma clearance rates. Accordingly, the DAR of ADC agents has been set to around 2 to 4 for a while. As a result, a technology used for drug binding to cysteine or lysine residues of antibodies is mainly used.

Since many of the cytotoxic drugs and linkers mainly used are hydrophobic, problems such as ADC aggregation, loss of affinity for target antigens, or increased plasma clearance occur. Hydrophilic linkers containing sulfonate or polyethylene glycol (PEG) solve the problems caused by hydrophobic linkers. The PEG linkers have the advantages of being water-soluble, having low toxicity, and low immunogenicity.

It has been thought for a long time that a DAR of around 4 is optimal, but in fact, this applies to second-generation linkers using MMAE or DM1 as drugs, and a higher DAR is better for third-generation linkers. Many recently approved ADCs have DAR values close to 8, and new ADCs currently in clinical trials have DAR values ranging from 1 to 15.

Antibody-drug conjugates (ADCs) using camptothecin-based payloads have attracted significant attention as a novel approach to treat various cancers, especially solid tumors. Among these ADCs, novel ADCs (e.g., Enhertu) that include newly synthesized camptothecin-based payloads (e.g., DXd) optimized for ADC modality have recently attracted particular attention due to successful clinical outcomes.

The success of these ADCs is partly due to the fact that novel camptothecins have the following characteristics: (1) superior cell growth inhibition; (2) better safety profiles than existing ADC payloads; (3) optimized bystander effects; and (4) excellent physicochemical properties suitable for the generation of high-DAR ADCs. However, despite the remarkable success of ADCs utilizing camptothecin-based payloads such as Enhertu or DS-1062a, there are still clear unmet requirements, including improved safety profiles (minimization of interstitial lung disease (ILD) and neutropenia) as described above, and development of novel ADCs with multiple MoA payloads to cope with cancer heterogeneity.

To address these unmet requirements, the present invention provides active camptothecin derivatives of Chemical Formula 1, which are designed to bind to DDX5 protein and E3 ligase as molecular glue degraders, as candidate drugs offering diverse physicochemical properties, and further synthesized (Preparation Examples 1 to 4) and evaluated (Examples 2 to 10) novel camptothecin-based payloads represented by Chemical Formula 1 (PBX series compounds).

The novel PBX series compounds represented by Chemical Formula 1, which are derivatives having, as the parent nucleus, FL118 of Chemical Formula 2, which has excellent safety profile and is a potent dual Top1/anti-apoptotic pathway inhibitor, and exhibit potent exvivo cytotoxicity comparable to Dxd, exhibit an excellent safety profile in preliminary

toxicity studies in mice and rapid clearance in PK studies (Example 4, Tables 4 and 5). This will minimize systemic side effects when the linker is cleaved early when used as the payload of ADC.

As shown in FIG 17, which is an experimental result of conversion from lactone form to carboxylate form at pH 7.4 (Example 5), it was confirmed that compounds PBX-7014, PBX-7016, and PBX-7024 represented by Chemical Formula 1 were converted from lacton form (active form) to carboxylate form (inactive form), which is inactive as a TOP1 inhibitor, more rapidly and at a higher rate than other reference compounds (exatecan, DXd, SN-38, FL118) at pH 7.4, which is the pH of blood or extracellular fluid.

In particular, the degree (63-86%) and rate (0.4-0.8%/min) of formation of the carboxylate form, which is inactive as a TOP1 inhibitor, at blood pH 7.4, are different greatly depending on the type of camptothecin derivative (FIG. 17). PBX-7014 and PBX-7016 formed the carboxylate form of 86% or more, and both converted relatively quickly from the lactone form to the carboxylate form at a rate of 0.8% per minute. The DXd, a competing drug, formed the carboxylate form of about 76.5%, and converted from the lactone form to the carboxylate form at a rate of 0.6% per minute.

The formation rate of the lactone form (measured as the carboxylate form), which is active as a TOP1 inhibitor, at the pH of the tumor microenvironment (TME) of 6.0 was confirmed to be similar for all evaluated drugs (the carboxylate form of about 20% was formed after 800 minutes) (FIG. 18).

Through this, it is predicted that compounds represented by Chemical Formula 1, such as PBX-7014, PBX-7016, and PBX-7024, form an inactive carboxylate form rather than an active lacton form, which exhibits cytotoxicity, at a relatively rapid and high rate in blood or extracellular fluid (normal tissue other than tumor tissue) compared to reference compounds, thereby exhibiting relatively low toxicity, i.e., high safety in normal cells. However, in the tumor microenvironment, all compounds PBX-7014, PBX-7016, and PBX-7024 represented by Chemical Formula 1 form active lactone forms at a level equivalent to that of other TOP1 inhibitors, so there is no factor to reduce the efficacy.

Among the PBX series compounds represented by Chemical Formula 1, two leading compounds, PBX-7014 and PBX-7016, were selected for further evaluation as ADC payloads linked to various antibodies, including trastuzumab (HER2 antibody), nimotuzumab, and sacituzumab.

Trastuzumab-7014 and trastuzumab-7016 (DAR 7-8) were easily synthesized without aggregation problems by linking PBX-7014 or PBX-7016 to trastuzumab using a conventional enzyme-cleavable linker in the same manner for comparative evaluation with Enhertu (dxdtrastuzumab) (Preparation Example 5, FIGS. 19 and 20).

In Her2-high cell line (MDA-MB-453) and Her2-low/mid cell line (FaDu), these two new ADCs showed better cytotoxicity than DS-8201a (anti-HER2-DXd), whereas in Her2-negative cell line (MDA-MB-468), they showed similar cytotoxicity to DS-8201a (Example 7 and FIG. 22).

In the NCI-N87CDX model with high Her2 expression, trastuzumab-7014 and trastuzumab-7016 showed clear dose-dependent anticancer efficacy similar to or superior to DS-8201 at the same dose (Example 8, FIGS. 23 and 24), and trastuzumab-7016 also showed bystander effect similar to Enhertu (Example 9, FIGS. 25 and 26).

Trastuzumab-7014 and trastuzumab-7016 not only showed tumor regression effects for more than 20 days through the bystander effect in tumor tissue after administration, but also showed different ADMEs with only a difference in the methyl group, thereby showing dose-dependent anticancer efficacy differences (conc. vs. effect) as well as time-dependent anticancer efficacy differences (effect vs. time) in targeted tumor tissue (Example 8, FIGS. 23 and 24).

When the hydrophobicity of a linker and a drug increases, problems such as an increase in the aggregation of the ADC and thus a decrease in the therapeutic coefficient of the drug are caused. Surprisingly, trastuzumab-7016 showed superior dose-dependent anticancer efficacy (conc. vs. effect) and time-dependent anticancer efficacy (effect vs. time) despite its increased hydrophobicity with only a difference in the methyl group from trastuzumab-7014 (Example 8, FIGS. 23 and 24).

However, in some cases, the superior time-dependent anticancer efficacy may also increase side effects (continuous T cell activation and suppression signal accumulation due to chronic antigen exposure and T cell exhaustion resulting therefrom, side effects due to bystander effect on fibroblasts derived from connective tissues surrounding tumor tissues, ILD, neutropenia). For example, depending on the drug modality (small molecule vs. ADC) of the active camptothecin derivative of Chemical Formula 1, it may be important to exceed a certain drug concentration (high), and in some cases, the time maintained above a certain drug concentration (low) may be important, and it may be important to provide a desired degree of hydrophobicity and/or control aggregation to ensure rapid or slow removal through lymphatic drainage.

Therefore, considering not only various side effects but also problems such as reduced therapeutic coefficient, the selection range of payloads of ADCs that exhibit appropriate anticancer efficacy may be expanded to various candidates including the active camptothecin derivatives of Chemical Formula 1 designed to bind to DDX5 protein and E3 ligase as molecular glue degraders, which is also a key feature of the present invention.

Despite the remarkable success of ADCs utilizing the camptothecin-based payloads such as Enhertu or DS-1062a, as candidates for multiple MoA payloads to improve the safety profile (minimize ILD and neutropenia) and cope with cancer heterogeneity, the present invention provides various active camptothecin derivatives represented by Chemical Formula 1, which are designed to bind to DDX5 protein and E3 ligase, thereby allowing selection of one or more

appropriate drugs among the active camptothecin derivatives of Chemical Formula 1, further selecting appropriate linkers, and applying appropriate dose-dosage to precisely control desired efficacy (e.g., anticancer, combination therapy) and side effects (e.g., pro-carcinogenic inflammation, drug resistance, ILD, neutropenia).

**[Toxicity profile, non-selective uptake, and bystander effect of ADCs]**

The therapeutic index of a drug is a measure of the safety and efficacy of a drug in medical treatment. It is defined as a ratio between the dose of a drug that causes a therapeutic effect and the dose that causes toxicity or adverse effects. In other words, it indicates a range between the therapeutic dose and the toxic dose of a drug.

A high therapeutic index indicates a wide margin of safety in which the effective dose is significantly lower than the toxic dose. This means that the drug can be administered at a therapeutic level without causing serious side effects or toxicity. Drugs with a high therapeutic index are generally considered safer and more desirable for clinical use.

On the other hand, a low therapeutic index means a narrow margin of safety. In such cases, the effective dose and the toxic dose are relatively close, and the risk of side effects or toxicity is high when the drug is used. Drugs with a low therapeutic index require careful monitoring and accurate dosing so as not to harm the patients.

The therapeutic index is an important consideration in drug development because it helps determine the dose range that will provide the desired therapeutic effect while minimizing the risk of adverse effects. It provides useful information when prescribing a drug and allows for an assessment of the overall benefit-to-risk ratio of the drug.

Antibody-drug conjugates (ADCs) are a rapidly growing class of anticancer therapeutics, with 100 or more ADCs in clinical trials. Currently, 12 ADCs have been approved by the US Food and Drug Administration (FDA), including gemtuzumab ozogamicin (Mylotarg), brentuximab vedotin (Adcetris), inotuzumab ozogamicin (Besponsa), trastuzumab emtansine (Kadcyla), polatuzumab vedotin (Polivy), enfortumab vedotin (Padcev), trastuzumab deruxtecan (Enhertu), sacituzumab govitecan (Trodelvy), belantamab mafodotin (Blenrep), loncastuximab tesirine (Zynlonta), tisotumab vedotin (Tivdak), and mirvetuximab soravtansine (Elahere). Furthermore, a relatively small number of payload molecules (e.g., MMAE, MMAF, DM1, DM4, calicemicin, SN38, Dxd, PBD) are used in most of approved and developing ADCs.

While several ADCs have demonstrated sufficient efficacy and safety to gain FDA approval, the clinical use of all ADCs causes significant toxicity in treated patients, and many ADCs have failed during clinical development due to their unacceptable toxicity profile. This is because off-site toxicities remain a problem, limiting tolerable ADC doses to below the level required for substantial anticancer efficacy. Even for FDA-approved ADCs, a significant proportion of treated patients require adjuvant therapy to reduce the severity of ADC-related toxicities, and many patients require dose reduction, treatment delay, or treatment discontinuation.

Analysis of clinical data has demonstrated that dose-limiting toxicities (DLTs) are often shared by multiple ADCs delivering the same cytotoxic payload, regardless of the target antigen and/or the cancer type being treated. The DLTs are typically associated with cells and tissues that do not express the target antigen (i.e., off-target toxicity) and often limit ADC doses below the level required for optimal anticancer effect.

The ADC has the potential to safely enhance the efficacy of cytotoxic drugs compared to when used as a single drug. The ADC attaches a drug to an antibody so that the antibody specifically targets only the lesion site, and thus the drug is delivered only to the lesion site and not to normal tissue. In the case of cancer cells, antibodies that specifically bind to specific antigens expressed on the surface of cancer cells are used to specifically deliver highly toxic drugs to cancer cells, thereby killing only cancer cells.

For ADC to work, the ADC must enter the target cell. The ADC antibody specifically binds to a specific antigen expressed on the surface of a target cell such as a cancer cell, and then enters the target cell through the clathrin-coated pit mechanism of action of the cell membrane.

The ADC incorporated into the cell is separated from clathrin, is fused with other vesicles within the cell, and then follows the endosome-lysosome pathway. After reaching the endosome, the drug is separated from the antibody by specific elements of the specific tumor cell internal environment there. The free cytotoxic drug separated from the antibody penetrates the lysosomal membrane and enters the cytoplasm. The activated drug exerts a pharmacological effect by binding to its own molecular target in the vicinity, thereby inducing cell death to kill cancer cells.

Among these activities, some cytotoxic drugs passively diffuse, are actively transported, or are released from the cell through dead cells. When the drug that has spread to the surroundings penetrates the cell membrane, it enters adjacent cells and causes a bystander cell-killing effect that kills the surrounding cells as well (the so-called bystander cell-killing phenomenon).

It was also reported in the study results that in contrast to DM1 of Kadcyla, Enhertu's DXd had high membrane permeability, and as a result, the payload released from the inside of the cell was delivered to cells that are adjacent to the target cell and do not express HER2. Accordingly, the Enhertu can have a potential bystander effect due to the nature of its payload and provide clinical benefits to patients who are refractory to Herceptin or Kadcyla.

A significant number of cancer-specific antigens are limitedly expressed on the surface of cancer cells. In such cases, it is not easy to deliver a sufficient amount of cytotoxic drugs into cancer cells with ADCs, so the strength of the toxin

is increased as an alternative.

In addition, since the drug must be conjugated while having little effect on the antibody, the amount of payload that can be delivered is limited. This indicates that the cytotoxic drug to be applied to ADCs must be able to kill most tumor cells at low concentrations (nM or pM) and must exhibit therapeutic effects while controlling drug release.

As the cytotoxic drug bound to ADCs, a drug that was stronger than a general anticancer drug was used. The potency of cytotoxic drugs (payload) bound to ADC is usually 100 to 1,000 times greater than the potency of the toxic drug alone.

Most of the potent cytotoxic drugs introduced into ADCs are too toxic, and the drug payload released from ADCs can also affect normal cells due to the bystander effect (FIG. 31).

Therefore, it is necessary to develop ADCs that act highly specifically on target cancer cells without causing serious side effects in normal cells.

Fibroblasts, a member of connective tissue, are predominant cells in the stroma, especially in the case of breast cancer, prostate cancer, and pancreatic cancer.

Stromal fibroblasts, also known as cancer-associated fibroblasts (CAF), are a specific type of fibroblast found in the tumor microenvironment. Stromal fibroblasts are receiving much attention because they play an important role in tumor growth, invasion, and metastasis. The CAF are responsible for the production of paracrine growth factors, proteases, and ECM components.

The camptothecin-based drug represented by Chemical Formula 1 according to the present invention is a hydrophobic small molecule that can penetrate cell membranes, so it can accumulate at high concentrations while penetrating deep into cancer tissues, and can penetrate the cell membrane and exert cytotoxicity inside the cell to cause cell death, and then are released and continuously penetrate the cell membrane of surrounding cells to move into the cell and act. In this case, the surrounding cell killing effect is high and thus may be advantageous in the treatment of heterogeneous tumors. However, in some cases, in order to provide a modality for alleviating or preventing camptothecin-based payload-based ADC toxicity such as ILD, a kind of autoimmune disease, for example, a payload that does not penetrate the cell membrane of fibroblasts, has a high IC50, or is rapidly removed through lymphatic drainage and thus does not accumulate at high concentrations can be designed/selected from a variety of candidates including the active camptothecin derivatives of Chemical Formula 1 so that the camptothecin-based payload released from the ADC does not exert a surrounding cell killing effect on fibroblasts around cancer cells. Furthermore, by appropriately applying the dose-dosage thereof, the desired efficacy (e.g., anticancer, combination therapy) and side effects (chronic cancer antigen exposure, drug resistance, ILD) can be precisely controlled.

In addition, in order to suppress non-selective uptake of the camptothecin-based drug and/or ADC released from the apoptotic cells while increasing the therapeutic index of the payload camptothecin-based drug, the present invention may select the payload of the ADC from the camptothecin-based drug candidate group represented by Chemical Formula 1. For example, by selecting a camptothecin-based drug having a high killing effect on target cancer cells at a low concentration of the drug (payload) from the candidate drug represented by Chemical Formula 1, the by-stander cell-killing effect of the free drug (payload) released from the apoptotic cells on normal cells can be suppressed through the ADC dosage that lowers the total concentration of the ADC payload, as shown in FIG. 31, or the problem of off-target toxicity of the free camptothecin-based drug (payload) released from the target/non-target apoptotic cells can be overcome.

**[Cell death of fibroblasts, a member of the connective tissue surrounding cancer cells or tumor tissues, and immune response resulting therefrom]**

Unlike SN-38, the camptothecin compound represented by Chemical Formula 1 is a small molecule drug that inhibits the Bcl family, such as Survivin, a resistance protein involved in the resistance mechanism, or binds to and degrades the oncoprotein DDX5 (p68) that controls c-Myc, survivin, and mutant Kras.

In addition, the camptothecin compound represented by Chemical Formula 1 is a camptothecin-based cytotoxic drug that degrades the DDX5 protein, and is designed to bind to the DDX5 protein and E3 ligase, and can kill cells through a mechanism of action (MoA) that degrades the oncoprotein DDX5 along with the ability to inhibit type 1 topoisomerase.

The signaling pathway that regulates cell death is a complex network of intracellular signaling pathways that are tightly regulated by various proteins and can be activated by various internal or external stimuli such as DNA damage, oxidative stress, and growth factor deprivation. Dysregulation of these pathways can lead to a variety of diseases, including cancer and neurodegenerative disorders.

All of the major signaling pathways that regulate cell death converge on the activation of caspases, a family of cysteine proteases that cleave a variety of cellular substrates and ultimately lead to cell death. Caspase activation is tightly regulated by a variety of proteins, including those of the Bcl-2 family, which can either promote or inhibit apoptosis.

The Bcl-2 family of proteins includes both pro-apoptotic and anti-apoptotic members, which can either promote or inhibit apoptosis, respectively. The balance between pro- and anti-apoptotic Bcl-2 family members is important in determining cell fate and sensitivity to cell death.

Other signaling pathways, such as the p53 pathway and the PI3K/Akt pathway, can also regulate cell death. The p53 pathway is activated in response to DNA damage and leads to the transcription of pro-apoptotic genes, such as Bax and Puma. On the other hand, the PI3K/Akt pathway can inhibit apoptosis by phosphorylating and inactivating pro-apoptotic proteins such as Bad and Bim.

Some forms of necroptosis may be programmed cellular responses to stimuli such as infection or DNA damage.

Immunogenic cell death involves changes in the cell surface, and the release of cancer antigens and damage-associated molecular patterns in the process of cancer cells being damaged and killed. The released immune-inducing substances can stimulate dendritic cells and macrophages, which are immune cells, to induce an antitumor immune response.

Apoptosis is a type of method in which damaged or aged cells die on their own, and is accompanied by the transformation and decomposition of various intracellular substances. In this process, immune-inducing substances such as cancer antigens and damage-associated molecular patterns (DAMPs), which cause the body's immunity against cancer cells, may be damaged by proteases, oxidation, etc., which may limit the effect of immunotherapy on residual cancer or metastatic cancer after treatment.

cell death caused by reactive oxygen species generated from chemotherapy is mainly apoptosis in which caspase, a protease, is involved.

Unlike apoptosis in which decomposition enzymes such as caspases is involved, necroptosis, a type of necrosis that causes cell membrane collapse and self-destruction, does not cause protein degradation, oxidation, or the like, thereby minimizing damage to cancer markers or immune-inducing substances.

Damage-associated molecular patterns (DAMPs) are molecules that can induce immune responses within cells that are released during the cell death process, and stimulate immune cells. Representatively, there are calreticulin, heat shock protein 70/90, high-mobility group box 1, and ATP.

Meanwhile, inflammation is a natural response of the body to infection or injury, and includes the release of inflammatory cytokines and the recruitment of immune cells to the affected sites. In addition, if inflammation persists for a long time, it can damage tissues and increase the risk of developing cancer.

In relation to cancer, chronic antigen exposure can cause T cell exhaustion. Tumor cells express antigens that T cells can recognize, but persistent exposure to these antigens can exhaust T cells. This weakens the antitumor response, allowing tumors to grow and spread.

Overall, the relationship between T cell exhaustion, chronic antigen exposure, and disease is complex and multifaceted.

As described above with respect to the clinical results and side effects of ADCs with Camptothecin Payloads, the ADCs with Camptothecin Payloads may also be drugs that induce immune responses through cell death (in some cases, pneumonia/ILD side effects).

One aspect of the present invention provides an anticancer formulation characterized in that (a) an active camptothecin derivative represented by Chemical Formula 1 above, which is designed to bind to DDX5 protein and E3 ligase; or (b) a prodrug thereof, preferably a carrier-drug-conjugate (CDC), more preferably an antibody-drug conjugate (ADC), which is designed to release the active camptothecin derivative (a) at a target site in vivo is administered in a dosage capable of stimulating antigen-presenting cells (APCs) through cell death of cancer cells to induce an antitumor immune response.

Here, the antigen presenting cell (APC) may be a dendritic cell or a macrophage.

In addition, one aspect of the present invention provides a method for preparing an active camptothecin derivative (a)-based anticancer agent in which the surrounding cell killing effect (bystander effect) and/or ADME profile are controlled of the active camptothecin derivative (a) so that T cell exhaustion does not occur due to chronic antigen exposure, or so that it is rapidly or slowly removed via lymphatic drainage by providing a desired degree of hydrophobicity and/or controlling aggregation,

wherein the active camptothecin derivative (a)-based anticancer agent is an anticancer formulations including (a) an active camptothecin derivative designed to bind to DDX5 protein and E3 ligase as a molecular glue degrader; or (b) a prodrug thereof, preferably a carrier-drug-conjugate (CDC), more preferably an antibody-drug conjugate (ADC), which is designed to release the active camptothecin derivative (a) at a target site in vivo,
the method characterized by comprising a step of designing the active camptothecin derivative (a) or the prodrug thereof (b) to include a camptothecin-based skeleton represented by Chemical Formula 1 as a parent nucleus, selecting a compound designed in this way, or synthesizing a compound designed in this way.

As mentioned above in relation to the clinical results and side effects of trastuzumab deruxtecan (Enhertu), the anticancer cell-mediated action that can be induced by camptothecin-based payload-based ADCs can provide a treatment that targets cancer cells via the immune system. The anticancer cell-mediated action mainly refers to attacking cancer cells using T cells and natural killer cells (NK cells).

The anticancer cell-mediated action induced by trastuzumab deruxtecan (Enhertu) is a very useful method for cancer treatment, but may often cause side effects. The most common side effect is the occurrence of autoimmune diseases. The autoimmune diseases are conditions in which the immune system mistakenly attacks its own normal tissues.

The occurrence of autoimmune diseases is possible during anticancer cell-mediated action therapy because the anticancer cell-mediated action can attack not only cancer cells but also normal cells. Common side effects may include inflammation, fatigue, rash, fever, etc. caused by drugs that induce an immune response. However, in the case of serious autoimmune diseases, it may affect various tissues and organs such as skin, joints, kidneys, liver, and lungs (e.g., ILD).

Camptothecin-based payloads released from ADCs based on camptothecin-based payloads may be beneficial in the treatment of heterogeneous tumors because they have a high effect on not only target cell death but also surrounding cell killing, but in some cases, autoimmune diseases such as ILD may occur due to cell death of fibroblasts derived from connective tissues surrounding tumor tissues.

Considering these points, although trastuzumab-7016 showed superior dose-dependent anticancer efficacy and time-dependent anticancer efficacy despite increased hydrophobicity due to only a difference in the methyl group from trastuzumab-7014 (Example 8, FIGS. 23 and 24), treatment with trastuzumab-7014 as well as trastuzumab-7016 at low concentrations/doses may be required to control surrounding cell killing effects in terms of side effects such as T cell exhaustion, ILD, and/or neutropenia.

It is required to know the properties of a drug to use it properly. Pharmacodynamic and pharmacokinetic parameters of a drug help to understand the properties of a drug.

Pharmacodynamics explains the magnitude and pattern of changes (effects) (cell viability, clinical effect) (therapeutic action, toxic effect, adverse effect) that occur in cells or the body after a drug binds to a receptor, in relation to drug concentration.

PK(Pharmaco-kinetics) shows how drug concentration changes when moving through different parts of the body via ADME according to drug or drug modality. Example 8, Table 6, FIGS. 23 and 24 can predict drug concentration of active camptothecin derivative (a) in tumor tissue over time for various drugs/modalities.

The reasons for failure in new drug development in terms of pharmacokinetics are that toxic drugs, such as camptothecin payloads released from camptothecin payload-based ADCs, can accumulate, useful drugs may have no benefit because the dose is too low to establish a treatment, and the drugs can be rapidly metabolized.

Therefore, in order to properly use the drug, for example, to control the hydrophobicity and/or aggregation of the camptothecin payload of Chemical Formula 1 to control the surrounding cell killing effect through lymphatic drainage, it is necessary to select an appropriate camptothecin payload of Chemical Formula 1, as well as apply an appropriate dose-dosage.

Dose refers to the amount of a drug administered at one time. The dose of a drug is usually prescribed by a healthcare provider based on factors such as the patient's age, weight, and health status. On the other hand, dosage refers to the frequency and duration of drug administration. It is a measure of the total amount of a drug given over a period of time, and is usually expressed as a daily or weekly amount. Dose and dosage are important considerations for the safe and effective use of a drug.

In this respect, the present invention provides, as various drug candidates, various active camptothecin derivatives of Chemical Formula 1 that bind to DDX5 protein and E3 ligase as molecular glue degraders, so that by selecting one or more appropriate drugs and appropriately applying the dose-dosage thereof, the desired efficacy (e.g., anticancer, combination therapy) and side effects (chronic cancer antigen exposure, drug resistance, ILD) can be precisely controlled.

For example, in the active camptothecin derivative of Chemical Formula 1, depending on the drug modality (small molecule, ADC), it may be important to exceed a certain drug concentration (high) in tumor tissue to control the surrounding cell killing effect in terms of cell death of heterogeneous tumor cells and/or connective tissue-derived fibroblasts surrounding tumor tissue, and in some cases, the time for which a specific drug concentration (low) or more is maintained in tumor tissue may be important, and it may be important to be removed quickly or slowly through lymphatic drainage. This can be achieved by selecting an appropriate camptothecin-based payload of Chemical Formula 1 to provide the desired degree of hydrophobicity and/or control aggregation.

**[Prodrug]**

In this specification, a prodrug includes a prodrug that becomes biologically active or more active after being placed in a predetermined physiological environment, but is not limited thereto.

For example, it refers to a compound that is designed to chemically modify a physiologically active substance or a therapeutically active organic compound and release or liberate the parent compound in vivo under enzymatic or other conditions. The prodrug is changed into the desired compound in vivo after administration. Drugs that are not suitable in terms of side effects, stability, solubility, absorption, duration of action, etc. despite their usefulness are chemically

modified to enable clinical use.

As an example of a prodrug, an antibody-drug conjugate (ADC), in which a cytotoxic drug is bound to an antibody that is expressed on the surface of cancer cells and bind to antigens that can be internalized in cells, can be expected to selectively deliver drugs to cancer cells, thereby accumulating the drugs in cancer cells and killing the cancer cells.

Among the components of ADCs, a linker is what binds the antibody and the cytotoxic drug.

The linker must be stable in the bloodstream to prevent the drug from being separated from the antibody and maintain it in a prodrug state until it reaches the target, thereby minimizing damage to normal tissues. The most ideal linker is one that is stable when the ADC is circulated throughout the body, while being cleaved in the target cell to appropriately release the cytotoxic drug, thereby safely delivering the drug to the target, and ensuring that the ADC has both efficacy and safety.

When a drug is bound to an antibody by the linker, the drug should not affect the structural stability, substrate binding characteristics, and pharmacokinetics of the antibody. One of the reasons for the failure of early ADC drugs is known to be the early release of the drug.

A considerable number of ADCs currently in clinical trials adopt chemical linkers such as hydrazone, disulfide, peptide, or thioether bonds. The process of releasing the drug from the linker utilizes differences in specific pH or enzyme concentrations within cancer cells. Usually, hydrazone and disulfide linkers have limited stability in plasma. On the other hand, peptide-based linkers have excellent stability in plasma and easy control of drug release.

Some chemical linkers control the balance of hydrophobicity between the antibody and the drug to prevent ADC aggregation in the bloodstream, which is a hydrophilic environment. Hydrophilic linkers and spacers include cyclodextrin, polyethylene glycol (PEG), or other polymers, which play a role in stability and pharmacokinetic properties in the bloodstream.

The linkers used in ADCs are classified into non-cleavable and cleavable depending on their cleavage ability.

A representative non-cleavable linker is a thioether linker, which has relatively high plasma stability compared to cleavable linkers and is resistant to proteolysis. Unlike cleavable linkers, non-cleavable linkers do not undergo degradation of the linker itself, so they have the characteristic that the drug can be released only when the antibody is degraded after introduction into cells in the form of ADC.

The drug released in this way is charged and has a low possibility of diffusing to surrounding cells (bystander effect). Although there is no bystander effect that shows toxicity to surrounding cells, it means that it is relatively safe because the effect appears only in the target cell after target cell internalization, and it can be seen that ADCs manufactured by non-cleavable linkers are more dependent on the biological mechanism within the target cell. In many studies, ADCs with non-cleavable linkers have shown high stability and efficacy and thus are being utilized as linkers for ADC development. Currently, the ADC to which this technology has been applied is Kadcycla®.

The cleavage linker is cleaved in response to specific environmental factors and releases the drug into the cytoplasm. There are enzymatic cleavage and non-enzymatic cleavage types.

The enzymatic cleavage type is cleaved by enzymes such as cathepsin B, β-glucuronidase, phosphatase, pyrophosphatase, and sulfatase.

Peptide linkers are degraded by proteases, and has high plasma stability due to the presence of protease inhibitors in the plasma. The representative protease used in ADC is cathepsin B. The cathepsin B exists at high levels in tumor tissues, giving tumor selectivity to the ADC. The peptide linkers are mainly developed as dipeptides with two amino acids attached, and were applied to Adcetris®.

The peptide linker composed of dipeptides or tetrapeptides that are recognized and cleaved by proteases in lysosomes when the ADC is internalized. The tetrapeptides were used in the early stages of development and showed limitations such as relatively slow drug release and the possibility of aggregation when combined with hydrophobic drugs. These problems were solved by the development of dipeptide linkers such as Val-Cit, Phe-Lys, Val-Lys, and Val-Ala, and were successfully applied to several ADCs such as Adcetris® and Vedotin®.

The β-glucuronide linker is degraded by β-glucuronidase, a glycolytic enzyme present in lysosomes. β-glucuronidase is overexpressed in some tumor cells to give tumor specificity. This enzyme is highly active at low pH, but decreases to 10% at neutral pH. Due to this characteristic, ADCs with β-glucuronide linkers have improved stability in plasma, preventing drug release off-targets.

Non-enzymatic cleavage types include acid-labile linkers and oxidation-reduction reaction linkers.

The acid-labile linkers are chemically unstable linkers that were developed in the early stages of ADC development and are still used despite their low stability. A representative linker is a hydrazone linker, which is stable at pH 7.3~7.5, which is the neutral environment of blood, but has a mechanism in which a drug is released by hydrolysis in a weakly acidic environment, such as around tumor cells (pH 6.5-7.2) or the endosome (pH 5.0-6.5) and lysosome (pH 4.5-5.0) when internalized into the cell. However, since the acidic state is not limited to the tumor microenvironment and is often found outside the cell, nonspecific drug release may occur.

A typical redox linker is a disulfide linker. The disulfide linker is also a type of chemically unstable linker, and is based on redox reactions. After internalization, the linker is degraded by disulfide exchange or a reducing agent such as

glutathione to release cytotoxic drugs.

Glutathione is a low-molecular-weight thiol, and is known as an antioxidant that regulates cell proliferation and death and protects cells from inflammation and oxidative stress. The glutathione exists in cells at a concentration of 0.5-10 mM, but in tumors under hypoxic conditions, it exists at a concentration up to 1000 times higher. Since it exists at a low concentration (2-20 μM) in plasma, the disulfide linkers are relatively safe and tumor-specific linkers that reduce nonspecific drug release by having high plasma stability.

The efficient release of cytotoxic drugs is affected depending on which linker is used.

In the development of linkers, it is important that the monoclonal antibodies (mAbs) should be stable during systemic circulation, reflecting the long half-life, which is an advantage of the mAbs, and that the combination of the linker and cytotoxic drugs does not affect the stability and pharmacokinetics of the antibody.

In general, the catabolism that can occur during systemic circulation of linker-cytotoxic drugs is as follows, and there are various catabolisms that are not yet known: hydrazone cleavage, protease-mediated dipeptide cleavage, esterase-mediated carbamate cleavage, hydrolysis of acetate ester, disulfide cleavage, succinimde ring opening.

Catabolism that occurs at specific sites of linker-cytotoxic drugs may sometimes maintain cytotoxic effects and exhibit activity at the target, and may also cause toxicity during systemic circulation. Conversely, if cytotoxic effects are lost, it may be difficult to expect pharmacological effects even if the drug reaches the target. For example, thailanstatin, which was developed as a payload as a splicesome inhibitor, maintains activity even when ester group hydrolysis occurs, whereas tubulin inhibitors such as cyptophycin or tubulysin lose activity when ester group hydrolysis occurs.

The stability of ADC during migration to the target is very important to achieve the desired therapeutic index regardless of the type of linker used, such as a cleavable or non-cleavable linker.

In an example of a prodrug of the active camptothecin derivative (a) represented by Chemical Formula 1, designed to bind to DDX5 protein and E3 ligase according to the present invention, the active camptothecin derivative (a) represented by Chemical Formula 1 may be used instead of exatecan in DE-310 to be described later.

As described in WO97/46260 (corresponding Korean Registration No. 10-2087017 B1), DE-310 is a complex in which exatecan is linked to a biodegradable carboxymethyldextran polyalcohol polymer via a GGFG peptide spacer. By converting exatecan into a polymeric prodrug, high blood retention is maintained, and the targeting to the tumor site is passively increased by utilizing the enhancement of permeability of tumor neovascularization and tumor tissue retention. The DE-310 continuously liberates the exatecans as an active body and the exatecans in which glycine is bound to an amino group, through cleavage of the peptide spacer by an enzyme. As a result, the pharmacokinetics are improved, and in various tumor evaluation models in nonclinical trials, DE-310 showed higher efficacy than when exatecan was administered as a single agent, even though the dosage of exatecan was reduced compared to when exatecan was administered as a single agent. Regarding DE-310, clinical trials were conducted, and it was reported that efficacy was confirmed in humans, and that the active body was confirmed to accumulate in tumors rather than in normal tissues, while there was also a report that the accumulation of DE-310 and the active body in tumors in humans was not significantly different from the accumulation in normal tissues, and that passive targeting was not observed in humans.

As a compound related to DE-310, a complex is also known in which a structural part represented by $-NH(CH_2)_4C(=O)-$ is inserted between the -GGFG- spacer and exatecan, and $-$ $GGFG-NH(CH_2)_4C(=O)-$ is used as a spacer structure.

An example of a prodrug of the active camptothecin derivative (a) represented by Chemical Formula 1, designed to bind to DDX5 protein and E3 ligase according to the present invention, may include a linker and/or spacer designed to release the active camptothecin derivative (a) at a target site in vivo.

A non-limiting example of a self immolative spacer is illustrated in FIG. 32.

According to the present invention, an active camptothecin derivative represented by Chemical Formula 1, designed to bind to DDX5 protein and E3 ligase, such as a camptothecin drug of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8 or Chemical Formula 9, can be used as a payload for various carrier-drug conjugates. That is, various carrier-drug conjugates in which the active camptothecin derivative is linked as a payload are a kind of prodrug of the active camptothecin derivative that releases the active camptothecin derivative in vivo. Accordingly, the present invention provides a carrier-drug conjugate comprising a compound which is an active camptothecin derivative represented by Chemical Formula 1, preferably a compound of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9.

In the carrier-drug conjugate according to the present invention, the carrier may be an antibody, a peptide, a repebody, and/or an aptamer, but is not limited thereto.

| | ADC | PDC | ApDC | Repebody |
|---|---|---|---|---|
| Size | > 150 kDa | 2~5 KDA | >20 KDa | 30~50 kDa |
| Advantages | ● Next-generation anticancer drug that is gaining attention in the global market because it can simultaneously exert two functions: target selectivity of antibodies and cytotoxicity of drugs | ● High tissue permeability<br>● Low immunogenicity<br>● Precise controllability of DAR<br>● Possible to introduce modified amino acids such as non-natural amino acids | ● High tissue permeability<br>● Low immunogenicity<br>● Precise controllability of DAR<br>● Easy to chemically modify | ● Relatively low immunogenicity<br>● Relatively easy to control DAR<br>● High structural stability against high temperature and pH |
| Disadvantages | ● High immunogenicity<br>● Difficult to control DAR<br>● Low tissue permeability | ● Low in vivo safety | ● Low in vivo safety | ● Early stage of development, and requiring relevant research and optimization technology |

The antibody-drug conjugate (ADC), which is a type of prodrug of the present invention, includes an immunoconjugate comprising an antibody or an antigen-binding site-containing fragment thereof, designed to release the active camptothecin derivative (a) of the present invention in vivo, or a pharmaceutically acceptable salt thereof.

Monoclonal antibodies used in ADC include IgG1, IgG2, and IgG4, among which IgG1 is most commonly used. In traditional ADCs, a full-length antigen was used. As a strategy to enhance absorption and penetration, attempts are being made to use smaller Fab, scFv, and diabody instead of monoclonal antibodies.

The immunoconjugate of the present invention can be usefully used for the treatment or prevention of cancer because it specifically binds to the antigen of cancer cells and releases the drug inside and outside the cancer cells to exhibit cytotoxicity.

For example, the antibody-drug conjugates (ADCs) are a novel drug platform that selectively delivers payloads with strong anticancer efficacy only to cancer tissues by utilizing the high tissue selectivity of antibodies that specifically bind to specific antigens expressed on the surface of cancer cells. The ADC selectively delivers a potent payload that kills cancer cells even at low concentrations of pM only to cancer tissues, and minimizes systemic drug exposure, thereby simultaneously securing anticancer efficacy and safety.

Most of the ADCs are introduced into cells through the clathrin-coated pit function. The ADCs that have moved into cells are released from clathrin, fuse with other vesicles in the cell, and then proceed to the endosome-lysosome pathway. Then, proteases in the acidic environment of endosomes cleave the linker, and the activated "free" drugs pass through the lysosomal membrane into the cytoplasm, and then bind to the molecular target of the drug, thereby arresting the cell cycle of tumor cells and causing cancer cells to die due to apoptosis. Among them, a certain amount of the drug is passively diffused in the cell, actively transported, or leaked out of the cell through dead cells. In this case, if the released drug has cell membrane permeability, it may enter surrounding cells, causing the so-called by-stander cell-killing phenomenon.

Aptamer-drug conjugate (ApDC) introduces aptamers instead of ADC's antibodies. The aptamers are single-stranded nucleic acids with a three-dimensional structure. They are discovered through the 'SELEX' (Systematic Evolution of Ligands by Exponential Enrichment) process. The SELEX is a technology that obtains functional nucleic acids that bind to target protein molecules by inserting them into a compound library.

The aptamer can bind to a target very strongly and selectively, so it is also called a chemical antibody. The aptamer is about 20 kDa in size and is known to have excellent cell penetration and low immunogenicity compared to an antibody.

Since the aptamers can be chemically synthesized, precise design of the conjugation location and number of conjugated drugs is possible when manufacturing aptamer-drug conjugates. The production costs are lower than ADCs.

The aptamers are generally composed of natural nucleic acids, so they are degraded by nucleases in the body, making them less stable in vivo. However, the ease of chemical modification of aptamers can be used to overcome the limitations in the stability of modified aptamers.

Peptide-drug conjugate (PDC) is a form in which a peptide instead of an antibody is introduced in the ADC. The peptides are composed of amino acids and have a size ranging from 500 to 5,000 Da (daltons). This is a very small size compared to antibodies that are over 150 kDa (kilodaltons). Therefore, peptide-based PDCs have superior cell penetration

ability compared to ADCs and have a very low possibility of developing immunogenicity. In addition, the peptides can be chemically synthesized. For this reason, the PDCs not only have very low production costs, but also allow for precise control of the conjugation location and ratio of peptides and drugs.

In general, the peptides are easily degraded by proteases, so they have a short biological half-life. To overcome the limitations of peptide-based drug conjugates, strategies that utilize modified peptides, such as cyclic peptides and the introduction of non-natural amino acids, are being proposed.

Repebody is a type of artificial body that does not have an antibody skeleton but has the function of recognizing antigens like antibodies. Repebody specific to target proteins can be discovered through phage display technology.

The phage display is a technology that expresses a desired protein on the surface of bacteriophage. The repebody is about 30 kDa in size, which is about 20% of the size of antibodies. Therefore, it is known to have relatively low immunogenicity and improved cell penetration compared to antibodies. In addition, it is expected that the thermal and pH stability of the repebody can be controlled, which will increase its structural stability. The production cost is also evaluated to be relatively low compared to antibodies. Due to these advantages of the repebody, interest in the development of repebody-drug conjugates (repebody-DC) as a strategy to replace antibodies with repebody is also increasing.

The carrier-drug conjugate according to the present invention may be characterized by a form in which a carrier is conjugated to a drug, i.e., a compound which is an active camptothecin derivative represented by Chemical Formula 1, preferably a compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8 or Chemical Formula 9, through a linker, but is not limited thereto.

In the carrier-drug conjugate according to the present invention, the compound which is an active camptothecin derivative represented by Chemical Formula 1, preferably the compound of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8 or Chemical Formula 9, can be linked to the linker at an appropriate site as long as its properties such as anticancer activity do not change, and accordingly, the present invention provides a drug-linker in which the compound which is an active camptothecin derivative represented by Chemical Formula 1, preferably the compound of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8 or Chemical Formula 9, is linked to the linker.

Preferably, the drug-linker according to the present invention may have a structure of Chemical Formula 3a, Chemical Formula 3a-1, Chemical Formula 4a, Chemical Formula 4a-1, Chemical Formula 5a, Chemical Formula 5a-1, Chemical Formula 6a, Chemical Formula 7a, Chemical Formula 8a, or Chemical Formula 9a, but is not limited thereto.

[Chemical Formula 3a]

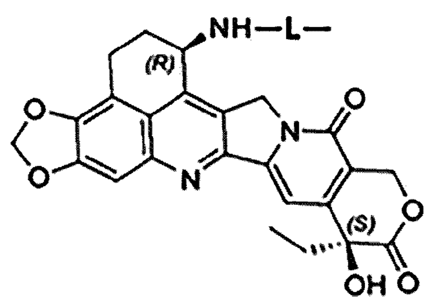

[Chemical Formula 3a-1]

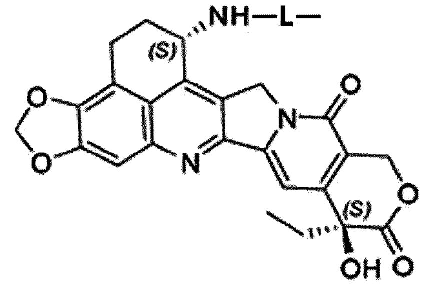

[Chemical Formula 4a]

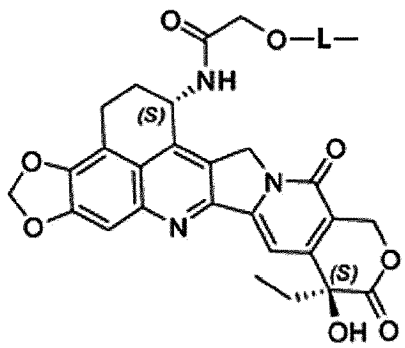

[Chemical Formula 4a-1]

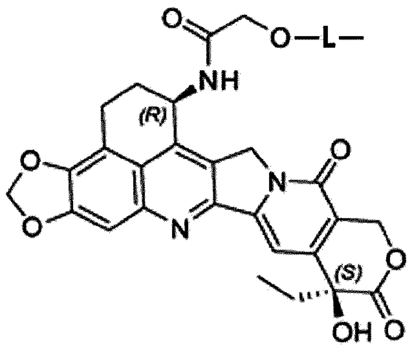

[Chemical Formula 5a]

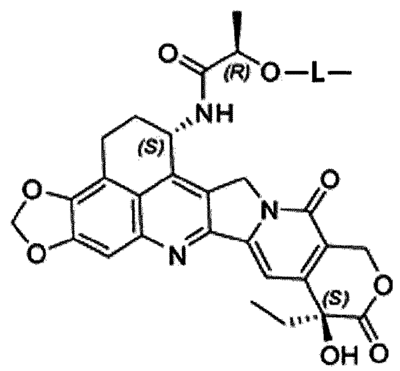

[Chemical Formula 5a-1]

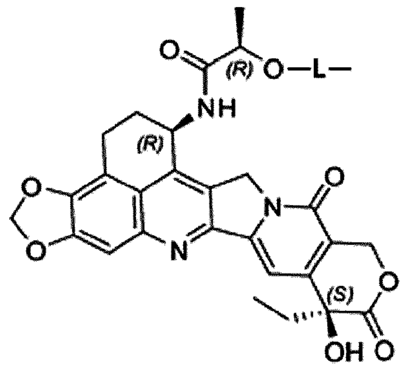

[Chemical Formula 6a]

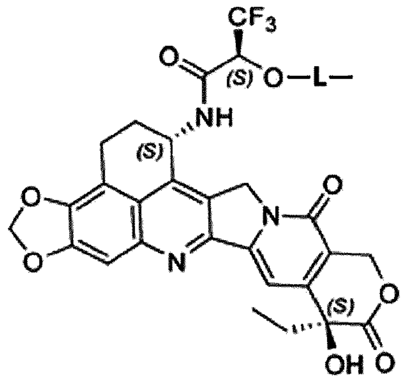

[Chemical Formula 7a]

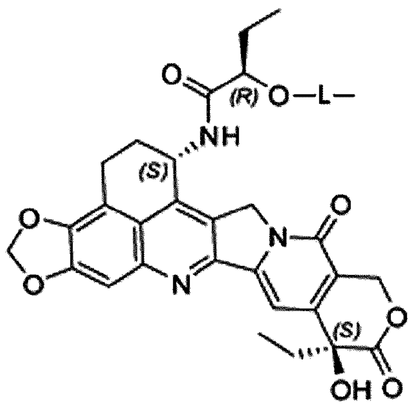

[Chemical Formula 8a]

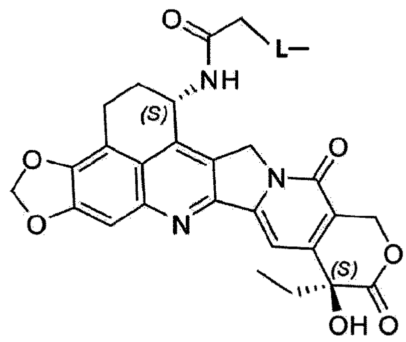

[Chemical Formula 9a]

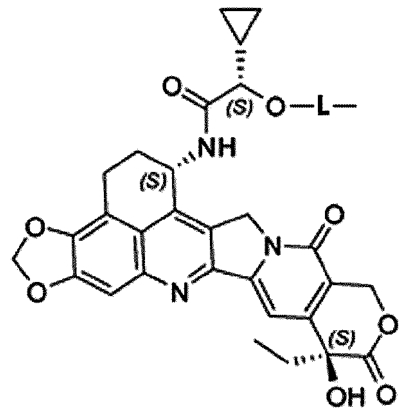

In the above structural formula, L represents a linker.

**[Target antigen and antibody]**

In ADC, the interaction between the antibody and the target antigen is important to ensure safety and obtain therapeutic effects. The two variables of antigen selection are tumor specificity and expression level. Ideally, the antigen is specifically expressed only in the tumor, and expression is absent or minimal in normal cells. Specificity is crucial to reducing toxicity and determines the success of ADC. Cancer-specific antigens are expressed as surface receptors on the surface of tumor cells or are expressed within the tumor vasculature and tumor microenvironment.

As in homogeneous tumors, cancer-specific antigens are expressed homogeneously in tumor tissues, so that all cancer cells respond to drugs, making cancer treatment easier. In the case of heterogeneous tumors, non-responsive cancer cells are mixed therein, so cancer cells that survive ADC treatment may exist. If ADCs have a surrounding cell killing effect, the problem of such heterogeneous tumors can be overcome.

Targeting antigens internalized in tumor cells is not easy in solid tumors with abundant intracellular matrix. In this case, the tumor microenvironment is targeted and approached rather than cancer cells. Cytotoxic drugs are released outside the cell by using components such as extracellular proteins, acidic substances of the extracellular matrix, or glutathione.

Among the ADCs currently undergoing clinical trials, the target antigen that is attractively used due to its excellent marketability is known to be HER2, and three HER2-targeting ADCs are in phase 3 clinical trials (Dean et al., 2021).

In the case of HER2-positive cancer, the active camptothecin derivative (a) or ADC, a prodrug thereof, drug-targeting the DDX5 protein according to the present invention can be a new treatment that can solve anti-HER2 treatment resistance.

**In** order to deliver potent cytotoxic drugs only to specific cancer cells, determining the antigen to be targeted is the first major step in ADC development. By using antibodies, long-term systemic circulation is possible with high specificity for the target and long half-life, which allows cytotoxic drugs to be selectively accumulated only in tumor cells and reduces

damage by minimizing the exposure of normal tissues, thereby reducing side effects and increasing the therapeutic effects. To this end, it is necessary to find a target antigen that can specify tumor cells, and the following conditions are required. First, the target antigen must be uniformly overexpressed on the surface of tumor cells, and must be relatively little or not expressed in normal cells. A representative example is a human epidermal growth factor receptor 2 (HER2) receptor, which is known to be expressed more than 100 times more in HER2-positive breast cancer than in normal cells. Therefore, when the tumor expression of the target antigen is analyzed through various profiling before the antibody is made, and the overexpression of a specific antigen is confirmed, a monoclonal antibody that recognizes this antigen is produced. The second is the binding force to an antigen, because the stronger the force to bind to the epitope of the antigen due to the characteristics of the antibody in which internalization occurs through the receptor, the more internalization may occur, thereby increasing the therapeutic effect. Additionally, there is low immunogenicity. Initially, the first-generation ADC was produced using antibodies produced through mice. The first-generation ADC injected mice antibodies into humans, but it was difficult to show an anticancer effect due to side effects and antibody neutralization caused by the immune response in the body to the administered mouse antibody. The problem of immune response has been greatly improved with the development of genetic engineering technology to produce chimeric antibodies, humanized antibodies, and fully human antibodies.

Antibodies that recognize antigens on cancer cells must be internalized in cells together with the drug. Bispecific antibodies are being developed to enhance cell internalization in cancer cells.

MEDI4267 (Trastuzumab-META), which is being developed by Medimmune and Astrazeneca, has shown that a biparatopic antibody targeting two non-overlapping epitopes in HER2 induces HER2 receptor clustering, thereby promoting cellular internalization, lysosomal trafficking, and degradation.

In addition, bispecific antibodies that introduce lysosomal markers CD63 or APLP2 and prolactin receptor together with tumor-targeting antibodies have shown enhanced tumor antigen recognition and cellular internalization compared to single antibodies. There is a bispecific ADC of trastuzumab and CD63 targeting HER2 (HER2xCD63-duostatin-3, Creative biolabs), which has showed a strong anticancer effect by reducing delivery to normal tissues and delivering specifically to cancer cells.

When designing the antibody-drug conjugate (ADC), immunoconjugate or carrier-drug conjugate of the present invention, the target to be targeted may be expanded to not only cancer cells, but also infectious disease organisms and/or cells associated with autoimmune diseases.

Therefore, the cell targeted by the antibody or its antigen-binding site-containing fragment may be a cancer cell, an infectious disease organism and/or a cell associated with autoimmune diseases.

Non-limiting examples of target antigens include antigens selectively distributed on the surface of cancer cells, such as Her2, FolR, and PSMA, and cancer cell-overexpressed antigens that are also distributed in small numbers in normal tissues, such as Trop2.

The cancer cell target antigen may be, for example, 5T4, ABL, ABCF1, ACVR1, ACVR1B, ACVR2, ACVR2B, ACVRL1, ADORA2A, AFP, Aggrecan, AGR2, AICDA, AIF1, AIGI, AKAP1, AKAP2, ALCAM, ALK, AMH, AMHR2, ANGPT1, ANGPT2, ANGPTL3, ANGPTL4, ANPEP, APC, APOCl, AR, aromatase, ASPH, ATX, AX1, AXL, AZGP1 (zinc-a-glycoprotein), B4GALNT1, B7, B7.1, B7.2, B7-H1, B7-H3, B7-H4, B7-H6, BAD, BAFF, BAG1, BAI1, BCR, BCL2, BCL6, BCMA, BDNF, BLNK, BLR1 (MDR15), BlyS, BMP1, BMP2, BMP3B (GDFIO), BMP4, BMP6, BMP8, BMP10, BMPR1A, BMPR1B, BMPR2, BPAG1 (Plectin), BRCA1, C19orflO (IL27w), C3, C4A, C5, C5R1, CA6, CA9, CANT1, CAPRIN-1, CASP1, CASP4, CAV1, CCBP2 (D6/JAB61), CCL1 (1-309), CCLI1 (eotaxin), CCL13 (MCP-4), CCL15 (MIP-Id), CCL16 (HCC-4), CCL17 (TARC), CCL18 (PARC), CCL19 (MIP-3b), CCL2 (MCP-1), MCAF, CCL20 (MIP-3a), CCL21 (MEP-2), SLC, exodus-2, CCL22(MDC/STC-I), CCL23 (MPIF-I), CCL24 (MPIF-2/eotaxin-2), CCL25 (TECK), CCL26(eotaxin-3), CCL27 (CTACK/ILC), CCL28, CCL3 (MIP-Ia), CCL4 (MIPIb), CCL5(RANTES), CCL7 (MCP-3), CCL8 (mcp-2), CCNA1, CCNA2, CCND1, CCNE1, CCNE2, CCR1 (CKR1/HM145), CCR2 (mcp-IRB/RA), CCR3 (CKR3/CMKBR3), CCR4, CCR5(CMKBR5/ChemR13), CCR6 (CMKBR6/CKR-L3/STRL22/DRY6), CCR7 (CKR7/EBI1), CCR8 or CDw198 (CMKBR8/TERI/CKR-L1), CCR9 (GPR-9-6), CCRL1 (VSHK1), CCRL2 (L-CCR), CD13, CD164, CD19, CDH6, CDIC, CD2, CD20, CD21, CD200, CD22, CD23, CD24, CD27, CD28, CD29, CD3, CD33, CD35, CD37, CD38, CD3E, CD3G, CD3Z, CD4, CD40, CD40L, CD44, CD45RB, CD47, CD52, CD56, CD69, CD70, CD72, CD74, CD79A, CD79B, CD8, CD80, CD81, CD83, CD86, CD97, CD99, CD117, CD125, CD137, CD147, CD179b, CD223, CD279, CD152, CD274, CDH1 (E-cadherin), CDH1O, CDH12, CDH13, CDH18, CDH19, CDH2O, CDH3, CDH5, CDH7, CDH8, CDH9, CDH17, CDK2, CDK3, CDK4, CDK5, CDK6, CDK7, CDK9, CDKN1A (p21Wap1/Cip1), CDKN1B (p27Kip1), CDKN1C, CDKN2A (p16INK4a), CDKN2B, CDKN2C, CDKN3, CEA, CEACAM5, CEACAM6, CEBPB, CERI, CFC1B, CHGA, CHGB, Chitinase, CHST1O, CIK, CKLFSF2, CKLFSF3, CKLFSF4, CKLFSF5, CKLFSF6, CKLFSF7, CKLFSF8, CLDN3, CLDN6, CLDN7 (Claudin-7), CLDN18, CLEC5A, CLEC6A, CLEC11A, CLEC14A, CLN3, CLU (clusterin), CMKLR1, CMKOR1 (RDC1), CNR1, C-MET, COL18A1, COLIA1, COL4A3, COL6A1, CR2, Cripto, CRP, CSF1 (M-CSF), CSF2 (GM-CSF), CSF3 (GCSF), CTAG1B (NY-ESO-1), CTLA4, CTL8, CTNNB1 (b--catenin), CTSB (cathepsin B), CX3CL1 (SCYD1), CX3CR1 (V28), CXCL1 (GRO1), CXCL1O (IP-IO), CXCLI1 (1-TAC/IP-9), CXCL12 (SDF1), CXCL13, CXCL14, CXCL16, CXCL2 (GRO2), CXCL3 (GRO3), CXCL5 (ENA-78/LIX), CXCL6 (GCP-2), CXCL9 (MIG), CXCR3 (GPR9/CKR-L2), CXCR4, CXCR6 (TYMSTR/STRL33/Bonzo), CYB5, CYC1, CYSLTR1, DAB2IP, DES, DKFZp451J0118, DLK1, DNCL1, DPP4, E2F1, Engel, Edge, Fennel, EFNA3, EFNB2, EGF, EGFR, ELAC2, ENG, Enola, ENO2, ENO3, EpCAM, EPHA1, EPHA2, EPHA3, EPHA4, EPHA5, EPHA6, EPHA7, EPHA8, EPHA9, EPHA10, EPHB1, EPHB2, EPHB3, EPHB4, EPHB5, EPHB6, EPHRIN-A1, EPHRIN-A2, EPHRINA3, EPHRIN-A4, EPHRIN-A5, EPHRIN-A6, EPHRIN-B1, EPHRIN-B2, EPHRIN-B3, EPHB4, EPG, ERBB2 (HER-2), ERBB3, ERBB4, EREG, ERK8, Estrogen receptor, Earl, ESR2, F3 (TF), FADD, FAP, Farnesyltransferase, FasL, FASNf, FCER1A, FCER2, FCGR3A, FGF, FGF1 (aFGF), FGF10, FGF1 1, FGF12, FGF12B, FGF13, FGF14, FGF16, FGF17, FGF18, FGF19, FGF2 (bFGF), FGF20, FGF21, FGF22, FGF23, FGF3 (int-2), FGF4 (HST), FGF5, FGF6 (HST-2), FGF7 (KGF), FGF8, FGF9, FGFR1, FGFR2, FGFR3, FGFR4, FIGF (VEGFD), FIL1(EPSILON), FBL1 (ZETA), FLJ12584, FLJ25530, FLRT1 (fibronectin), FLT1, FLT-3, FOLR1, FOS, FOSL1(FRA-1), FR-alpha, FY (DARC), GABRP (GABAa), GAGEB1, GAGEC1, GALNAC4S-6ST, GATA3, GD2, GD3, GDF5, GFI1, GFRA1, GGT1, GM-CSF, GNAS1, GNRH1, GPC1, GPC3, GPNB, GPR2 (CCR10), GPR31, GPR44, GPR81 (FKSG80), GRCC1O (C1O), GRP, GSN (Gelsolin), GSTP1, GUCY2C, HAVCR1, HAVCR2, HDAC, HDAC4, **HDAC5, HDAC7A,** HDAC9, Hedgehog, HER3, HGF, HIF1A, HIP1, Histamine and histamine receptors, HLA-A, HLA-DR, HLA-DRA, HLA-E, HM74, HMOXI, HSP90, HUMCYT2A, ICEBERG, ICOSL, ID2, IFN-a, IFNA1, IFNA2, IFNA4, IFNA5, EFNA6, BFNA7, IFNB1, IFNgamma, IFNW1, IGBP1, IGF1, IGFIR, IGF2, IGFBP2, IGFBP3, IGFBP6, DL-1, ILIO, ILIORA, ILIORB, IL-1, IL1R1 (CD121a), IL1R2(CD121b), IL-IRA, IL-2, IL2RA (CD25), IL2RB(CD122), IL2RG(CD132), IL-4, IL-4R(CD123), IL-5, IL5RA(CD125), IL3RB(CD131), IL-6, IL6RA, (CD126), IR6RB(CD130), IL-7, IL7RA(CD127), IL-8, CXCR1 (IL8RA), CXCR2, (IL8RB/CD128), IL-9, IL9R(CD129), IL-10, IL10RA(CD210), IL10RB(CDW210B), IL-11, IL11RA, IL-12, IL-12A, IL-12B, IL-12RB1, IL-12RB2, IL-13, IL13RA1, IL13RA2, IL14, IL15, IL15RA, IL16, IL17, IL17A, IL17B, IL17C, IL17R, IL18, IL18BP, IL18R1, IL18RAP, IL19, ILIA, ILIB, ILIF10, ILIF5, IL1F6, ILIF7, IL1F8, DL1F9, ILIHYI, ILIR1, IL1R2, ILIRAP, ILIRAPLI, ILIRAPL2, ILIRL1, ILIRL2, ILIRN, IL2, IL20, IL20RA, IL21R, IL22, IL22R, IL22RA2, IL23, DL24, IL25, IL26, IL27, IL28A, IL28B, IL29, IL2RA, IL2RB, IL2RG, IL3, IL30, IL3RA, IL4, 1L4, IL6ST (Glycoprotein 130), ILK, INHA, INHBA, INSL3, INSL4, IRAK1, IRAK2, ITGA1, ITGA2, ITGA3, ITGA6 (α6 integrin), ITGAV, ITGB3, ITGB4 (β4 integrin), JAG1, JAK1, JAK3, JTB, JUN, K6HF, KAI1, KDR, KIT, KITLG, KLF5 (GC Box BP), KLF6, KLK10, KLK12, KLK13, KLK14, KLK15, KLK3, KLK4, KLK5, KLK6, KLK9, KRT1, KRT19 (keratin 19), KRT2A, KRTHB6 (hair-specific type II keratin), L1CAM, LAG3, LAMA5, LAMP1, LEP (leptin), Lewis Yantigen ("LeY"), LILRB1, Lingo-p75, Lingo-Troy, LGALS3BP, LRRC15, LPS, LTA (TNF-b), LTB, LTB4R (GPR16), LTB4R2, LTBR, LY75, LYPD3, MACMARCKS, MAG or OMgp, MAGEA3, MAGEA6, MAP2K7 (c-Jun), MCP-1, MDK, MIB1, midkine, MIF, MISRII, MJP-2, MLSN, MK, MKI67 (Ki-67), MMP2, MMP9, MS4A1, MSMB, MT3 (Metallothionectin-UI), mTOR, MTSS1, MUC1 (mucin), MUC16, MYC, MYD88, NCK2, NCR3LG1, neurocan, NFKBI, NFKB2, NGFB (NGF), NGFR, NgR-Lingo, NgRNogo66, (Nogo), NgR-p75, NgR-Troy, NMEI (NM23A), NOTCH, NOTCH1, NOTCH3, NOX5, NPPB, NROB1, NROB2, NRID1, NR1D2, NR1H2, NR1H3, NR1H4, NR112, NR113, NR2C1, NR2C2, NR2E1, NR2E3, NR2F1, NR2F2, NR2F6, NR3C1, NR3C2, NR4A1, NR4A2, NR4A3, NR5A1, NR5A2, NR6A1, NRP1, NRP2, NT5E, NTN4, NY-ESO1, ODZI, OPRDI, P2RX7, PAP, PART1, PATE, PAWR, P-cardherin, PCA3, PCD1, PD-L1, PCDGF, PCNA, PDGFA, PDGFB, PDGFRA, PDGFRB, PECAMI, L1-CAM, peg-asparaginase, PF4 (CXCL4), PGF, PGR, phosphacan, PIAS2, PI3 kinase, PIK3CG, PLAU (uPA), PLG, PLXDCI, PKC, PKC-beta, PPBP (CXCL7), PPID, PR1, PRAME, PRKCQ, PRKD1, PRL, PROC, PROK2, PSAP, PSCA, PSMA, PTAFR, PTEN, PTHR2, PTGS2 (COX-2), PTN, PVRIG, RAC2 (P21Rac2), RANK, RANK ligand, RARB, RGS1, RGS13, RGS3, RNFI1O (ZNF144), Ron, ROBO2, ROR1, RXR, S100A2, SCGB 1D2 (lipophilin B), SCGB2A1 (mammaglobin 2), SCGB2A2 (mammaglobin 1), SCYE1 (endothelial monocyte-activating cytokine), SDF2, SERPENA1, SERPINA3, SERPINB5 (maspin), SERPINEI (PAI-I), SERPINFI, SHIP-1, SHIP-2, SHB1, SHB2, SHBG, SfcAZ, SLAMF7, SLC2A2, SLC33A1, SLC43A1, SLC44A4, SLC34A2, SLIT2, SPP1, SPRR1B (Spr1), ST6GAL1, ST8SIA1, STAB1, STATE, STEAP, STEAP2, TB4R2, TBX21, TCP1O, TDGF1, TEK, TGFA, TGFB1, TGFB1I1, TGFB2, TGFB3, TGFBI, TGFBR1, TGFBR2, TGFBR3, THIL, THBS1 (thrombospondin-1), THBS2, THBS4, THPO, TIE (Tie-1), TIMP3, tissue factor, TLR1, TLR2, TLR3, TLR4, TLR5, TLR6, TLR7, TLR8, TLR9, TLR10, TLR11, TNF, TNF-a, TNFAIP2 (B94), TNFAIP3, TNFRSFI1A, TNFRSF1A, TNFRSF1B, TNFRSF21, TNFRSF5, TNFRSF6 (Fas), TNFRSF7, TNFRSF8, TNFRSF9, TNFSF1O (TRAIL), TNFRSF10A, TNFRSF10B, TNFRSF12A, TNFRSF17, TNFSF1 1 (TRANCE), TNFSF12 (APO3L), TNFSF13 (April), TNFSF13B, TNFSF14 (HVEM-L), TNFRSF14 (HVEM), TNFSF15 (VEGI), TNFSF18, TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TOLLIP, Toll-like receptor, TOP2A (topoisomerase Iia), TP53, TPM1, TPM2, TRADD, TRAF1, TRAF2, TRAF3, TRAF4, TRAF5, TRAF6, TRKA, TREM1, TREM2, TROP2, TRPC6, TSLP, TWEAK, Tyrosinase, uPAR, VEGF, VEGFB, VEGFC, versican, VHL C5, VLA-4, WT1, Wnt-1, XCL1 (lympotactin), XCL2 (SCM-Ib), XCRI (GPR5/CCXCR1), YY1, ZFPM2, CLEC4C (BDCA-2, DLEC, CD303, CDH6, CLECSF7), CLEC4D (MCL, CLECSF8), CLEC4E (Mincle), CLEC6A (dectin-2), CLEC5A (MDL-1, CLECSF5), CLEC1B (CLEC-2), CLEC9A (DNGR-1), CLEC7A (dectin-1), CLEC11A, PDGFRa, SLAMF7, GP6 (GPVI), LILRA1 (CD85I), LILRA2 (CD85H, ILT1), LILRA4 (CD85G, ILT7), LILRA5 (CD85F, ILT11), LILRA6 (CD85b, ILT8), LILRB1, NCR1 (CD335, LY94, NKp46), NCR3 (CD335, LY94, NKp46), NCR3 (CD337, NKp30), OSCAR, TARM1, CD30, CD300C, CD300E, CD300LB (CD300B), CD300LD (CD300D), KIR2DL4 (CD158D), KIR2DS, KLRC2 (CD159C, NKG2C), KLRK1 (CD314, NKG2D), NCR2 (CD336, NKp44), PILRB, SIGLEC1 (CD169, SN), SIGLEC5, SIGLEC6, SIGLEC7, SIGLEC8, SIGLEC9, SIGLEC10, SIGLEC11, SIGLEC12,

SIGLEC14, SIGLEC15 (CD33L3), SIGLEC16, SIRPA, SIRPB1 (CD172B), TREM1 (CD354), TREM2, KLRF1 (NKp80), 17-1A, SLAM7, MSLN, CTAG1B/NY-ESO-1, MAGEA3/A6, ATP5I (Q06185), OAT (P29758), AIFM1 (Q9Z0X1), AOFA (Q64133), MTDC (P18155), CMC1 (Q8BH59), PREP (Q8K411), YMEL1 (O88967), LPPRC (Q6PB66), LONM (Q8CGK3), ACON (Q99KI0), ODO1 (Q60597), IDHP (P54071), ALDH2 (P47738), ATPB (P56480), AATM (P05202), TMM93 (Q9CQW0), ERGI3 (Q9CQE7), RTN4 (Q99P72), CL041 (Q8BQR4), ERLN2 (Q8BFZ9), TERA (Q01853), DAD1 (P61804), CALX (P35564), CALU (O35887), VAPA (Q9WV55), MOGS (Q80UM7), GANAB (Q8BHN3), ERO1A (Q8R180), UGGG1 (Q6P5E4), P4HA1 (Q60715), HYEP (Q9D379), CALR (P14211), AT2A2 (055143), PDIA4 (P08003), PDIA1 (P09103), PDIA3 (P27773), PDIA6 (Q922R8), CLH (Q68FD5), PPIB (P24369), TCPG (P80318), MOT4 (P57787), NICA (P57716), BASI (P18572), VAPA (Q9WV55), ENV2 (P11370), VAT1 (Q62465), 4F2 (P10852), ENOA (P17182), ILK (O55222), GPNMB (Q99P91), ENV1 (P10404), ERO1A (Q8R180), CLH (Q68FD5), DSG1A (Q61495), AT1A1 (Q8VDN2), HYOU1 (Q9JKR6), TRAP1 (Q9CQN1), GRP75 (P38647), ENPL (P08113), CH60 (P63038), or CH10 (Q64433), but is not limited thereto.

The target antigen may be an antigen that is distributed 10 times or more abundantly in cancer cells than in normal cells.

Non-limiting examples of antibodies in the present invention that can be used in antibody-drug conjugates (ADCs) according to the present invention include Urelumab, Utomilumab, Bebtelovimab, Aducanumab, Bapinezumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, and Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Dapirolizumab, Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Datopotamab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, Dupilumab, Defemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Fabezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociferlimab, Tiragolumab, Vibostolimab, Surzebiclimab, Cobolimab, Sabatolimab, Concizumab, Marstacimab, Adalimumab, Golimumab, Infliximab, Certolizumab, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab, Obrindatamab, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Naptumomab, Belantamab, Pivekimab, Praluzatamab, Coltuximab, Denintuzumab, Loncastuximab, Ibritumomab, Inotuzumab, Epratuzumab, Moxetumomab, Brentuximab, Gemtuzumab, Vadastuximab, Lorvotuzumab, Polatuzumab, Tusamitamab, Telisotuzumab, Rovalpituzumab, Depatuxizumab, Farletuzumab, Mirvetuximab, Disitamab, Anetumab, Enfortumab, Sacituzumab, Vobarilizumab, Cadonilimab, Vudalimab, Tebotelimab, Ivonescimab, Erfonrilimab, Ozoralizumab, Faricimab, Vanucizumab, Navicixizumab, and the like.

**[Pharmaceutical composition for preventing or treating cancer]**

The present invention provides a pharmaceutical composition for preventing or treating cancer, including a compound represented by Chemical Formula 1, for example, Chemical Formula 3, Chemical Formula 3-1, Chemical

Formula 4, Chemical Formula 4-1, Chemical Formula 5, Chemical Formula 5-1, Chemical Formula 6, Chemical Formula 7, Chemical Formula 8, or Chemical Formula 9 as mentioned above, a pharmaceutically acceptable salt thereof, a solvate thereof, or a prodrug thereof, preferably a carrier-drug conjugate (CDC), more preferably an antibody-drug conjugate (ADC), as an active ingredient.

According to the present invention, the active camptothecin derivative (a) represented by Chemical Formula 1 or the prodrug (b) thereof, preferably a carrier-drug conjugate (CDC), more preferably an antibody-drug conjugate (ADC), may be included as an active ingredient in a pharmaceutical composition for administration to a patient group in which DDX5 functions as an oncoprotein in cells.

For example, a patient group to which the active camptothecin derivative (a) or the prodrug thereof (b) is to be administered may be identified by quantitatively or qualitatively analyzing intracellular DDX5 protein using the complex containing a DDX5 protein-targeting ligand, in which the ability of the FL118 compound of Chemical Formula 2 or the active camptothecin derivative (a) to inhibit type 1 topoisomerase is preferably inactivated through a non-cleavable linker connection, in a tissue biopsy or liquid biopsy.

According to the present invention, the active camptothecin derivative (a) represented by Chemical Formula 1, for example, the compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, is a camptothecin derivative having a novel structure. Therefore, through this, a drug candidate having more desirable properties in terms of efficacy, toxicity, selectivity, duration of action, administration, handling, stability, production possibility, and/or the like of a Dxd drug may be provided. In addition, it is possible to optimize the interaction between the target and the drug candidate, that is, the pharmacodynamic properties, and to improve the ability to optimize the drug's access to the target, that is, the pharmacodynamic ability.

In addition, one embodiment of the present invention provides a method for treating or preventing cancer, including administering to a subject in need thereof a therapeutically effective amount of a compound represented by Chemical Formula 1, for example, Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 as mentioned above, a pharmaceutically acceptable salt thereof, a solvate thereof, or a prodrug thereof, preferably a carrier-drug conjugate (CDC), more preferably an antibody-drug conjugate (ADC). The subject may be a mammal including a human.

In the present invention, the cancer includes all cancers treatable by inhibition of topoisomerase I and/or inhibition of one or more cancer-associated survival genes selected from the group consisting of DDX5, survivin, Mcl-1, XIAP and cIAP2, and may be solid cancer or hematological cancer. For example, it may be one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, bile duct cancer, colon cancer, chronic myeloid leukemia, chronic lymphocytic leukemia, retinoblastoma, choroidal melanoma, ampulla of Vater, bladder cancer, peritoneal cancer, parathyroid cancer, adrenal cancer, paranasal sinus cancer, non-small cell lung cancer, tongue cancer, astrocytoma, small cell lung cancer, pediatric brain cancer, pediatric lymphoma, pediatric leukemia, small intestinal cancer, meningioma, esophageal cancer, glioma, renal pelvis cancer, kidney cancer, cardiac cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms' cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic neuroma, pancreatic cancer, salivary gland cancer, Kaposi sarcoma, Paget's disease, tonsil cancer, squamous cell carcinoma, pulmonary adenocarcinoma, lung cancer, lung squamous cell carcinoma, skin cancer, anal cancer, rhabdomyosarcoma, laryngeal cancer, pleural cancer, blood cancer, and thymic cancer, but is not limited thereto. In addition, the cancer includes not only primary cancer but also metastatic cancer.

In this specification, "patient" and "subject" refer to an animal such as a mammal. In certain embodiments, the patient is a human. In another embodiment, the patient is a non-human animal such as a dog, a cat, a domestic animal (e.g., a horse, a pig, or a donkey), a chimpanzee or a monkey.

The term "therapeutically effective amount" as used herein refers to an amount of an active camptothecin derivative (a) represented by Chemical Formula 1, such as a compound represented by Chemical Formula 3, Chemical Formula 3-1, Chemical Formula 4, Chemical Formula 4-1, Chemical Formula 5 or Chemical Formula 5-1, a pharmaceutically acceptable salt thereof, a solvate thereof, or a prodrug thereof, which is effective for treating or preventing cancer. Specifically, a "therapeutically effective amount" means an amount sufficient to treat a disease with a reasonable benefit/risk ratio applicable to medical treatment, and the effective dose level can be determined according to factors, including individual type and severity, age, sex, disease type, drug activity, drug sensitivity, administration time, administration route and discharge rate, treatment period, drugs used concurrently, and other factors well known in the medical field. The pharmaceutical composition of the present invention may be administered individually or in combination with other therapeutic agents, and may be administered sequentially or simultaneously with commercially available therapeutic agents. It may also be administered singly or multiply. Considering all of the above factors, it is important to administer an amount that can achieve the maximum effect with the minimum amount without side effects, and the compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8 or Chemical Formula 9 of the present invention and its pharmaceutically acceptable salt exhibit a dose-dependent effect, so the administration dose may be easily determined by those skilled in the art according to various factors such as the patient's condition, age, gender and complications. Since the active ingredient of the pharmaceutical composition of the present invention has excellent safety, it may be used even at a determined administration dose or higher.

Furthermore, according to one specific embodiment of the present invention, the present invention provides a use of an active camptothecin derivative (a) represented by Chemical Formula 1, for example, a compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, a pharmaceutically acceptable salt thereof, a solvate thereof, or a prodrug thereof, preferably a carrier-drug conjugate (CDC), more preferably an antibody-drug conjugate (ADC), for use in the manufacture of a medicament for the treatment or prevention of cancer. The compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, a pharmaceutically acceptable salt thereof, a solvate thereof, or a prodrug thereof may be mixed with an acceptable excipient, diluent, carrier, etc., and may be manufactured into a composite preparation together with other active agents to have a synergistic effect of the active ingredients.

Matters mentioned in the uses, compositions, and treatment methods of the present invention are equally applicable unless they are contradictory.

In this specification, the anticancer effect or therapeutic effect of an anticancer agent may refer to an action that reduces the severity of cancer, reduces tumor size, or delays or slows down the progression of cancer that occurs while a patient is suffering from a specific cancer.

For example, the anticancer effect of an anticancer agent may be a cell viability (change in the degree of cytotoxicity or cell number) of cancer cells after treating the cancer cells with an anticancer agent in vitro and/or in vivo. For example, it can be indirectly confirmed through a drug response test using a cell line or a non-clinical animal model (xenograft). In addition, the anticancer effect of an anticancer agent can also be directly confirmed in cancer patients, and data related thereto can be derived and used as a database. In addition, animal model PK parameters and/or toxicity profiles may be considered in parallel when designing a dosing guideline for an anticancer agent.

The anticancer effect of an anticancer agent can be inferred from in-vitro data, such as the % maximum effect of the anticancer agent, such as $IC_{50}$, $IC_{60}$, $IC_{70}$, $IC_{80}$ and $IC_{90}$, and can also be confirmed in nonclinical animal models and clinical cancer patients through in-vivo data, such as the drug's maximum blood concentration (Cmax) and/or the area under the blood drug concentration-time curve (AUC).

The reactivity of an anticancer agent refers to a clinical sensitivity in terms of the anticancer effect.

When referring to treatment using an anticancer agent, "sensitivity" and "sensitive" are relative terms referring to the degree of the compound's effect in alleviating or reducing the progression of the tumor or disease being treated.

An "effective patient anti-cancer effect/response" may be, for example, a 5%, 10%, 15%, 20%, 25%, 30%, 40%, 50%, 60%, 70%, 80%, 90%, 100%, 200%, or more inhibition in patient response as measured by any suitable means, such as gene expression, cell counts, assay results, etc.

In this specification, the dosage is a dose at which the efficacy is expected. In the present invention, the efficacy may be an anticancer effect. The reactivity (anticancer effect) of the anticancer agent is the degree of response, and may be the % maximum effect of the anticancer agent, such as $IC_{50}$, $IC_{60}$, $IC_{70}$, $IC_{80}$ and $IC_{90}$, and the value at which it exerts toxicity to normal cells ($LC_{50}$).

For example, oral formulations may be formulated using various formulation techniques known in the art. For example, it may include a biodegradable (hydrolyzable) polymeric carrier used for attachment to the oral mucosa. It is manufactured to be slowly eroded over a predetermined period of time, where the drug delivery is essentially provided in its entirety.

In the oral formulation, the drug delivery avoids the disadvantages encountered with oral drug administration, such as slow absorption, degradation of the active agent by fluids present in the gastrointestinal tract, and/or first-pass inactivation in the liver. For the biodegradable (hydrolyzable) polymeric carrier, virtually any such carrier may be used so long as the desired drug release profile is not impaired, and the carrier is compatible with any other ingredients present in the oral dosage unit. Typically, the polymeric carrier comprises a hydrophilic (water-soluble and water-swellable) polymer that adheres to the wet surface of the oral mucosa. An example of a polymeric carrier useful herein is an acrylic acid polymer (e.g., a carbomer). In some embodiments, non-limiting examples of other ingredients that may be incorporated

into the oral formulation include disintegrants, diluents, binders, lubricants, flavoring agents, coloring agents, preservatives, and the like. In some embodiments, for oral or sublingual administration, it may be in the form of tablets, rosgenes, or gels formulated by the conventional manner.

In some embodiments, when the patient's condition improves, administration of the compound may be continued at the physician's discretion; alternatively, the dose of the drug being administered may be temporarily reduced or temporarily discontinued for a certain length of time (i.e., a "drug holiday"). The length of a drug holiday may vary from 2 days to 1 year, and includes, by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, or 365 days. In some embodiments, the dose reduction during the drug holiday is 10%-100%, including, by way of example only, 10%, 15%, 20%, 25%, 30%, 35%, 40%, 45%, 50%, 55%, 60%, 65%, 70%, 75%, 80%, 85%, 90%, 95%, or 100%.

When the patient's condition improves, a maintenance dose is administered, if necessary. Thereafter, the dosage or frequency of administration, or both, may be reduced as a function of symptoms to a level at which the improved disease, disorder or condition is maintained. However, the patient requires intermittent treatment over a long period of time if any symptoms recur.

The amount of a given formulation that corresponds to such an amount will vary depending on factors of the subject requiring treatment, such as the particular compound, the severity of the disease, the identity (e.g., body weight), but can nevertheless be routinely determined in a manner known in the art, for example, depending on the formulation to be administered, the route of administration, and the particular circumstances surrounding the subject being treated. In general, however, the dose used for adult human treatment will typically range from about 0.02 to about 5000 mg/day, or about 1 to about 1500 mg/day.

A single dose herein may be provided as a single dose or as divided doses administered simultaneously, for example, as 2, 3, 4 or more sub-doses.

In some embodiments, the oral formulation is a unit dosage form suitable for single administration of precise dosages. In the unit dosage form, the formulation is divided into unit doses containing appropriate amounts of one or more compounds. In some embodiments, the unit dosage is in the form of a package containing a separate amount of the formulation. Non-limiting examples thereof are packaged tablets or capsules, and powder vials or ampoules. The aqueous suspension composition may be packaged in a single-dose non-reclosable container. Alternatively, a multi-dose reclosable container may be used, and in this case, it is typical to include a preservative in the composition.

In some embodiments, the parenteral injectable formulation is provided in unit dosage form, including but not limited to ampoules, or in multi-dose containers, with an added preservative.

Typically, it is prepared in unit dosage injectable form with a pharmaceutically acceptable parenteral vehicle, for parenteral administration, i.e., bolus, intravenous, and intratumoral injection. It is optionally mixed with pharmaceutically acceptable diluents, carriers, excipients, or stabilizers (Remington's Pharmaceutical Sciences (1980) 16th edition, Osol, A. Ed.) in the form of a lyophilized preparation or an aqueous solution.

**[Ligand that targets DDX5 protein]**

In addition to FL118, the active camptothecin derivative represented by Chemical Formula 1 according to the present invention is a molecular glue that activates the degradation of oncoprotein DDX5, and therefore, can be used as a ligand that targets DDX5 protein or a ligand that binds to DDX5 protein. Accordingly, there may be provided a complex containing a DDX5 protein-targeting ligand, in which the ability of the FL118 compound or the active camptothecin derivative (a) to inhibit type 1 topoisomerase is inactivated through a linker connection.

The linker may be a non-cleavable linker, and a non-limiting example of the non-cleavable linker is a thioether linker.

The FL118 compound or active camptothecin derivative (a) represented by Chemical Formula 1 binds to the DDX5 protein, and the DDX5 protein is a direct target of the active camptothecin derivative (a).

Therefore, the DDX5 can be used as a biomarker for predicting drug sensitivity or tumor sensitivity.

The patient group to which the active camptothecin derivative (a) or the prodrug thereof (b) is to be administered can be determined, or the recurrence rate and/or prognosis during chemotherapy can be predicted by quantitatively or qualitatively analyzing intracellular DDX5 protein using the complex containing a DDX5 protein-targeting ligand, in which the ability of the FL118 compound of Chemical Formula 2 or the active camptothecin derivative (a) to inhibit type 1 topoisomerase represented by Chemical Formula 1 is inactivated through a linker connection, in a tissue biopsy or liquid biopsy.

**[Cancer diagnostic composition]**

The present invention provides a cancer diagnostic composition including a complex containing a DDX5 protein-targeting ligand (c), in which the ability of the FL118 compound of Chemical Formula 2 or the active camptothecin derivative (a) to inhibit type 1 topoisomerase represented by Chemical Formula 1 is inactivated through a linker connection.

According to the present invention, the cancer diagnostic composition can be used to determine a patient group to which the active camptothecin derivative (a) or the prodrug thereof (b) is to be administered, or to predict drug response, recurrence rate and/or prognosis during chemotherapy.

**[Advantageous Effects]**

According to the present invention, the camptothecin derivative represented by Chemical Formula 1, for example, the camptothecin derivative represented by Chemical Formulas 3 to 9 or an isomer thereof, is a compound having a new structure. Through this, a drug candidate having more desirable properties in terms of efficacy, toxicity, selectivity, duration of action, administration, handling, stability, production possibility, and/or the like of a Dxd drug may be provided. In addition, it is possible to optimize the interaction between the target and the drug candidate, that is, the pharmacodynamic properties, and to improve the ability to optimize the drug's access to the target, that is, the pharmacodynamic ability.

The present invention provides a compound of Chemical Formula 1, for example, a compound of Chemical Formula 3 to 9 or an isomer thereof, which maintains the structure of a FL118 drug that maintains the advantage of the FL118 drug that can maximize the efficacy of the main drug target modulation through an additional mechanism of action of (i) simultaneously inhibiting the Bcl family, such as Survivin, which is a resistance protein, and/or (ii) inhibiting the action of an efflux pump.

Therefore, like the FL118 drug, the compound of Chemical Formula 1, for example, the compound of Chemical Formulas 3 to 9 or its isomers, is designed based on a well-designed polypharmacology-based approach that achieves excellent therapeutic efficacy by simultaneously targeting multiple drug targets that require control together, rather than the conventional drug development method having limited therapeutic efficacy by targeting one drug target with one drug, and can treat intractable diseases for which existing treatments have not shown a clear therapeutic effect. In addition, in the case of recurrent/resistant cancer, sufficient efficacy cannot be obtained by controlling the activity of only one drug target, and there is a problem that safety cannot be guaranteed when multiple unspecified drug targets are targeted at the same time. However, like the FL118 drug, by utilizing two or more well-selected drug targets, synergy in terms of efficacy can be achieved while ensuring sufficient safety at the same time.

**[Description of Drawings]**

FIG. 1 shows the synthetic design concept of a novel active camptothecin derivative (a) having a dual mechanism of action (dual MoA) that degrades oncoprotein DDX5 along with the ability to inhibit type 1 topoisomerase through structural improvement of FL118.

FIG. 2 shows the structural formula of camptothecin (CPT) and its binding to type 1 topoisomerase (topoisomerase-1), and shows the extension of the molecular design concept of FL118 and PBX-7011 from the camptothecin, the derivation of new-structure compounds PBX-7014 and PBX-7016 extended from PBX-7011, and the results of hydrophilicity calculations thereof.

FIG. 3 illustrates the synthesis method of 25-4 and 25-6 from PBX-7014 and PBX-7016, similar to the process of synthesizing Deruxtecan from Dxd.

FIG. 4 shows molecular dynamics simulations and docking scores that analyze the interaction between existing camptothecin drugs and TOP1.

FIG. 5 shows molecular dynamics simulations and docking scores that analyze the interaction between novel camptothecin drugs of the present invention and TOP1.

FIG. 6 is a molecular dynamics simulation that analyzes the interaction between camptothecin and mutant TOP1 (E418K).

FIG. 7 shows the SN38-TOP1 interaction in normal TOP1.

FIG. 8 shows the SN38-TOP1 interaction in E418K mutated TOP1.

FIG. 9 shows the compound of Chemical Formula 1-1 (Compound A)-TOP1 interaction in E418K mutated TOP1.

FIGS. 10 to 14 are Western blot results showing the degree of inhibition of anti-apoptotic proteins by various camptothecin drugs (FL118 drug, SN-38 drug, Exatecan drug, PBX-7011, PBX-7014, and PBX-7016) in FaDu cell line and A549 cell line to verify dual MoA (Topoisomerase I inhibition & DDX5 degradation) according to Example 2-1.

FIGS. 12 and 14 are the results of numerically plotting the concentrations in the western blot experiments (FIGS. 11 and 13) performed in FaDu cell lines and A549 cell lines.

FIG. 15 is the results of comparative evaluation of in vitro cell viability for various camptothecin drugs in FaDu cell lines

or A549 cell lines.

FIG. 16 is the results of comparative evaluation of in vitro cell viability for various camptothecin drugs in MDA-MB-453 (HER2++) cell lines and FaDu (HER2+) cell lines.

FIG. 17 is the results of carboxylate to lactone form conversion in pH 7.4.

FIG. 18 is the results of carboxylate to lactone form conversion in pH 6.0.

FIGS. 19 and 20 are the results of confirming the production of ADC using SEC and SDS PAGE.

FIG. 21 is the result of comparing the binding forces to Her2 of ADC in which trastuzumab, trastuzumab & PBX-7014, and trastuzumab & PBX-7016 were conjugated.

FIG. 22 is the result of comparing the in vitro cell viability of various camptothecin drugs and ADCs with them as payloads in MDA-MB-453 (HER2++) cell lines, FaDu (HER2+) cell lines, and MDA-MB-468 (HER2-) cell lines.

FIG. 23 is the result of an efficacy test for ADCs with various camptothecin drugs as payloads in an in vivo xenograft model in mice using JIMT-1 cell lines.

FIG. 24 is the result of mouse weight changes for ADCs with various camptothecin drugs as payloads in an in vivo xenograft model in mice using JIMT-1 cell lines.

FIG. 25 shows the results of counting the number of cells by FACS based on the presence or absence of GFP expression for ADCs with camtothecin drugs as payload after co-culturing MDA-MB-453 (HER2++) cell lines and MDA-MB-468 (HER2-) cell lines with GFP expressed.

FIG. 26 shows the results of counting the number of cells by FACS based on the presence or absence of HER2 expression using HER2-FITC antibody for ADCs with camtothecin drugs as payload after co-culturing MDA-MB-453 (HER2++) cell lines and MDA-MB-468 (HER2-) cell lines.

FIG. 27 shows the results of comparing and evaluating in vitro cell viability (ADC 6 days Treatment) for Cetuximab-25-6 in MDA-MB-468 (EGFR positive) cell lines and SW480 (EGFR negative) cell lines (FIG. 27A); and the results of comparing and evaluating in vitro cell viability (ADC 6 days Treatment) for Sacituzumab-25-6 in FaDu (Trop2+++) cell lines and Calu-6 (Trop2--) cell lines (FIG. 27B).

FIG. 28 compares LogP, cLogP and tPSA (topological polar surface area) of FL118, Exatecan, SN-38, Dxd, Compound A (PBX-7011), Compound B (PBX-7012), Compound C (PBX-7014), Compound D (PBX-7015), Compound E (PBX-7016), and Compound F (PBX-7017).

FIG. 29 compares LogP, cLogP and tPSA (topological polar surface area) of various camptothecin drugs derivable from Chemical Formula 1, Compound G, Compound H, Compound I, Compound J, Compound G', Compound H', Compound I', and Compound J'.

FIG. 30 is a detailed classification diagram of cell death related to programmed cell death.

FIG. 31 illustrates the mechanism of ADC toxicity (Source: Cancers 2023, 15(3), 713; https://doi.org/10.3390/cancers15030713).

FIG. 32 illustrates the mechanism of action of various self-immolative spacers.

**[Best Modes of the Invention]**

Hereinafter, the present invention will be described more specifically by way of examples. However, the following examples are only intended to clearly illustrate the technical features of the present invention and do not limit the protection scope of the present invention.

**Preparation Example 1: Synthesis of active camptothecin derivatives of Chemical Formula 3 or Chemical Formula 3-1 (PBX-7011 and PBX-7012)**

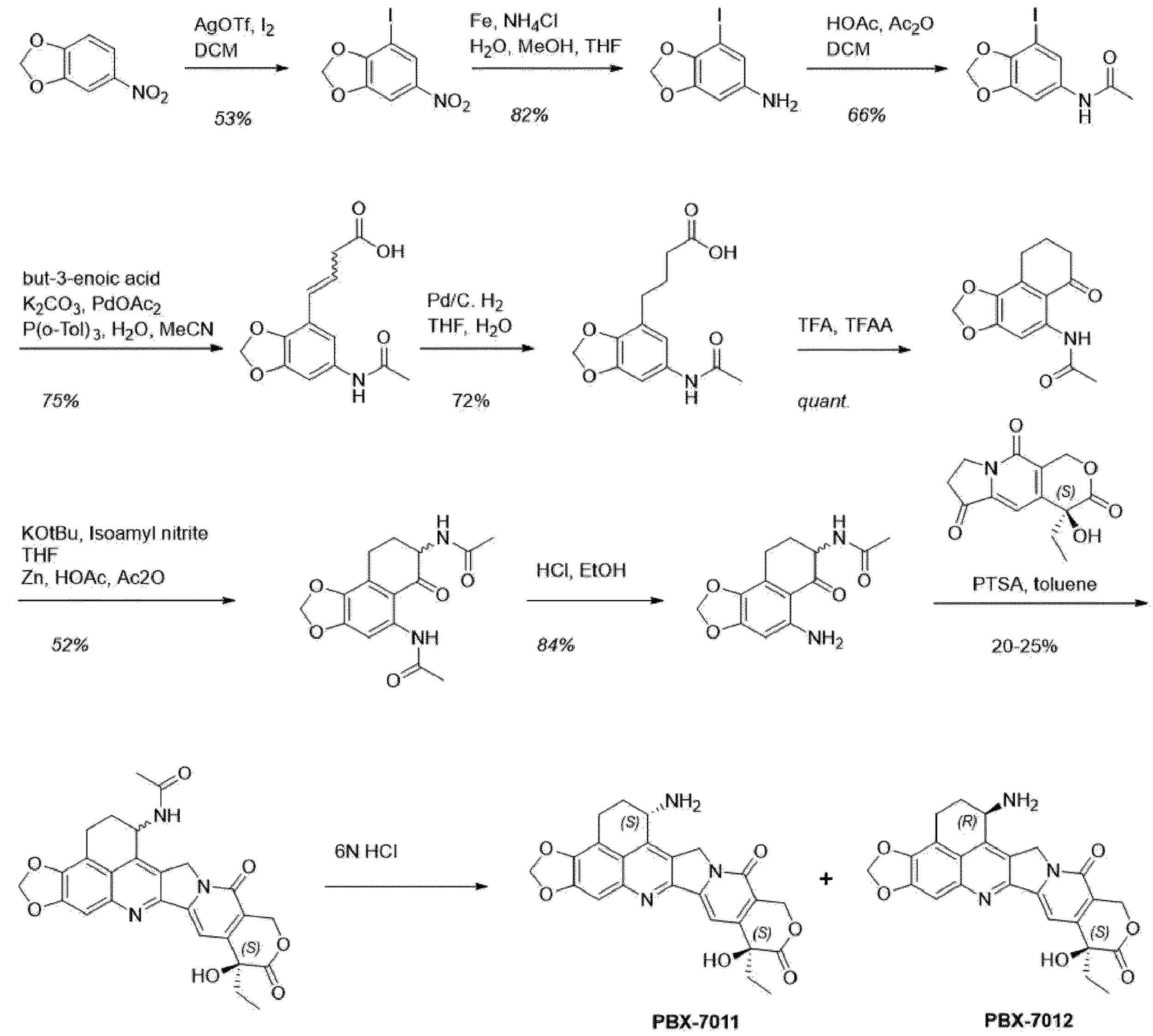


PBX-7011

PBX-7012

**1-1. Synthesis of compound 2**

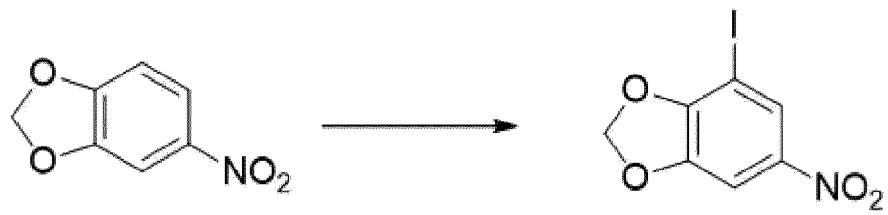

1

2

To a solution of 5-nitrobenzo[d][1,3]dioxole (25 g, 150 mmol) in a dichloromethane (748 mL) in a flask was added silver triflate (57.7 g, 224 mmol) and iodine (57.0 g, 224 mmol). The solution was stirred in the dark at room temperature under an $N_2$ atmosphere.

AgI was removed by filtration and the solid was washed with dichloromethane (100 mL). The solvent was removed under reduced pressure and the residue was partitioned between EtOAc (250 mL) and 5% (v/v) $NH_4OH/H_2O$ solution (200 mL). The organic layer was separated and washed with 1 M $Na_2SO_3$ (5 × 200 mL) and brine (200 mL), dried

over $Na_2SO_4$, treated with activated carbon, and filtered through Celite. The solvent was evaporated under reduced pressure to give the crude as a brown solid. This was triturated in ice-cold EtOH (400 mL) and filtered, and the solid was washed with ice-cold EtOH (100 mL) to give the product as a pale brown-gray solid (14.3 g). The solvent was removed from the filtrate, and the crude was triturated a second time in ice-cold EtOH (300 mL). The solid was collected by filtration and washed with EtOH (50 mL) to give additional amount of product (10.2 g) as a dark brown-gray solid. Both batches were used as they were without further purification.
SC_ACID: m/z 294.2 $[M+H]^+$

**1-2. Synthesis of compound 3**

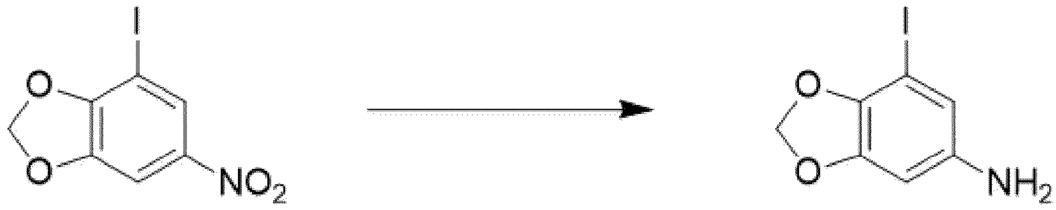

2 3

To a suspension of 4-iodo-6-nitrobenzo[d][1,3]dioxole (8.75 g, 29.9 mmol) in a mixture of water (80 mL), methanol (40.0 mL), and tetrahydrofuran (40.0 mL) were added iron powder (6.67 g, 119 mmol) and ammonium chloride (6.39 g, 119 mmol). The suspension was heated to 75°C and stirred for 16.5 hours. The reaction mixture was cooled to room temperature and concentrated under reduced pressure. The resulting black solid was suspended in ethyl acetate (100 mL), and the solution was decanted. The residue was washed with EtOAc (3 × 50 mL). The mixture was transferred to a separatory funnel, water was added, and the aqueous layer was removed. The organic layer was washed with saturated aqueous $NaHCO_3$ solution (150 mL) and brine (150 mL). The organic layer was dried over $Na_2SO_4$, and the solvent was removed under reduced pressure to give a brown solid (4.75 g, 60% yield). The iron residue was thoroughly washed with ethyl acetate to recover additional product. The organic fraction was then washed with saturated aqueous $NaHCO_3$ solution (150 mL) and brine (150 mL), and dried over $Na_2SO_4$. The solvent was removed under reduced pressure to give a brown solid (1.74 g, 22% yield).
SC_ACID: m/z 264.0 $[M+H]^+$

**1-3. Synthesis of compound 4**

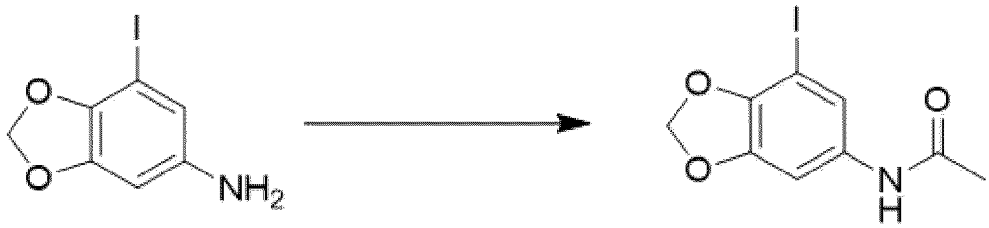

3 4

To a solution of 7-iodobenzo[d][1,3]dioxol-5-amine (6.39 g, 24.29 mmol) in dichloromethane (49 mL) was added acetic anhydride (2.75 mL, 29.2 mmol) and triethylamine (4.06 mL, 29.2 mmol). The reaction mixture was stirred at room temperature overnight. After several hours, additional DCM (10 mL) was added. The suspension was filtered through a sintered funnel and washed with ice-cold DCM (10 mL). The product was further dried under reduced pressure to give a light gray solid (4.46 g). The filtrate was concentrated under reduced pressure, and the residue was dissolved in EtOAc, washed with water and brine, dried over $Na_2SO_4$, and concentrated under reduced pressure to give a brown solid (~3 g). The brown solid was purified by flash column chromatography (80 g Si, 0-100% ethyl acetate in heptane). All batches of the

product were triturated in ice-cold EtOAc (5-10 ml). The two batches were combined and further dried to give an off-white solid.

Total yield: 5.0 g, 66%
SC_ACID: m/z 306.0 $[M+H]^+$
$^1H$ NMR (400 MHz, DMSO-d6) δ 9.88 (s, 1H), 7.39 (d, *J* = 1.9 Hz, 1H), 7.17 (d, *J* = 1.9 Hz, 1H), 6.04 (s, 2H), 1.99 (s, 3H).

**1-4. Synthesis of compound 5**

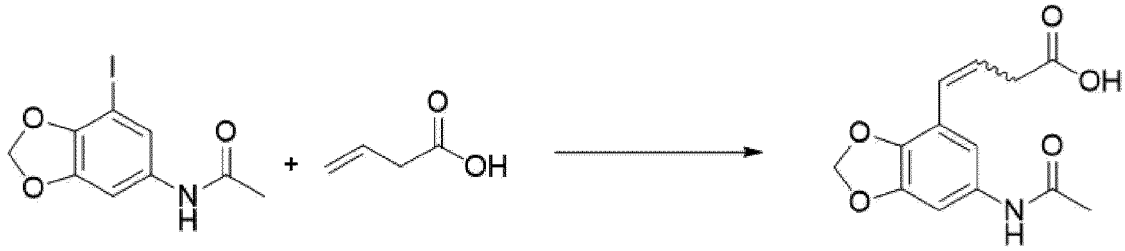

4 5

A slurry of N-(7-iodobenzo[d][1,3]dioxol-5-yl)acetamide (5.06 g, 16.59 mmol), but-3-enoic acid (1.69 mL, 19.90 mmol), and potassium carbonate (2.98 g, 21.56 mmol) in acetonitrile (40 mL) in a 3-neck flask equipped with a condenser was cooled to 0 to 5°C. Water (13.33 mL) was slowly added to evolve gas. When the gas evolution ceased, the mixture was degassed with Ar for 30 min. Tri-o-tolylphosphine (0.505 g, 1.659 mmol) and palladium acetate (0.186 g, 0.829 mmol) were added, and the mixture was again degassed for 30 min and then heated to reflux under Ar. The reaction was cooled to room temperature and filtered through celite. The filter cake was washed with $H_2O$ and EtOAc. The organic solvent was removed from the filtrate under vacuum, and the aqueous material was acidified with concentrated HCl solution until pH = 1-2. The aqueous layer was extracted with EtOAc, and the combined organic phases were washed with brine, dried over $Na_2SO_4$, filtered, and concentrated under reduced pressure to give the crude product. The crude material was triturated in ice-cold EtOAc (30 mL), and the solid was collected by filtration to give the product as a brown solid. The mother liquor was concentrated under reduced pressure and purified by flash chromatography (80 g Si, 0 - 100% EtOAc in heptane). The product fractions were concentrated under reduced pressure to give a light brown foam. Total yield: 3.46 g, 75%. A mixture of E/Z isomers was obtained.
SC_ACID: m/z 264.4 $[M+H]^+$

**1-5. Synthesis of compound 6**

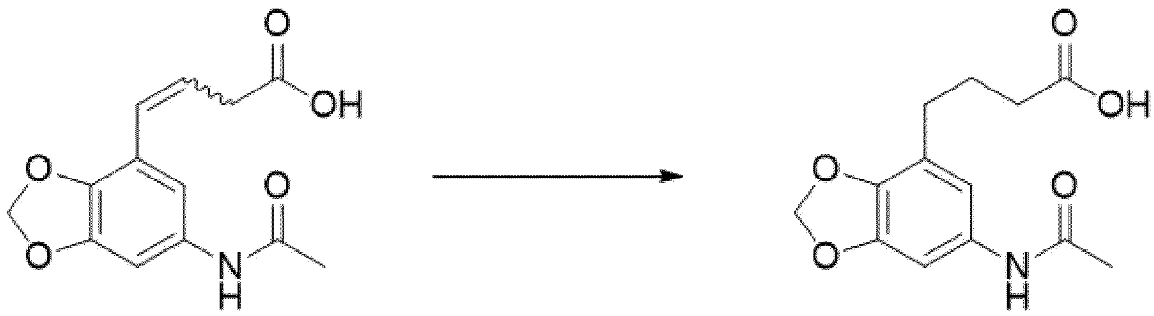

5 6

A suspension of 4-(6-acetamidobenzo[d][1,3]dioxol-4-yl)but-3-enoic acid (3.47 g, 13.18 mmol) in tetrahydrofuran (50 mL)/water (50 mL) was degassed with $N_2$ for 15 min. Pd/C (2.97 g, 1.397 mmol) was added and the suspension was

degassed with $H_2$ for 5 min before heating to 40°C under $H_2$ (balloon). After 24 hours, nitrogen and additional Pd/C (2.97 g, 1.397 mmol) were added to the reaction mixture. The reaction mixture was bubbled with hydrogen for 10 min and stirred overnight at 45°C under hydrogen. The reaction was filtered through Celite. The filter cake was washed with $H_2O$ (50 mL) and EtOAc (50 mL). The organic solvent was removed from the filtrate under vacuum. The aqueous solution was acidified with concentrated HCl until pH = 1, and a dark brown/green precipitate was collected by filtration through a sintered funnel. The aqueous filtrate was extracted. The combined organic phases were washed with brine, dried over $Na_2SO_4$, filtered, and the solvent was removed under vacuum to give a brown oil. Since the product recovery was low, the filter cake was washed with EtOAc (50 ml), water (200 ml) and MeOH (400 ml). The water/EtOAc flush was added to the flask containing the solid from the MeOH flush. The aqueous layer was acidified with concentrated HCl until pH = 1, and an off-white precipitate was formed, which was collected by filtration through a sintered funnel. The aqueous filtrate was extracted with EtOAc (3x200 mL), and LCMS showed that all product was removed from the aqueous. The combined organic phases were washed with brine, dried over $Na_2SO_4$, filtered, and the solvent was removed under vacuum to give a light brown solid. All product batches were combined and purified by flash column chromatography (40 g Si, 0-10% MeOH in DCM). The product fractions were concentrated to give a light brown solid. Yield: 2.52 g, 72%.

SC_ACID: 266.2 $[M+H]^+$
$^1$H NMR (400 MHz, DMSO-d6) δ 12.07 (s, 1H), 9.78 (s, 1H), 7.14 (d, *J* = 2.0 Hz, 1H), 6.80 (d, *J* = 2.2 Hz, 1H), 5.95 (s, 2H), 2.50 - 2.46 (m, 2H), 2.23 (t, *J* = 7.4 Hz, 2H), 1.98 (s, 3H), 1.84 - 1.70 (m, 2H).

**1-6. Synthesis of compound 7**

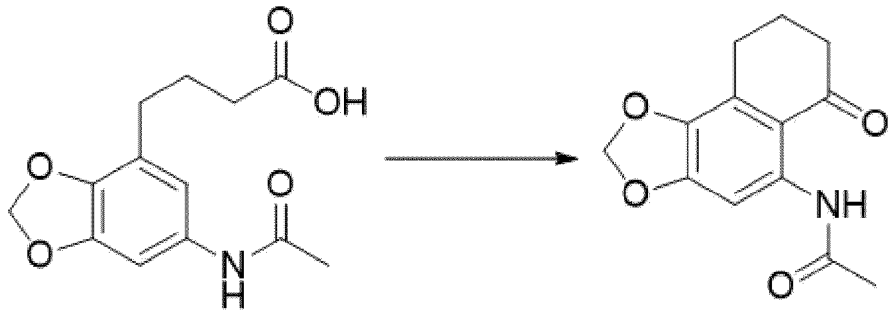

6 7

A suspension of 4-(6-acetamidobenzo[d][1,3]dioxol-4-yl)butanoic acid (150 mg, 0.565 mmol) in TFA (433 μL, 5.65 mmol) was cooled on ice. TFAA (157 μL, 1.131 mmol) was added. The mixture was stirred at 0-5°C for 1 hour, and turned into a dark solution over time. The reaction mixture was added dropwise to ice-cold saturated aqueous $NaHCO_3$ solution (10 mL), and the aqueous solution was extracted with ethyl acetate (3 × 25 mL). The combined organic layers were washed with sat. $NaHCO_3$ and brine, dried over $Na_2SO_4$, filtered, and the solvent was removed in vacuum to give a salmon pink solid. Yield: 140 mg, 100%

SC_ACID: m/z 248.2 $[M+H]^+$
$^1$H NMR (400 MHz, DMSO-d6) δ 12.34 (s, 1H), 8.11 (s, 1H), 6.13 (s, 2H), 2.80 (t, *J* = 6.2 Hz, 2H), 2.66 - 2.58 (m, 2H), 2.12 (s, 3H), 2.01 - 1.91 (m, 2H).

**1-7. Synthesis of compound 8**

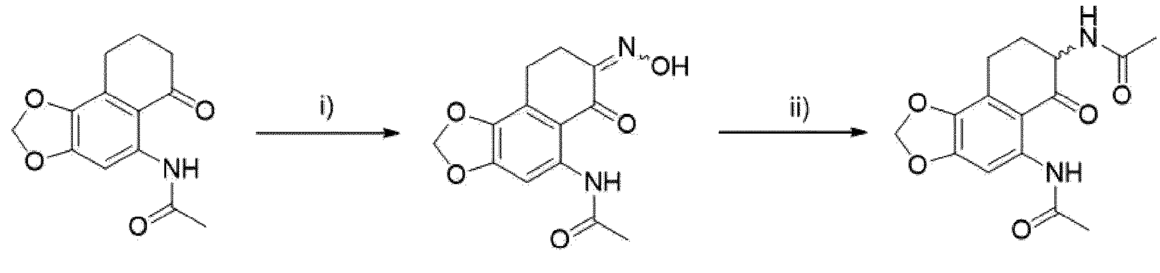


7 8

A suspension of N-(6-oxo-6,7,8,9-tetrahydronaphtho[1,2-d][1,3]dioxol-5-yl)acetamide (50 mg, 0.202 mmol) in tetrahydrofuran (1.2 mL) was cooled to 0°C, and potassium tert-butoxide (27.2 mg, 0.243 mmol) and isoamyl nitrite (35.0 μL, 0.263 mmol) were added. The dark green mixture was stirred on ice (< 5°C) for 1.5 hours. Acetic acid (170 μL, 2.94 mmol), acetic anhydride (170 μL, 1.810 mmol), and zinc dust (66.1 mg, 1.011 mmol) were added to the reaction mixture. The suspension was stirred at 0°C for 2 hours. The reaction mixture was filtered over celite and flushed with DCM. The filtrate was concentrated under reduced pressure to give a black oily substance. The crude product was purified by flash column chromatography (4 g Si, 0-4% MeOH in DCM). The product fractions were concentrated to give a gray solid (32 mg, 52%) with 80-90% purity. A higher purity sample can be obtained when purified by preparative MPLC.

SC_ACID: m/z 305.4 $[M+H]^+$
1H NMR (400 MHz, DMSO-d6) δ 12.10 (s, 1H), 8.21 (d, J = 8.0 Hz, 1H), 8.12 (s, 1H), 6.15 (d, J = 9.3 Hz, 2H), 4.66 - 4.56 (m, 1H), 2.93 (dd, J = 8.9, 4.0 Hz, 2H), 2.14 (s, 3H), 2.13 - 1.93 (m, 2H), 1.91 (s, 3H).

**1-8. Synthesis of compound 9**

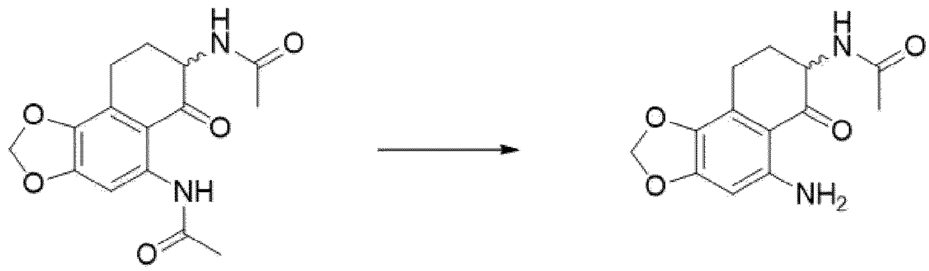

8 9

N,N'-(6-Oxo-6,7,8,9-tetrahydronaphtho[1,2-d][1,3]dioxole-5,7-diyl)diacetamide (244 mg, 0.802 mmol) was suspended in 2 M hydrochloric acid (4.69 mL, 9.38 mmol) in ethanol/water (5/1). The mixture was heated to 55°C for 4 hours. The black mixture was cooled to 0-5°C. Triethylamine (1.4 mL, 10.04 mmol) was added dropwise while stirring. The mixture was then diluted with EtOH and evaporated to dryness. The residue was partitioned between water and DCM. The layers were separated, and the aqueous layer was extracted once with DCM. The combined organic layers were dried over $Na_2SO_4$ and concentrated to give the product as a brown solid (178 mg, 73%) with 86% purity.

SC_ACID: m/z 263.0 $[M+H]^+$
1H NMR (400 MHz, DMSO) δ 8.04 (d, J = 8.0 Hz, 1H), 6.20 (s, 1H), 5.94 (d, J = 6.0 Hz, 2H), 4.48 - 4.41 (m, 1H), 3.08 (s, 2H), 2.88 - 2.69 (m, 2H), 2.15 - 2.03 (m, 1H), 1.88 (s, 3H), 1.86 - 1.75 (m, 1H).

**1-9. Synthesis of compound 10**

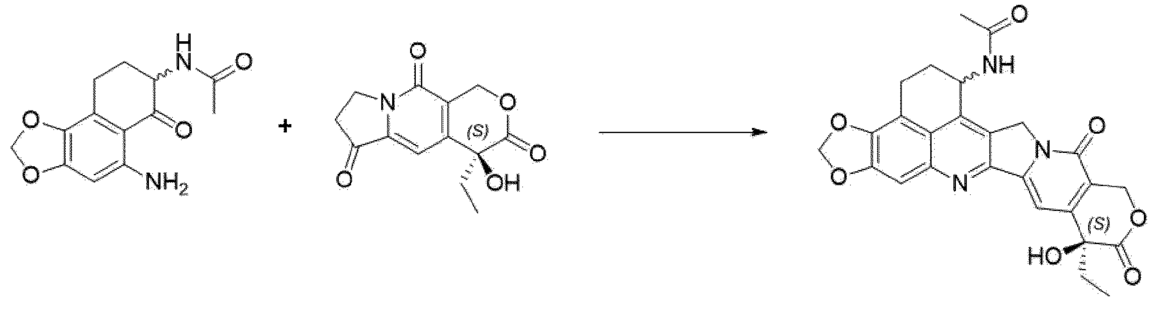

9 10

(4S)-4-Ethyl-7,8-dihydro-4-hydroxy-1H-pyrano[3,4-f]indolizine-3,6,10(4H)-trione (146 mg, 0.555 mmol) and N-(5-amino-6-oxo-6,7,8,9-tetrahydronaphtho[1,2-d][1,3]dioxol-7-yl)acetamide (112 mg, 0.427 mmol) were added to dry toluene (4.5 mL). PPTS (21 mg, 0.085 mmol) was added, and the reaction mixture was stirred at 115°C for 40 hours.

The reaction mixture was cooled to room temperature. The suspension was diluted with 2 mL DCM and filtered. A black residue (210 mg) was obtained.

The crude product was purified by column chromatography (12 g Si, 0-7% methanol in DCM) to give the product (45 mg, 21%) as a brown solid.

LCMS analysis showed two diastereoisomers.

SC_ACID: m/z 490.2 $[M+H]^+$

$^1$H NMR (400 MHz, DMSO-d6) δ 8.47 (t, *J* = 9.3 Hz, 1H), 7.42 (s, 1H), 7.24 (s, 1H), 6.49 (s, 1H), 6.29 (d, *J* = 5.2 Hz, 2H), 5.57 - 5.49 (m, 1H), 5.41 (s, 2H), 5.23 - 5.07 (m, 2H), 3.09 - 3.00 (m, 2H), 2.11 - 2.01 (m, 2H), 1.91 (s, 3H), 1.89 - 1.79 (m, 2H), 0.87 (t, *J* = 7.1 Hz, 3H).

**1-10. Synthesis of PBX-7011 and PBX-7012**

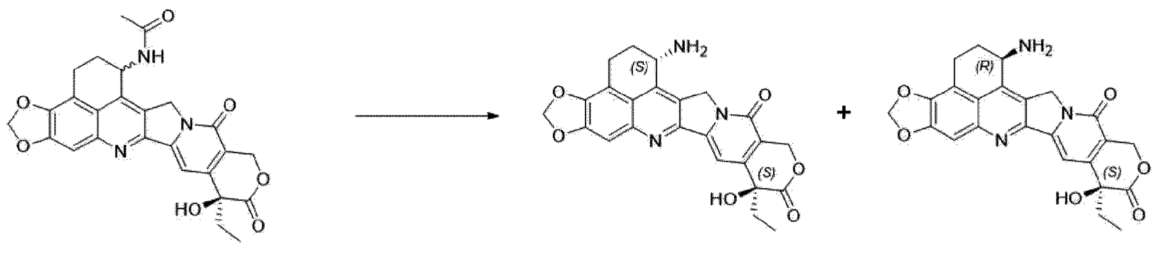

10 PBX-7011 PBX-7012

N-((10S)-10-ethyl-10-hydroxy-11,14-dioxo-2,3,10,11,14,16-hexahydro-1H,13H-benzo[de][1,3]dioxolo[4,5-g]pyrano[3',4':6,7]indolizino[1,2-b]quinolin-1-yl)acetamide (73.5 mg, 0.150 mmol) was dissolved in 1.0 mL 6N HCl and stirred at 90°C for 8 h and then at room temperature overnight. The reaction mixture was concentrated under reduced pressure and purified by running twice acidic preparative MPLC (Luna2-30). The fractions containing the separated diastereomers were acidified with 5 drops of 3N HCl and lyophilized to give the product as a yellow solid.

1st eluting isomer: PBX-7011, 23 mg, 34% yield

U_AN_ACID: m/z 448.4 $[M+H]^+$

$^1$H NMR (400 MHz, DMSO-d6) δ 8.54 (s, 3H), 7.50 (s, 1H), 7.27 (s, 1H), 6.52 (s, 1H), 6.34 (d, *J* = 13.3 Hz, 2H), 5.77 (d, *J* = 19.3 Hz, 1H), 5.44 (s, 2H), 5.37 (d, *J* = 19.3 Hz, 1H), 5.05 (s, 1H), 3.19 - 3.02 (m, 2H), 2.46 (s, 1H), 2.20 - 2.03 (m, 1H), 1.94 - 1.81 (m, 2H), 0.88 (t, *J*= 7.3 Hz, 3H).

2nd eluting isomer: PBX-7012, 28 mg, 41% yield

U_AN_ACID: m/z 448.2 [M+H]+

$^1$H NMR (400 MHz, DMSO-d6) δ 8.57 (d, *J* = 4.7 Hz, 3H), 7.51 (s, 1H), 7.27 (s, 1H), 6.52 (s, 1H), 6.34 (d, *J* = 12.2 Hz, 2H), 5.76 (d, *J* = 19.4 Hz, 1H), 5.44 (s, 2H), 5.37 (d, *J* = 19.4 Hz, 1H), 5.08 (s, 1H), 3.16 - 2.98 (m, 2H), 2.46 (s, 1H), 2.18 - 2.06 (m, 1H), 1.94 - 1.80 (m, 2H), 0.87 (t, *J* = 7.3 Hz, 3H).

**Preparation Example 2: Synthesis of active camptothecin derivatives of Chemical Formula 4 or Chemical Formula 4-1 (PBX-7014 and PBX-7015)**

This example describes the synthesis of compounds PBX-7014 and PBX-7015 starting from two separated diastereomers of the Exatecan-hybrid compound (PBX-7011 and PBX-7012).

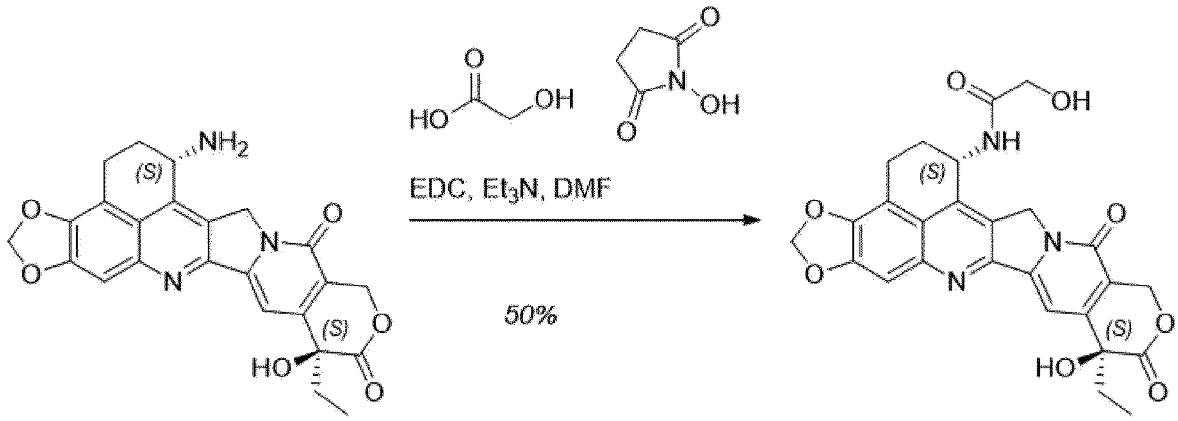


**PBX-7011** **PBX-7014**

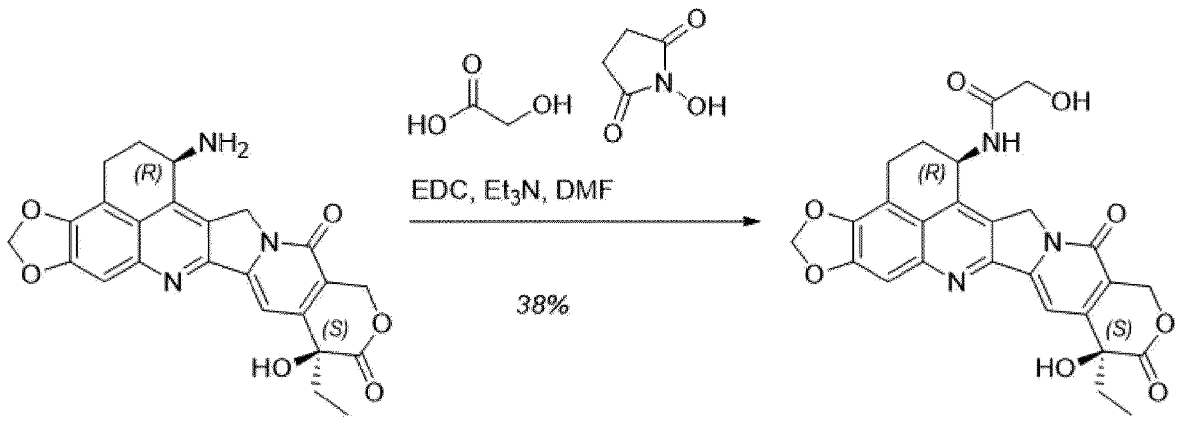


**PBX-7012** **PBX-7015**

2-1: Synthesis of PBX-7014

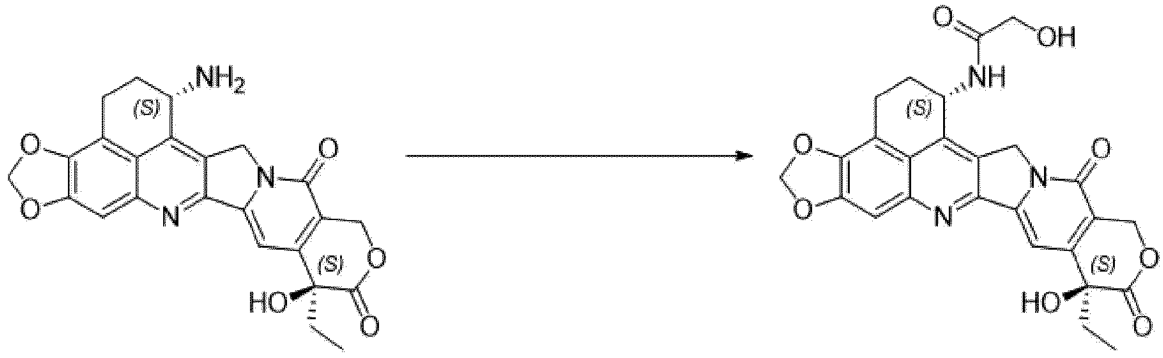

PBX-7011 PBX-7014

A stock solution of activated glycolic acid was prepared according to the following procedure: Glycolic acid (17 mg, 0.224 mmol) was dissolved in 1 mL of N,N-dimethylformamide. HOSu (25.7 mg, 0.223 mmol) and EDC (42.8 mg, 0.223 mmol) were added. The reaction mixture was stirred at room temperature for 1 hour.

Next, 0.4 mL of the activated acid solution was added to a suspension of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (40 mg, 0.089 mmol ) and triethylamine (0.025 mL, 0.179 mmol) in N,N-dimethylformamide (2.5 mL). The mixture was stirred at room temperature for 3 hours. 0.05 mL of freshly prepared activated acid solution was added. The reaction mixture was then stirred at room temperature for an additional 2 hours. The reaction mixture was evaporated to dryness. The crude product was purified by column chromatography (0-8% methanol in chloroform). This gave a yellow solid containing the PBX-7014 product and residual succinimide. The product was further purified by acidic preparative MPLC (Luna5-40), and the product fractions were lyophilized to give a light yellow solid. Yield: 20mg, 50%

U_AN_ACID: m/z 506.2 $[M+H]^+$

$^1$H NMR (400 MHz, DMSO-d6) δ 8.40 (d, *J* = 8.9 Hz, 1H), 7.40 (s, 1H), 7.23 (s, 1H), 6.49 (s, 1H), 6.28 (d, *J* = 4.6 Hz, 2H), 5.61 - 5.45 (m, 2H), 5.45 - 5.35 (m, 2H), 5.19 - 5.06 (m, 2H), 3.95 (s, 2H), 3.13 - 2.96 (m, 2H), 2.21 - 2.02 (m, 2H), 1.94 - 1.77 (m, 2H), 0.87 (t, *J*= 7.3 Hz, 3H).

2-2: Synthesis of PBX-7015

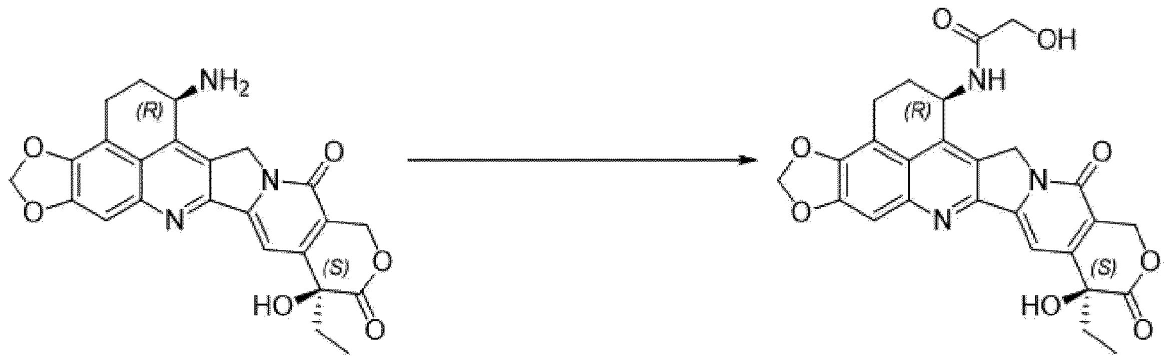

PBX-7012 PBX-7015

A stock solution of the activated acid was prepared according to the following procedure: Glycolic acid (17.00 mg, 0.223 mmol) was dissolved in 1 mL of N,N-dimethylformamide. HOSu (25.7 mg, 0.223 mmol) and EDC (42.8 mg, 0.223 mmol) were added. The reaction mixture was stirred at room temperature for 1 hour.

Next, 0.25 mL of the activated acid solution was added to a suspension of (1R,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (25 mg, 0.056 mmol) and triethylamine (0.016 mL, 0.112 mmol) in N,N-dimethylformamide (2.5 mL). The mixture was stirred at room temperature overnight. 0.03 mL of freshly prepared activated acid solution was added. The

reaction mixture was then stirred at room temperature for 2 hours. The reaction mixture was combined with the previous smaller batch and evaporated to dryness. The crude product was purified by column chromatography. This gave a yellow solid containing the PBX-7015 product and residual succinimide. The product was purified by acidic preparative MPLC (Luna5-40). The product fractions were lyophilized to give a light yellow solid. Yield: 15mg, 38%

U_AN_ACID: 506.2 $[M+H]^+$
$^1$H NMR (400 MHz, DMSO) δ 8.44 (d, *J* = 9.0 Hz, 1H), 7.41 (s, 1H), 7.24 (s, 1H), 6.48 (s, 1H), 6.29 (d, *J* = 2.3 Hz, 2H), 5.61 - 5.44 (m, 2H), 5.44 - 5.36 (m, 2H), 5.20 - 5.07 (m, 2H), 3.96 (s, 2H), 3.10 - 2.96 (m, 2H), 2.20 - 2.06 (m, 2H), 1.95 - 1.79 (m, *J* = 7.3 Hz, 2H), 0.87 (t, *J*= 7.3 Hz, 3H).

**Preparation Example 3 Synthesis of an active camptothecin derivative of Chemical Formula 5 (PBX-7016)**

This example describes the synthesis of compound PBX-7016 starting from an Exatecan-hybrid compound (PBX-7011).

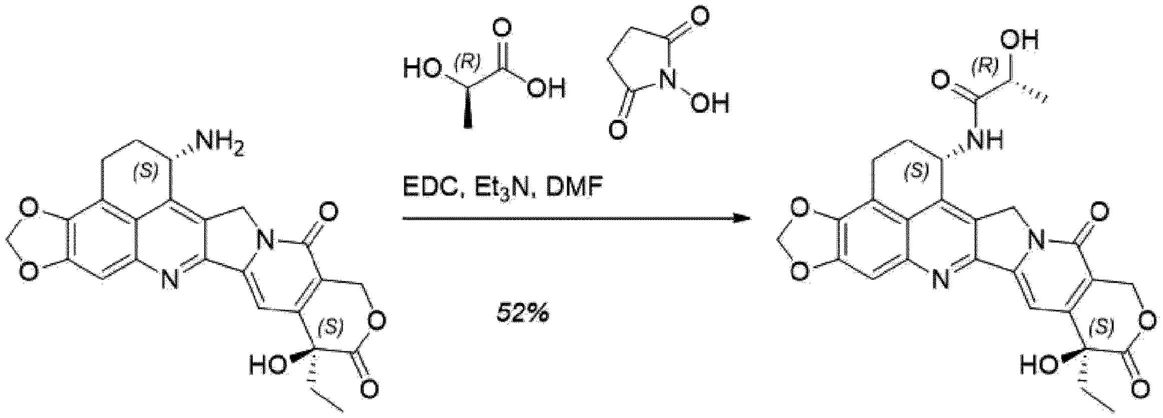


PBX-7011 PBX-7016

A stock solution of activated D-lactic acid was prepared according to the following procedure.
D-lactic acid (22 mg, 0.244 mmol) was dissolved in 1 mL of N,N-dimethylformamide. HOSu (27 mg, 0.235 mmol) and EDC (38.6 mg, 0.201 mmol) were added. The reaction mixture was stirred at room temperature for 2 hours.
Next, 0.3 mL of the activated acid solution was added to a solution of (1S, 10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (36 mg, 0.080 mmol) and triethylamine (0.022 ml, 0.161 mmol) in N,N-dimethylformamide (2.5 mL). The mixture was stirred at room temperature for 6 hours. 0.05 mL of the prepared activated acid solution was added. The reaction mixture was then stirred at room temperature overnight. The reaction mixture was purified directly by acid preparative MPLC (Luna10-50), and the product fraction was lyophilized to give a light yellow solid.

Yield: 22mg, 52%
U_AN_ACID: m/z 520.2 $[M+H]^+$
$^1$H NMR (400 MHz, DMSO) δ 8.43 (d, J = 9.1 Hz, 1H), 7.41 (s, 1H), 7.23 (s, 1H), 6.50 (s, 1H), 6.29 (d, J = 2.1 Hz, 2H), 5.62 - 5.58 (m, 1H), 5.58 - 5.51 (m, 1H), 5.41 (s, 2H), 5.21 - 5.01 (m, 2H), 4.17 - 4.07 (m, 1H), 3.14 - 2.95 (m, 2H), 2.19 - 2.04 (m, 2H), 1.92 - 1.78 (m, 2H), 1.39 (d, J = 6.8 Hz, 3H), 0.87 (t, J = 7.3 Hz, 3H).

As described above, PBX-7016 of Chemical Formula 5 can be synthesized from PBX-7011 of Chemical Formula 3, and thus PBX-7017 of Chemical Formula 5-1 can be synthesized from PBX-7012 of Chemical Formula 3-1 in the same manner.

**Preparation Example 4: Synthesis of an active camptothecin derivative of Chemical Formula 9 (PBX-7024)**

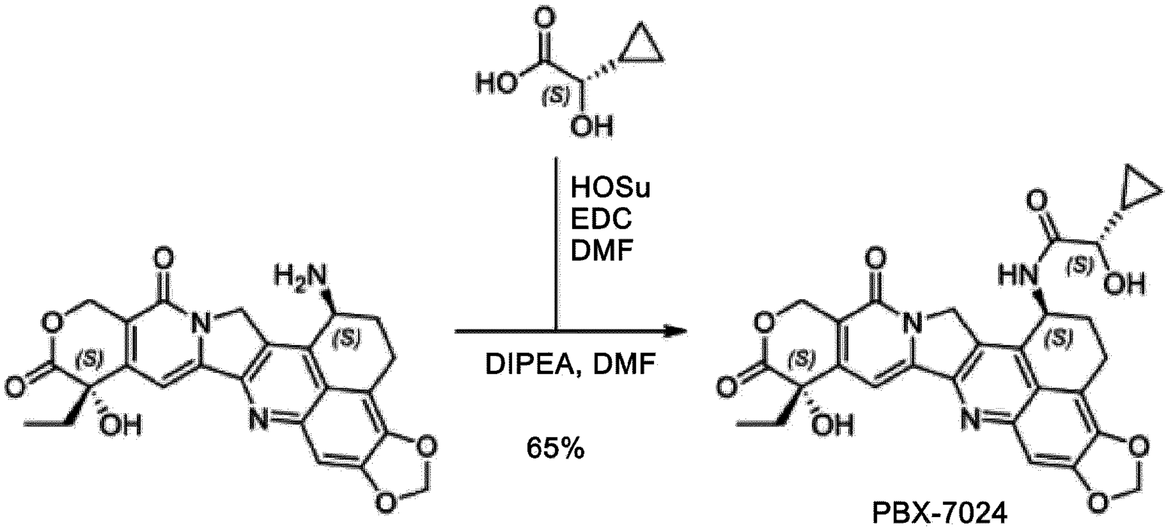


PBX-7011

PBX-7024

20 mg shipped

PBX-7024 was prepared in high yield by binding (2S)-2-cyclopropyl-2-hydroxyacetic acid to PBX-7011 compound.

(2S)-2-cyclopropyl-2-hydroxyacetic acid (26 mg, 0.244 mmol) was dissolved in 1 mL of N,N-dimethylformamide. HOSu (26 mg, 0.226 mmol) and EDC (42 mg, 0.219 mmol) were added. The reaction mixture was stirred at room temperature for 2 hours. Next, 0.6 mL of the activated acid solution was added to a solution of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11, 14-dione (40 mg, 0.089 mmol) and DIPEA (0.047 ml, 0.268 mmol) in N,N-dimethylformamide (2.5 mL). The mixture was stirred at room temperature overnight. The reaction mixture was purified directly by acid preparative MPLC (Luna10-50), and the product fraction was lyophilized to give a light white solid.

Yield: 32 mg, 65%

U_AN_ACID: m/z 520.2 $[M+H]^+$

$^1$H NMR (400 MHz, DMSO-d6) $\delta$ 8.33 (d, *J* = 8.7 Hz, 1H), 7.39 (s, 1H), 7.23 (s, 1H), 6.48 (s, 1H), 6.28 (d, *J* = 4.9 Hz, 2H), 5.50 (q, *J* = 6.7 Hz, 1H), 5.40 (s, 3H), 5.23 - 5.06 (m, 2H), 3.62 (d, *J* = 6.3 Hz, 1H), 3.03 (q, *J* = 6.2 Hz, 2H), 2.21 - 2.02 (m, 2H), 1.92 - 1.79 (m, 2H), 1.18 - 1.08 (m, 1H), 0.87 (t, *J* = 7.3 Hz, 3H), 0.47 - 0.30 (m, 4H).

Example 1: Analysis of binding mode of camptothecin derivatives with TOP1/DNA

[Table 2]

| Camptothecin derivatives | Docking Score (The smaller, the more stable) | |
|---|---|---|
| | Normal TOP1 | Mutation TOP1 |
| 1. Sn-38 | -10.310 | -10.314 |
| 2. Exatecan | -11.023 | -10.362 |
| 3. Dxd | -9.989 | -10.377 |
| 4. FL118 | -9.969 | -9.361 |
| PBX-7011 of Chemical Formula 3 | -11.328 | -9.094 |
| PBX-7012 of Chemical Formula 3-1 | -10.358 | -10.061 |

According to the results of binding mode prediction (Docking Score) through molecular dynamics simulation, all camptothecin derivatives in Table 2 basically bind through strong $\pi$-$\pi$ stacking between DNA bases (FIGS. 4 and 5), and the ester of the lactone is hydrogen-bonded with Arg488 of TOP1, and the ethyl and OH groups are hydrogen-bonded with Asp533 of TOP1 (FIG. 1), which supports the camptothecin derivatives to bind well between TOP1 Protein and DNA.

The SN-38 drug is stabilized by being hydrogen-bonded with Glu356 and Arg364 of TOP1 (FIG. 4).
Exatecan drug is further stabilized by being hydrogen-bonded (H-bonded) with surrounding DNA bases (FIG. 4).
The $-OCH_2O-$ (methylenedioxo) pentagonal ring of FL118 drug is weaker than hydrogen bonding (different from actual cytotoxicity) and shows a worse docking score than SN-38 drug or Exatecan drug, but this bond perfectly binds between DNA bases and also helps solvation (FIG. 4).
As shown in FIG. 5, the compound of Chemical Formula 3 (PBX-7011) and the compound of Chemical Formula 3-1 (PBX-7012), which were designed by mixing the functional groups of FL118 drug and Exatecan drug according to the present invention, are diastereomeric, and thus share the same binding pose and are expected to stably bind to TOP1. In particular, the compound of Chemical Formula 3 (PBX-7011) was calculated to have a higher docking score than SN-38 drug.
Compound of Chemical Formula 3 (PBX-7011), which has chirality similar to that of the Exatecan drug, showed slightly better binding than compound of Chemical Formula 3-1 (PBX-7012).
Meanwhile, regarding the TOP1 mutation (E418K) that showed resistance to Trodelvy (payload: SN-38), as shown in FIG. 6, the E418K mutation is far from the drug binding site.
As shown in FIGS. 7 and 8, as a result of energy minimization after E418K mutation, it was confirmed by MD simulation that the newly introduced Lys418 changes some of the drug binding sites to a relatively polar environment and is also continuously ion-binding with the surrounding Glu356. When some of the binding sites become polar due to mutation, it was observed in MD simulations that substances with polar functional groups, such as SN-38 drugs, move slightly away from the existing binding site to interact with Lys418 and Glu356. This can weaken the interaction between lactone and Asp533 and Lys532, which is an inhibitory activity principle common to the TOP1 inhibitors of the same series.
When SN-38 drugs were docked to the mutated TOP1 structure, although SN-38 drugs moved away from the original binding site and moved to the side, they showed good binding docking scores by obtaining new hydrogen bonds, etc., so the docking score is limited in explaining the phenomenon in which mutated TOP1 shows resistance to SN-38.
As shown in FIGS. 7 and 8, when analyzing the interaction between SN-38 drug and TOP1 during MD simulation, it was confirmed that the interaction of SN-38 with Asp533 and Arg488 (89%) was reduced to 10% or less by mutation, but the interaction with Asn352 and Glu356 was increased from 40% to 80%.
When docking is performed after TOP1 mutation, the SN-38 drug moves to the newly modified binding site and binds in a new form, showing a score similar to the existing one (Table 2).
On the other hand, as shown in Table 2, it was confirmed that Exatecan drug, FL118 drug, compound of Chemical Formula 3 (PBX-7011) and compound of Chemical Formula 3-1 (PBX-7012), which do not have a polar functional group (-OH) at the 10th carbon of the A-ring, had a worse relative docking score with the mutated TOP1 due to some environmental changes in the new binding site. Nevertheless, it was confirmed by MD simulation that Exatecan drug, FL118 drug, compound of Chemical Formula 3 (PBX-7011) and compound of Chemical Formula 3-1 (PBX-7012) maintained the interaction the existing lactone and the Asp533 and Lys532.
As shown in FIG. 9, even with TOP1 mutation, compound of Chemical Formula 3 (PBX-7011) maintains the existing binding mode (especially, lactone and -OH group).
In conclusion, although the E418K mutation does not interact directly with TOP1 inhibitors, in order to explain a situation in which the FL118 drug inhibits the TOP1 mutation showing resistance to SN-38 drugs by E418K at a high level, it may be assumed that unlike the docking result, the new binding of SN-38 to the binding site modified by the mutation proceeds in a form in which the action of lactone with Asp533 and Lys532 is weakened, and thus does not inhibit the action of TOP1.
Under this assumption, the Exatecan drug, the FL118 drug, the compound of Chemical Formula 3 (PBX-7011), and the compound of Chemical Formula 3-1 (PBX-7012) are considered to show less resistance by continuously acting with Asp533 and Lys532 of the mutated TOP1.

Example 2

For FL118, Exatecan, SN-38, Dxd, Exatecan-Lactic acid, PBX-7011, PBX-7014, PBX-7016, and PBX-7024, the expression inhibition analysis of anti-apoptotic proteins and the in vitro cell viability assay were performed as follows.

2-1: Confirmation of the expression inhibition activity of cancer-associated survival genes of DDXS, survivin, Mcl-1, XIAP, and cIAP2 through Western blot

(1) Protein extraction

200,000 FaDu cell lines per well were seeded in a 6-well plate and incubated at constant temperature (37°C, 5% $CO_2$). After 24 hours, the wells were treated with drugs (FL118 drug, SN-38 drug, exatecan drug, PBX-7011, PBX-7014, PBX-7016) at concentrations of 0 nM, 10 nM, and 100 nM, respectively. Incubation at constant temperature was performed

(37°C, 5% $CO_2$) for 24 hours. The well was treated with 100 ul of RIPA buffer in which protein inhibitor cocktails were dissolved. The plate was placed on ice and incubated at constant temperature for 2 hours in an orbital shaker. The RIPA buffer containing the lysed cells was transferred to an ep tube and centrifuged (16,000 rcf, 20 min, 4°C). Then, only the supernatant was transferred to a new ep tube. The protein concentration was confirmed through protein assay.

(2) Protein separation through electrophoresis

The protein sample and 4x SDS-PAGE Loading Buffer were mixed in a 3:1 ratio and boiled at 95°C for 10 minutes and then cooled. The samples were loaded into the wells of the gel so that the amount of protein was the same. Electrophoresis was performed on the gel at 60V.

(3) Transfer of proteins from gel to membrane

7 activated filter papers, PVDF membranes, gels, and 7 filter papers were placed in the cassettes of the Trans-Blot® Turbo™ Transfer System in order, closed with the lid, plugged into the machine, and the protocol was run.

(4) Antibody incubation

The transferred membrane was immersed in blocking buffer and incubated at constant temperature (RT, 1 h). Then, it was incubated at constant temperature (4°C, overnight) in the first antibody solution. It was rinsed with TBST buffer for 3 min (repeated 3 times). It was incubated at constant temperature (RT, 1 h) in the secondary antibody solution conjugated with HRP. It was rinsed with TBST buffer for 3 min (repeated 3 times).

(5) Imaging and analysis of results

After immersing the membrane in the ECL substrate for 3 to 5 minutes, the signal was confirmed using the ChemiDoc™ MP Imaging System. The light emitted by the luminol of ECL being oxidized by HRP bound to the secondary antibody was detected and displayed as an image. Since the thickness of the band is proportional to the amount of protein, the amount of protein can be compared through the thickness of the band.

As shown in FIGS. 10 to 12, it was confirmed how the protein expression level was changed by the treatment of the drugs (FL118 drug, SN-38 drug, exatecan drug, PBX-7011, PBX-7014, PBX-7016). GAPDH is an enzyme involved in glycolysis, an essential metabolic process in cells, and is a gene that is always expressed in cells and whose expression level is not well changed, so it is an indicator that can tell whether the samples have the same amount of protein loaded in the gel.

In the same way, instead of the FaDu cell line that does not express ABCG2, A549, which overexpresses ABCG2, was seeded, and 4 ug of protein was loaded, and Western blot was performed (FIG. 13 and FIG. 14).

**Discussion: Evaluation of PBX-7011, PBX-7014, and PBX-7016**

The degree of inhibition of the expression of anti-apoptotic proteins in FaDu cell lines was confirmed through Western blot, and as a result, PBX-7011, PBX-7014, and PBX-7016 all showed a decrease in the expression of Survivin, cIAP2, Mcl-1, and XIAP as the concentration increased, which was shown to be at a similar level to FL118 and Exatecan drugs, and better than SN-38 when compared to the existing FL118, SN-38, and Exatecan drugs.

In Example 2-1, it was confirmed that it had a heterogeneous characteristic of DDX5 degradation and the resulting anticancer effect by showing a dual inhibition effect that inhibits Topoisomerase 1 while degrading DDX5.

Specifically, as a result of treating two cell lines (FaDu, A549) with a certain concentration (0, 10, 100 nM) of the drug and checking the expression level of each protein (DDX, Survivin, Mcl-1, XIAP) by Western blot, FL118 was superior to other substances in DDX5 degradation, followed by 7011, 7016, and 7014 in that order.

In addition to DDX5, other anti-apoptotic proteins downstream of DDX5 showed similar trends.

In short, PBX-7014 and PBX-7016 not only inhibit type 1 topoisomerase, but also show dual MoA that acts as a degrader of DDX5 (p68), an oncoprotein that regulates Survivin, Mcl-1, XIAP, etc.

As a result of the evaluation, it was confirmed that among PBX-7014 and PBX-7016, PBX-7016 inhibited DDX5 in a concentration-dependent manner, and as a result, by observing that Survivin, Mcl-1, and XIAP were inhibited, the PBX-7016 was shown to act as a DDX5 degrader of FL118 as well as a topoisomerase I inhibitor, and further, the PBX-7016 was working more excellently than PBX-7014 (FIGS. 11 to 14).

### 2-2: Cell viability assay for FaDu cell line / A549 cell line

3,000 FaDu cell lines (cancer cells that do not express ABCG2) or A549 cell lines (cancer cells that overexpress ABCG2) per well were seeded in a 96-well plate and incubated at constant temperature (37°C, 5% $CO_2$). After 24 hours, the cells were treated with 100 μl of drugs at 9 concentrations (serial dilutions of 1/5 each from 1,000 nM). In this case, Dxd drugs, PBX-7014, PBX-7016, and PBX-7024 were treated. Also, a control group (drug concentration 0) that was not treated with drugs was prepared. Incubation at constant temperature (37°C, 5% $CO_2$) was performed for 3 or 6 days. 100 ul of CellTiter-Glo reagent (using CellTiter-Glo® Luminescent Cell Viability Assay kit (Promega, G7571)) was added to each well and pipetted. After incubating at constant temperature (RT) for 10 minutes, luminescence was measured. When the luminescence value at 0 drug concentration is considered 100%, the concentration that shows 50% of the luminescence value is an $IC_{50}$ value.

Two cell lines (FaDu, A549) were treated with two compounds (PBX-7016, PBX-7024) and reference compounds (Dxd, SN-38, exatecan, FL118), and cell viability was observed through 3-day incubation.

As shown in FIG. 15, PBX-7024 shows strong cell killing efficacy not only in the FaDu cell line that does not express ABCG2, but also in the A549, a cancer cell line that overexpresses ABCG2. In other words, it was confirmed that it had an $IC_{50}$ level that is equal to or higher than that of Dxd, the payload used in the existing Enhertu.

In A549, a cancer cell line overexpressing ABCG2, it can be confirmed that the camptothecin compounds, including Dxd, show increased $IC_{50}$ values as shown in FIG. 15, whereas PBX-7016 and PBX-7024 still maintain strong efficacy.

That is, when using the PBX-7016 and PBX-7024, which are new camptothecin compounds according to the present invention, unlike ADCs using existing camptothecin compounds such as SN-38 and DXd, they have the effect of overcoming the resistance mechanism caused by ABCG2 overexpression.

### Example 3. New PBX-series compounds as candidate for ADC payload: in vitro cell viability test

Cell viability assays were performed on MDA-MB-453 (HER2++) cell lines and FaDu (HER2+) cell lines using the same method as Example 2-2.

Two cell lines (MDA-MB-453, FaDu) were treated with two compounds (PBX-7014, PBX-7016, PBX-7018, PBX-7020, PBX-7022) and one reference compound (Dxd), and cell viability was observed through incubation for 3 days. The results are shown in FIG. 16.

As shown in FIG. 16, the evaluation results confirmed that PBX-7016, PBX-7018, PBX-7020, and PBX-7022 had $IC_{50}$ levels equivalent to or higher than Dxd. In addition, it was confirmed that it had an $IC_{50}$ value equivalent to or higher than that of Dxd in the FaDu cell line that does not express ABCG2.

### Example 4. PBX-7016 IV and IP Pharmacokinetic Profile in NOD SCID Mice

The experimental method is as shown in Table 3 below. The IV Plasma concentration-time data of PBX-7016 are shown in Table 4, and the IP Plasma concentration-time data of PBX-7016 are shown in Table 5.

[Table 3]

| | | | In-life | | |
|---|---|---|---|---|---|
| Standard PK | | | | **Species/strain:** | NOD SCID Mice |
| 3 | | | | **Sex:** | Female |
| IV | 1 | mg/kg | | | |
| IP | 1 | mg/kg | | | |
| IV | 5% DMSO and 95%(0.1% (w/v) hydroxypropyl-beta-cyclodextrin in saline) adjust PH to 8~9 | | | | |
| IP | 5% DMSO and 95%(0.1% (w/v) hydroxypropyl-beta-cyclodextrin in saline) adjust PH to 8~9 | | | | |
| IV | 0.5 | mg/mL | | | |
| IP | 0.2 | mg/mL | | | |
| IV | 0.167, 1, 4, 12, 24 and 48 hr post dose. | | | | |
| IP | 0.167, 1, 4, 12, 24 and 48 hr post dose. | | | | |

[Table 4]

| IV Dose | 1 | mg/kg | | | | |
|---|---|---|---|---|---|---|
| Time (h) | Concentration (ng/mL) Mouse 1 | Mouse 2 | Mouse 3 | Mean (ng/mL) | SD (ng/mL) | CV (%) |
| 0.167 | 76.2 | 54.8 | 46.8 | 59.3 | 15.2 | 25.6 |
| 1 | 10.6 | 6.85 | 3.35 | 6.9 | 3.6 | 52.3 |
| 4 | BLOQ | BLOQ | BLOQ | NA | NA | NA |
| 12 | BLOQ | BLOQ | BLOQ | NA | NA | NA |
| 24 | BLOQ | BLOQ | BLOQ | NA | NA | NA |
| 48 | BLOQ | BLOQ | BLOQ | NA | NA | NA |

[Table 5]

| IP Dose | 1 | mg/kg | | | | |
|---|---|---|---|---|---|---|
| Time (h) | Concentration (ng/mL) Mouse 4 | Mouse 5 | Mouse 6 | Mean (ng/mL) | SD (ng/mL) | CV (%) |
| 0.167 | 158 | 186 | 149 | 164 | 19 | 11.7 |
| 1 | 10.5 | 23.9 | 14.0 | 16.1 | 7.0 | 43.1 |
| 4 | BLOQ | BLOQ | BLOQ | NA | NA | NA |
| 12 | BLOQ | BLOQ | BLOQ | NA | NA | NA |
| 24 | BLOQ | BLOQ | BLOQ | NA | NA | NA |
| 48 | BLOQ | BLOQ | BLOQ | NA | NA | NA |

Through the results in Tables 4 and 5, it was confirmed that the PBX-7016 compound cleared from the blood very quickly when administered intravascularly or intraperitoneally to mice, and thus it is expected that the PBX-7016 compound alone, rather than in the form of a prodrug or ADC, will exhibit high safety when administered in vivo. Since PBX-7016 compound is released out of the body very quickly when administered IV, it can act as a safety device that can reduce systemic exposure of the drug even when the drug is released orematurelv after enterine the body.

In addition, this experiment suggests that it is the PK of the PBX-7016 compound alone used as a payload, and is a safe payload that clears the PBX-7016 drug in the blood within a short time. In other words, it is suggested that after the PBX-7016 compound exerts the bystander killing effect at the tumor site, it is safely released out of the body within a very short time when circulating in the blood.

**Example 5. Carboxylate to Lactone conversion rate of PBX-7016 at pH 6.0**

Example 5 (FIGS. 17 and 18) is an experiment related to the activation of the lacto moiety of camptothecin, known as an activating factor of Top1 inhibitors.

**5-1. Carboxylate to lactone form conversion in pH 6.0**

PBX-7016 powder was prepared by dissolving it in DMSO at 5 mM. Since PBX-7016 dissolved in DMSO exists in the lactone form, it was diluted 1/10 with 0.1 N NaOH and incubated in a 25°C water bath for 30 minutes to be converted to the carboxylate form. PBX-7016 in the carboxylate form was diluted to 3 μM using 4°C PBS (pH 6.0) containing 10% DMSO. At this time, the same equivalent of HCl as the 0.1 N NaOH used was added to minimize the pH change. After dilution, centrifugation was performed at 4°C, 13000 RCF for 2 min, and the supernatant was used as a sample.

When the PBX-7016 lactone form was generated, two solvents were used to separate it by RP-HPLC: Solvent A: 100 mM acetate buffer pH 5.5, and Solvent B: ACN. The column used was Cortecs C18 2.7μm, 4.6x50mm Column, and the analysis was conducted under the following conditions: 1) 20 min : 5% B, 2) sample injection, 3) 2 min : 5% B, 4) 10 min : 5% B → 50% B, 5) 1 min : 50% B.

To measure the change over time, the sample chamber was maintained at 37°C, and the sample was set to be injected at each measurement time. The measurement times were 0, 20, 40, 60, 90, 120, 180, 240, 300, 360, 480, 600, 720, 840, 960, 1200, and 1440 min.

The content was obtained from the peak area of each lactone and carboxylate. Since the absorption coefficients of lactone and carboxylate are different, the lactone area was obtained by multiplying the carboxylate area by the following

equation:

$$\text{lactone EC}_{390} / \text{carboxylate EC}_{390} = 0.918$$

The same experiment as in Example 5-1 was performed except that FL118, SN38, DxD, exatecan, PBX-7014, and PBX-7024 were used instead of PBX-7016.

The results of the conversion experiment from carboxylate to lactone form at pH 6.0 are shown in FIG. 18. As shown in FIG. 18, it was confirmed that various camptothecin compounds changed their form from carboxylate to lactone, which is an active form that inhibits topoisomerase I, at similar rates and in equivalent amounts at pH 6.0, and thus it is predicted that they will exhibit equivalent activity in the tumor micro-environment where pH is generally 6.0.

### 5-2. Lactone to carboxylate form conversion in pH 7.4

PBX-7016 powder was prepared by dissolving it in DMSO at 5 mM, and diluted with 4°C PBS (pH 7.4) containing 10% DMSO to prepare 3 μM PBX-7016. Thereafter, it was centrifuged at 4°C, 13000 RCF for 2 min, and the supernatant was used as a sample. PBX-7016 dissolved in DMSO was present in the form of Lactone.

When the PBX-7016 carboxylate form was generated, two solvents were used to separate it by RP-HPLC: Solvent A: 100 mM acetate buffer pH 5.5, and Solvent B: ACN. The column used was Cortecs C18 2.7μm, 4.6x50mm Column, and the analysis was conducted under the following conditions: 1) 20 min : 5% B, 2) sample injection, 3) 2 min : 5% B, 4) 10 min : 5% B → 50% B, 5) 1 min : 50% B.

To measure the change over time, the sample chamber was maintained at 37°C, and the sample was set to be injected at each measurement time. The measurement times were 0, 20, 40, 60, 90, 120, 180, 240, 300, 360, 480, 600, and 720 min.

The content was obtained from the peak area of each lactone and carboxylate. Since the absorption coefficients of lactone and carboxylate are different, the lactone area was obtained by multiplying the carboxylate area by the following equation:

$$\text{lactone EC}_{390} / \text{carboxylate EC}_{390} = 0.918$$

The same experiment as in Example 5-2 was performed except that FL118, SN38, DxD, exatecan, PBX-7014, and PBX-7024 were used instead of PBX-7016.

As shown in FIG 17, which is an experimental result of conversion from lactone form to carboxylate form at pH 7.4, it was confirmed that compounds PBX-7014, PBX-7016, and PBX-7024 represented by Chemical Formula 1 were converted from lacton form (active form) to carboxylate form (inactive form), which is inactive as TOP1 inhibitors, more rapidly and at a higher rate than other reference compounds (exatecan, DXd, SN-38, FL118) at pH 7.4, which is the pH of blood or extracellular fluid.

In particular, the degree (63-86%) and rate (0.4-0.8%/min) of formation of the carboxylate form, which is inactive as a TOP1 inhibitor, at blood pH 7.4, are different greatly depending on the type of camptothecin derivative. PBX-7014 and PBX-7016 formed the carboxylate form of 86% or more, and both converted relatively quickly from the lactone form to the carboxylate form at a rate of 0.8% per minute. The DXd, a competing drug, formed the carboxylate form of about 76.5%, and converted from the lactone form to the carboxylate form at a rate of 0.6% per minute.

Through this, it is predicted that PBX-7014 and PBX-7016 compounds exist as inactive carboxylates rather than active lactons, which exhibit cytotoxicity in the blood at a relatively fast and high rate compared to reference compounds, thereby exhibiting high safety in normal cells.

### Preparation Example 5: Synthesis of 25-4 and 25-6 from PBX-7014 and PBX-7016, and Preparation of ADC (DAR7-8)thereof (Trastuzumab-25-4 and Trastuzumab-25-6)

25-4 (Chemical Formula 10) and 25-6 (Chemical Formula 11), which are linker-payloads containing two compounds (PBX-7014, PBX-7016) and introducing GGFG, an enzymatically cleavable linker system, were synthesized.

[Chemical Formula 10]

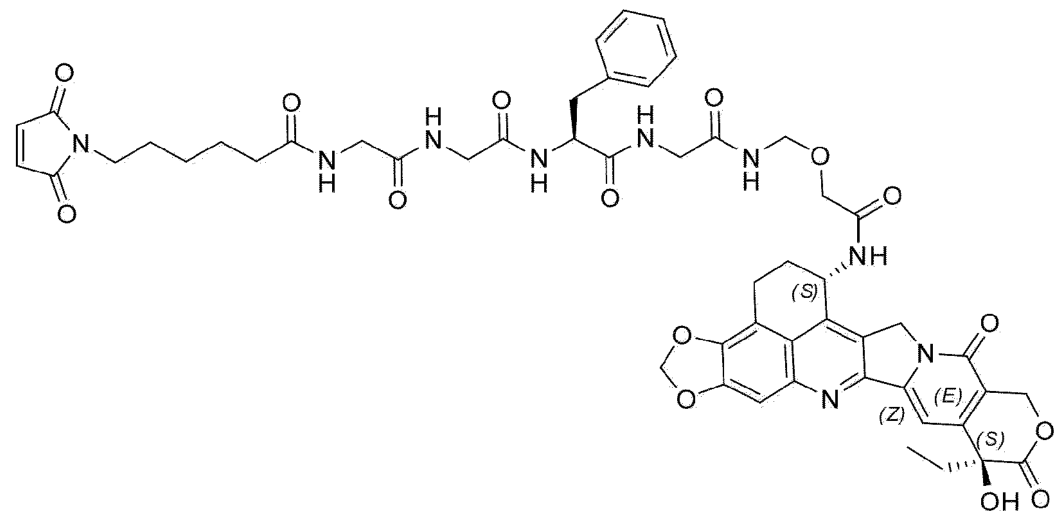

[Chemical Formula 11]

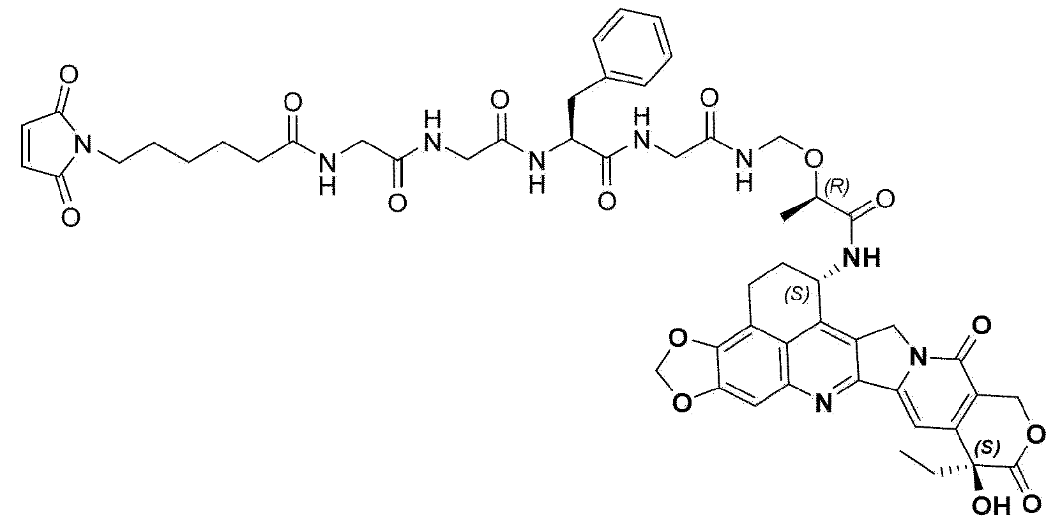

The molecular structure of 25-4 is GGFG-PBX-7014, and the molecular structure of 25-6 is GGFG-PBX-7016. That is, they each used the same GGFG linker as Enhertu®.

Furthermore, using the same GGFG linker and trastuzumab antibody as Enhertu®, ADCs were manufactured with PBX-7014 and PBX-7016 as payloads.

The linker-payload compounds of Chemical Formula 10 and Chemical Formula 11 were conjugated with trastuzumab, a Her2 target antibody, to synthesize ADCs. (Tra-25-4 and Tra-25-6, both DAR 8).

Trastuzumab-25-4 (Trastuzumab-7014) was manufactured by the following method. The prepared trastuzumab was buffer exchanged with reaction buffer (150 mM NaCl, 50 mM Histidine pH 6.0) using PD-10 desalting column, and then 825 uM TCEP was treated with 27.5 uM antibody at 25°C for 2 hours to create thiol sites required for the reaction from disulfide of the antibody.

Thereafter, extra TCEP was removed using PD-10 desalting column, and 61.9 uM 25-4 drug linker (Chemical Formula 10) and 13.8 uM reduced trastuzumab were reacted at 25°C for 1 hour in reaction buffer containing 10% DMSO to perform the first conjugation reaction.

Trastuzumab-25-6 (Trastuzumab-7016) was manufactured through the same process, but the same concentration of 25-6 drug linker (Chemical Formula 11) was used instead of 61.9uM 25-4 drug linker (Chemical Formula 10).

Thereafter, each ADC was purified using SEC, and it was confirmed that it was purified as a monomer without

aggregation (FIG. 19). It was confirmed that the drug was conjugated through the band shift of the light chain and heavy chain through SDS-PAGE (FIG. 20A).

**Preparation Example 6: Synthesis of 25-6 from PBX-7016, and preparation of ADC (DAR7-8) thereof (Nimotuzumab-25-6 and Sacituzumab-25-6)**

Nimotuzumab-25-6, Sacituzumab-25-6, and Cetuximab-25-6 were synthesized in the same manner as in Preparation Example 5, except that the Nimotuzumab, Sacituzumab, or Cetuximab were used instead of Trastuzumab.
Then, each ADC was purified using SEC, and it was confirmed that it was purified as a monomer without aggregation. It was confirmed that the drug was conjugated through the band shift of the light chain and heavy chain through SDS-PAGE (FIGS. 20B, 20C, and 20D).

**Example 6. Anti HER2 ADCs with PBX-payloads: binding affinity analysis**

Coating buffer was prepared by diluting the target antigen to 0.66 μg/ml using ELISA plate coating buffer (R&D systems, #DY006). Coating was performed using 100 μL of the prepared coating buffer per well. The plate cover was closed and kept at 2 to 8 °C overnight (12 to 18 hours). The plate used was Corning, #3590. It was washed three times with 200 μL or more of TBS Tween-20 buffer (thermo, #28360). When washing, the liquid was first removed with a pipette, and then extra buffer was removed by gently tapping on a paper towel. Blocking was performed at room temperature for 1 hour using 200 μL of assay buffer. The liquid was removed with a pipette, and extra buffer was removed by gently tapping on a paper towel. Control antibody and ADC to be measured were prepared in assay buffer at 1 μg/ml, and then prepared in the required amount or more through 1/5 serial dilution at the following concentrations. For example, samples may be prepared by diluting to the following concentrations based on duplication [240 μl assay buffer + 60 μl of sample of the previous concentration]: 1μg/ml, 0.4μg/ml, 0,08μg/ml, 0.016μg/ml, 0.0032μg/ml, 0.00064μg/ml, 0.000128μg/ml. The diluted control antibody and ADC were bound in duplicate at 100 μL per coated well. It was performed at room temperature for 1 hour. It was washed three times with 100 μL of TBS Tween-20 buffer (thermo, #28360). When washing, the liquid was first removed with a pipette, and then extra buffer was removed by gently tapping on a paper towel. The detection antibody was diluted in assay buffer and prepared according to the manufacturer's manual. Human antibody was prepared by using Goat anti-Human Fc-HRP (Novex, #A18817) as a detection antibody and diluting to 1/2000. 100 μL of the diluted detection antibody was added to each well and incubated at room temperature for 1 hour. It was washed three times with 100 μL of TBS Tween-20 buffer (thermo, #28360). When washing, the liquid was first removed with a pipette, and then extra buffer was removed by gently tapping on a paper towel. It was reacted at room temperature for 30 minutes using 100 μL of TMB substrate solution (Thermo, #N301). The reaction was stopped using 100 μL of stop solution (Thermo, N600). The reaction was stopped and the absorbance was measured at 450 nm. It was completed within 30 minutes after the addition of the stop solution. When bubbles existed, 100 to 150 μl was transferred to a new plate and measured. Through this, trastuzumab, and ADCs in which trastuzumab & PBX-7014 were conjugated, and ADCs in which trastuzumab & PBX-7016 were conjugated were measured to obtain the results of FIG. 21. As shown in FIG. 21, it was confirmed that when the binding intensity to the Her2 antigen of ADCs in which PBX-7014 or PBX-7016 was conjugated to trastuzumab was tested at various concentrations, there was no difference in the binding intensity compared to the binding intensity of trastuzumab to the Her2 antigen.

**Example 7. Anti HER2 ADCs with PBX-payloads : in vitro potency and target selectivity**

For ADCs with Dxd, PBX-7014, and PBX-7016 as payloads, respectively, in vitro cell viability assays were performed on MDA-MB-453 (HER2++) cell lines, FaDu (HER2+) cell lines, and MDA-MB-468 (HER2-) cell lines using the same method as in Example 2-2 as follows.
In this case, Herceptin (ingredient name: trastuzumab), which is a HER2-targeting therapeutic, Dxd drug, PBX-7014 (Preparation Example 2), PBX-7016 (Preparation Example 3), Tra-25-4 and Tra-25-6 (Preparation Example 5), which are ADCs thereof, and Tra-Dxd (Enhertu®) were treated.
Enhertu (Tra-Dxd, DAR 8) as a reference was treated to three cell lines, MDA-MB-453 (Her2++), FaDu (Her2+), and MDA-MB-468 (Her2-), according to the Her2 expression level, and cell viability was observed through incubation for 3 days (FIG. 22).
As shown in FIG. 22, it was confirmed that the anti-Her2 ADC with PBX-payload had excellent target selectivity.

**Discussion: Evaluation of PBX-7014 and PBX-7016 as ADC payloads**

As shown in FIG. 22, it was confirmed that ADC (Tra-25-6) with PBX-7016 as a payload had an $IC_{50}$ value that was approximately 5 times better than Enhertu (Tra-Dxd) in MDA-MB-453, which is a Her2++, and had an equivalent $IC_{50}$

value in the MDA-MB-468, which is a negative cell line, thereby confirming that the linker-payload system of Chemical Formula 10 and Chemical Formula 11 not only operates by distinguishing positive/negative cell lines when developed as ADC, but also shows superior efficacy compared to the Enhertu as a reference.

**Discussion: Originality and Excellence of PBX-7016**

PBX-7016, a camptothecin compound newly designed and synthesized in Chemical Formula 1 according to the present invention is a compound newly derived from FL118, which is an MD-CPT skeleton, and presents a new topoismerase 1 inhibitor that degrades DDX5, similar to FL118, which acts not only as a topoismerase I inhibitor but also as a DDX5 degrader (FIGS. 11 to 14).

In addition, when cell viability was simply compared, PBX-7016 showed an $IC_{50}$ value that was 1.5 to 2 times higher than that of Dxd, but surprisingly, when used as an ADC payload, it was confirmed that the ADC containing PBX-7016 had about 5 times better cell viability than the ADC containing Dxd (Enhertu) (FIG. 22).

**Example 8. Anti HER2 ADCs with PBX-payloads : in vivo efficacy**

**8-1. Cell culture**

JIMT-1 breast cancer cells were cultured in DMEM containing 10% FBS, 100 U/mL penicillin, and 100 μg/mL streptomycin under the conditions of 5% $CO_2$ and 37°C, and routinely subcultured twice a week by trypsin-EDTA treatment. Cells growing in the exponential growth phase were harvested and counted for tumor inoculation.

**8-2. BALB/c nude mice**

Both SCID mice and BALB/c nude mice are used as animal models to study the immune system and test treatments for various diseases.

Although both SCID mice and BALB/c nude mice have compromised immune systems, the underlying mechanisms of their immune defects are different.

The SCID mice are mice that have been genetically engineered to lack functional T cells and B cells, and have a severely compromised immune system. They are used to study immune system development and function and to test potential treatments for diseases that affect the immune system, such as autoimmune disorders and certain types of cancer.

On the other hand, the BALB/c nude mice are mice that do not have a thymus and therefore cannot produce T cells. They are also born hairless due to a defect in the hairless gene. These mice are used to study the role of T cells in immune responses and to test potential treatments for diseases that affect T cell function, such as certain types of cancer.

**8-3. Tumor inoculation and drug treatment**

JIMT-1 tumor cells ($5 \times 10^6$) in 0.2 mL DPBS with Matrigel were subcutaneously inoculated into the right anterior flank of each BALB/c nude mice. When the average tumor volume reached approximately 215 $mm^3$, the animals were randomly grouped and then the treatment for drug efficacy study was initiated as follows.

**Treatment:** I.V., Q1W

Vehicle group: Vehicle,0 mg/kg,5 μL/g,i.v.,Twice(Day 0,day 6)
Isotype-Dxd(3) group: CTP63-Dxd, 3 mg/kg,5 μL/g,i.v.,Twice(Day 0,day 6)
Isotype-Dxd(10) group: CTP63-Dxd, 10 mg/kg,5 μL/g,i.v.,Twice(Day 0,day 6)
Isotype-PBX7016(3) group; CTP63-PBX7016, 3 mg/kg,5 μL/g,i.v.,Twice(Day 0,day 6)
Isotype-PBX7016(10) group: CTP63-PBX7016,10 mg/kg,5 μL/g,i.v.,Twice(Day 0,day 6)
Isotype-PBX7014(3) group: CTP63-PBX7014, 3 mg/kg,5 μL/g,i.v.,Twice(Day 0,day 6)
Isotype-PBX7014(10) group: CTP63-PBX7014,10 mg/kg,5 μL/g,i.v.,Twice(Day 0,day 6)
Trastuzumab-PBX7014(3) group: CTP6-PBX7014,3 mg/kg,5 μL/g,i.v.,Twice(Day 0,day 6)
Trastuzumab -PBX7014(10) group: CTP6-PBX7014, 10 mg/kg,5 μL/g,i.v.,Twice(Day 0,day 6)
Trastuzumab -PBX7016(3) group: CTP6-PBX7016, 3 mg/kg,5 μL/g,i.v.,Twice(Day 0,day 6)
Trastuzumab -PBX7016(10) group: CTP6-PBX7016, 10 mg/kg,5 μL/g,i.v.,Twice(Day 0,day 6)
Enhertu (3) group: Enhertu, 3 mg/kg,5 μL/g,i.v.,Twice(Day 0,day 6)

Enhertu (10) group: Enhertu,10 mg/kg,5 μL/g,i.v.,Twice(Day 0,day 6)

### 8-4. Tumor measurement and endpoint

The time point at which tumor growth could be delayed or the mouse could be treated was taken as the main endpoint. The two-dimensional size of the tumor was measured twice a week using a caliper, and the volume was expressed in $mm^3$ using the formula ($V = 0.5a \times b^2$, where a and b are the long and short diameters of the tumor, respectively).

Tumor growth inhibition (TGI) was calculated for each group:

$$TGI (\%) = [1 - (T_i - T_0) / (V_i - V_0)] \times 100$$

Wherein $T_i$ is the average tumor volume of the treatment group on a given date, $T_0$ is the average tumor volume of the treatment group on the first day of treatment, $V_i$ is the average tumor volume of the vehicle control group on the same day as $T_i$, and $V_0$ is the average tumor volume of the vehicle group on the first day of treatment.

Animals with a weight loss of 15% or more or a tumor volume of 3000 $mm^3$ or more were euthanized as a humane endpoint.

### 8-5. Statistical analysis

The results are expressed as mean and standard error (mean ± SEM). Data were analyzed by a multiple comparison test of two-way RM ANOVA Dunnett using Graphpad Prism 6.0 software, and $p < 0.05$ was considered statistically significant.

Table 6 below shows the trend of the tumor volume ($mm^3$) over time (effect vs. time).

[Table 6]

| Group | 6+0 days | 6+3 days | 6+7 days | 6+10 days | 6+14 days |
|---|---|---|---|---|---|
| Vehicle group | 175.16 | 262.35 | 414.51 | 458.39 | 658.48 |
| Isotype-Dxd(3) group | 175.16 | 264.78 | 356.71 | 396.97 | 498.12 |
| Isotype-Dxd(10) group | 174.99 | 272.38 | 348.88 | 362.98 | 451.89 |
| Isotype-PBX7016(3) group | 175.73 | 277.19 | 418.38 | 462.90 | 530.39 |
| Isotype-PBX7016(10) group | 175.95 | 264.88 | 334.74 | 366.55 | 428.60 |
| Isotype-PBX7014(3) group | 175.46 | 252.90 | 335.66 | 388.03 | 468.99 |
| Isotype-PBX7014(10) group | 175.23 | 298.40 | 383.04 | 420.42 | 506.52 |
| Trastuzumab-PBX7014(3) group | 175.63 | 279.28 | 331.04 | 367.43 | 392.67 |
| Trastuzumab-PBX7014(10) group | 176.33 | 273.52 | 261.47 | 260.96 | 274.31 |
| Trastuzumab-PBX7016(3) group | 174.79 | 269.91 | 266.46 | 267.00 | 251.11 |
| Trastuzumab-PBX7016(10) group | 175.76 | 314.16 | 274.22 | 257.78 | 225.68 |
| Enhertu(3) group | 175.58 | 295.80 | 325.74 | 354.87 | 405.93 |
| Enhertu(10) group | 175.87 | 270.11 | 254.45 | 249.13 | 249.18 |

FIG. 23, which is a graphical representation of Table 6, shows the results of efficacy tests on ADCs with various camptothecin-based drugs as payloads in mice transplanted with JIMT-1 cell lines, i.e., in vivo xenograft models.

### 8-6. Body weight of JIMT-1 tumor model

The body weight of JIMT-1 tumor model mice was regularly monitored (FIG. 24).

### Example 9: Anti HER2 ADCs with PBX-payload: Bystander effect study

The bystander effect is one of the factors that significantly affects the efficacy of ADC drugs. To confirm the

bystander effect, the analysis was performed by FACS under 40 nM ADC treatment conditions.

In order to verify the efficacy and effectiveness as an ADC drug using PBX-7016, the leading substance of the present invention, as a payload in an antibody-drug conjugate (ADC) in which a payload-linker is bound to a trastuzumab antibody targeting HER2 protein, the bystander effect was confirmed in vitro through flow cytometric analysis experiments. In this example, the bystander effect is defined as the fact that MDA-MB-453, a HER2-positive cell line, is treated with ADC to induce cell death, and the drug (payload) released during the cell death process causes toxicity to surrounding HER2-negative cell lines, leading to cell death.

In order to evaluate the in vitro bystander effect of the ADC prepared in Preparation Example 5, HER2 expression-negative cell lines and HER2 expression-positive cell lines were mixed in a 6-well scale cell culture plate and cocultured at different ratios to evaluate whether there was the bystander effect due to ADC treatment.

Specifically, GFP-MDA-MB-468 cells (3 x 10^5 cells), which were prepared to express green fluorescent protein (GFP), and MDA-MB-453 cells (1 x 10^5 cells), a HER2 expression-positive cell line, were mixed in a 3:1 ratio to MDA-MB-468, a HER2 expression-negative cell line, cocultured for 24 hours, and then were treated with trastuzumab antibody and a total of 5 types of ADC samples (Trastuzumab-DXd, Isotype IgG-DXd, Trastuzumab-25-6, Isotype IgG-25-6, Enhertu) at a concentration of 40 nM each, and cell-cultured for 6 days. Then, flow cytometry was used to measure GFP fluorescence and determine cell counts in GFP-MDA-MB-468 cells, a HER2 expression-negative cell line, among living cells.

In addition, at the same time, MDA-MB-468 cells (3 x 10^5 cells), a HER2 expression-negative cell line, and MDA-MB-453 cells (1 x 10^5 cells), a HER2 expression-positive cell line, were mixed in a 3:1 ratio in 6-well cell culture plate, cocultured for 24 hours, and then were treated with trastuzumab antibody and a total of 5 types of ADC samples (Trastuzumab-DXd, Isotype IgG-DXd, Trastuzumab-25-6, Isotype IgG-25-6, Enhertu) at a concentration of 40 nM each, and cell-cultured for 6 days. Then, the cells were stained using anti-HER2-FITC antibodies that are fluorescent by labeling FITC (fluorescein isothiocyanate) fluorochrome dye on an antibody capable of recognizing HER2 protein. Then, flow cytometry was used to measure FITC fluorescence and determine cell counts in MDA-MB-453 cells, a HER2 expression-positive cell line, among living cells.

The flow cytometry was performed by collecting cells from each well of a 6-well cell culture plate, diluting them with buffer (400 ul), and then measuring GFP or FITC fluorescence for each sample at the same time (1 minute each) and flow using an FL1 laser.

As shown in FIGS. 25 and 26, as a result of flow cytometry, it was determined that Trastuzumab-25-6, a PBX-7016-based ADC, not only killed HER2 expression-positive cells but also simultaneously killed HER2 expression-negative cells compared to Trastuzumab-Dxd and Enhertu, which are DXd-based ADCs, indicating that it exhibited the bystander effect at the same level as the existing DXd-based Enhertu. In contrast, it was confirmed that Isotype IgG-DXd and Isotype IgG-25-6, negative control ADCs, did not significantly induce HER2 expression-negative cell death compared to the untreated condition where ADC was not treated.

In summary, Anti-HER2 ADC using PBX-7016 showed a bystander effect similar to that of Enhertu.

### Example 10: In vitro cell cytotoxicity test of Cetuximab-25-6 and Sacituzumab-25-6

In the same manner as Example 2-2, Cetuximab-25-6 (FIG. 27A) was treated on MDA-MB-468 (EGFR positive) cell lines and SW480 (EGFR negative) cell lines; and Sacituzumab-25-6 (FIG. 27B) was treated on FaDu (Trop2+++) cell lines and Calu-6 (Trop2--) cell lines, and cell viability assay was performed through incubation for 6 days. The results are shown in FIG. 27.

FIG. 27 shows that high-affinity mAb binding to cell membrane proteins can localize a significant portion of mAbs to target cell populations even in ADCs such as Cetuximab-25-6 and Sacituzumab-25-6, and that chemical conjugation of the PBX-7016 payload to the anticancer mAb increases the selectivity with which the PBX-7016 payload is delivered to cancer cells, thereby increasing the therapeutic index of the payload.

In particular, as a result of comparing and evaluating the in vitro cell viability (ADC 6 days treatment) for Sacituzumab-25-6 and PBX-7016 in FaDu (Trop2+++) cell lines and Calu-6 (Trop2--) cell lines (FIG. 27b), it is suggested that PBX-7016, which is an example of an active camptothecin derivative (a) represented by Chemical Formula 1 and designed to be released at the target site in vivo, can penetrate the cell membrane of non-target cells, move into the cell, and exert a cell death mechanism, that is, PBX-7016-based ADC can exert a bystander effect.

## Claims

**1.** (a) an active camptothecin derivative represented by Chemical Formula 1 below, which is designed to bind to DDX5 protein and E3 ligase; or (b) a prodrug thereof, preferably an antibody-drug conjugate (ADC) thereof, which is designed to release the active camptothecin derivative (a) at a target site in vivo; or (c) a complex containing a DDX5

protein-targeting ligand, in which the ability of the active camptothecin derivative (a) or an FL118 compound of Chemical Formula 2 to inhibit type 1 topoisomerase is inactivated through a linker connection.

[Chemical Formula 1]

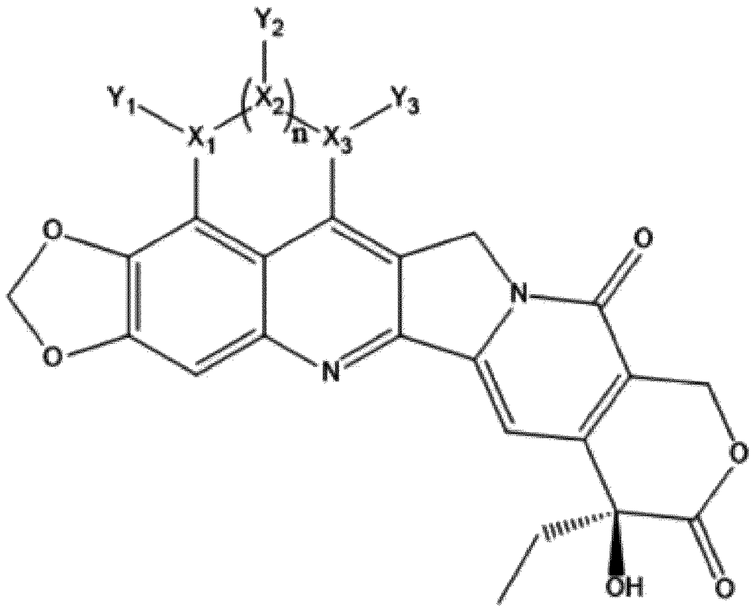

wherein $X_1$ and $X_3$ are each independently carbon, oxygen, nitrogen, or sulfur, and $X_1$ and $X_3$ may be the same or different,

$X_2$ is carbon, oxygen, nitrogen, sulfur, a single bond, or a double bond,
$X_1$, $(X_2)n$, and $X_3$ may form a six-membered or seven-membered ring (n=1 to 2),
$Y_1$, $Y_2$, and $Y_3$ may each independently be hydrogen, or a functional group containing oxygen, nitrogen, phosphorus, or sulfur.

**2.** The camptothecin derivative (a) or the prodrug thereof (b) according to claim 1, wherein the active camptothecin derivative (a) designed to bind to DDX5 protein and E3 ligase can serve as a ligand (binder) that binds to E3 ligase as a molecular glue degrader.

**3.** The camptothecin derivative (a) or the prodrug thereof (b) according to claim 1, wherein the active camptothecin derivative (a) designed to bind to DDX5 protein and E3 ligase kills target cells expressing DDX5 protein through a molecular glue degrader mechanism of action (MoA).

**4.** The camptothecin derivative (a) or the prodrug thereof (b) according to claim 1, wherein the active camptothecin derivative (a) designed to bind to DDX5 protein and E3 ligase has a mechanism of action (MoA) that degrades oncoprotein DDX5 together with the ability to inhibit type 1 topoisomerase.

**5.** The camptothecin derivative (a) or the prodrug thereof (b) according to claim 4, wherein (1) in General Formula 1 or Chemical Formula 2, the Group C site optionally binds to type 1 topoisomerase and the Group A site binds to DNA to stabilize the covalent bond of the topoisomerase-DNA complex, thereby preventing the cleaved DNA fragments from being reconnected, and/or (2) in General Formula 1 or Chemical Formula 2, the Group A site binds to DDX5 and the Group C site optionally binds to E3 ligase to induce DDX5 degradation.

[General Formula 1]

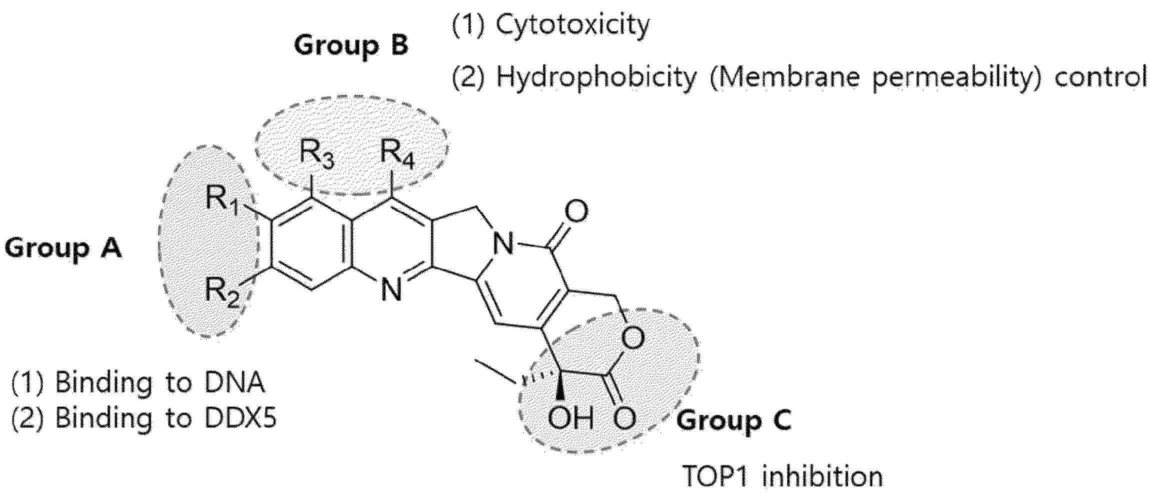


[Chemical Formula 2]

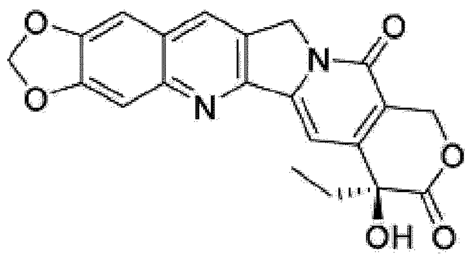

FL118

6. A pharmaceutical composition for preventing or treating cancer or a composition for diagnosing cancer, including the active camptothecin derivative (a), the prodrug thereof (b), preferably the antibody-drug conjugate (ADC), or the complex containing a DDX5 protein-targeting ligand (c) as described in any one of claims 1 to 5.

7. The pharmaceutical composition according to claim 6, which is used as a first-line treatment after cancer diagnosis, or is administered to an individual that (over)expresses DDX5 in cancer tissue.

8. The pharmaceutical composition according to claim 6, which is administered to treat HER2 positive cancer, breast cancer, lung cancer, or colon cancer.

9. The pharmaceutical composition or cancer diagnostic composition according to claim 6, wherein the DDX5 protein is used as a biomarker for predicting drug sensitivity or tumor sensitivity.

10. The pharmaceutical composition according to claim 6, wherein the DDX5 protein is a drug target of the active camptothecin derivative, and thus drug resistance, resistance to targeted therapy, and/or resistance during treatment can be avoided.

11. The pharmaceutical composition according to claim 6, wherein the active camptothecin derivative offs the transcription induction of anti-apoptotic genes.

12. The pharmaceutical composition according to claim 6, wherein the active camptothecin derivative degrades the

DDX5 protein, which is a transcription cofactor, thereby maintaining or enhancing the sensitivity of cancer cells to chemotherapy or radiotherapy.

**13.** The pharmaceutical composition according to claim 6, which converts a solid cancer aggregated with high cell density into a scattered tumor with a low cell density and/or converts a cold tumor with low immune activity into a hot tumor with high immune activity.

**14.** The cancer diagnostic composition including the complex containing a DDX5 protein-targeting ligand (c) according to claim 6, which is used to determine a patient group to which the active camptothecin derivative (a) or the prodrug thereof (b) is to be administered, or to predict drug response, recurrence rate and/or prognosis during chemotherapy.

**15.** A method for preparing (a) an active camptothecin derivative designed to bind to DDX5 protein and E3 ligase, (b) a prodrug thereof, preferably an antibody-drug conjugate (ADC) thereof, which is designed to release the active camptothecin derivative (a) at a target site in vivo, or (c) a complex containing a DDX5 protein-targeting ligand, in which the ability of the active camptothecin derivative (a) or an FL118 compound of Chemical Formula 2 to inhibit type 1 topoisomerase is inactivated through a linker connection,

the method including a step of designing the active camptothecin derivative (a) to include a camptothecin-based skeleton represented by Chemical Formula 1 below as a parent nucleus, selecting a compound designed in this way, or synthesizing a compound designed in this way.

[Chemical Formula 1]

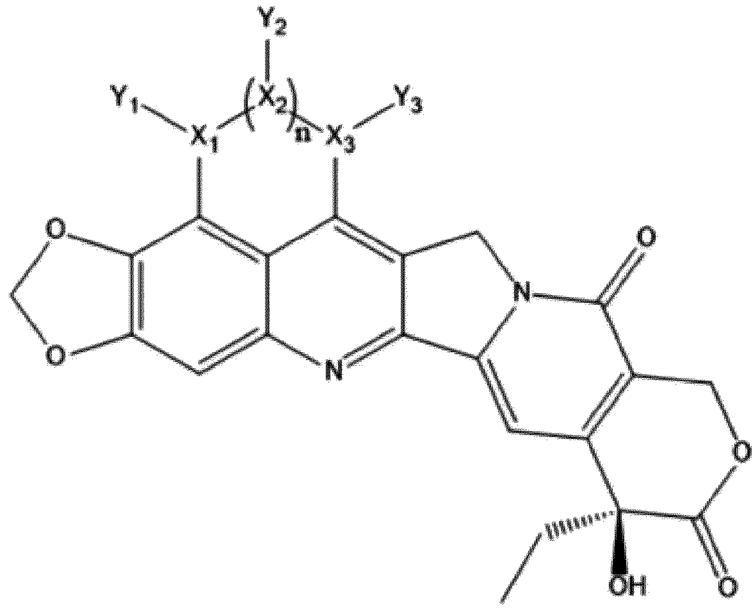

wherein $X_1$ and $X_3$ are each independently carbon, oxygen, nitrogen, or sulfur, and $X_1$ and $X_3$ may be the same or different,
$X_2$ is carbon, oxygen, nitrogen, sulfur, a single bond, or a double bond,
$X_1$, $(X_2)$n, and $X_3$ may form a six-membered or seven-membered ring (n=1 to 2),
$Y_1$, $Y_2$, and $Y_3$ may each independently be hydrogen, or a functional group containing oxygen, nitrogen, phosphorus, or sulfur.

**16.** The method for preparing the active camptothecin derivative, the prodrug thereof, or the complex containing a DDX5 protein-targeting ligand according to claim 15, wherein the active camptothecin derivative (a) designed to bind to DDX5 protein and E3 ligase has a mechanism of action (MoA) that degrades oncoprotein DDX5 together with the ability to inhibit type 1 topoisomerase.

**17.** The method for preparing the active camptothecin derivative, the prodrug thereof, or the complex containing a DDX5

protein-targeting ligand according to claim 15, wherein the step of designing the active camptothecin derivative (a) to include a camptothecin-based skeleton represented by Chemical Formula 1 as a parent nucleus or selecting a compound designed in this way is:

(1) a step of selecting an active camptothecin derivative designed to have a mechanism of action (MoA) for inhibiting type 1 topoisomerase and/or a mechanism of action (MoA) for degrading oncoprotein DDX5 from a compound library including a camptothecin-based skeleton represented by Chemical Formula 1 as a parent nucleus, and/or
(2) a step of confirming through in vitro experiments and/or in vivo experiments whether a compound including a camptothecin-based skeleton represented by Chemical Formula 1 as a parent nucleus inhibits type 1 topoisomerase and/or degrades oncoprotein DDX5.

**18.** The method for preparing the active camptothecin derivative, the prodrug thereof, or the complex containing a DDX5 protein-targeting ligand according to claim 15, wherein the active camptothecin derivative is designed to exert a surrounding cell killing effect (bystander effect) in cancer tissue or control the degree of the effect by modifying $X_1$, $(X_2)$ n, $X_3$, $Y_1$, $Y_2$ and/or $Y_3$ to control a hydrophobicity or cell membrane permeability thereof as desired.

**19.** The method for preparing the active camptothecin derivative, the prodrug thereof, or the complex containing a DDX5 protein-targeting ligand according to claim 15, wherein the active camptothecin derivative (a) or the prodrug (b) thereof is for administration to a patient group in which DDX5 functions as an oncoprotein in cells.

**20.** The method for preparing the active camptothecin derivative, the prodrug thereof, or the complex containing a DDX5 protein-targeting ligand according to claim 15, wherein the method further including a step of identifying a patient group to which the active camptothecin derivative (a) or the prodrug thereof (b) is to be administered, by quantitatively or qualitatively analyzing intracellular DDX5 protein using the complex containing a DDX5 protein-targeting ligand, in which the ability of the FL118 compound of Chemical Formula 2 or the active camptothecin derivative (a) to inhibit type 1 topoisomerase is inactivated through a linker connection, in a tissue biopsy or liquid biopsy.

**21.** A method for preparing (a) an active camptothecin derivative that binds to DDX5, which acts as an oncoprotein in a cell, and induces cell death through DDX5 protein degradation, (b) a prodrug thereof, preferably an antibody-drug conjugate (ADC), which is designed to release the active camptothecin derivative (a) in vivo, or (c) a complex containing a DDX5 protein-targeting ligand, in which the ability of the active camptothecin derivative (a) to inhibit type 1 topoisomerase is inactivated through a linker connection, the method including:

a first step of selecting the active camptothecin derivative (a) from the group consisting of a compound of Chemical Formula 3, a compound of Chemical Formula 4, a compound of Chemical Formula 5, a compound of Chemical Formula 6, a compound of Chemical Formula 7, a compound of Chemical Formula 8, a compound of Chemical Formula 9, and isomers thereof; and
optionally, a second step of selecting a prodrug (b) thereof designed to release the active camptothecin derivative (a) selected in the first step in vivo:

[Chemical Formula 3]

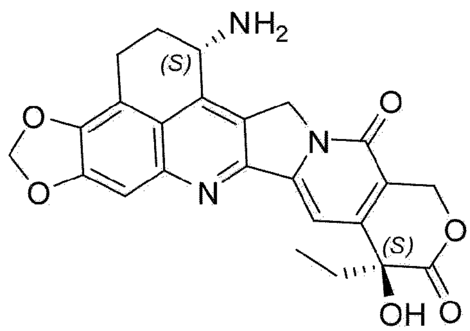

[Chemical Formula 4]

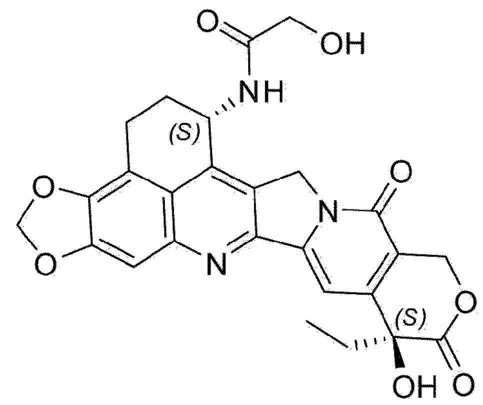

[Chemical Formula 5]

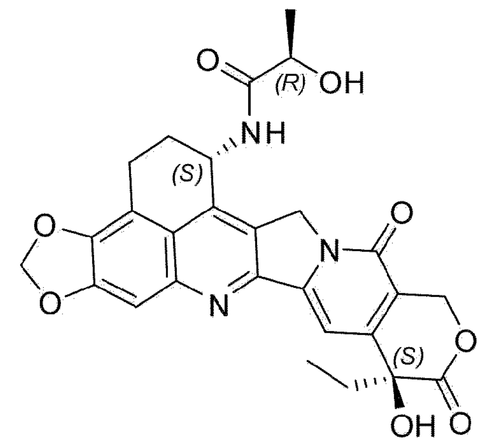

[Chemical Formula 6]

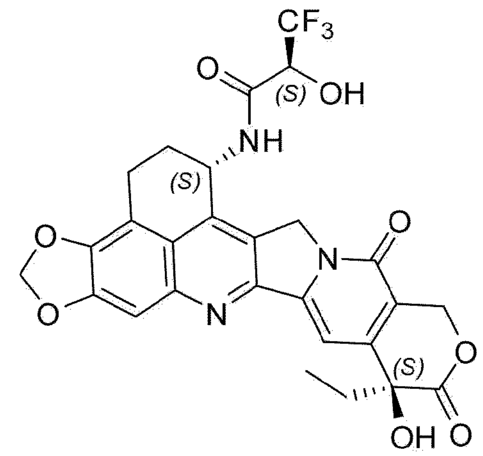

[Chemical Formula 7]

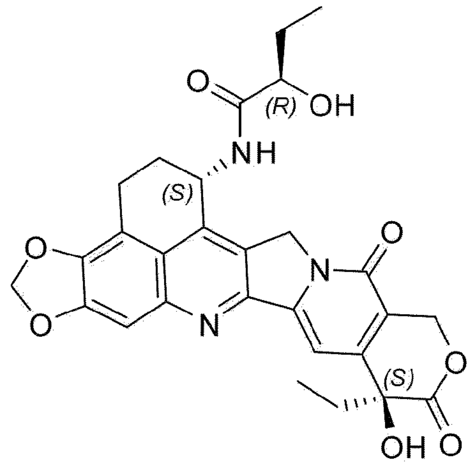

[Chemical Formula 8]

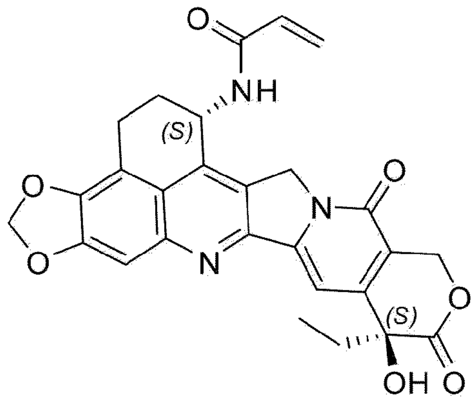

[Chemical Formula 9]

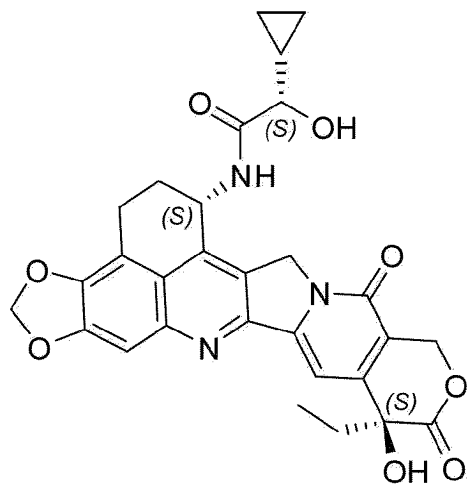

**22.** The preparation method according to claim 21, wherein the active camptothecin derivative (a) or the prodrug (b)

thereof is for administration to a patient group in which DDX5 functions as an oncoprotein in cells.

**23.** The preparation method according to claim 21, wherein the method further including a step of identifying a patient group to which the active camptothecin derivative (a) or the prodrug thereof (b) is to be administered, by quantitatively or qualitatively analyzing intracellular DDX5 protein using the complex containing a DDX5 protein-targeting ligand, in which the ability of the FL118 compound of Chemical Formula 2 or the active camptothecin derivative (a) to inhibit type 1 topoisomerase is inactivated through a linker connection, in a tissue biopsy or liquid biopsy.

**24.** A compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 below, or a pharmaceutically acceptable salt thereof:

[Chemical Formula 3]

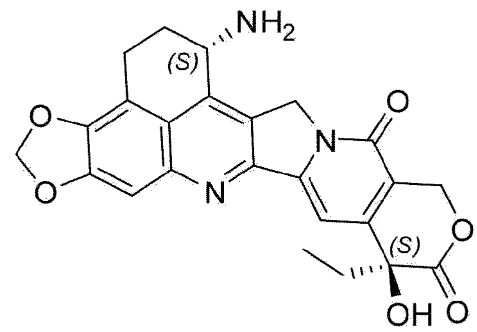

[Chemical Formula 3-1]

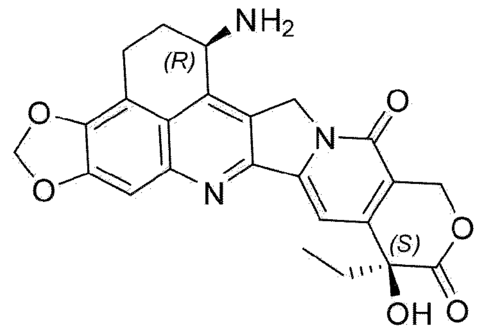

[Chemical Formula 4]

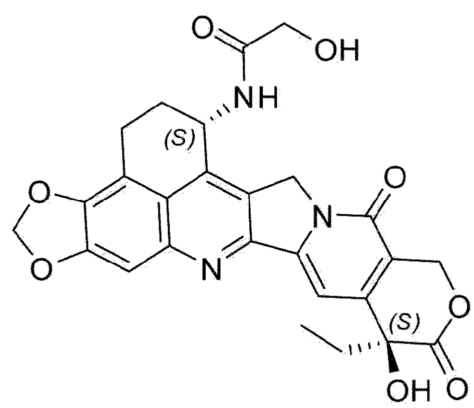

[Chemical Formula 4-1]

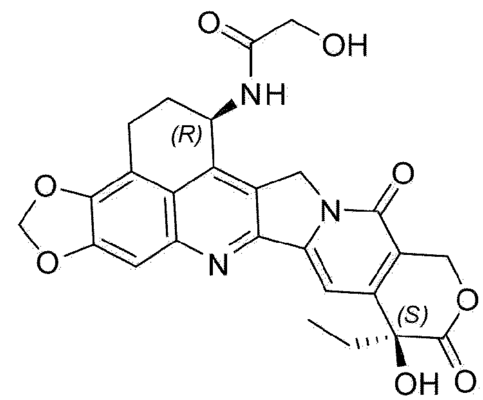

[Chemical Formula 5]

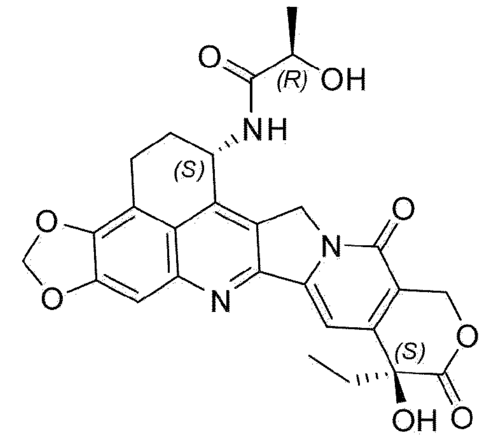

[Chemical Formula 5-1]

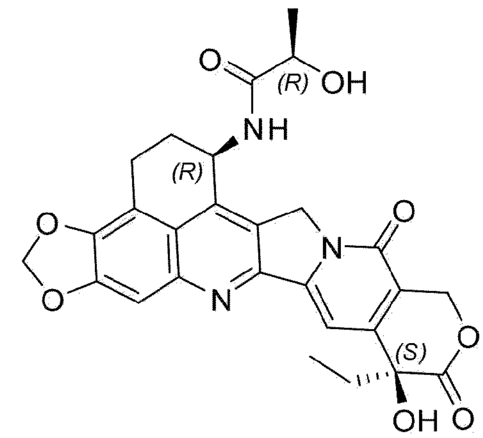

[Chemical Formula 6]

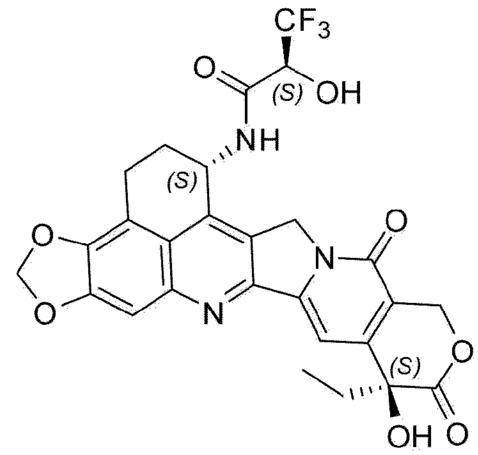

[Chemical Formula 7]

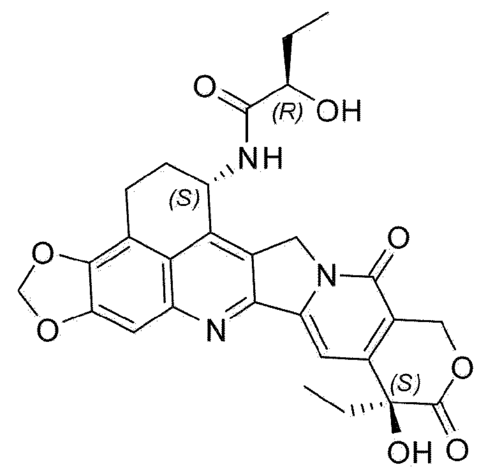

[Chemical Formula 8]

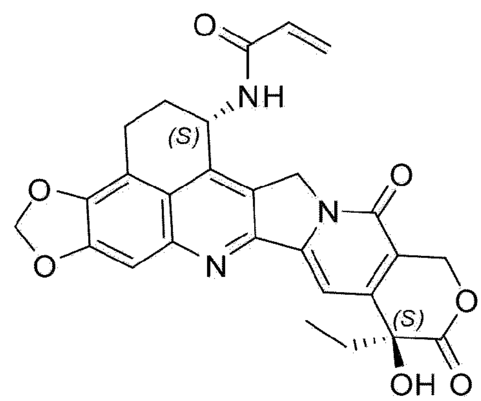

[Chemical Formula 9]

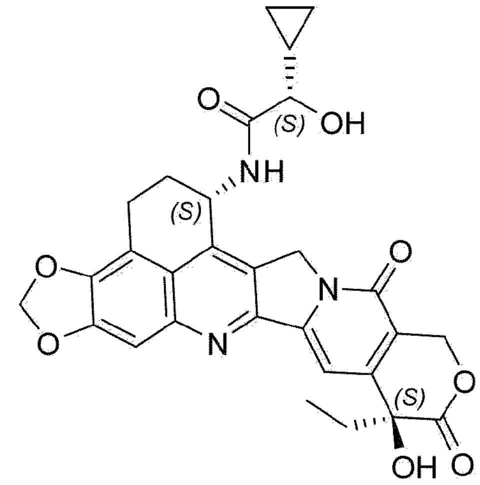

25. A pharmaceutical composition for preventing or treating cancer, including a compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, a pharmaceutically acceptable salt thereof, a solvate thereof, or a prodrug thereof, preferably an antibody-drug conjugate (ADC):

[Chemical Formula 3]

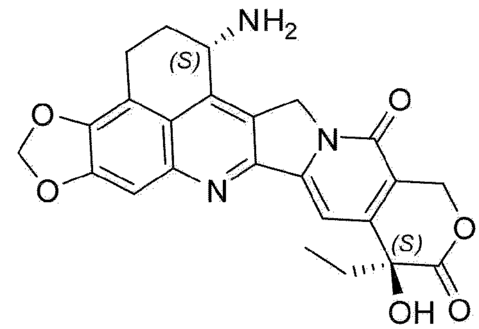

[Chemical Formula 3-1]

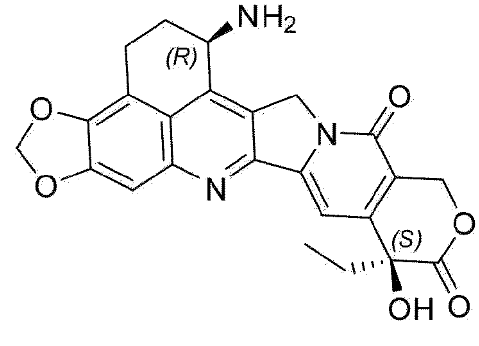

[Chemical Formula 4]

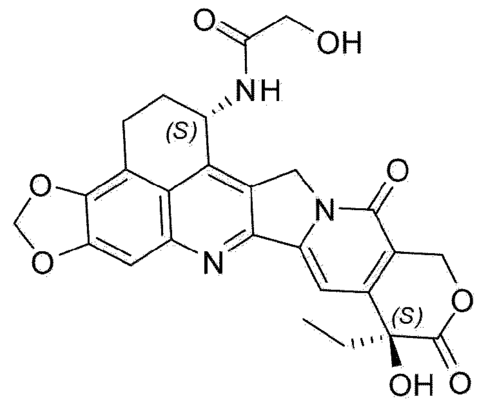

[Chemical Formula 4-1]

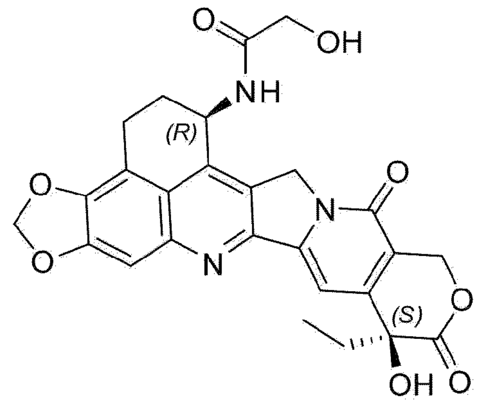

[Chemical Formula 5]

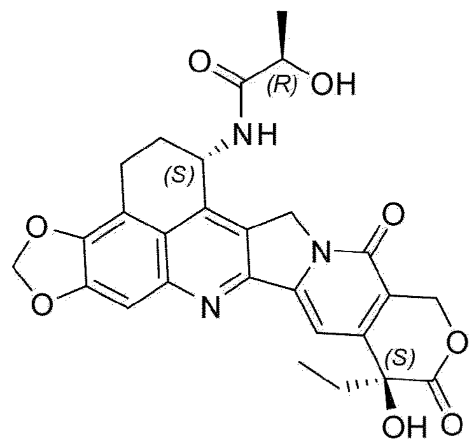

[Chemical Formula 5-1]

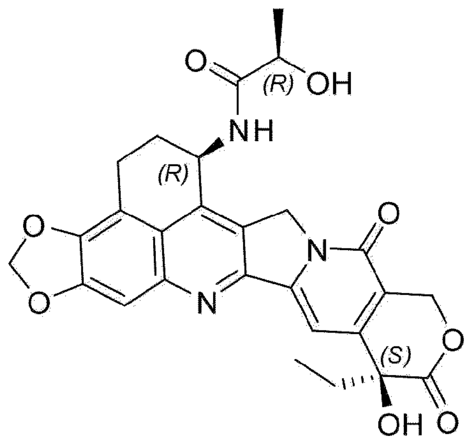

[Chemical Formula 6]

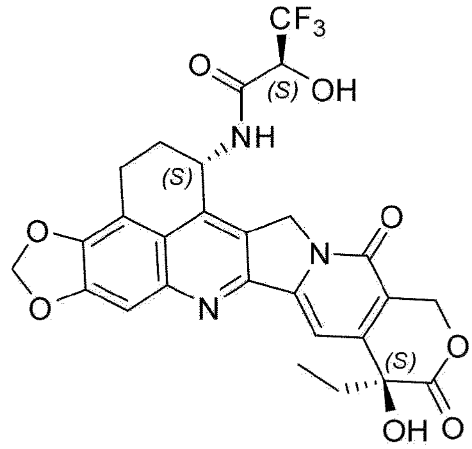

[Chemical Formula 7]

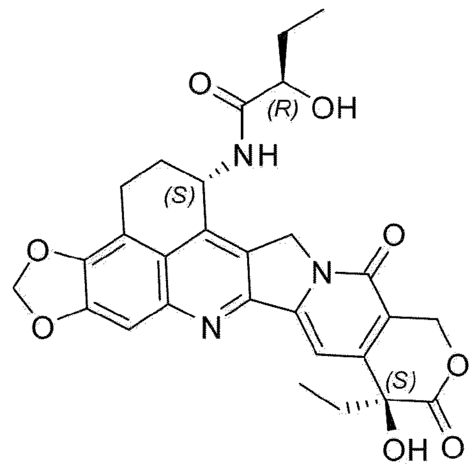

[Chemical Formula 8]

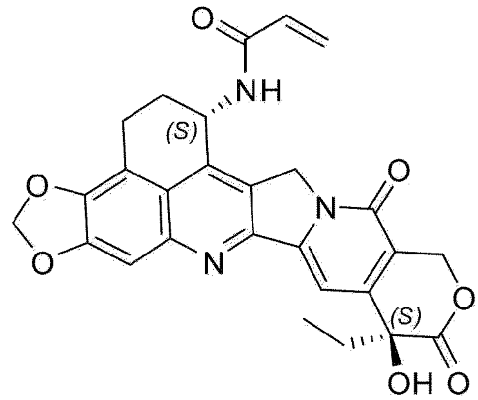

[Chemical Formula 9]

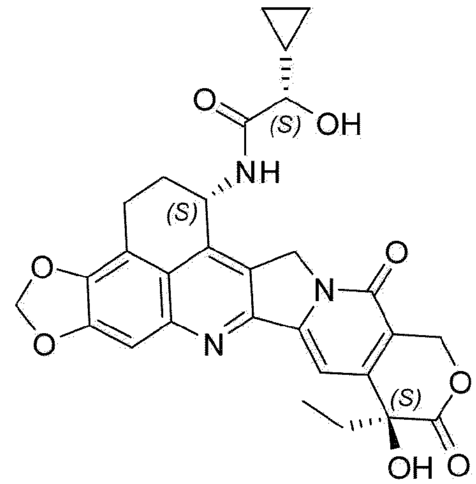

**26.** A pharmaceutical composition for cell death, including (a) an active camptothecin derivative represented by Chemical Formula 1 below, which is designed to bind to DDX5 protein and E3 ligase; or (b) a prodrug thereof, preferably an antibody-drug conjugate (ADC) thereof, which is designed to release the active camptothecin derivative (a) at a target site in vivo:

[Chemical Formula 1]

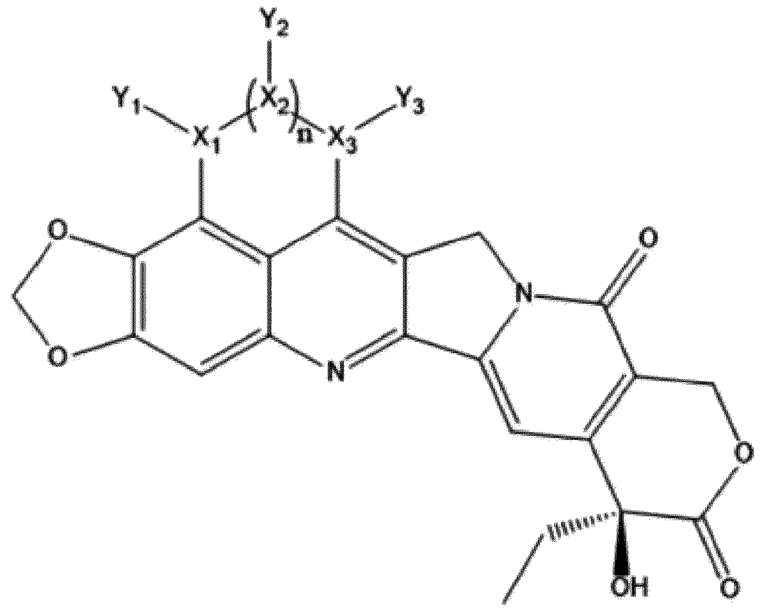

wherein $X_1$ and $X_3$ are each independently carbon, oxygen, nitrogen, or sulfur, and $X_1$ and $X_3$ may be the same or different,
$X_2$ is carbon, oxygen, nitrogen, sulfur, a single bond, or a double bond,
$X_1$, $(X_2)n$, and $X_3$ may form a six-membered or seven-membered ring (n=1 to 2),
$Y_1$, $Y_2$, and $Y_3$ may each independently be hydrogen, or a functional group containing oxygen, nitrogen, phosphorus, or sulfur.

**27.** The pharmaceutical composition for cell death according to claim 26, wherein the active camptothecin derivative (a) designed to bind to DDX5 protein and E3 ligase kills target cells expressing DDX5 protein through a molecular glue degrader mechanism of action (MoA).

**28.** The pharmaceutical composition for cell death according to claim 26, wherein the active camptothecin derivative (a) designed to bind to DDX5 protein and E3 ligase has a mechanism of action (MoA) that degrades oncoprotein DDX5 together with the ability to inhibit type 1 topoisomerase.

**29.** The pharmaceutical composition for cell death according to claim 26, wherein the active camptothecin derivative is designed to exert a surrounding cell killing effect (bystander effect) in cancer tissue or control the degree of the effect by modifying $X_1$, $(X_2)_n$, $X_3$, $Y_1$, $Y_2$ and/or $Y_3$ to control a hydrophobicity or cell membrane permeability thereof as desired.

**30.** The pharmaceutical composition for cell death according to claim 27, wherein the target cells are cancer cells or senescent cells.

**31.** An anticancer formulation including: (a) an active camptothecin derivative represented by Chemical Formula 1 below, which is designed to bind to DDX5 protein and E3 ligase; or (b) a prodrug thereof, preferably an antibody-drug conjugate (ADC) thereof, which is designed to release the active camptothecin derivative (a) at a target site in vivo, which is administered in a dosage capable of stimulating antigen-presenting cells (APCs) through cell death of cancer cells to induce an antitumor immune response.

[Chemical Formula 1]

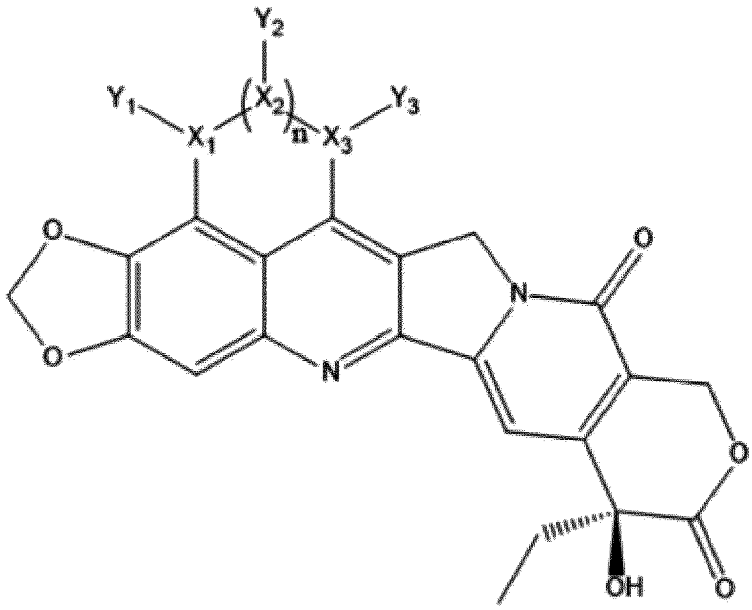

wherein $X_1$ and $X_3$ are each independently carbon, oxygen, nitrogen, or sulfur, and $X_1$ and $X_3$ may be the same or different,

$X_2$ is carbon, oxygen, nitrogen, sulfur, a single bond, or a double bond,

$X_1$, $(X_2)n$, and $X_3$ may form a six-membered or seven-membered ring (n=1 to 2),

$Y_1$, $Y_2$, and $Y_3$ may each independently be hydrogen, or a functional group containing oxygen, nitrogen, phosphorus, or sulfur.

**32.** The anticancer formulation according to claim 31, wherein the anticancer formulation is designed to target tumor tissue.

**33.** A method for preparing an active camptothecin derivative (a)-based anticancer agent in which a surrounding cell killing effect (bystander effect) and/or ADME profile of the active camptothecin derivative (a) are controlled so that T cell exhaustion does not occur due to chronic antigen exposure, or so that the active camptothecin derivative (a) is rapidly or slowly removed via lymphatic drainage by providing a desired degree of hydrophobicity and/or controlling aggregation,

wherein the active camptothecin derivative (a)-based anticancer agent is an anticancer formulations including (a) an active camptothecin derivative designed to bind to DDX5 protein and E3 ligase as a molecular glue degrader; or (b) a prodrug thereof, preferably an antibody-drug conjugate (ADC) thereof, which is designed to release the active camptothecin derivative (a) at a target site in vivo,

the method including a step of designing the active camptothecin derivative (a) or the prodrug thereof (b) to include a camptothecin-based skeleton represented by Chemical Formula 1 below as a parent nucleus, selecting a compound designed in this way, or synthesizing a compound designed in this way:

[Chemical Formula 1]

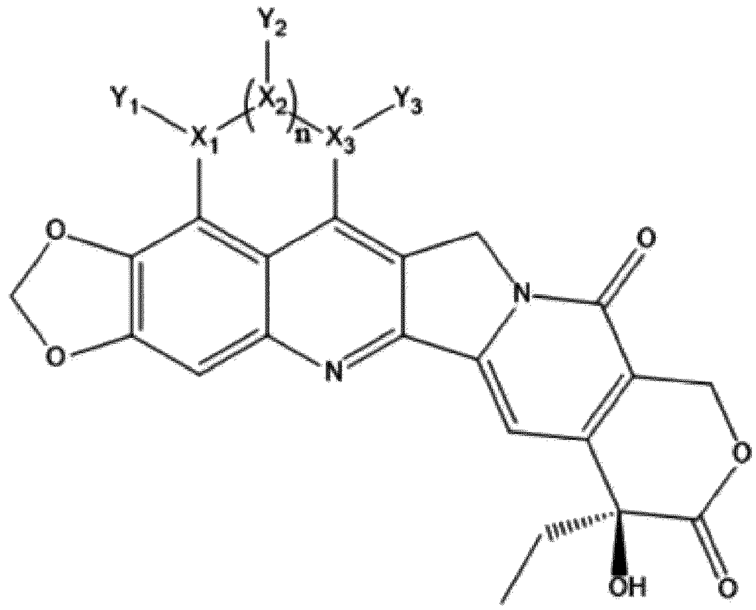

wherein $X_1$ and $X_3$ are each independently carbon, oxygen, nitrogen, or sulfur, and $X_1$ and $X_3$ may be the same or different,
$X_2$ is carbon, oxygen, nitrogen, sulfur, a single bond, or a double bond,
$X_1$, $(X_2)n$, and $X_3$ may form a six-membered or seven-membered ring (n=1 to 2),
$Y_1$, $Y_2$, and $Y_3$ may each independently be hydrogen, or a functional group containing oxygen, nitrogen, phosphorus, or sulfur.

**34.** The preparation method according to claim 33, wherein the active camptothecin derivative (a)-based anticancer agent is a formulation including (a) an active camptothecin derivative designed to have a mechanism of action (MoA) that degrades oncoprotein DDX5 together with the ability to inhibit type 1 topoisomerase, or (b) a prodrug thereof designed to release the active camptothecin derivative (a) in vivo.

**35.** A carrier-drug conjugate comprising: an active camptothecin derivative represented by Chemical Formula 1 according to claim 1, a pharmaceutically acceptable salt thereof, or a solvate thereof.

**36.** The carrier-drug conjugate according to claim 35, wherein the active camptothecin derivative of Chemical Formula 1 is selected from the group consisting of Chemical Formulas 3 to 9:

[Chemical Formula 3]

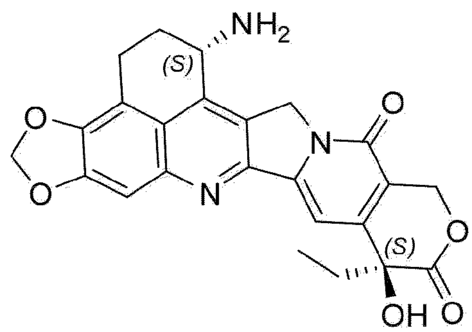

[Chemical Formula 3-1]

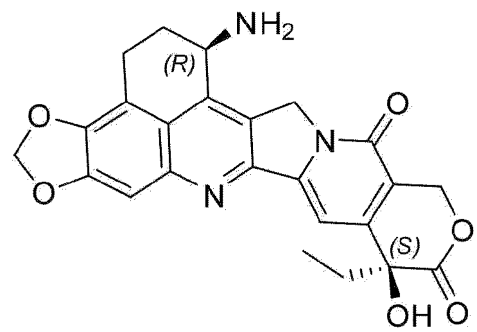

[Chemical Formula 4]

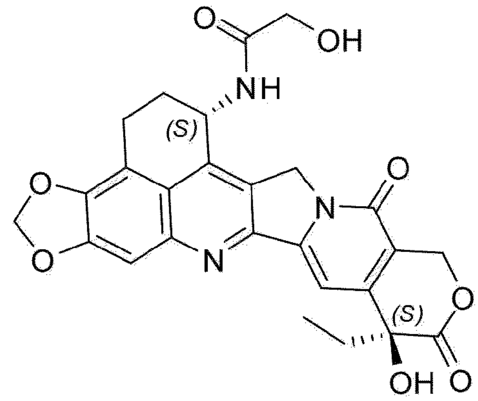

[Chemical Formula 4-1]

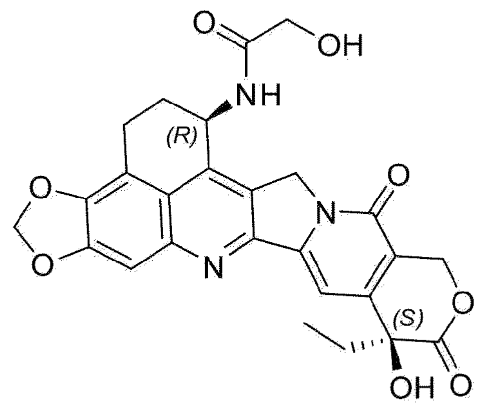

[Chemical Formula 5]

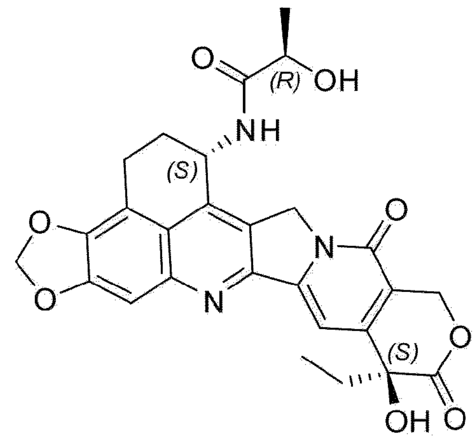

[Chemical Formula 5-1]

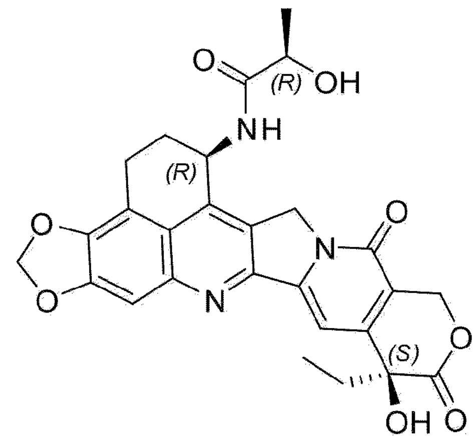

[Chemical Formula 6]

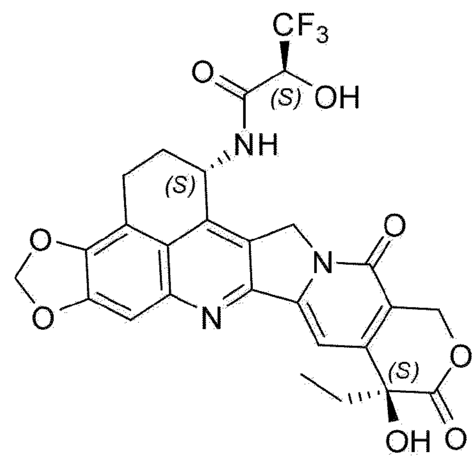

[Chemical Formula 7]

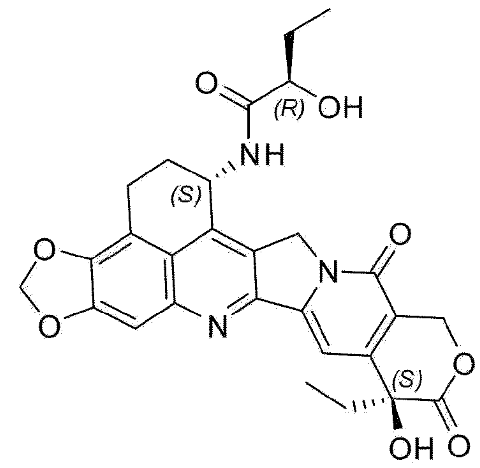

[Chemical Formula 8]

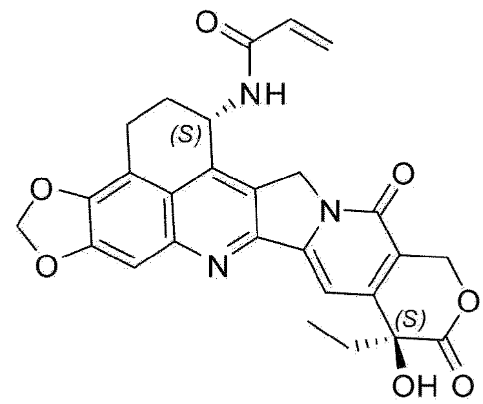

[Chemical Formula 9]

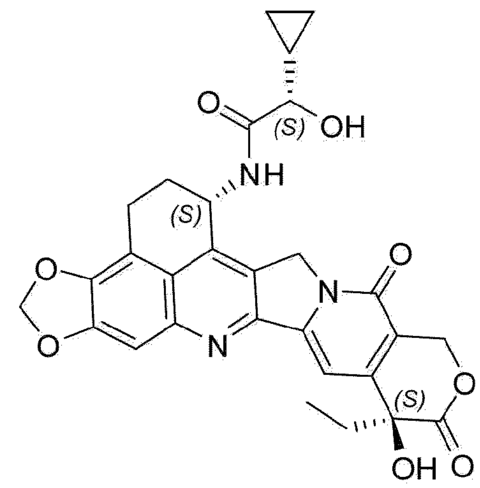

**37.** The carrier-drug conjugate according to claim 35, wherein the carrier is an antibody, a peptide, a repebody, or an

aptamer.

**38.** The carrier-drug conjugate according to claim 37, wherein the carrier-drug conjugate is in a form in which the carrier is conjugated to the camptothecin derivative represented by Chemical Formula 1 via a linker.

**39.** The carrier-drug conjugate according to claim 38, wherein the linker comprises GGFG.

**40.** The carrier-drug conjugate according to claim 37, wherein the carrier is an antibody, a peptide, a repebody, or an aptamer that specifically binds to one or more substances (antigens) selected from the group consisting of 4-1BB, 5T4, integrin, activin, amyloid beta, angiopoietin (angiopoietin 1 or 2), angiopoietin-like substance 3, B cell maturation antigen (BCMA), B-cell activating factor (BAFF), B7-H3, complement 5, CCR4, CCR5, CCL11, CD2, CD3, CD4, CD6, CD11a, CD16A, CD19, CD20, CD22, CD25, CD27, CD28, CD30, CD32B, CD33, CD38, CD40, CD45, CD46, CD47, CD52, CD56, CD62, CD70, CD73, CD74, CD79b, CD80, CD105, CD123, CD154, CD166, CD262, CD278, CD319, CD326, carcinoembryonic antigen (CEA), CGRP, claudin-18, c-Met, CSF-1, CSF-1 receptor, CTLA4, DLL3, EGF receptor, hemophilia factor, Fc receptor, FGF23, folate receptor, GD2, glucocorticoid-induced TNF receptor (GITR), glypican 3, GM-CSF, HER2, HER3, TROP2, hepatocyte growth factor (HGF), interferon receptor, interferon gamma, IgE, IGF-1 receptor, interleukin 1, interleukin 2 receptor, interleukin 4, interleukin 4 receptor, interleukin 5, interleukin 5 receptor, interleukin 6, interleukin 6 receptor, interleukin 8, interleukin 12/23, interleukin 13, interleukin 17A, interleukin 17 receptor A, interleukin 23, interleukin 31 receptor, interleukin 36 receptor, lymphocyte-activation gene 3 (LAG3), lysyl oxidase homolog 2 (LOXL2), mesothelin, mucin-1, mucin-16, Netin-4, nerve growth factor (NGF), OX40, proprotein convertase subtilisin/kexin type 9 (PCSK9), PD-1, PD-L1, phospholipase C, receptor activator of nuclear factors kappa B ligand (RANKL), tyrosine-protein kinase transmembrane receptor (ROR1), sialic acid binding ig-like lectin 15 (Siglec-15), transforming growth factor beta (TGFβ), T-cell innunoreceptor with immunoglobulin and ITIM domain (TIGIT), T cell immunoglobulin and mucin-domain containing-3 (Tim-3), tissue factor, tissue factor pathway inhibitor (TFPI), TORP-2, tumor necrosis factor (TNF), thymic stromal lymphopoietin (TSLB), colony stimulating factor 1 receptor (CSF1R), vascular endothelial growth factor (VEGF), VEGF receptor, and von Willebrand factor (vWF).

**41.** The carrier-drug conjugate according to claim 37, wherein the carrier is an antibody.

**42.** The carrier-drug conjugate according to claim 41, wherein the antibody is at least one selected from the group consisting of Urelumab, Utomilumab, Bebtelovimab, Aducanumab, Bapinezumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, and Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Dapirolizumab, Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Datopotamab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, Dupilumab, Defemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Fabezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociferlimab, Tiragolumab, Vibostolimab, Surzebiclimab, Cobolimab, Sabatolimab, Concizumab, Marstacimab,

Adalimumab, Golimumab, Infliximab, Certolizumab, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab, Obrindatamab, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Naptumomab, Belantamab, Pivekimab, Praluzatamab, Coltuximab, Denintuzumab, Loncastuximab, Ibritumomab, Inotuzumab, Epratuzumab, Moxetumomab, Brentuximab, Gemtuzumab, Vadastuximab, Lorvotuzumab, Polatuzumab, Tusamitamab, Telisotuzumab, Rovalpituzumab, Depatuxizumab, Farletuzumab, Mirvetuximab, Disitamab, Anetumab, Enfortumab, Sacituzumab, Vobarilizumab, Cadonilimab, Vudalimab, Tebotelimab, Ivonescimab, Erfonrilimab, Ozoralizumab, Faricimab, Vanucizumab, and Navicixizumab.

**43.** A pharmaceutical composition for preventing or treating cancer, including the carrier-drug conjugate according to any one of claims 35 to 42.

**44.** The pharmaceutical composition according to claim 43, wherein the cancer is at least one selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, bile duct cancer, colon cancer, chronic myeloid leukemia, chronic lymphocytic leukemia, retinoblastoma, choroidal melanoma, ampulla of Vater, bladder cancer, peritoneal cancer, parathyroid cancer, adrenal cancer, paranasal sinus cancer, non-small cell lung cancer, tongue cancer, astrocytoma, small cell lung cancer, pediatric brain cancer, pediatric lymphoma, pediatric leukemia, small intestinal cancer, meningioma, esophageal cancer, glioma, renal pelvis cancer, kidney cancer, cardiac cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms' cancer, breast cancer, triple negative breast cancer (TNBC), sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic neuroma, pancreatic cancer, salivary gland cancer, Kaposi sarcoma, Paget's disease, tonsil cancer, squamous cell carcinoma, pulmonary adenocarcinoma, lung cancer, lung squamous cell carcinoma, skin cancer, anal cancer, rhabdomyosarcoma, laryngeal cancer, pleural cancer, blood cancer, and thymic cancer.

[FIG. 1]

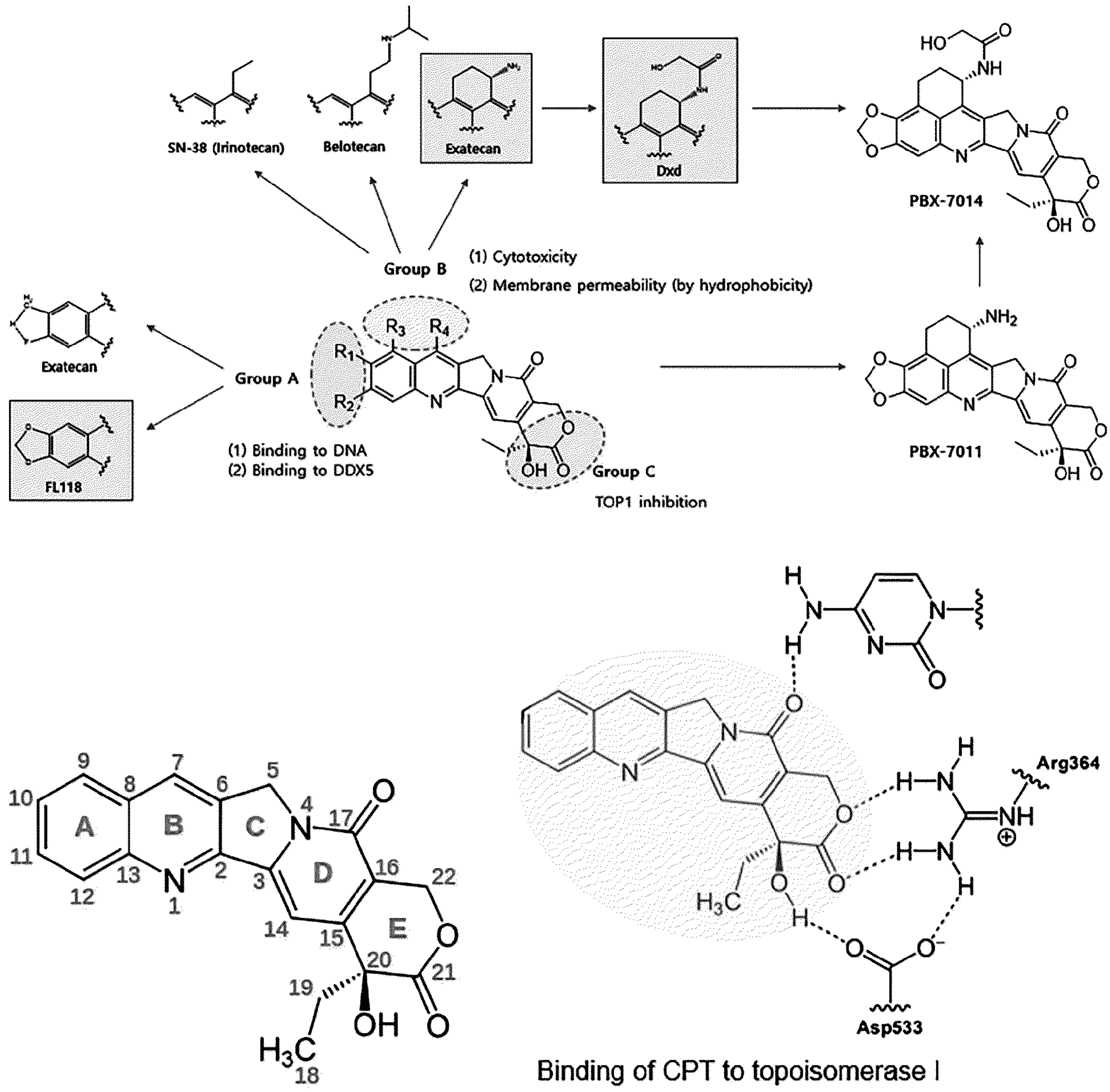


Binding of CPT to topoisomerase I

[FIG. 2]

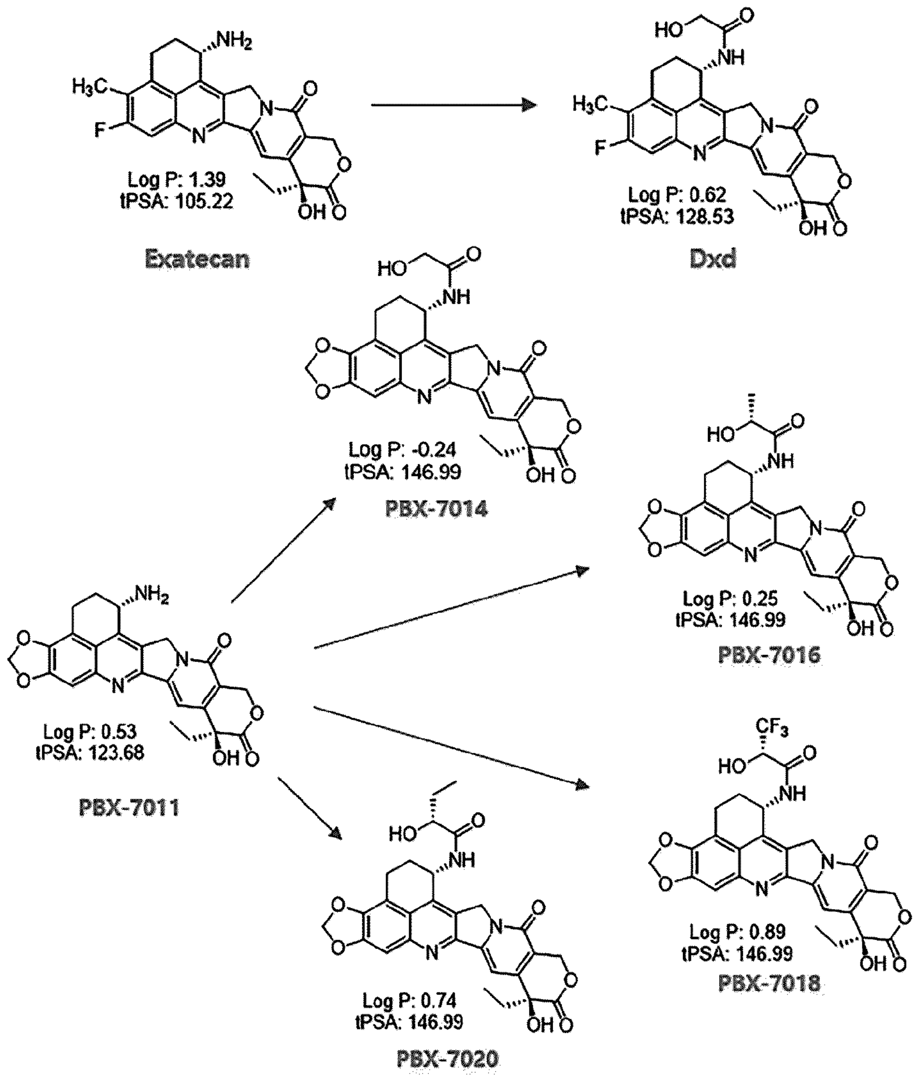
Log P: 1.39
tPSA: 105.22
Exatecan

Log P: 0.62
tPSA: 128.53
Dxd

Log P: -0.24
tPSA: 146.99
PBX-7014

Log P: 0.25
tPSA: 146.99
PBX-7016

Log P: 0.53
tPSA: 123.68
PBX-7011

Log P: 0.89
tPSA: 146.99
PBX-7018

Log P: 0.74
tPSA: 146.99
PBX-7020

| Compound | LogP | tPSA | Remarks |
|---|---|---|---|
| Exatecan | 1.39 | 105.22 | |
| **Dxd** | **0.62** | **128.53** | Exatecan + Glycolic acid |
| PBX-7011 | 0.53 | 123.68 | |
| **PBX-7014** | -0.24 | 146.99 | PBX-7011 + Glycolic acid |
| **PBX-7016** | **0.25** | **146.99** | |
| PBX-7018 | 0.89 | 146.99 | |
| PBX-7020 | **0.74** | **146.99** | |

* LogP and tPSA values were calculated by ChemDraw based on 2D structures

[FIG. 3]

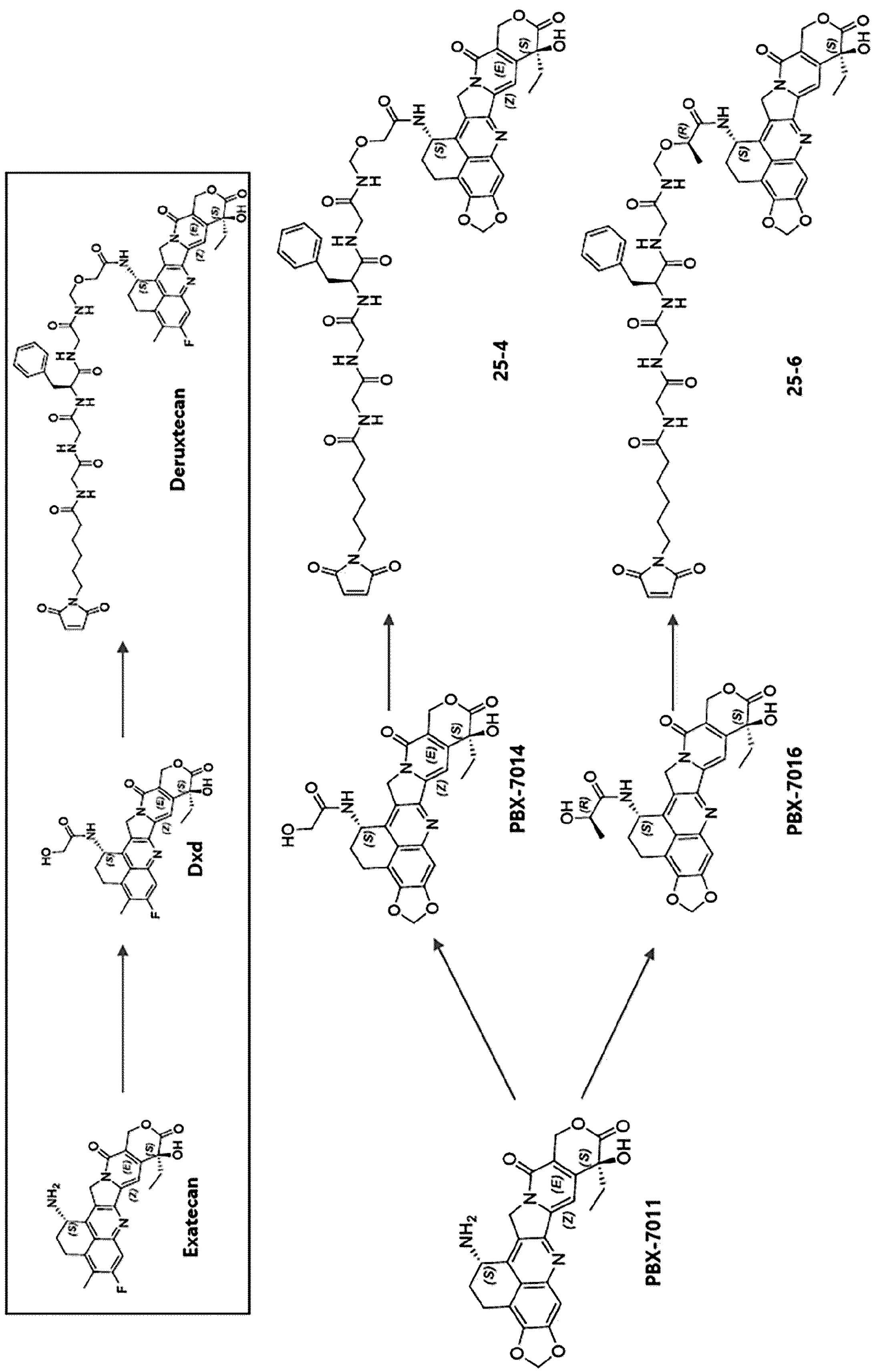

Exatecan

Dxd

Deruxtecan

PBX-7014

25-4

PBX-7016

25-6

PBX-7011

[FIG. 4]

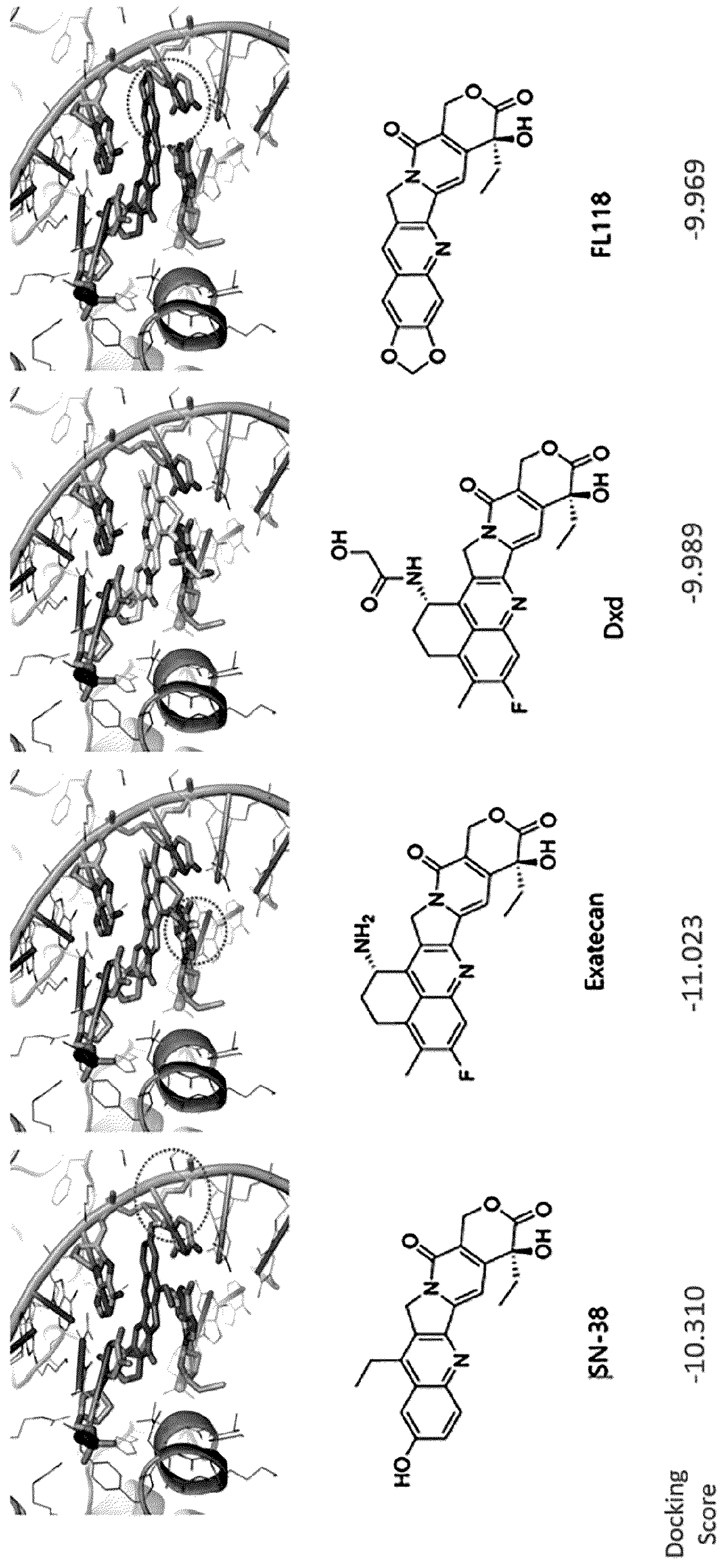
FL118
-9.969
Dxd
-9.989
Exatecan
-11.023
SN-38
-10.310
Docking Score

[FIG. 5]

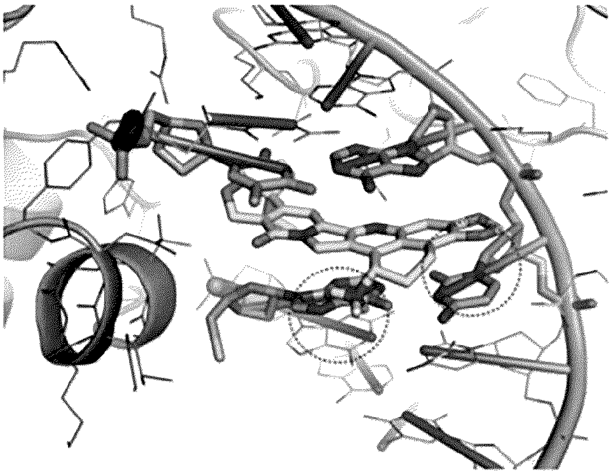

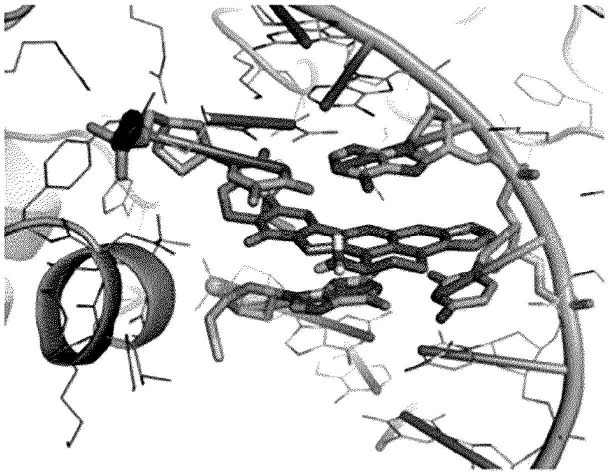

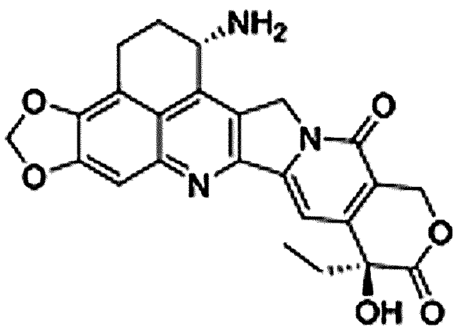

Hybrid-1 (isomer A)

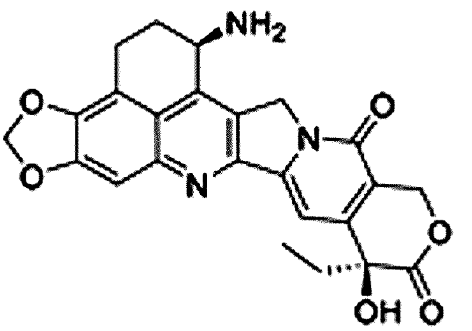

Hybrid-1 (isomer B)

| | | |
|---|---|---|
| Docking Score | -11.328 | -10.358 |

[FIG. 6]

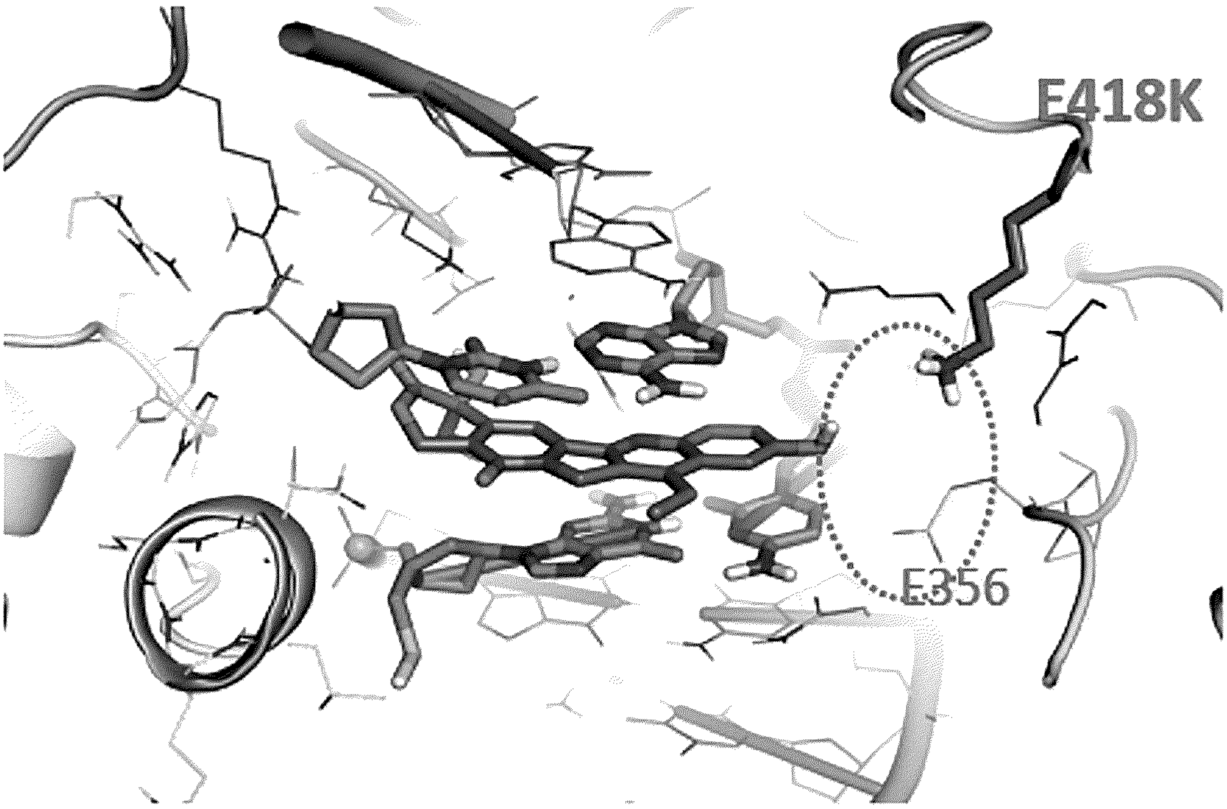

[FIG. 7]

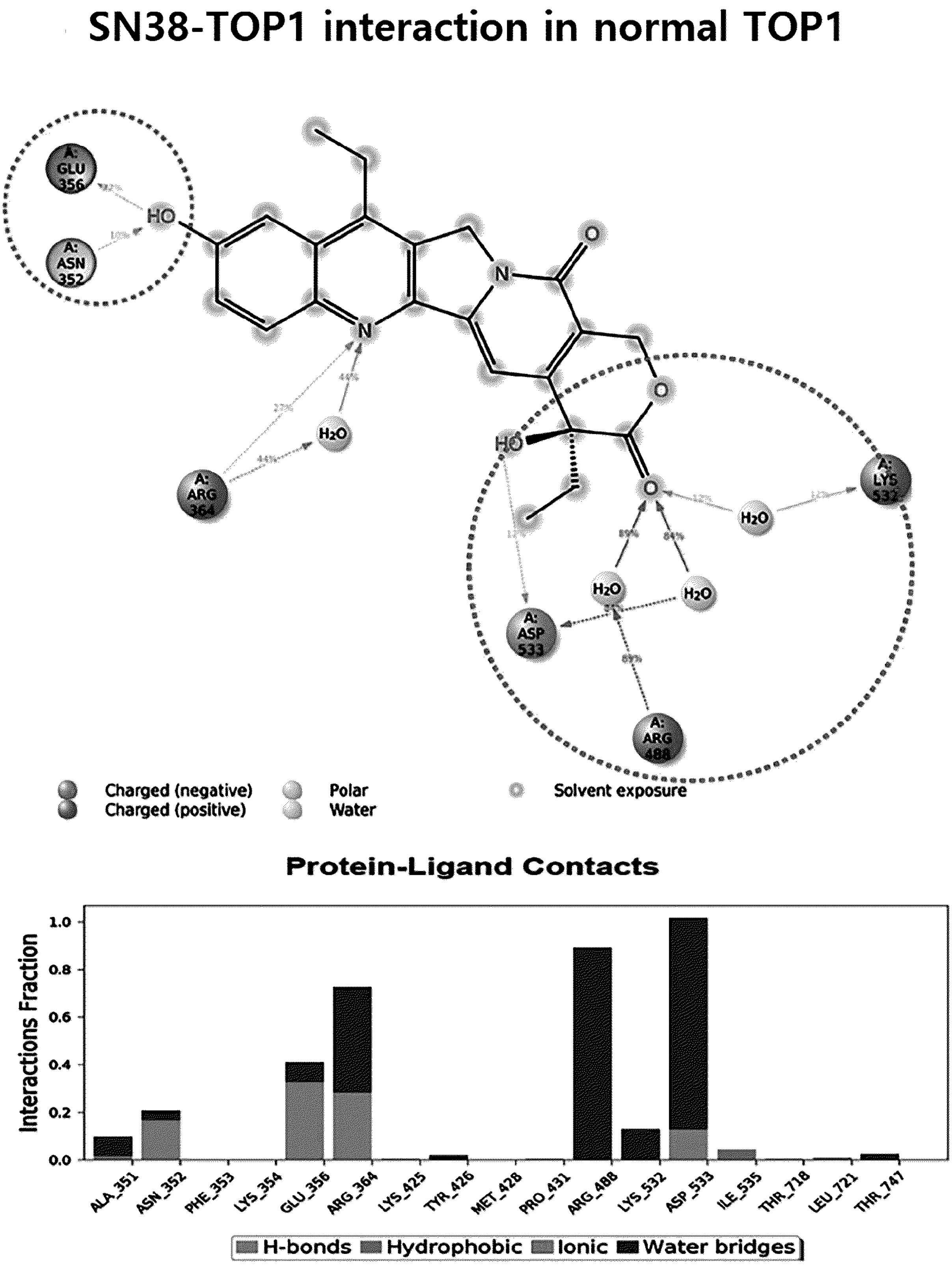
SN38-TOP1 interaction in normal TOP1
Charged (negative)
Charged (positive)
Polar
Water
Solvent exposure
Protein-Ligand Contacts
Interactions Fraction
1.0
0.8
0.6
0.4
0.2
0.0
ALA_351
ASN_352
PHE_353
LYS_354
GLU_356
ARG_364
LYS_425
TYR_426
MET_428
PRO_431
ARG_488
LYS_532
ASP_533
ILE_535
THR_718
LEU_721
THR_747
H-bonds
Hydrophobic
Ionic
Water bridges

[FIG. 8]

# SN38-TOP1 interaction in E418k mutated TOP1

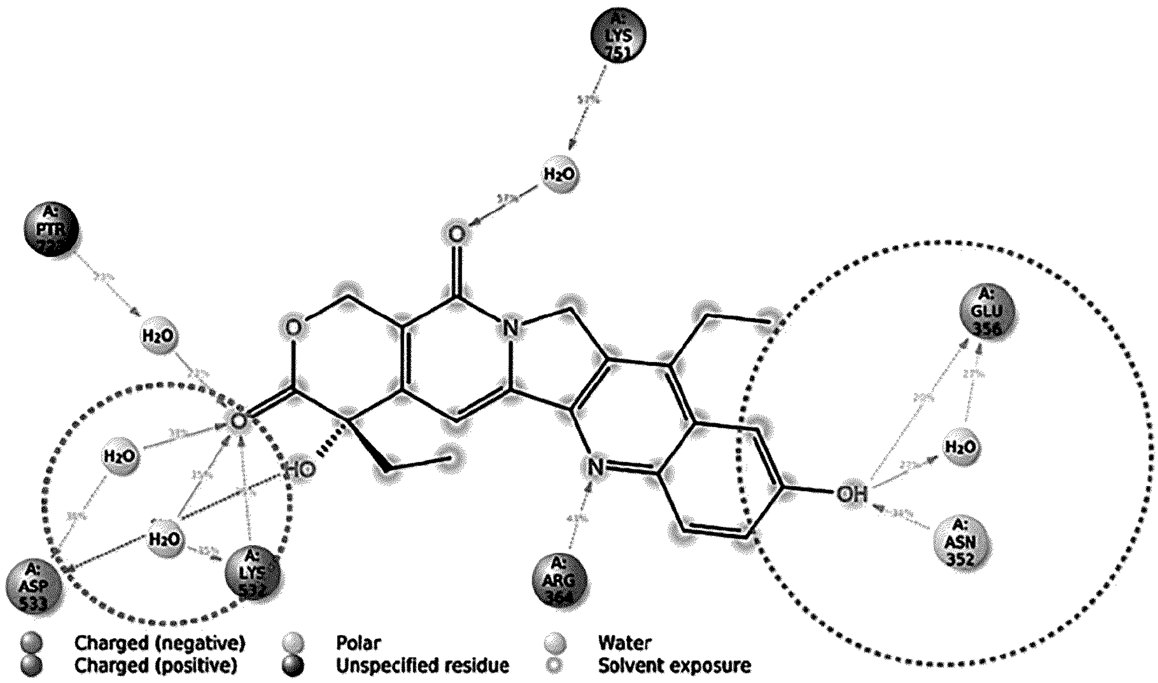


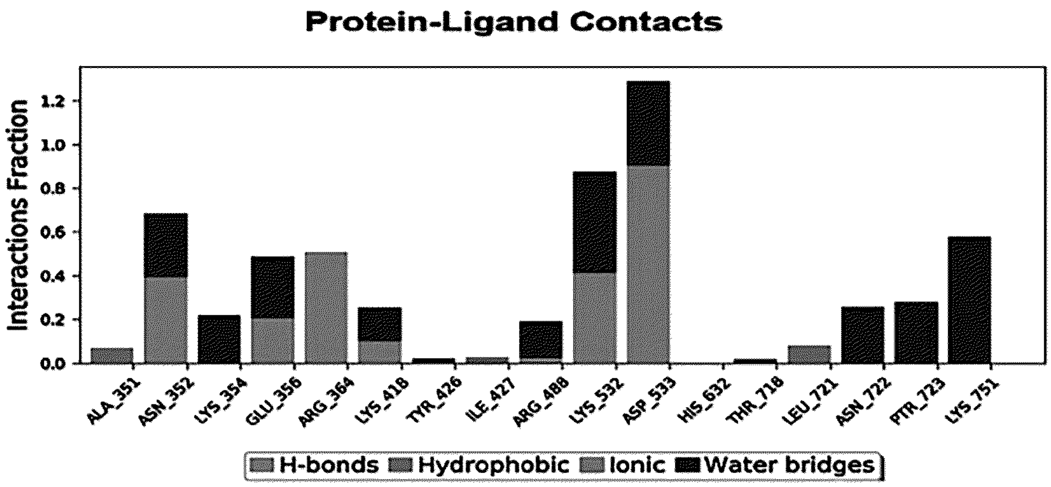

[FIG. 9]

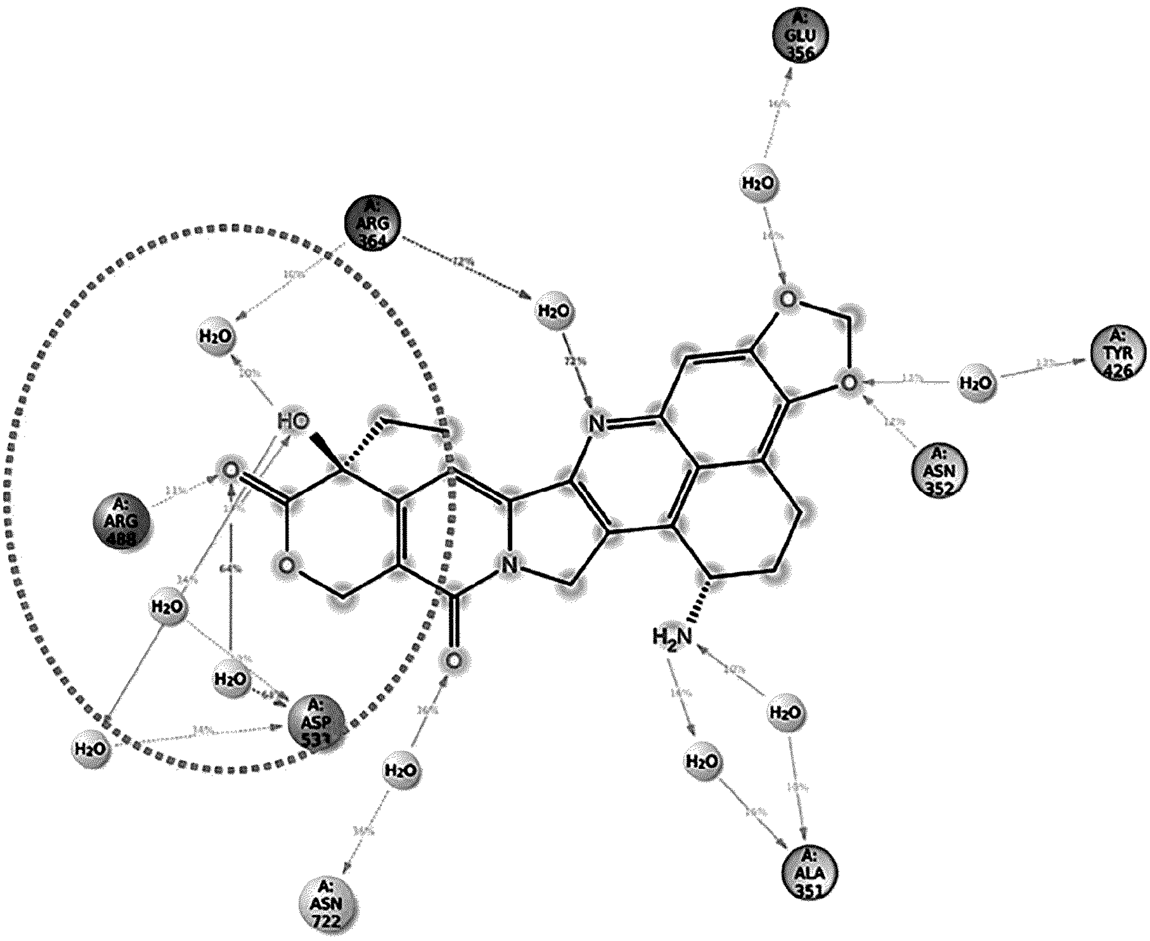

Charged (negative) Hydrophobic Water
Charged (positive) Polar Solvent exposure

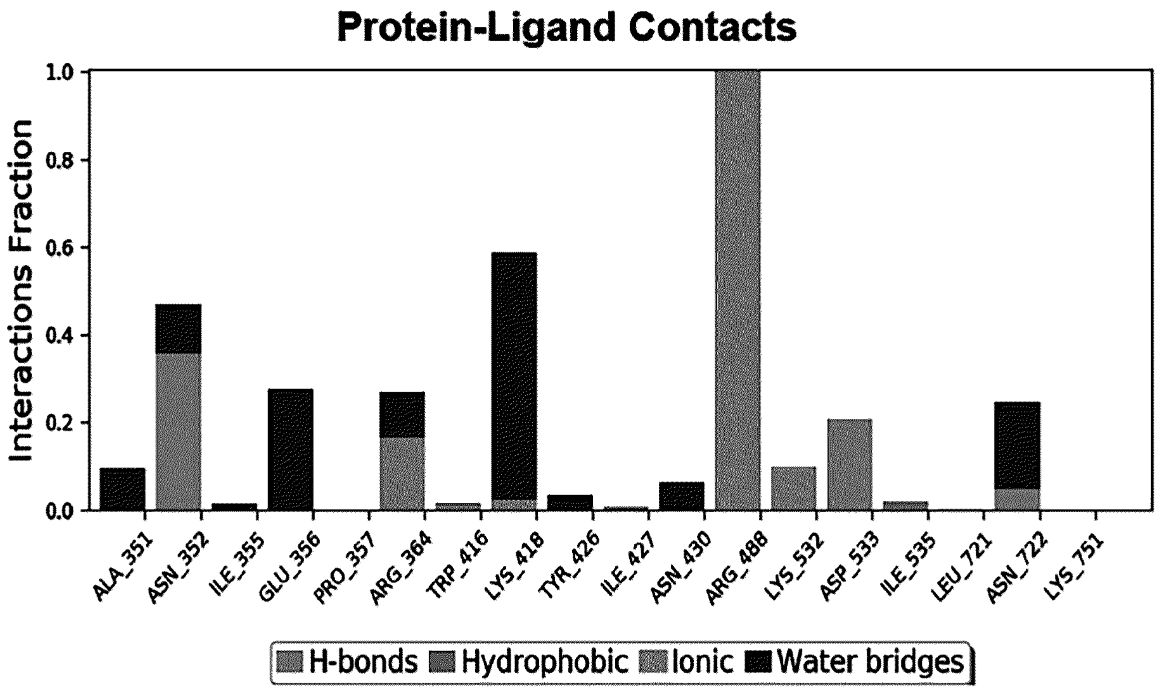

[FIG. 10]

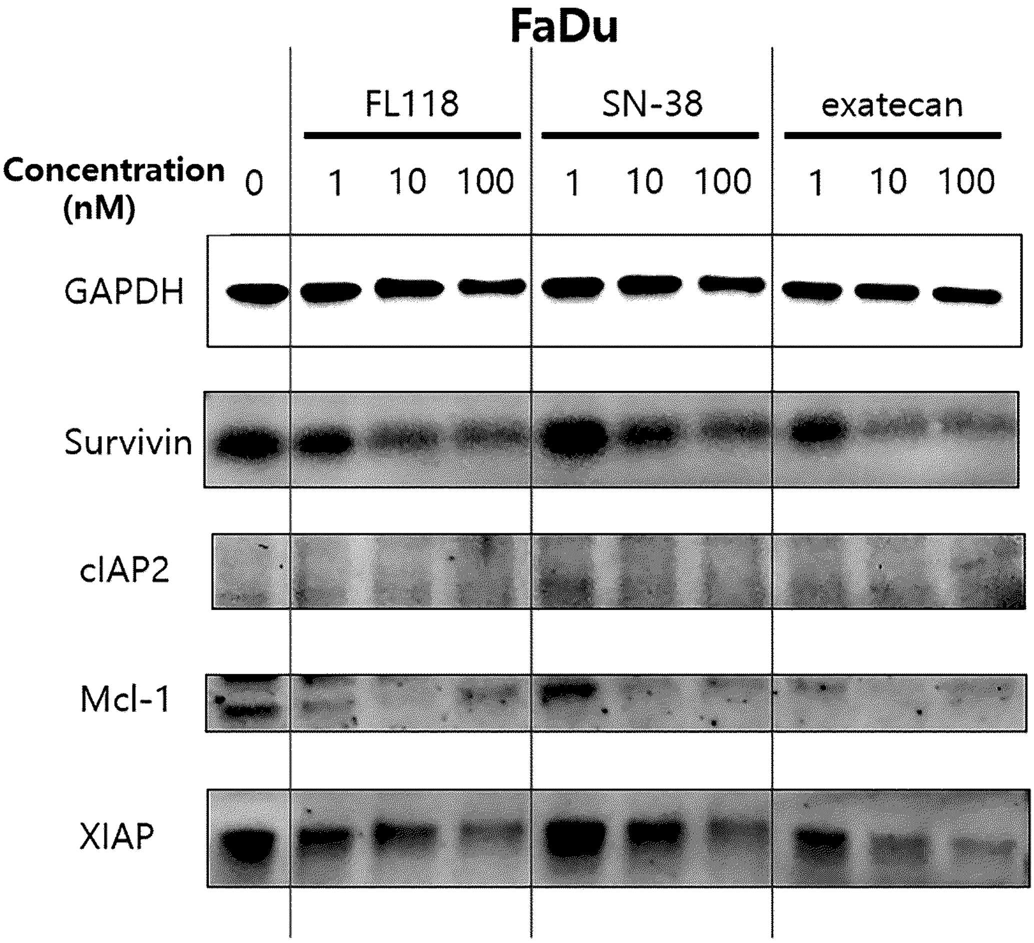
FaDu
FL118
SN-38
exatecan
Concentration (nM)
0
1 10 100
1 10 100
1 10 100
GAPDH
Survivin
cIAP2
Mcl-1
XIAP

[FIG. 11]

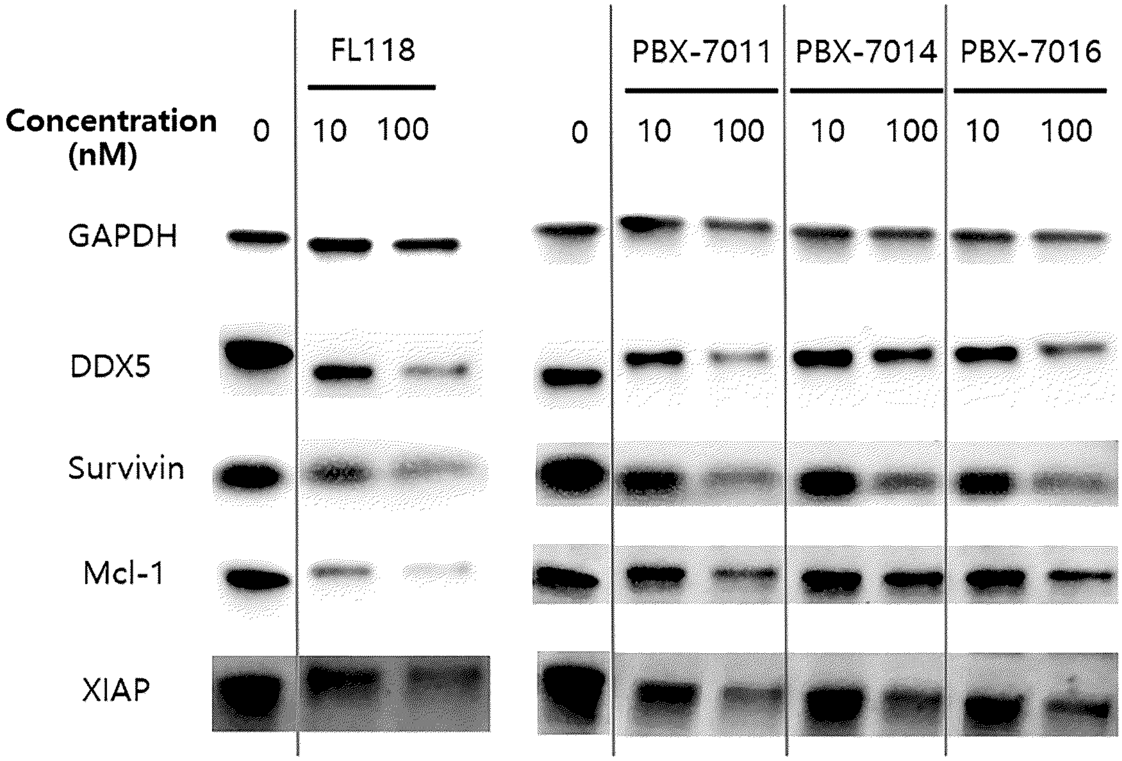
FL118
PBX-7011
PBX-7014
PBX-7016
Concentration (nM)
0
10
100
0
10
100
10
100
10
100
GAPDH
DDX5
Survivin
Mcl-1
XIAP

[FIG. 12]

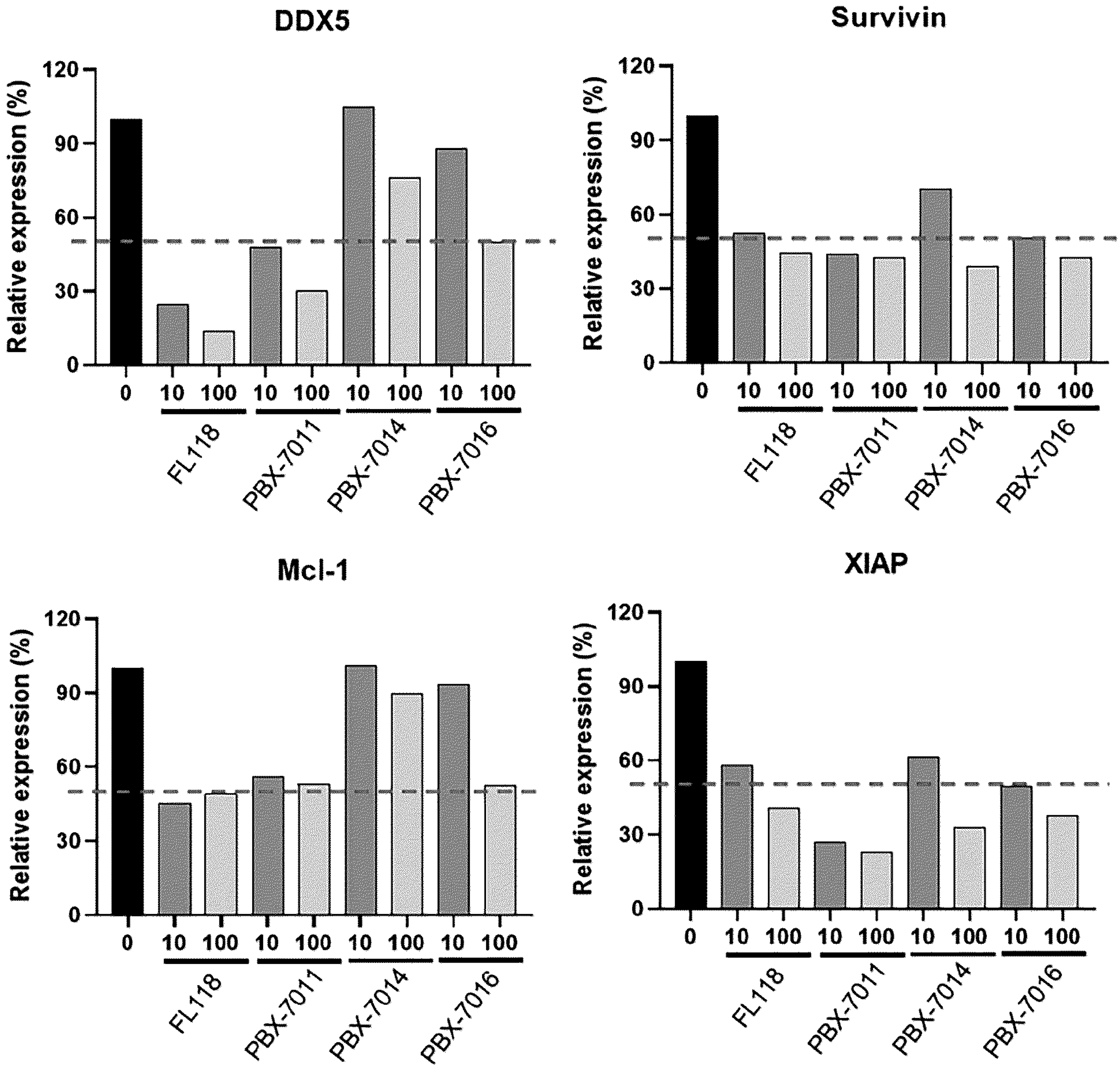
DDX5
Survivin
Mcl-1
XIAP
Relative expression (%)
120
90
60
30
0
0
10
100
FL118
PBX-7011
PBX-7014
PBX-7016

[FIG. 13]

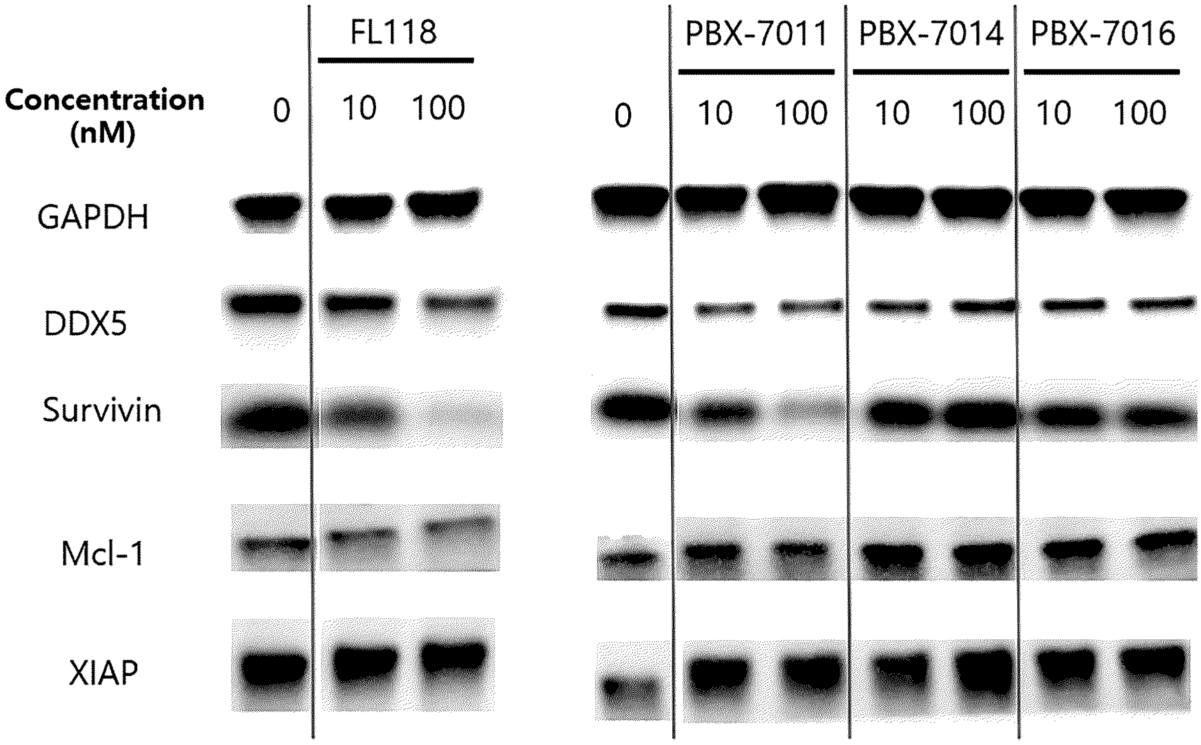
FL118
PBX-7011
PBX-7014
PBX-7016
Concentration (nM)
0
10
100
0
10
100
10
100
10
100
GAPDH
DDX5
Survivin
Mcl-1
XIAP

[FIG. 14]

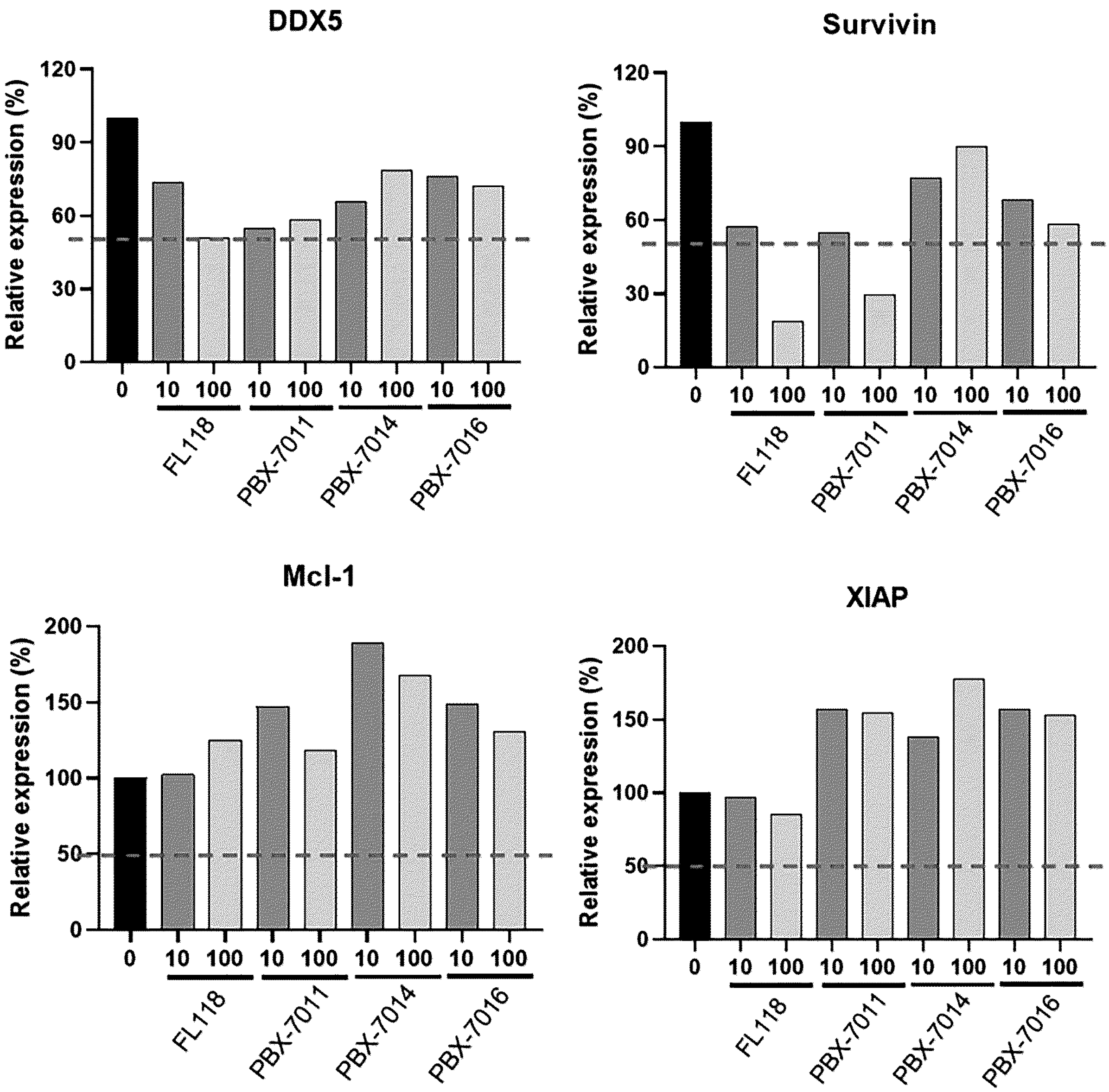
DDX5
Survivin
Mcl-1
XIAP
Relative expression (%)
0
10
100
FL118
PBX-7011
PBX-7014
PBX-7016

[FIG. 15]

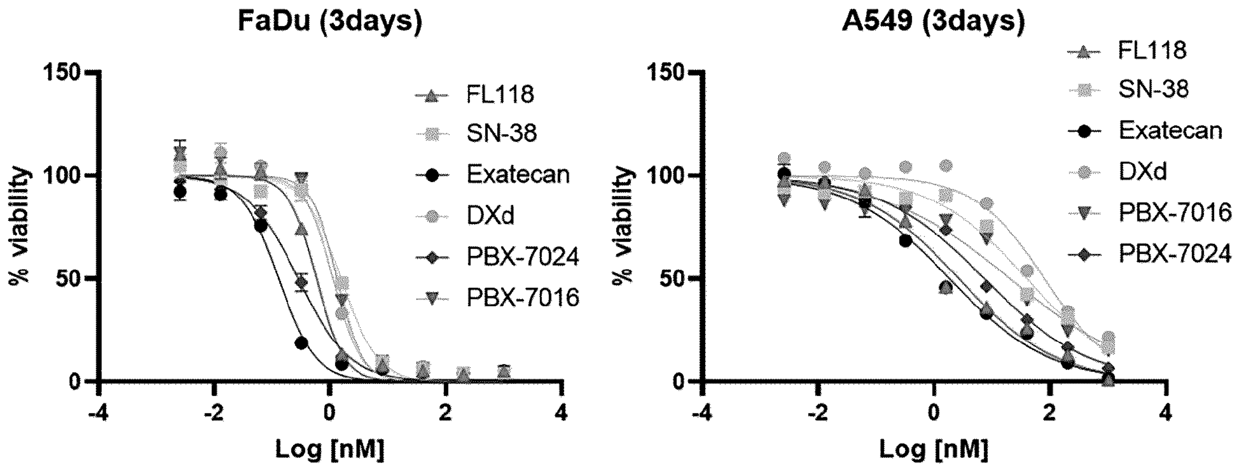


| Drug | $IC_{50}$ (nM) |
|---|---|
| FL118 | 0.5844 |
| SN-38 | 1.555 |
| Exatecan | 0.1298 |
| DXd | 1.130 |
| PBX-7016 | 1.331 |
| PBX-7024 | 0.2841 |

| Drug | $IC_{50}$ (nM) |
|---|---|
| FL118 | 2.670 |
| SN-38 | 39.70 |
| Exatecan | 1.845 |
| DXd | 80.25 |
| PBX-7016 | 20.47 |
| PBX-7024 | 7.387 |

[FIG. 16]

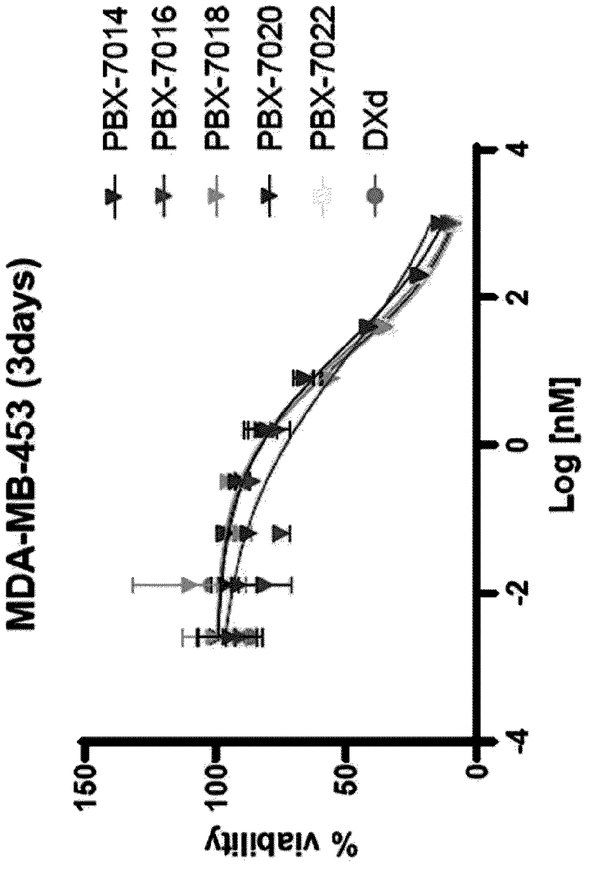


| Drug | $IC_{50}$ (nM) |
|---|---|
| PBX-7014 | 22.35 |
| PBX-7016 | 14.98 |
| PBX-7018 | 14.91 |
| PBX-7020 | 15.78 |
| PBX-7022 | 16.33 |
| DXd | 18.48 |

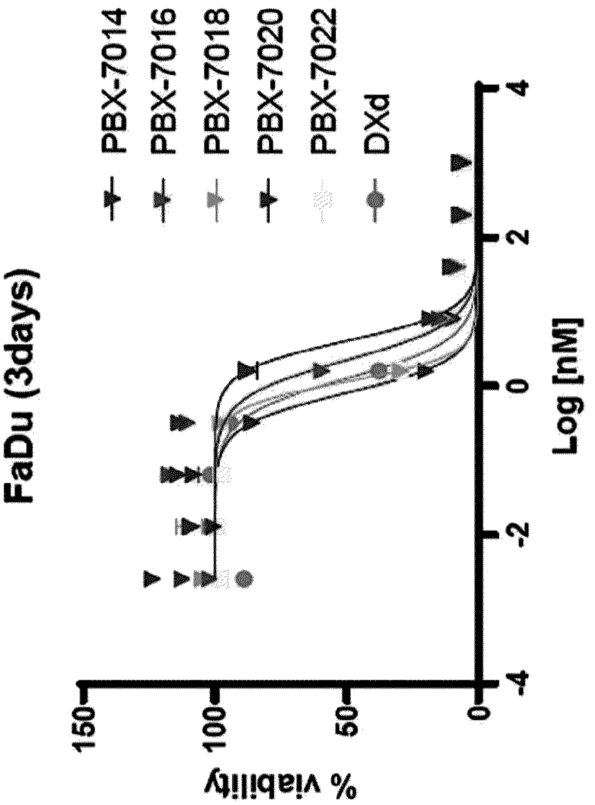


| Drug | $IC_{50}$ (nM) |
|---|---|
| PBX-7014 | 4.037 |
| PBX-7016 | 2.223 |
| PBX-7018 | 1.145 |
| PBX-7020 | 0.8072 |
| PBX-7022 | 1.145 |
| DXd | 1.262 |

[FIG. 17]

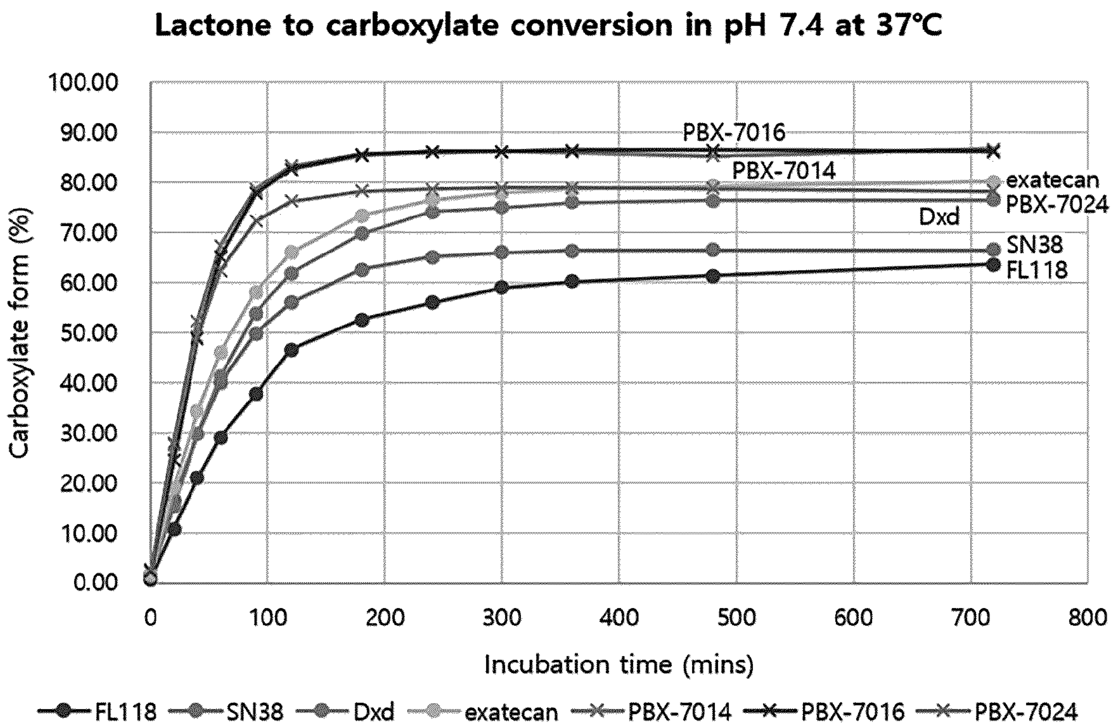


| Time (mins) | FL118 | SN38 | Dxd | exatecan | PBX-7014(1차) | PBX-7014(2차) | PBX-7014 | PBX-7016(1차) | PBX-7016 (2차) | PBX-7016 | PBX-7024(1차) | PBX-7024(2차) | PBX-7024 |
|---|---|---|---|---|---|---|---|---|---|---|---|---|---|
| 0 | 0.73 | 1.71 | 1.28 | 1.68 | 1.64 | 2.53 | 2.08 | 2.42 | 2.22 | 2.32 | 2.71 | 2.61 | 2.66 |
| 20 | 10.72 | 16.44 | 15.26 | 18.92 | 30.08 | 25.81 | 27.95 | 30.16 | 18.50 | 24.33 | 29.74 | 25.24 | 27.49 |
| 40 | 20.86 | 29.68 | 29.61 | 34.19 | 53.89 | 50.78 | 52.33 | 55.26 | 42.26 | 48.76 | 50.95 | 47.56 | 49.26 |
| 60 | 29.02 | 39.89 | 41.12 | 46.07 | 68.24 | 66.40 | 67.32 | 69.94 | 60.24 | 65.09 | 63.95 | 60.76 | 62.35 |
| 90 | 37.64 | 49.86 | 53.88 | 58.02 | 79.07 | 78.06 | 78.57 | 80.13 | 75.39 | 77.76 | 73.74 | 70.78 | 72.26 |
| 120 | 46.56 | 55.97 | 61.82 | 66.01 | 83.52 | 82.97 | 83.24 | 84.11 | 81.26 | 82.69 | 77.39 | 74.98 | 76.18 |
| 180 | 52.55 | 62.59 | 69.77 | 73.41 | 86.06 | 85.24 | 85.65 | 86.03 | 84.68 | 85.36 | 79.30 | 77.25 | 78.28 |
| 240 | 56.00 | 65.14 | 74.06 | 76.48 | 85.61 | 86.38 | 85.99 | 86.76 | 85.56 | 86.16 | 79.51 | 77.80 | 78.65 |
| 300 | 58.93 | 66.00 | 74.93 | 77.84 | 86.80 | 85.35 | 86.08 | 86.41 | 85.89 | 86.15 | 79.65 | 77.99 | 78.82 |
| 360 | 60.23 | 66.32 | 76.00 | 78.66 | 86.21 | 85.83 | 86.02 | 86.53 | 86.29 | 86.41 | 79.65 | 78.00 | 78.82 |
| 480 | 61.35 | 66.51 | 76.33 | 79.22 | 86.26 | 84.33 | 85.29 | 86.68 | 86.04 | 86.36 | 79.65 | 77.95 | 78.80 |
| 720 | 63.62 | 66.46 | 76.50 | 80.08 | 86.75 | 86.44 | 86.60 | 85.77 | 86.54 | 86.16 | 79.26 | 77.22 | 78.24 |

[FIG. 18]

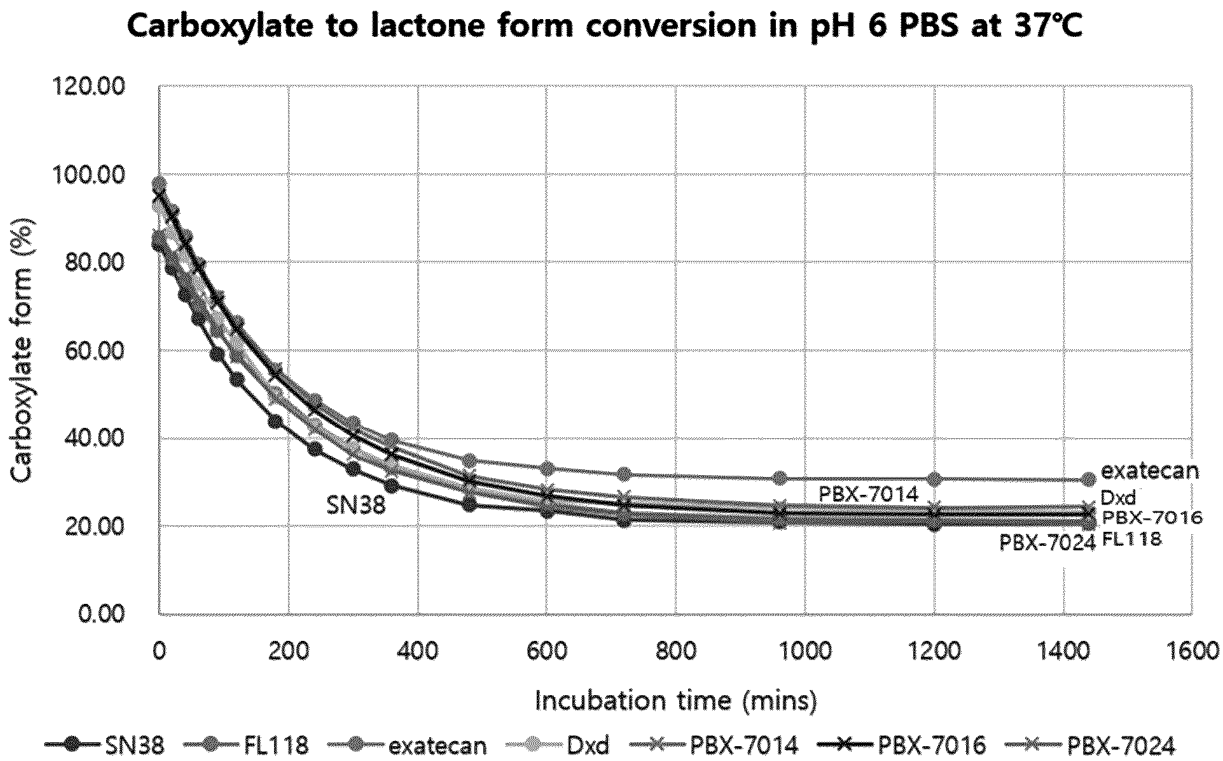


| Time (mins) | SN38 | FL118 | exatecan | Dxd | PBX-7014(1차) | PBX-7014(2차) | PBX-7014 | PBX-7016 (1차) | PBX-7016 (2차) | PBX-7016 | PBX-7024(1차) | PBX-7024(2차) | PBX-7024 |
|---|---|---|---|---|---|---|---|---|---|---|---|---|---|
| 0 | 84.02 | 85.67 | 97.90 | 92.78 | 94.97 | 94.60 | 94.79 | 95.40 | 95.01 | 95.21 | 85.88 | 86.45 | 86.17 |
| 20 | 78.64 | 80.70 | 91.68 | 86.70 | 90.23 | 91.13 | 90.68 | 90.38 | 90.11 | 90.25 | 80.96 | 82.37 | 81.67 |
| 40 | 72.63 | 76.03 | 85.82 | 81.70 | 84.62 | 84.40 | 84.51 | 84.61 | 83.61 | 84.11 | 75.58 | 78.26 | 76.92 |
| 60 | 67.09 | 70.14 | 79.59 | 75.10 | 79.09 | 78.83 | 78.96 | 79.30 | 77.97 | 78.63 | 70.34 | 73.28 | 71.81 |
| 90 | 59.20 | 64.39 | 71.96 | 67.12 | 71.91 | 71.88 | 71.89 | 71.40 | 70.50 | 70.95 | 63.57 | 65.58 | 64.57 |
| 120 | 53.26 | 58.90 | 66.17 | 61.67 | 65.73 | 65.54 | 65.64 | 64.83 | 64.57 | 64.70 | 57.45 | 59.68 | 58.56 |
| 180 | 43.78 | 50.10 | 55.61 | 49.46 | 55.86 | 55.65 | 55.75 | 54.34 | 54.16 | 54.25 | 48.29 | 49.45 | 48.87 |
| 240 | 37.43 | 42.95 | 48.59 | 42.58 | 48.45 | 47.06 | 47.76 | 46.52 | 46.25 | 46.39 | 40.90 | 43.07 | 41.99 |
| 300 | 32.84 | 37.57 | 43.33 | 37.57 | 42.55 | 41.83 | 42.19 | 40.38 | 40.80 | 40.59 | 35.34 | 37.06 | 36.20 |
| 360 | 29.18 | 33.22 | 39.53 | 33.77 | 38.39 | 37.51 | 37.95 | 36.06 | 36.40 | 36.23 | 31.86 | 32.93 | 32.40 |
| 480 | 24.73 | 28.36 | 34.90 | 28.59 | 32.43 | 30.37 | 31.40 | 30.26 | 29.94 | 30.10 | 26.68 | 28.42 | 27.55 |
| 600 | 23.41 | 25.06 | 33.07 | 25.66 | 29.16 | 27.37 | 28.26 | 26.91 | 26.57 | 26.74 | 23.16 | 25.51 | 24.34 |
| 720 | 21.37 | 22.83 | 31.62 | 25.24 | 27.35 | 25.64 | 26.50 | 24.64 | 24.66 | 24.65 | 22.28 | 22.67 | 22.47 |
| 960 | 20.86 | 21.78 | 30.73 | 24.05 | 25.57 | 23.66 | 24.62 | 23.05 | 22.43 | 22.74 | 19.90 | 21.45 | 20.67 |
| 1200 | 20.46 | 21.35 | 30.65 | 23.36 | 25.15 | 23.20 | 24.17 | 22.45 | 22.57 | 22.51 | 20.80 | 21.24 | 21.02 |
| 1440 | 20.56 | 21.05 | 30.45 | 23.17 | 24.96 | 23.53 | 24.24 | 22.09 | 23.30 | 22.70 | 19.48 | 21.39 | 20.44 |

[FIG. 19]

# ➢ SEC (Tra-PBX-7014)

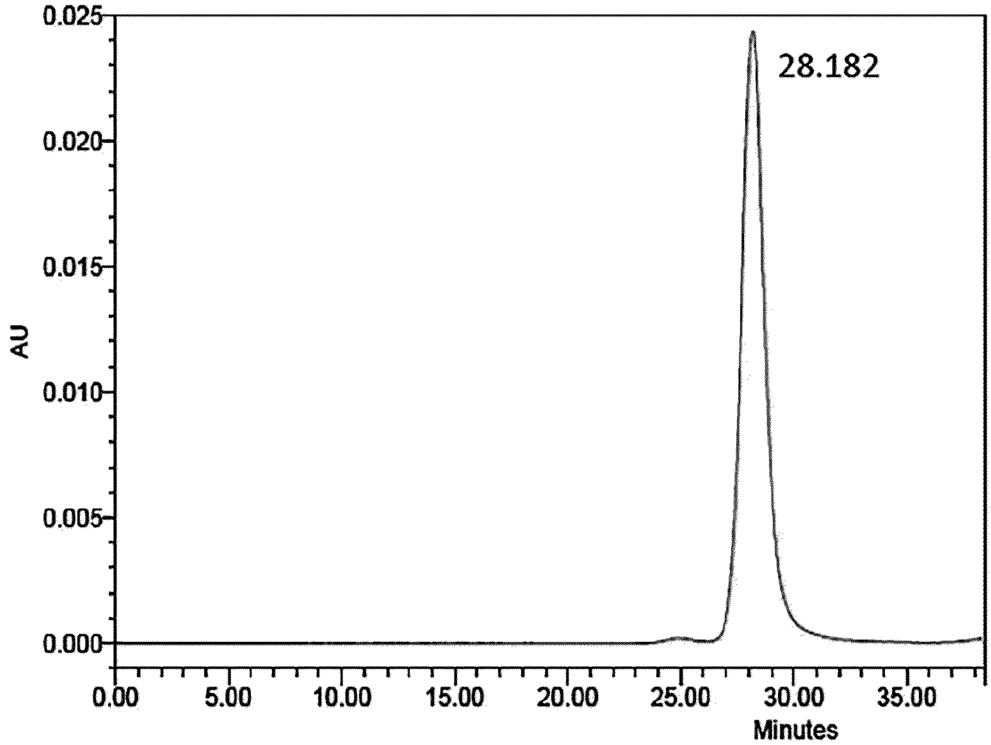


# ➢ SEC (Tra-PBX-7016)

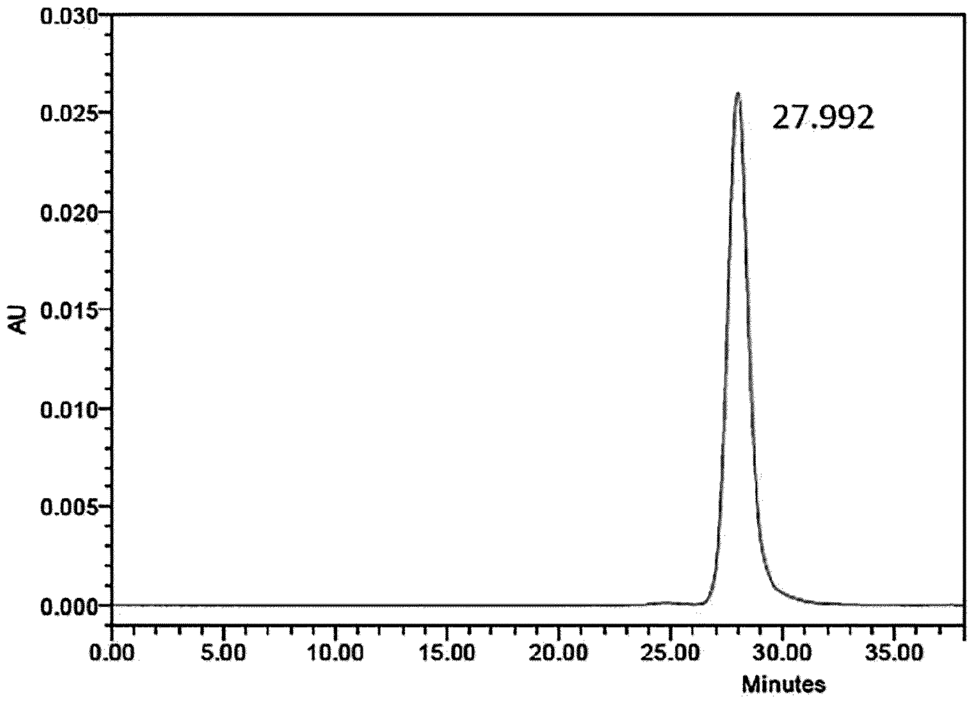

[FIG. 20a]

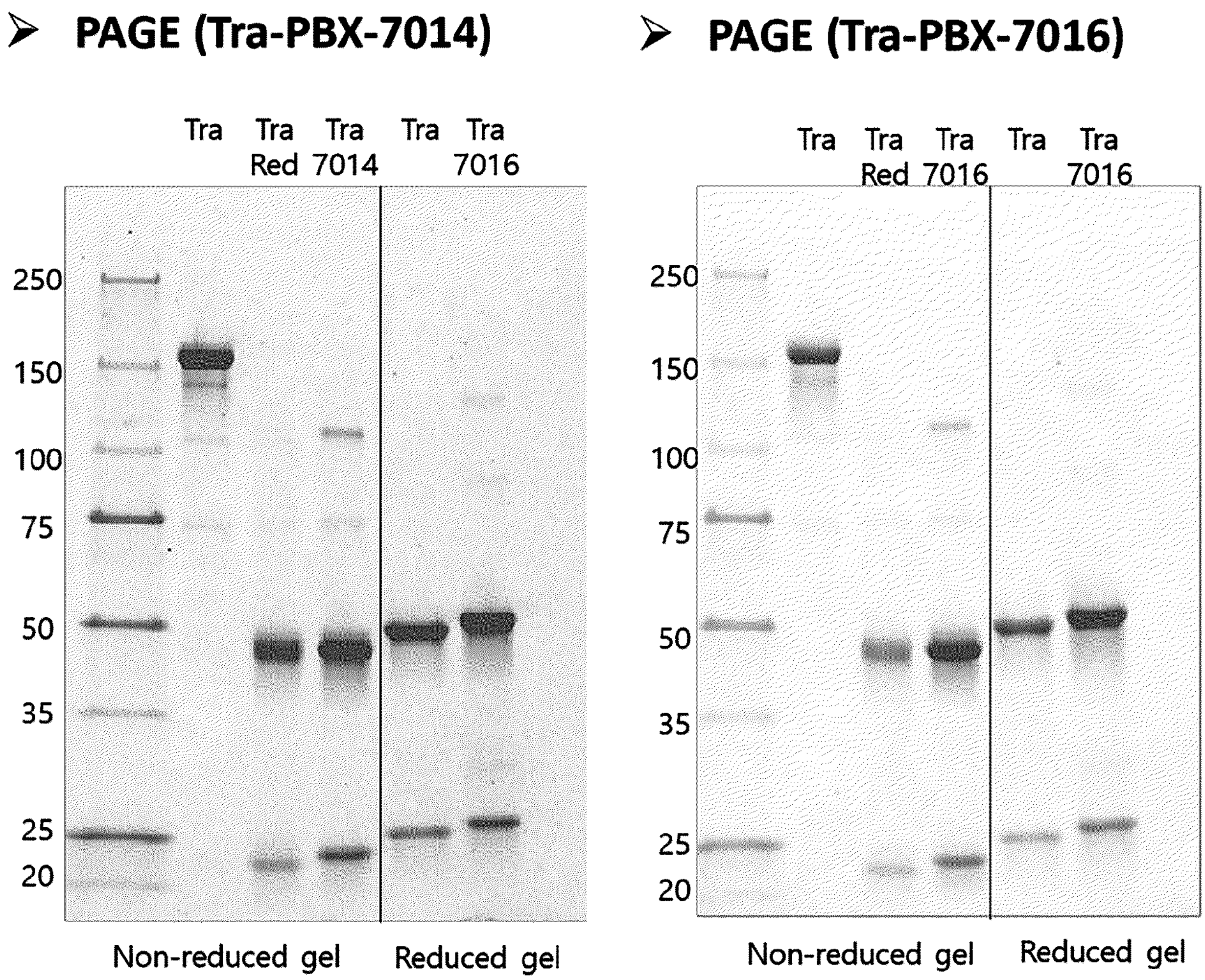
PAGE (Tra-PBX-7014)
Tra
Tra Red
Tra 7014
Tra
Tra 7016
250
150
100
75
50
35
25
20
Non-reduced gel
Reduced gel
PAGE (Tra-PBX-7016)
Tra
Tra Red
Tra 7016
Tra
Tra 7016
250
150
100
75
50
35
25
20
Non-reduced gel
Reduced gel

[FIG. 20b]

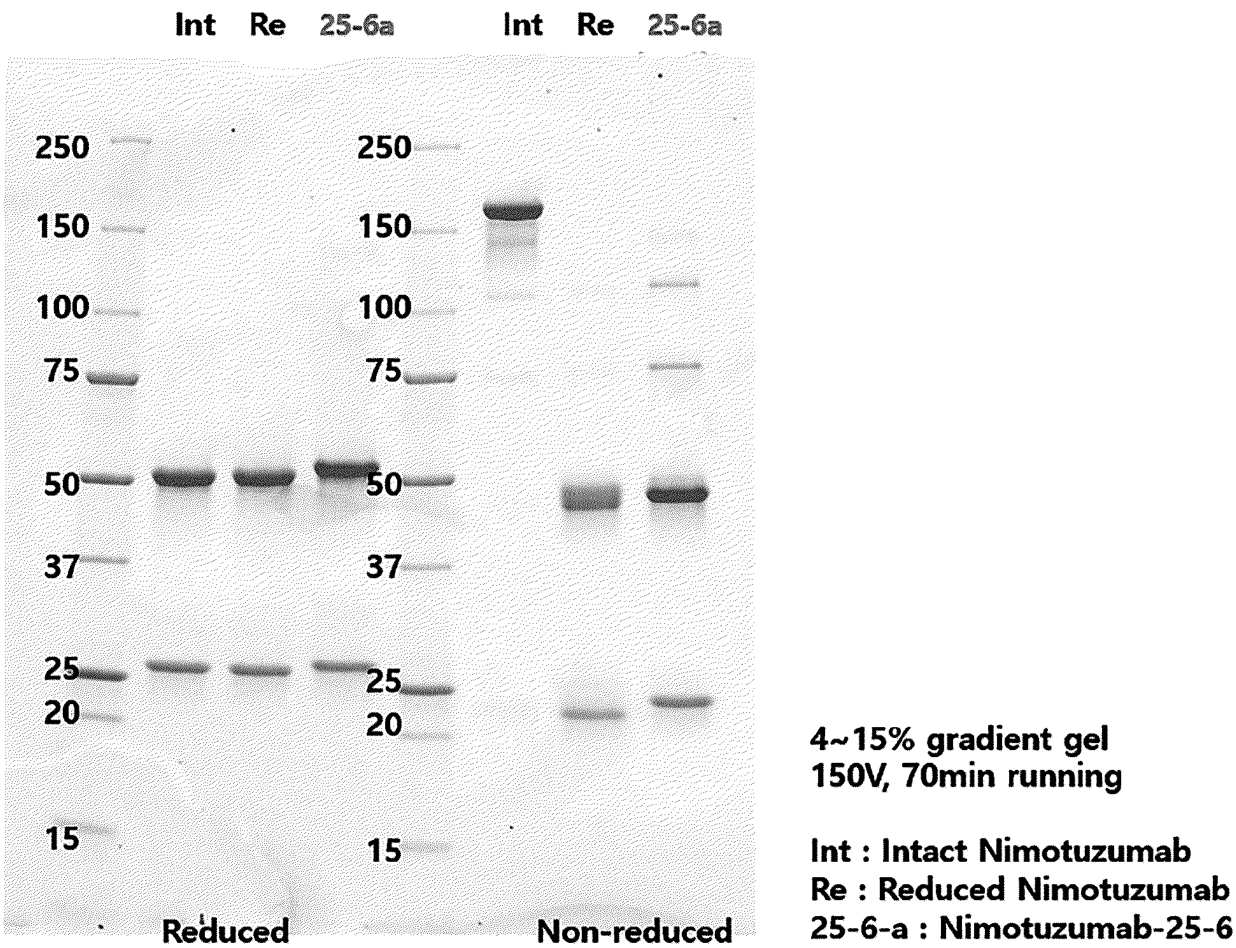
Int
Re
25-6a
Int
Re
25-6a
250
150
100
75
50
37
25
20
15
250
150
100
75
50
37
25
20
15
Reduced
Non-reduced
4~15% gradient gel
150V, 70min running
Int : Intact Nimotuzumab
Re : Reduced Nimotuzumab
25-6-a : Nimotuzumab-25-6

[FIG. 20c]

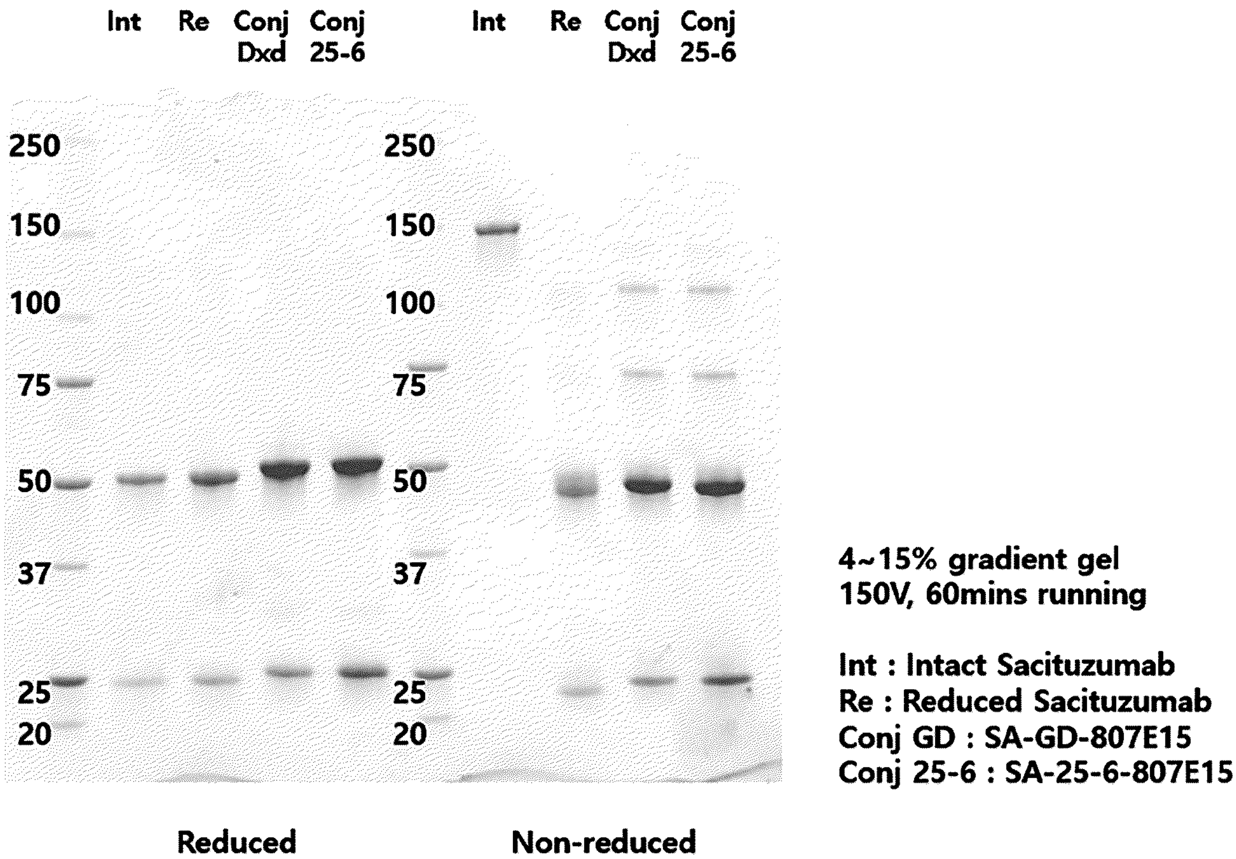

Int
Re
Conj Dxd
Conj 25-6
Int
Re
Conj Dxd
Conj 25-6
250
150
100
75
50
37
25
20
250
150
100
75
50
37
25
20
Reduced
Non-reduced
4~15% gradient gel
150V, 60mins running
Int : Intact Sacituzumab
Re : Reduced Sacituzumab
Conj GD : SA-GD-807E15
Conj 25-6 : SA-25-6-807E15

[FIG. 20d]

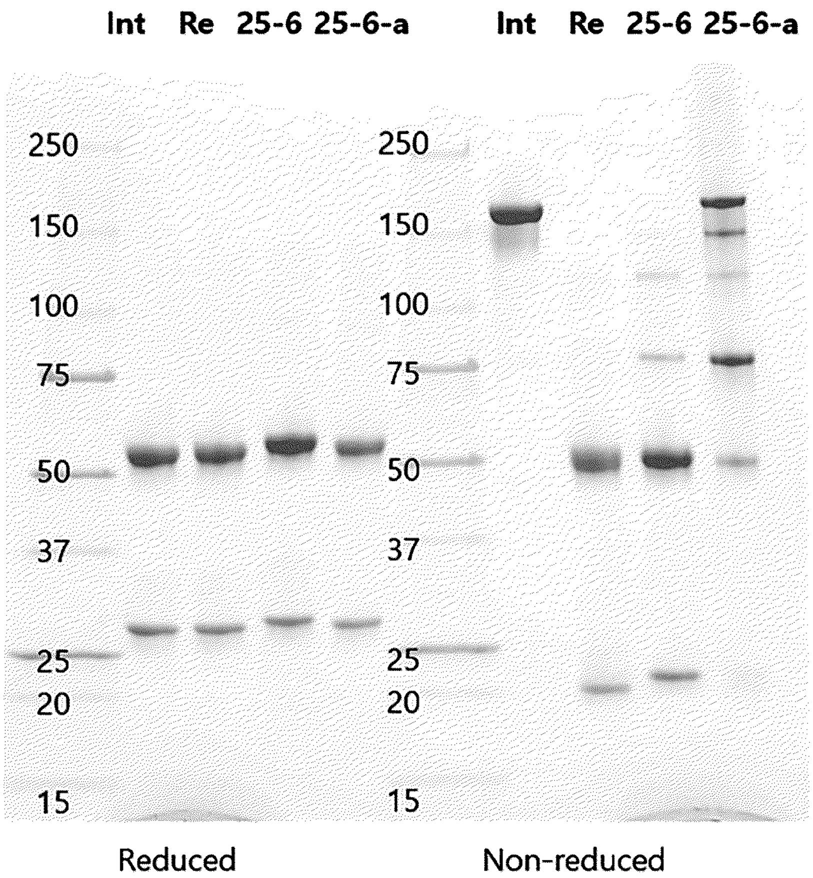


**4~15% gradient gel**
**150V, 60min running**

**Int : Intact Cetuximab**
**Re : Reduced Cetuximab**
**25-6 : CE-25-6-807D13**
**25-6-a : CE-25-6-a-407D13**

[FIG. 21]

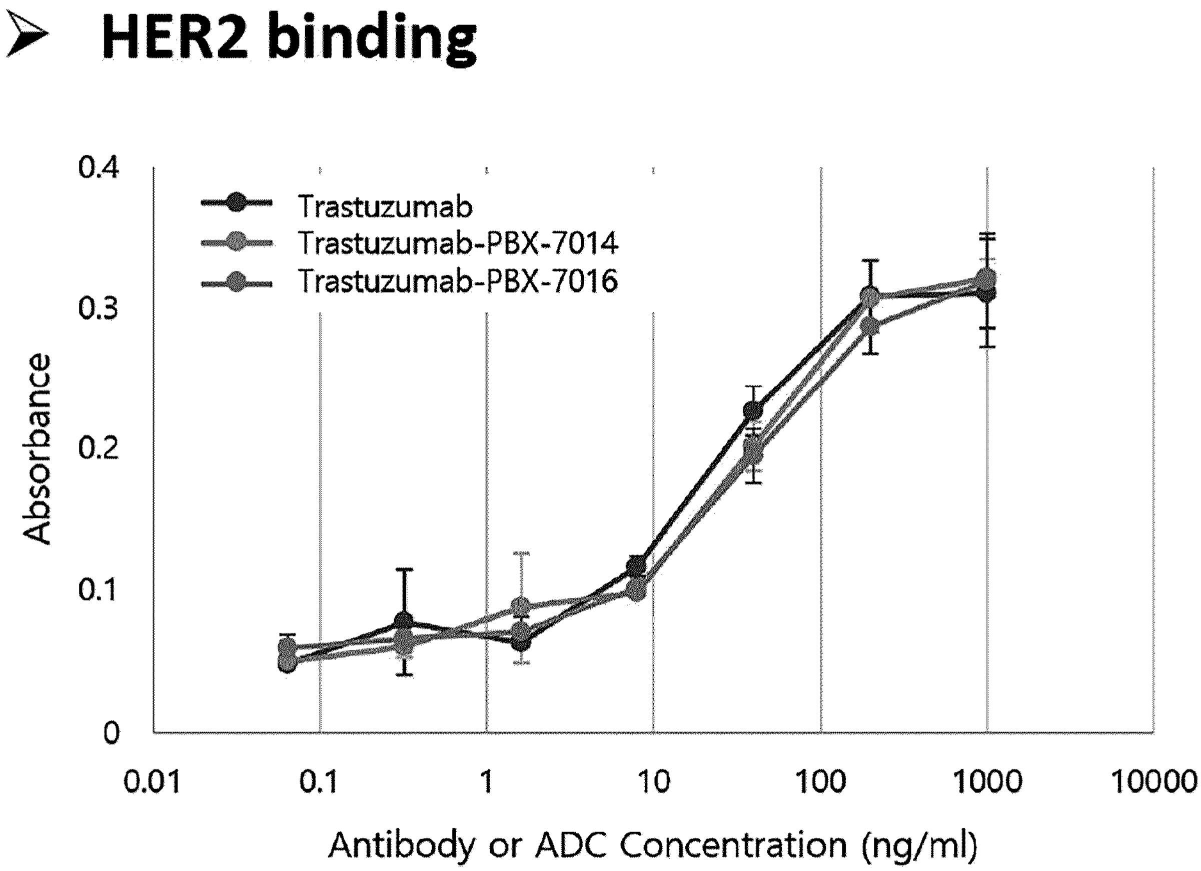
➢ HER2 binding
0.4
0.3
0.2
0.1
0
Absorbance
Trastuzumab
Trastuzumab-PBX-7014
Trastuzumab-PBX-7016
0.01
0.1
1
10
100
1000
10000
Antibody or ADC Concentration (ng/ml)

[FIG. 22]

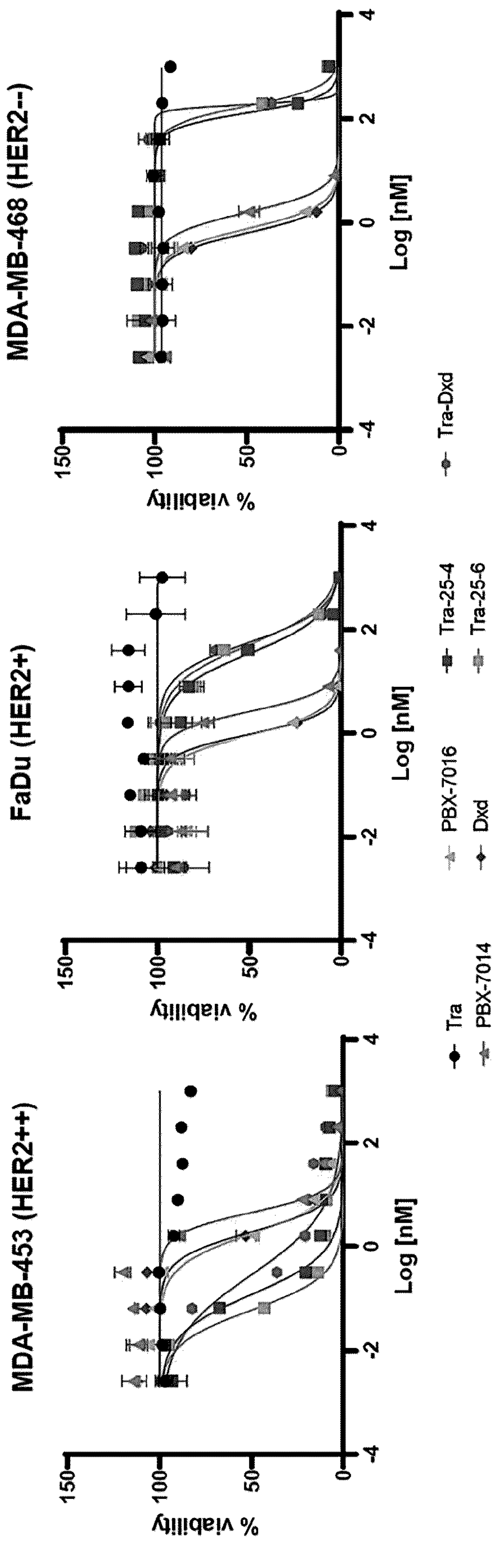


MDA-MB-453 (HER2++)

| Drug | | $IC_{50}$ (nM) |
|---|---|---|
| Tra | Ab only | - |
| PBX-7014 | Payload only | 4.539 |
| PBX-7016 | | 1.750 |
| Dxd | | 1.917 |
| Tra-25-4 | ADC | **0.1192** |
| Tra-25-6 | | **0.05973** |
| Tra-Dxd | | **0.2866** |

FaDu (HER2+)

| Drug | | $IC_{50}$ (nM) |
|---|---|---|
| Tra | Ab only | 1524 |
| PBX-7014 | Payload only | 2.607 |
| PBX-7016 | | 0.9129 |
| Dxd | | 0.9574 |
| Tra-25-4 | ADC | **34.50** |
| Tra-25-6 | | **51.02** |
| Tra-Dxd | | **56.86** |

MDA-MB-468 (HER2--)

| Drug | | $IC_{50}$ (nM) |
|---|---|---|
| Tra | Ab only | - |
| PBX-7014 | Payload only | 1.557 |
| PBX-7016 | | 0.7548 |
| Dxd | | 0.6222 |
| Tra-25-4 | ADC | **128.8** |
| Tra-25-6 | | **174.8** |
| Tra-Dxd | | **188.0** |

[FIG. 23]

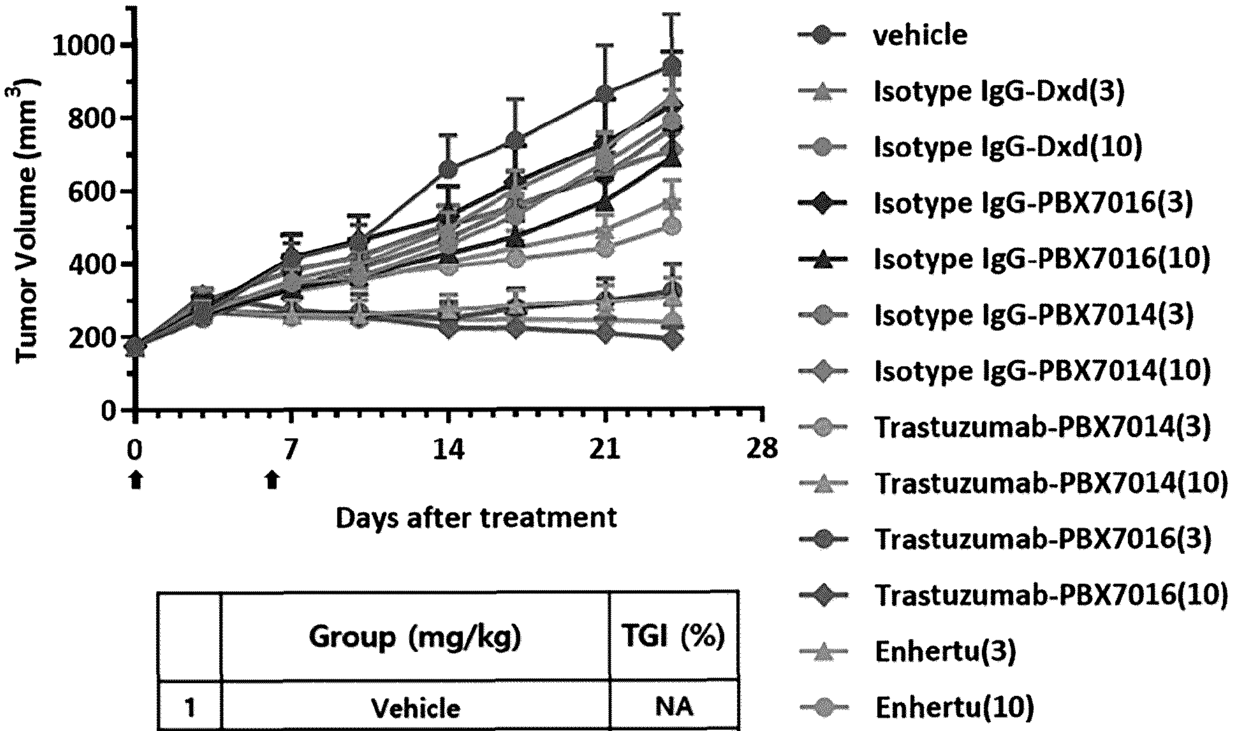


| | Group (mg/kg) | TGI (%) |
|---|---|---|
| 1 | Vehicle | NA |
| 2 | Isotype-Dxd (CTP63-Dxd (3)) | 11.27 |
| 3 | Isotype-Dxd (CTP63-Dxd (10)) | 19.74 |
| 4 | Isotype-PBX7016 (CTP63-PBX7016 (3)) | 14.38 |
| 5 | Isotype-PBX7016 (CTP63-PBX7016 (10)) | 32.95 |
| 6 | Isotype-PBX7014 (CTP63-PBX7014 (3)) | 22.52 |
| 7 | Isotype-PBX7016 (CTP63-PBX7014 (10)) | 30.09 |
| 8 | Trastuzumab-PBX7014 (CTP6-PBX7014 (3)) | 57.42 |
| 9 | Trastuzumab-PBX7014 (CTP6-PBX7014 (10)) | 82.61 |
| 10 | Trastuzumab-PBX7016 (CTP6-PBX7016 (3)) | 80.41 |
| 11 | Trastuzumab-PBX7016 (CTP6-PBX7016 (10)) | 97.80 |
| 12 | Enhertu (DP) (3) | 48.42 |
| 13 | Enhertu (DP) (10) | 91.68 |

[FIG. 24]

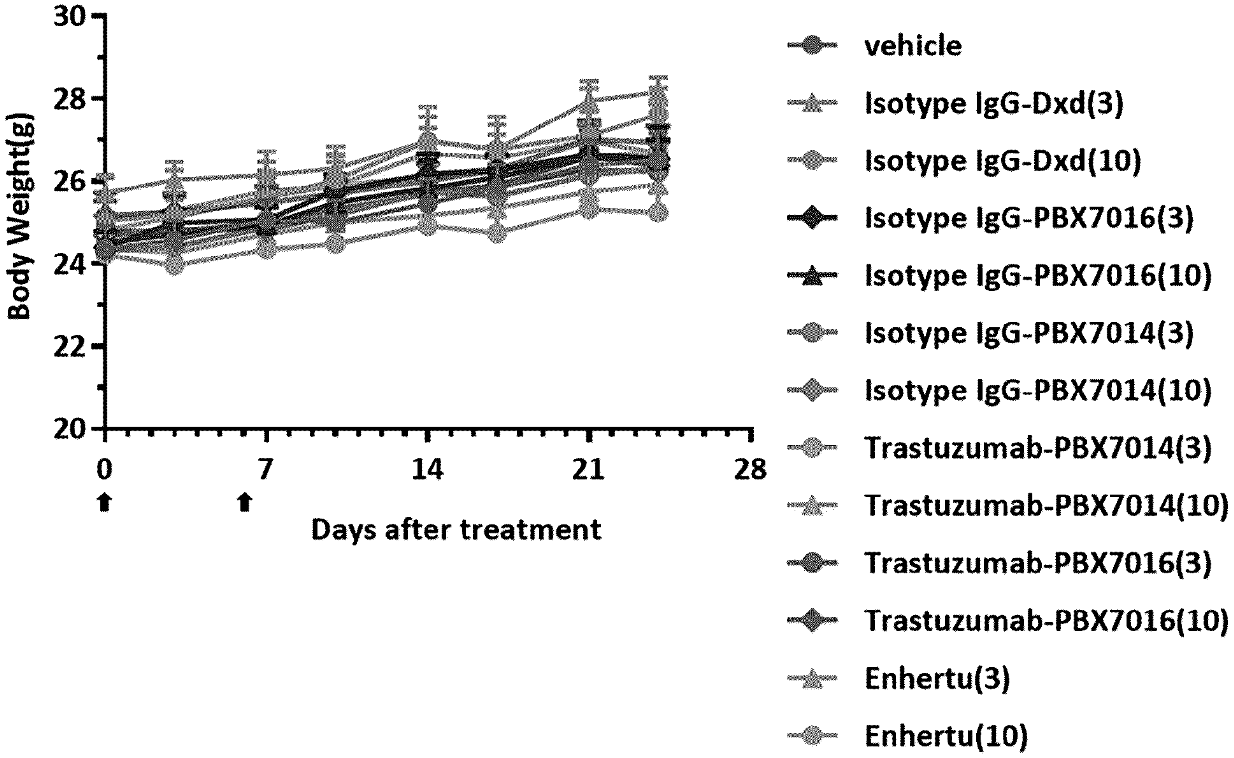
Body Weight(g)
30
28
26
24
22
20
0
7
14
21
28
Days after treatment
vehicle
Isotype IgG-Dxd(3)
Isotype IgG-Dxd(10)
Isotype IgG-PBX7016(3)
Isotype IgG-PBX7016(10)
Isotype IgG-PBX7014(3)
Isotype IgG-PBX7014(10)
Trastuzumab-PBX7014(3)
Trastuzumab-PBX7014(10)
Trastuzumab-PBX7016(3)
Trastuzumab-PBX7016(10)
Enhertu(3)
Enhertu(10)

[FIG. 25]

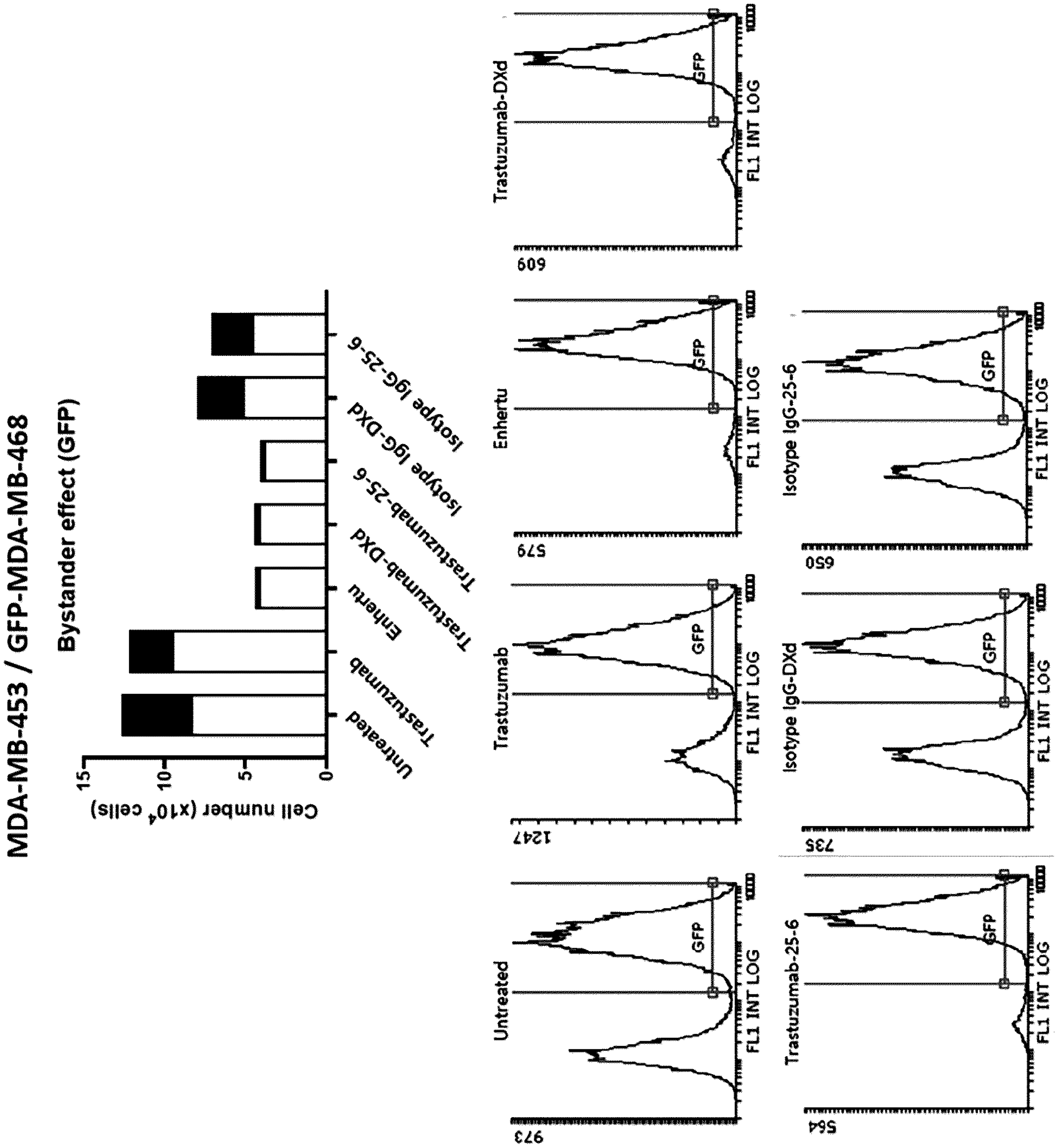
MDA-MB-453 / GFP-MDA-MB-468
Bystander effect (GFP)
Cell number (x10⁴ cells)
0
5
10
15
Untreated
Trastuzumab
Enhertu
Trastuzumab-DXd
Trastuzumab-25-6
Isotype IgG-DXd
Isotype IgG-25-6
Untreated
973
Trastuzumab
1247
Enhertu
579
Trastuzumab-DXd
609
Trastuzumab-25-6
564
Isotype IgG-DXd
735
Isotype IgG-25-6
650
GFP
FL1 INT LOG

[FIG. 26]

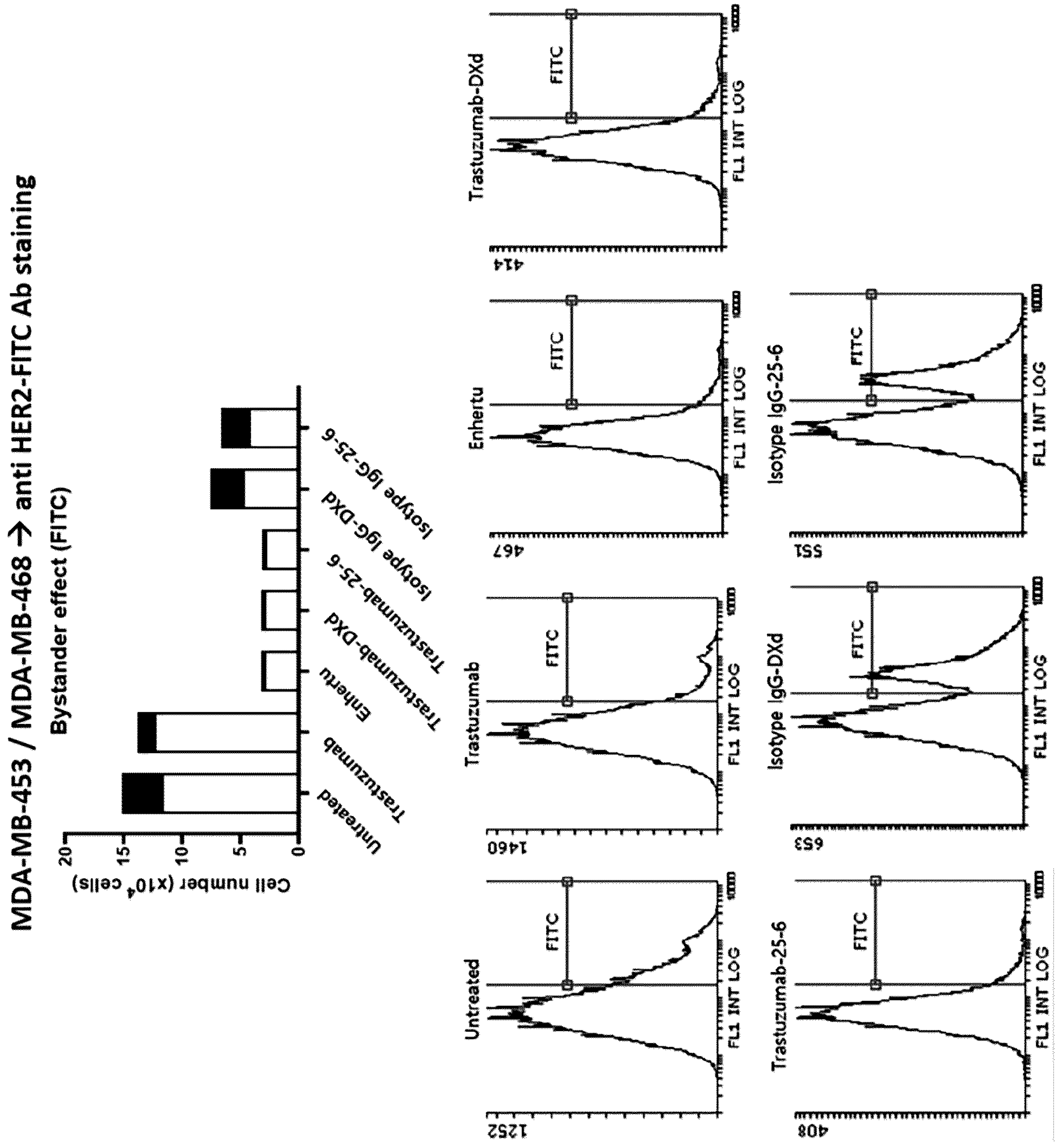
MDA-MB-453 / MDA-MB-468 → anti HER2-FITC Ab staining
Bystander effect (FITC)
Cell number (x10⁴ cells)
20
15
10
5
0
Untreated
Trastuzumab
Enhertu
Trastuzumab-DXd
Trastuzumab-25-6
Isotype IgG-DXd
Isotype IgG-25-6
Untreated
Trastuzumab
Enhertu
Trastuzumab-DXd
Trastuzumab-25-6
Isotype IgG-DXd
Isotype IgG-25-6
FITC
FL1 INT LOG
1252
1460
467
414
408
653
551

[FIG. 27a]

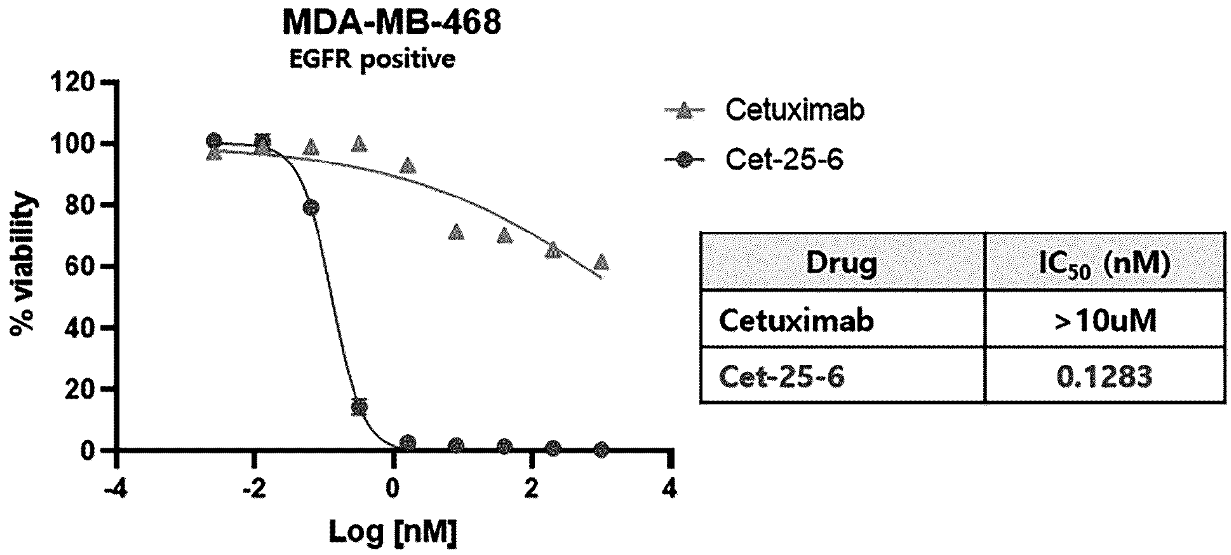


| Drug | $IC_{50}$ (nM) |
|---|---|
| Cetuximab | >10uM |
| Cet-25-6 | 0.1283 |

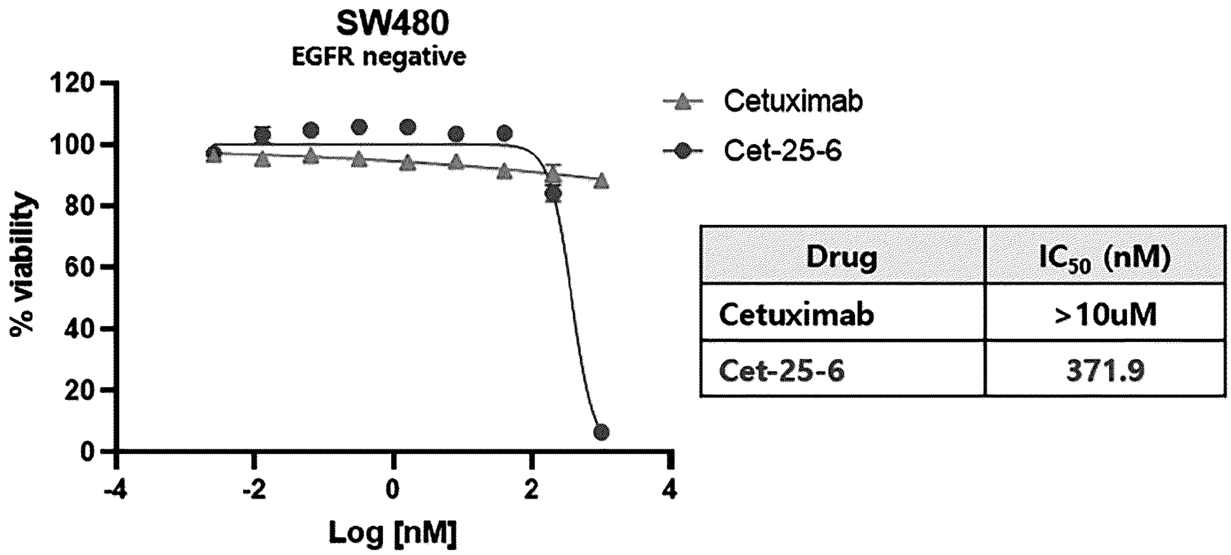


| Drug | $IC_{50}$ (nM) |
|---|---|
| Cetuximab | >10uM |
| Cet-25-6 | 371.9 |

[FIG. 27b]

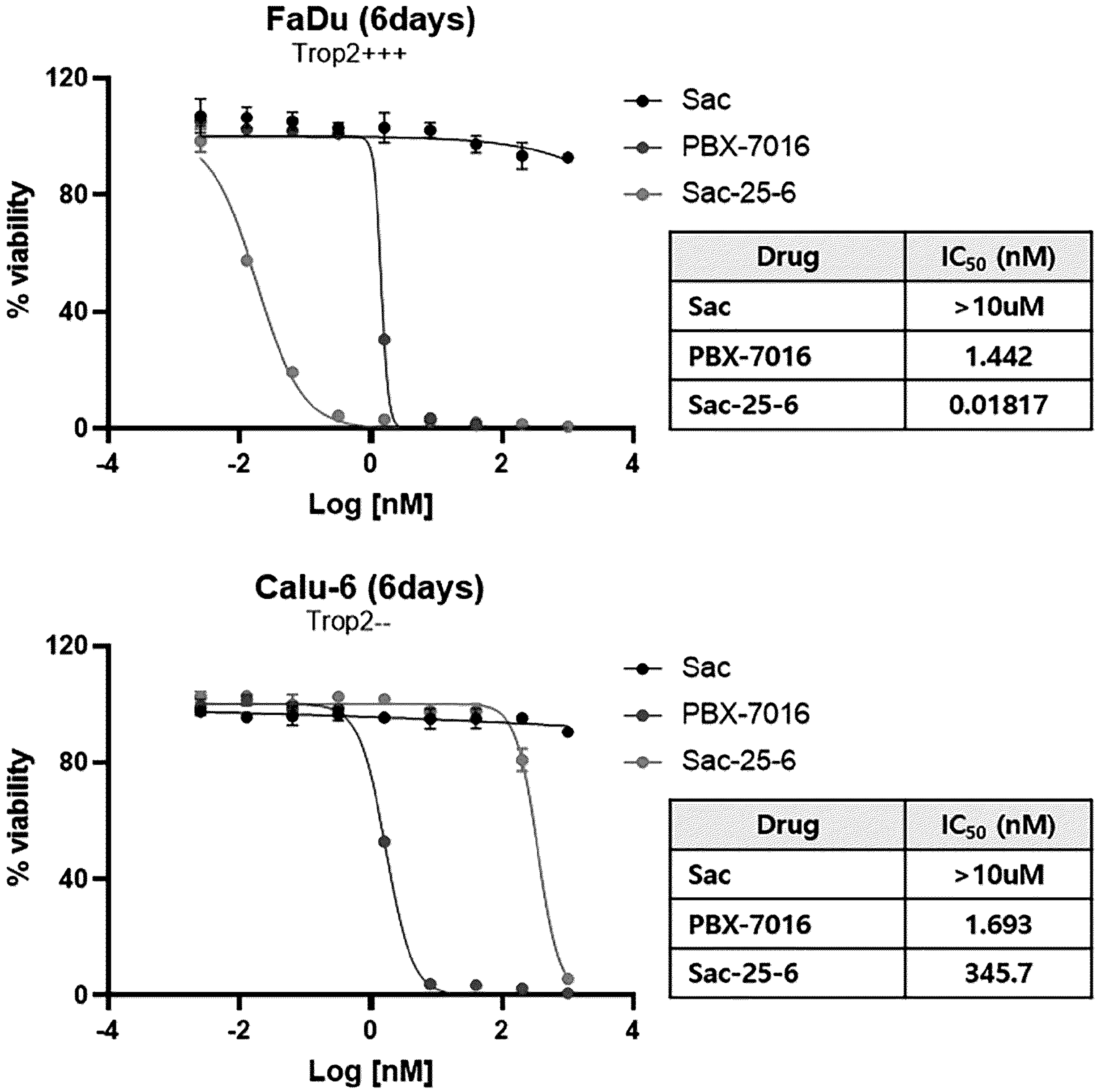


| Drug | $IC_{50}$ (nM) |
|---|---|
| Sac | >10uM |
| PBX-7016 | 1.442 |
| Sac-25-6 | 0.01817 |

| Drug | $IC_{50}$ (nM) |
|---|---|
| Sac | >10uM |
| PBX-7016 | 1.693 |
| Sac-25-6 | 345.7 |

[FIG. 28]

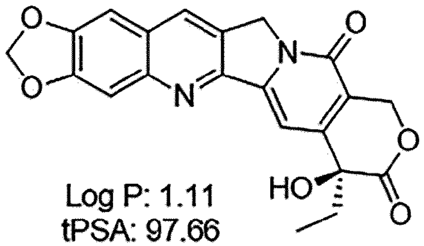

Log P: 1.11
tPSA: 97.66
CLogP: 1.41597

**FL118**

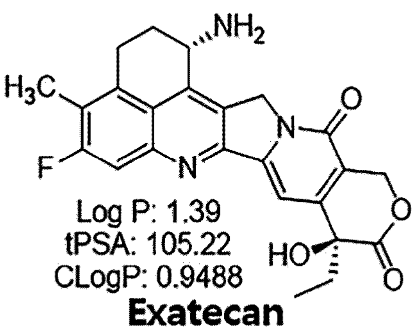

Log P: 1.39
tPSA: 105.22
CLogP: 0.9488

**Exatecan**

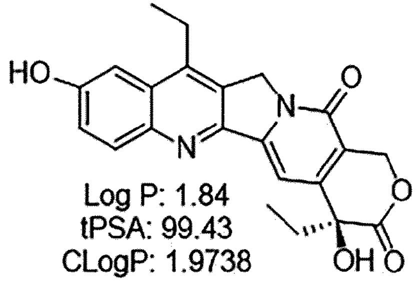

Log P: 1.84
tPSA: 99.43
CLogP: 1.9738

**SN-38**

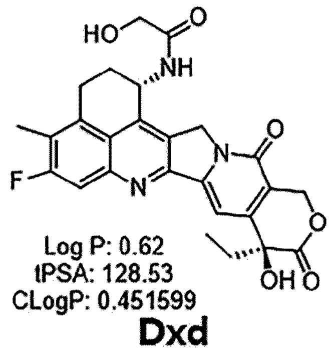

Log P: 0.62
tPSA: 128.53
CLogP: 0.451599

**Dxd**

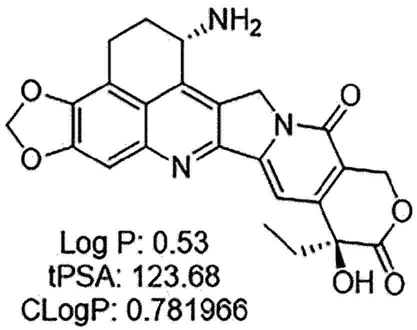

Log P: 0.53
tPSA: 123.68
CLogP: 0.781966

**Compound A**

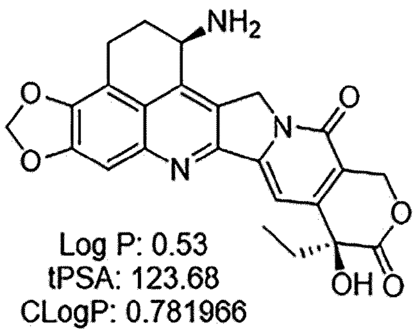

Log P: 0.53
tPSA: 123.68
CLogP: 0.781966

**Compound B**

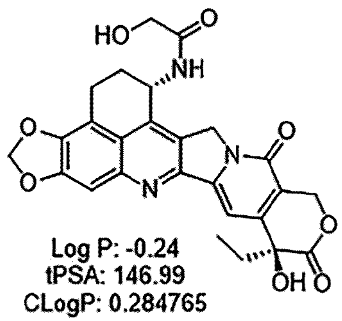

Log P: -0.24
tPSA: 146.99
CLogP: 0.284765

**Compound C**

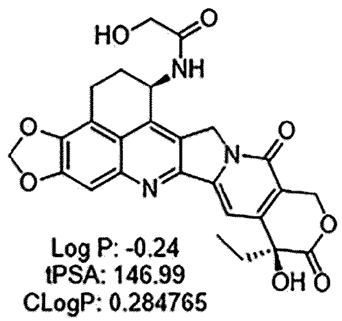

Log P: -0.24
tPSA: 146.99
CLogP: 0.284765

**Compound D**

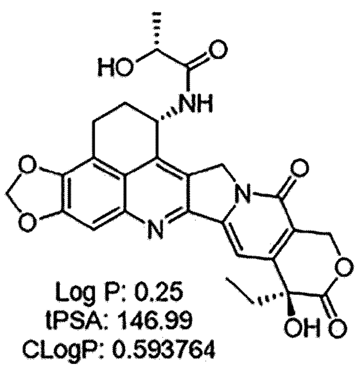

Log P: 0.25
tPSA: 146.99
CLogP: 0.593764

**Compound E**

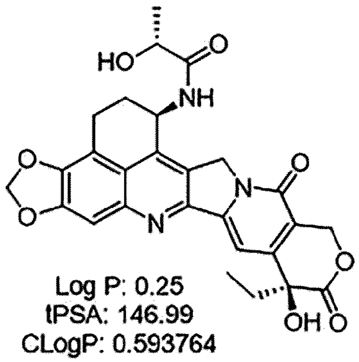

Log P: 0.25
tPSA: 146.99
CLogP: 0.593764

**Compound F**

[FIG. 29]

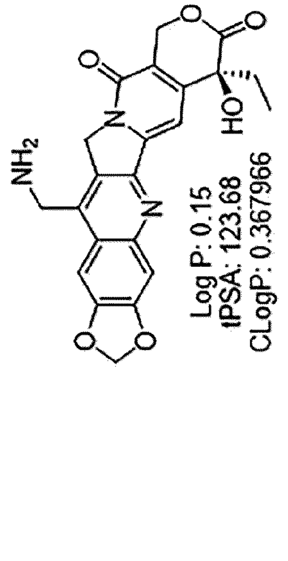

Log P: 0.15
tPSA: 123.68
CLogP: 0.367966

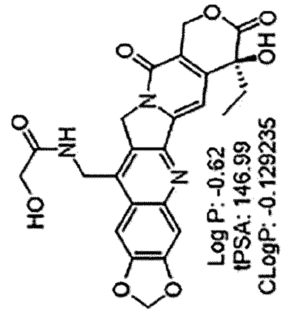

Log P: -0.62
tPSA: 146.99
CLogP: -0.129235

Compound G

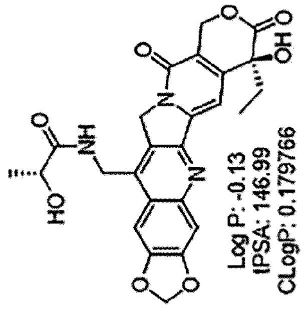

Log P: -0.13
tPSA: 146.99
CLogP: 0.179766

Compound H

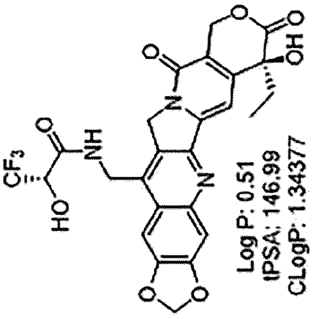

Log P: 0.51
tPSA: 146.99
CLogP: 1.34377

Compound I

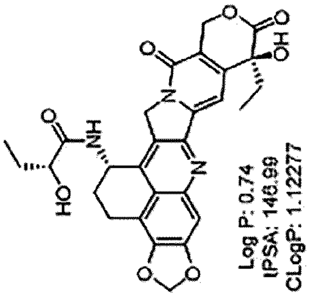

Log P: 0.74
tPSA: 146.99
CLogP: 1.12277

Compound J

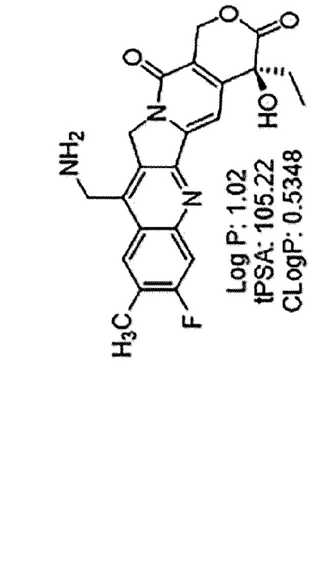

Log P: 1.02
tPSA: 105.22
CLogP: 0.5348

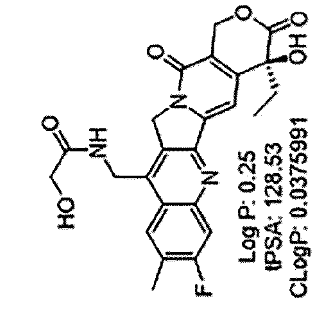

Log P: 0.25
tPSA: 128.53
CLogP: 0.0375991

Compound G'

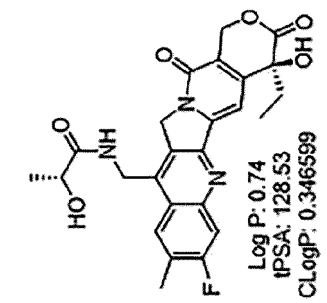

Log P: 0.74
tPSA: 128.53
CLogP: 0.346599

Compound H'

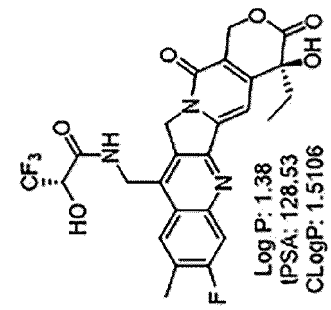

Log P: 1.38
tPSA: 128.53
CLogP: 1.5106

Compound I'

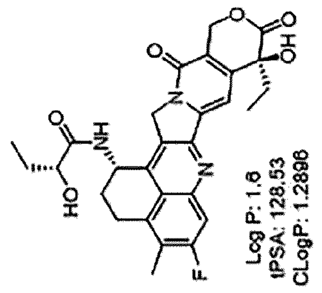

Log P: 1.6
tPSA: 128.53
CLogP: 1.2896

Compound J'

[FIG. 30]

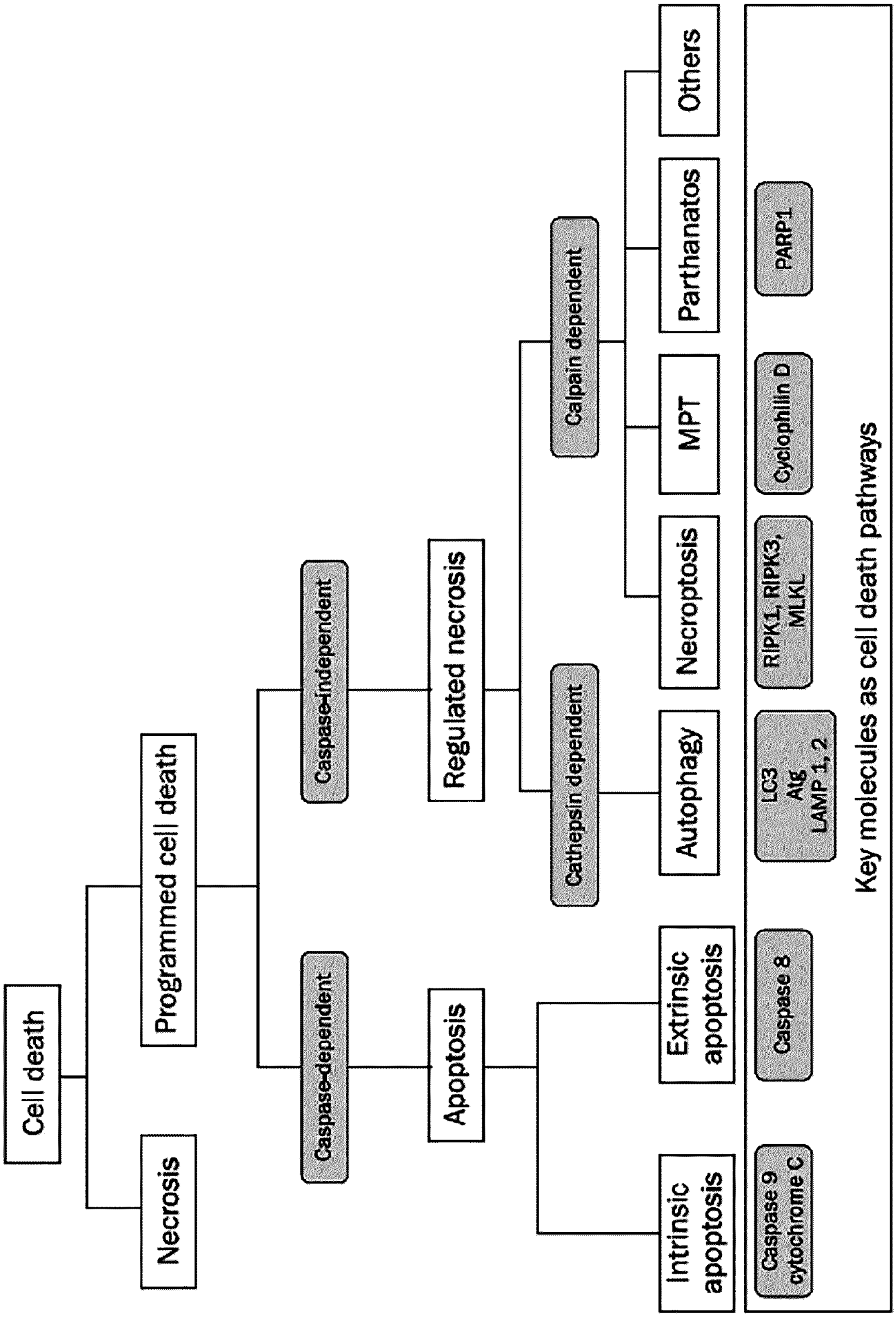
Cell death
Necrosis
Programmed cell death
Caspase-dependent
Caspase-independent
Apoptosis
Regulated necrosis
Cathepsin dependent
Calpain dependent
Intrinsic apoptosis
Extrinsic apoptosis
Autophagy
Necroptosis
MPT
Parthanatos
Others
Caspase 9 cytochrome C
Caspase 8
LC3 Atg LAMP 1, 2
RIPK1, RIPK3, MLKL
Cyclophilin D
PARP1
Key molecules as cell death pathways

[FIG. 31]

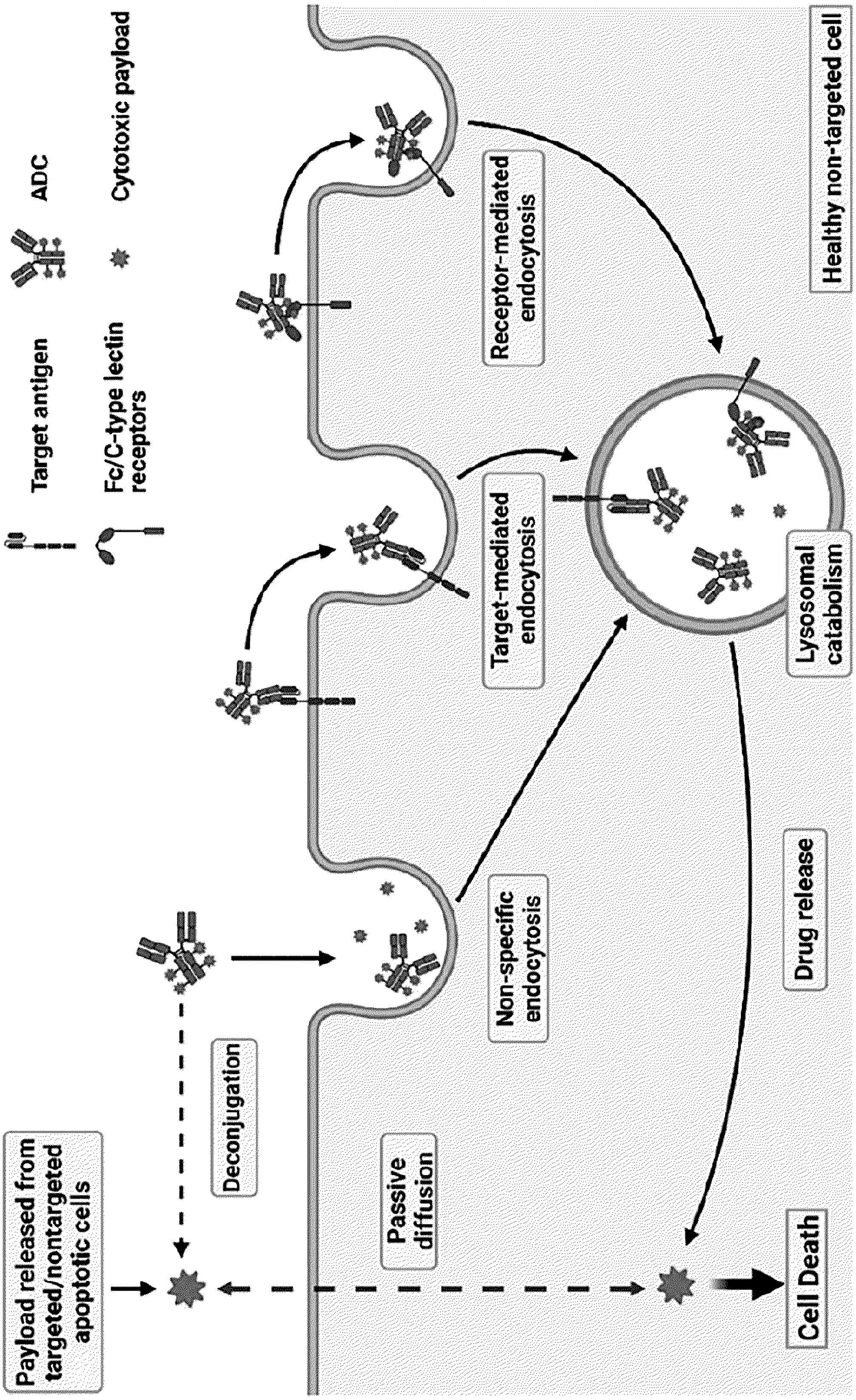
Target antigen
ADC
Fc/C-type lectin receptors
Cytotoxic payload
Receptor-mediated endocytosis
Target-mediated endocytosis
Non-specific endocytosis
Lysosomal catabolism
Healthy non-targeted cell
Drug release
Deconjugation
Passive diffusion
Payload released from targeted/nontargeted apoptotic cells
Cell Death

[FIG. 32]

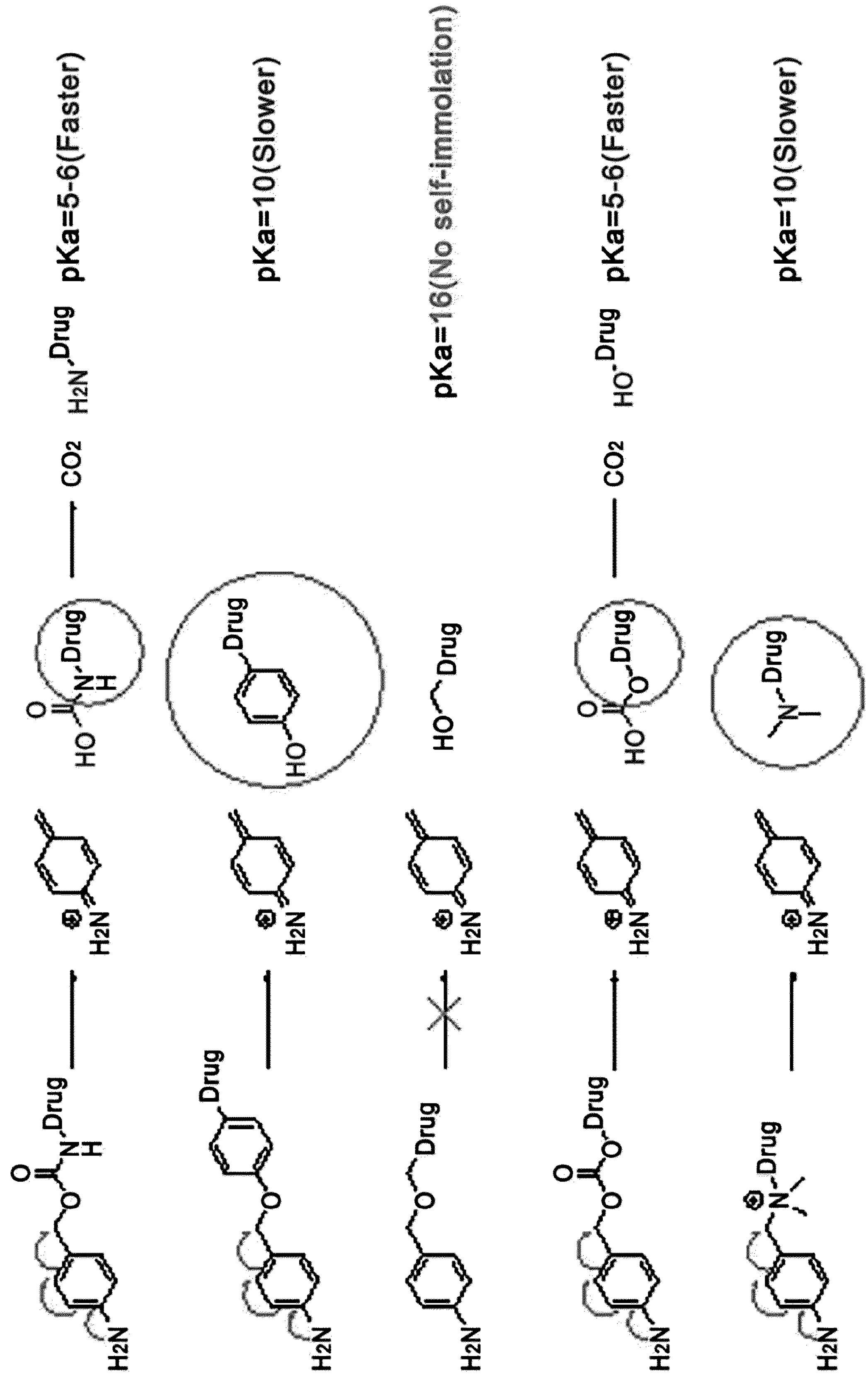
pKa=5-6(Faster)
pKa=10(Slower)
pKa=16(No self-immolation)
pKa=5-6(Faster)
pKa=10(Slower)

**INTERNATIONAL SEARCH REPORT**

International application No. **PCT/KR2023/009854**

**A. CLASSIFICATION OF SUBJECT MATTER**

**C07D 491/22**(2006.01)i; **A61K 31/4745**(2006.01)i; **A61K 47/68**(2017.01)i; **A61P 35/00**(2006.01)i; **G01N 33/574**(2006.01)i; **A61K 47/65**(2017.01)i

According to International Patent Classification (IPC) or to both national classification and IPC

**B. FIELDS SEARCHED**

Minimum documentation searched (classification system followed by classification symbols)

C07D 491/22(2006.01); A61K 31/4745(2006.01); A61K 31/675(2006.01); A61P 35/00(2006.01); C07D 495/22(2006.01); C07F 7/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 캄토테신 유도체 (camptothecin derivatives), DDX5 단백질 (DDX5 protein), 세포사멸 (cell death), 암 (cancer)

**C. DOCUMENTS CONSIDERED TO BE RELEVANT**

| Category* | Citation of document, with indication, where appropriate, of the relevant passages | Relevant to claim No. |
|---|---|---|
| X | WO 2022-121981 A1 (WIGEN BIOMEDICINE TECHNOLOGY (SHANGHAI) CO., LTD.) 16 June 2022 (2022-06-16) See abstract; claims 1-18; page 1; and examples 10 and 11. | 1-8,11,13-15,18, 21,24-26,29,35-44 |
| Y | | 9,10,12,16,19,20, 22,23,27,28,30-34 |
| Y | WO 2022-015110 A1 (PINOTBIO, INC.) 20 January 2022 (2022-01-20) See claim 1; and paragraph [0098]. | 9,10,12,16,19,20, 22,23,27,28,30-34 |
| A | US 2017-0360770 A1 (IMMUNOMEDICS, INC.) 21 December 2017 (2017-12-21) See entire document. | 1-16,18-44 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 October 2023** | **12 October 2023** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
PCT/KR2023/009854

C. DOCUMENTS CONSIDERED TO BE RELEVANT

| Category* | Citation of document, with indication, where appropriate, of the relevant passages | Relevant to claim No. |
|---|---|---|
| A | LING, X. et al. FL118, a novel camptothecin analogue, overcomes irinotecan and topotecan resistance in human tumor xenograft models. American journal of translational research. 2015, vol. 7, no. 10, pp. 1765-1781.<br>See entire document. | 1-16,18-44 |
| A | ZHANG, Y. et al. Self-assembling nanoparticles of dually hydrophobic prodrugs constructed from camptothecin analogue for cancer therapy. European journal of medicinal chemistry. 30 April 2020, vol. 200, 112365, pp. 1-11.<br>See entire document. | 1-16,18-44 |
| PX | WO 2022-170971 A1 (MEDILINK THERAPEUTICS (SUZHOU) CO., LTD.) 18 August 2022.<br>See abstract; claims 1-51; and pages 379 and 390. | 1-8,11,13-15,18, 21,24-26,29,35-44 |

Form PCT/ISA/210 (second sheet) (July 2022)

**INTERNATIONAL SEARCH REPORT**

International application No.
**PCT/KR2023/009854**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17**
because they relate to subject matter not required to be searched by this Authority, namely:

Claim 17 pertains to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2023/009854**

| Patent document cited in search report | Publication date (day/month/year) | Patent family member(s) | Publication date (day/month/year) |
|---|---|---|---|
| WO 2022-121981 A1 | 16 June 2022 | AU 2021-394411 A1 | 06 July 2023 |
| | | CA 3200649 A1 | 16 June 2022 |
| | | CN 116583526 A | 11 August 2023 |
| | | KR 10-2023-0118891 A | 14 August 2023 |
| WO 2022-015110 A1 | 20 January 2022 | AU 2021-308834 A1 | 16 February 2023 |
| | | CA 3189462 A1 | 20 January 2022 |
| | | CN 116075321 A | 05 May 2023 |
| | | EP 4183418 A1 | 24 May 2023 |
| | | JP 2023-534292 A | 08 August 2023 |
| | | KR 10-2022-0009916 A | 25 January 2022 |
| | | KR 10-2022-0009928 A | 25 January 2022 |
| | | KR 10-2349925 B1 | 12 January 2022 |
| | | US 2023-0270867 A1 | 31 August 2023 |
| US 2017-0360770 A1 | 21 December 2017 | AT 500845 T | 15 March 2011 |
| | | AU 2018-201403 A1 | 15 March 2018 |
| | | AU 2018-201403 B2 | 18 July 2019 |
| | | AU 2019-250192 A1 | 07 November 2019 |
| | | AU 2019-250192 B2 | 01 April 2021 |
| | | AU 4829600 A | 21 November 2000 |
| | | AU 5600500 A | 28 December 2000 |
| | | AU 6761098 A | 20 October 1998 |
| | | AU 728325 B2 | 04 January 2001 |
| | | AU 774044 B2 | 17 June 2004 |
| | | AU 782160 B2 | 07 July 2005 |
| | | BR 0311799 A | 10 May 2005 |
| | | BR 112012004269 A2 | 16 November 2016 |
| | | BR PI0406574 A | 17 January 2006 |
| | | BR PI0607486 A2 | 04 August 2009 |
| | | BR PI0607486 B1 | 16 April 2019 |
| | | BR PI0607486 B8 | 25 May 2021 |
| | | CA 2816418 A1 | 03 May 2012 |
| | | CA 2816418 C | 26 May 2020 |
| | | CA 2914438 A1 | 29 January 2015 |
| | | CA 2916671 A1 | 24 July 2008 |
| | | CA 2916671 C | 09 January 2018 |
| | | CA 2920192 A1 | 02 April 2015 |
| | | CA 2948013 A1 | 07 January 2016 |
| | | CA 2948693 A1 | 19 August 2010 |
| | | CA 2948693 C | 04 September 2018 |
| | | CA 2961774 A1 | 14 April 2016 |
| | | CA 3031737 A1 | 15 February 2018 |
| | | CA 3043766 A1 | 30 August 2018 |
| | | CA 3044096 A1 | 07 June 2018 |
| | | CN 103442565 A | 11 December 2013 |
| | | CN 103442565 B | 10 August 2016 |
| | | CN 106715443 A | 24 May 2017 |
| | | CN 107735090 A | 23 February 2018 |
| | | CN 107735090 B | 06 April 2021 |
| | | CN 107735104 A | 23 February 2018 |
| | | CN 107753954 A | 06 March 2018 |

Form PCT/ISA/210 (patent family annex) (July 2022)

**INTERNATIONAL SEARCH REPORT**
**Information on patent family members**

International application No.
**PCT/KR2023/009854**

| Patent document cited in search report | Publication date (day/month/year) | Patent family member(s) | Publication date (day/month/year) |
|---|---|---|---|
| | | CN 109562172 A | 02 April 2019 |
| | | CN 110075295 A | 02 August 2019 |
| | | CN 110248680 A | 17 September 2019 |
| | | CN 110392580 A | 29 October 2019 |
| | | CN 1649902 A | 03 August 2005 |
| | | CN 1649902 B | 13 April 2011 |
| | | CN 1649903 A | 03 August 2005 |
| | | CN 1675245 A | 28 September 2005 |
| | | CN 1675245 B | 12 January 2011 |
| | | CN 1764478 A | 26 April 2006 |
| | | CN 1764478 B | 21 October 2015 |
| | | CY 1110556 T1 | 29 April 2015 |
| | | DE 05075555 T1 | 08 February 2007 |
| | | DE 69830570 T2 | 03 November 2005 |
| | | DK 0969866 T3 | 03 October 2005 |
| | | DK 1194167 T3 | 16 November 2009 |
| | | DK 2396036 T3 | 16 October 2017 |
| | | DK 3332808 T3 | 14 December 2020 |
| | | DK 3483183 T3 | 21 June 2021 |
| | | EP 2632264 A2 | 04 September 2013 |
| | | EP 2632264 A4 | 23 April 2014 |
| | | EP 2632264 B1 | 02 October 2019 |
| | | EP 2674440 A2 | 18 December 2013 |
| | | EP 2674440 A3 | 05 March 2014 |
| | | EP 2674440 B1 | 03 July 2019 |
| | | EP 2675485 A2 | 25 December 2013 |
| | | EP 2675485 A4 | 15 October 2014 |
| | | EP 2704751 A1 | 12 March 2014 |
| | | EP 2704751 A4 | 03 June 2015 |
| | | EP 2704751 B1 | 17 April 2019 |
| | | EP 2764362 A1 | 13 August 2014 |
| | | EP 3122754 A2 | 01 February 2017 |
| | | EP 3122754 A4 | 21 February 2018 |
| | | JP 2016-534092 A | 04 November 2016 |
| | | JP 2017-536340 A | 07 December 2017 |
| | | JP 2018-520148 A | 26 July 2018 |
| | | JP 2020-105187 A | 09 July 2020 |
| | | JP 2020-183429 A | 12 November 2020 |
| | | JP 2020-508292 A | 19 March 2020 |
| | | JP 5214252 B2 | 19 June 2013 |
| | | JP 5231231 B2 | 10 July 2013 |
| | | JP 5314590 B2 | 16 October 2013 |
| | | JP 5335651 B2 | 06 November 2013 |
| | | JP 5388355 B2 | 15 January 2014 |
| | | JP 5435432 B2 | 05 March 2014 |
| | | JP 5682000 B2 | 11 March 2015 |
| | | JP 5699362 B2 | 08 April 2015 |
| | | JP 5740772 B2 | 01 July 2015 |
| | | JP 5779789 B2 | 16 September 2015 |
| | | JP 5789821 B2 | 07 October 2015 |

Form PCT/ISA/210 (patent family annex) (July 2022)

**INTERNATIONAL SEARCH REPORT**
**Information on patent family members**

International application No.
**PCT/KR2023/009854**

| Patent document cited in search report | Publication date (day/month/year) | Patent family member(s) | Publication date (day/month/year) |
|---|---|---|---|
| | | JP 6024025 B2 | 09 November 2016 |
| | | JP 6114936 B2 | 19 April 2017 |
| | | JP 6205621 B2 | 04 October 2017 |
| | | JP 6366111 B2 | 01 August 2018 |
| | | JP 6427789 B2 | 28 November 2018 |
| | | JP 6678941 B2 | 15 April 2020 |
| | | JP 6741349 B2 | 19 August 2020 |
| | | KR 10-1002443 B1 | 17 December 2010 |
| | | KR 10-1017732 B1 | 28 February 2011 |
| | | KR 10-1053739 B1 | 02 August 2011 |
| | | KR 10-1143035 B1 | 08 May 2012 |
| | | KR 10-1287280 B1 | 23 July 2013 |
| | | KR 10-1410521 B1 | 20 June 2014 |
| | | KR 10-1482295 B1 | 13 January 2015 |
| | | KR 10-1583388 B1 | 07 January 2016 |
| | | KR 10-2002-0020730 A | 15 March 2002 |
| | | KR 10-2004-0089694 A | 21 October 2004 |
| | | KR 10-2004-0089695 A | 21 October 2004 |
| | | KR 10-2005-0010054 A | 26 January 2005 |
| | | KR 10-2005-0104354 A | 02 November 2005 |
| | | KR 10-2008-0055932 A | 19 June 2008 |
| | | KR 10-2008-0097995 A | 06 November 2008 |
| | | KR 10-2010-0085932 A | 29 July 2010 |
| | | KR 10-2010-0132484 A | 17 December 2010 |
| | | KR 10-2015-0093159 A | 17 August 2015 |
| | | KR 10-2018-0023915 A | 07 March 2018 |
| | | MX 2010004547 A | 05 July 2010 |
| | | MX 2010006728 A | 05 November 2010 |
| | | MX 2015007343 A | 10 September 2015 |
| | | MX 342894 B | 18 October 2016 |
| | | PL 2396036 T3 | 29 December 2017 |
| | | PL 378012 A1 | 20 February 2006 |
| | | PT 1194167 E | 23 October 2009 |
| | | PT 1450891 E | 31 January 2013 |
| | | RU 2019119320 A3 | 12 January 2021 |
| | | RU 2349343 C2 | 20 March 2009 |
| | | SG 153825 A1 | 29 July 2009 |
| | | TR 201901824 T4 | 21 March 2019 |
| | | TR 201910848 T4 | 21 August 2019 |
| | | US 10344037 B2 | 09 July 2019 |
| | | US 10377829 B2 | 13 August 2019 |
| | | US 10413539 B2 | 17 September 2019 |
| | | US 10561738 B2 | 18 February 2020 |
| | | US 10653793 B2 | 19 May 2020 |
| | | US 10682347 B2 | 16 June 2020 |
| | | US 10709701 B2 | 14 July 2020 |
| | | US 10744129 B2 | 18 August 2020 |
| | | US 10751420 B2 | 25 August 2020 |
| | | US 1084828 B2 | 10 August 2021 |
| | | US 10849986 B2 | 01 December 2020 |

Form PCT/ISA/210 (patent family annex) (July 2022)

**INTERNATIONAL SEARCH REPORT**
**Information on patent family members**

International application No.
**PCT/KR2023/009854**

| Patent document cited in search report | Publication date (day/month/year) | Patent family member(s) | Publication date (day/month/year) |
|---|---|---|---|
| | | US 2002-0041847 A1 | 11 April 2002 |
| | | US 2002-0071807 A1 | 13 June 2002 |
| | | US 2003-0124058 A1 | 03 July 2003 |
| | | US 2003-0133930 A1 | 17 July 2003 |
| | | US 2003-0171637 A1 | 11 September 2003 |
| | | US 2004-0202666 A1 | 14 October 2004 |
| | | US 2004-0219203 A1 | 04 November 2004 |
| | | US 2004-0230087 A1 | 18 November 2004 |
| | | US 2005-0014207 A1 | 20 January 2005 |
| | | US 2005-0191300 A1 | 01 September 2005 |
| | | US 2005-0271671 A1 | 08 December 2005 |
| | | US 2006-0014245 A1 | 19 January 2006 |
| | | US 2006-0228300 A1 | 12 October 2006 |
| | | US 2006-0228357 A1 | 12 October 2006 |
| | | US 2006-0235365 A1 | 19 October 2006 |
| | | US 2006-0264688 A1 | 23 November 2006 |
| | | US 2007-0020265 A1 | 25 January 2007 |
| | | US 2007-0086942 A1 | 19 April 2007 |
| | | US 2007-0087001 A1 | 19 April 2007 |
| | | US 2007-0135674 A1 | 14 June 2007 |
| | | US 2008-0050311 A1 | 28 February 2008 |
| | | US 2008-0253964 A1 | 16 October 2008 |
| | | US 2009-0060862 A1 | 05 March 2009 |
| | | US 2010-0189641 A1 | 29 July 2010 |
| | | US 2010-0266497 A1 | 21 October 2010 |
| | | US 2010-0272636 A1 | 28 October 2010 |
| | | US 2010-0284906 A1 | 11 November 2010 |
| | | US 2011-0008251 A1 | 13 January 2011 |
| | | US 2011-0020273 A1 | 27 January 2011 |
| | | US 2011-0300105 A1 | 08 December 2011 |
| | | US 2011-0305631 A1 | 15 December 2011 |
| | | US 2011-0311531 A1 | 22 December 2011 |
| | | US 2011-0318306 A1 | 29 December 2011 |
| | | US 2012-0009149 A1 | 12 January 2012 |
| | | US 2012-0040431 A1 | 16 February 2012 |
| | | US 2012-0076727 A1 | 29 March 2012 |
| | | US 2013-0164816 A1 | 27 June 2013 |
| | | US 2013-0171064 A1 | 04 July 2013 |
| | | US 2013-0171065 A1 | 04 July 2013 |
| | | US 2014-0066470 A1 | 06 March 2014 |
| | | US 2014-0377177 A1 | 25 December 2014 |
| | | US 2014-0377287 A1 | 25 December 2014 |
| | | US 2015-0010982 A1 | 08 January 2015 |
| | | US 2015-0023870 A1 | 22 January 2015 |
| | | US 2015-0024458 A1 | 22 January 2015 |
| | | US 2015-0038686 A1 | 05 February 2015 |
| | | US 2015-0050715 A1 | 19 February 2015 |
| | | US 2015-0056680 A1 | 26 February 2015 |
| | | US 2015-0276812 A1 | 01 October 2015 |
| | | US 2015-0374846 A1 | 31 December 2015 |

Form PCT/ISA/210 (patent family annex) (July 2022)

**INTERNATIONAL SEARCH REPORT**
**Information on patent family members**

International application No.
**PCT/KR2023/009854**

| Patent document cited in search report | Publication date (day/month/year) | Patent family member(s) | Publication date (day/month/year) |
|---|---|---|---|
| | | US 2016-0024212 A1 | 28 January 2016 |
| | | US 2017-0320952 A1 | 09 November 2017 |
| | | US 2017-0334996 A1 | 23 November 2017 |
| | | US 2017-0360770 A1 | 21 December 2017 |
| | | US 2018-0079816 A1 | 22 March 2018 |
| | | US 2018-0085469 A1 | 29 March 2018 |
| | | US 2018-0170944 A1 | 21 June 2018 |
| | | US 2018-0327495 A1 | 15 November 2018 |
| | | US 2019-0015405 A1 | 17 January 2019 |
| | | US 2019-0367529 A1 | 05 December 2019 |
| | | US 2020-0276325 A1 | 03 September 2020 |
| | | US 2020-0316054 A1 | 08 October 2020 |
| | | US 6183744 B1 | 06 February 2001 |
| | | US 6306393 B1 | 23 October 2001 |
| | | US 6761680 B2 | 13 July 2004 |
| | | US 6786858 B2 | 07 September 2004 |
| | | US 6820318 B2 | 23 November 2004 |
| | | US 6997862 B2 | 14 February 2006 |
| | | US 7008368 B2 | 07 March 2006 |
| | | US 7060020 B2 | 13 June 2006 |
| | | US 7074291 B2 | 11 July 2006 |
| | | US 7074403 B1 | 11 July 2006 |
| | | US 7094198 B2 | 22 August 2006 |
| | | US 7211039 B2 | 01 May 2007 |
| | | US 7238785 B2 | 03 July 2007 |
| | | US 7244226 B2 | 17 July 2007 |
| | | US 7252630 B2 | 07 August 2007 |
| | | US 7282567 B2 | 16 October 2007 |
| | | US 7312318 B2 | 25 December 2007 |
| | | US 7407477 B2 | 05 August 2008 |
| | | US 7497818 B2 | 03 March 2009 |
| | | US 7517964 B2 | 14 April 2009 |
| | | US 7521056 B2 | 21 April 2009 |
| | | US 7597876 B2 | 06 October 2009 |
| | | US 7612180 B2 | 03 November 2009 |
| | | US 7641901 B2 | 05 January 2010 |
| | | US 7642239 B2 | 05 January 2010 |
| | | US 7666400 B2 | 23 February 2010 |
| | | US 7942803 B2 | 17 May 2011 |
| | | US 7981398 B2 | 19 July 2011 |
| | | US 7993626 B2 | 09 August 2011 |
| | | US 7999083 B2 | 16 August 2011 |
| | | US 8003111 B2 | 23 August 2011 |
| | | US 8343460 B2 | 01 January 2013 |
| | | US 8343496 B2 | 01 January 2013 |
| | | US 8349332 B2 | 08 January 2013 |
| | | US 8361464 B2 | 29 January 2013 |
| | | US 8367037 B2 | 05 February 2013 |
| | | US 8632752 B2 | 21 January 2014 |
| | | US 8652484 B2 | 18 February 2014 |

Form PCT/ISA/210 (patent family annex) (July 2022)

**INTERNATIONAL SEARCH REPORT**
**Information on patent family members**

International application No.
**PCT/KR2023/009854**

| Patent document cited in search report | Publication date (day/month/year) | Patent family member(s) | | | Publication date (day/month/year) |
|---|---|---|---|---|---|
| | | US | 8658773 | B2 | 25 February 2014 |
| | | US | 9422303 | B2 | 23 August 2016 |
| | | US | 9513293 | B2 | 06 December 2016 |
| | | US | 9522959 | B2 | 20 December 2016 |
| | | US | 9540435 | B2 | 10 January 2017 |
| | | US | 9709603 | B2 | 18 July 2017 |
| | | US | 9849176 | B2 | 26 December 2017 |
| | | US | 9855344 | B2 | 02 January 2018 |
| | | US | 9862770 | B2 | 09 January 2018 |
| | | US | 9872920 | B2 | 23 January 2018 |
| | | US | 9931413 | B2 | 03 April 2018 |
| | | US | 9931417 | B2 | 03 April 2018 |
| | | US | 9963516 | B2 | 08 May 2018 |
| | | US | 9999668 | B2 | 19 June 2018 |
| | | WO | 00-67795 | A1 | 16 November 2000 |
| | | WO | 00-74718 | A1 | 14 December 2000 |
| | | WO | 03-039463 | A2 | 15 May 2003 |
| | | WO | 03-039463 | A3 | 31 July 2003 |
| | | WO | 2004-110390 | A2 | 23 December 2004 |
| | | WO | 2004-110390 | A3 | 28 April 2005 |
| | | WO | 2005-080586 | A1 | 01 September 2005 |
| | | WO | 2006-094192 | A2 | 08 September 2006 |
| | | WO | 2006-094192 | A3 | 09 November 2006 |
| | | WO | 2007-046893 | A2 | 26 April 2007 |
| | | WO | 2007-046893 | A3 | 23 April 2009 |
| | | WO | 2007-112193 | A2 | 04 October 2007 |
| | | WO | 2007-112193 | A3 | 20 November 2008 |
| | | WO | 2007-134037 | A2 | 22 November 2007 |
| | | WO | 2007-134037 | A3 | 27 November 2008 |
| | | WO | 2008-088648 | A2 | 24 July 2008 |
| | | WO | 2008-088648 | A3 | 25 September 2008 |
| | | WO | 2008-088658 | A2 | 24 July 2008 |
| | | WO | 2008-088658 | A3 | 20 November 2008 |
| | | WO | 2009-055653 | A1 | 30 April 2009 |
| | | WO | 2009-079024 | A1 | 25 June 2009 |
| | | WO | 2009-100194 | A2 | 13 August 2009 |
| | | WO | 2009-100194 | A3 | 07 January 2010 |
| | | WO | 2009-126558 | A1 | 15 October 2009 |
| | | WO | 2009-135015 | A2 | 05 November 2009 |
| | | WO | 2009-135015 | A3 | 07 January 2010 |
| | | WO | 2009-135015 | A8 | 08 April 2010 |
| | | WO | 2010-017500 | A2 | 11 February 2010 |
| | | WO | 2010-017500 | A3 | 22 April 2010 |
| | | WO | 2010-022225 | A1 | 25 February 2010 |
| | | WO | 2010-022225 | A9 | 28 April 2011 |
| | | WO | 2010-093395 | A1 | 19 August 2010 |
| | | WO | 2010-117984 | A1 | 14 October 2010 |
| | | WO | 2011-025904 | A1 | 03 March 2011 |
| | | WO | 2012-024223 | A2 | 23 February 2012 |
| | | WO | 2012-024223 | A3 | 05 July 2012 |

Form PCT/ISA/210 (patent family annex) (July 2022)

**INTERNATIONAL SEARCH REPORT**
**Information on patent family members**

International application No.
**PCT/KR2023/009854**

| Patent document cited in search report | Publication date (day/month/year) | Patent family member(s) | Publication date (day/month/year) |
|---|---|---|---|
| | | WO 2012-058666 A2 | 03 May 2012 |
| | | WO 2012-058666 A3 | 10 January 2013 |
| | | WO 2012-061548 A1 | 10 May 2012 |
| | | WO 2012-075361 A2 | 07 June 2012 |
| | | WO 2012-075361 A3 | 26 July 2012 |
| | | WO 2012-082618 A2 | 21 June 2012 |
| | | WO 2012-082618 A3 | 07 September 2012 |
| | | WO 2012-162583 A1 | 29 November 2012 |
| | | WO 2013-052484 A1 | 11 April 2013 |
| | | WO 2013-082254 A1 | 06 June 2013 |
| | | WO 2013-112801 A1 | 01 August 2013 |
| | | WO 2014-092804 A1 | 19 June 2014 |
| | | WO 2014-165506 A1 | 09 October 2014 |
| | | WO 2015-012904 A2 | 29 January 2015 |
| | | WO 2015-012904 A3 | 07 May 2015 |
| | | WO 2015-047510 A1 | 02 April 2015 |
| | | WO 2015-148415 A2 | 01 October 2015 |
| | | WO 2015-148415 A3 | 10 December 2015 |
| | | WO 2015-153176 A1 | 08 October 2015 |
| | | WO 2016-003869 A1 | 07 January 2016 |
| | | WO 2016-057398 A1 | 14 April 2016 |
| | | WO 2016-077061 A2 | 19 May 2016 |
| | | WO 2016-077061 A3 | 18 August 2016 |
| | | WO 2016-118353 A1 | 28 July 2016 |
| | | WO 2016-210108 A1 | 29 December 2016 |
| | | WO 2017-004144 A1 | 05 January 2017 |
| | | WO 2017-034906 A1 | 02 March 2017 |
| | | WO 2017-034906 A8 | 12 October 2017 |
| | | WO 2017-180565 A1 | 19 October 2017 |
| | | WO 2018-031408 A1 | 15 February 2018 |
| | | WO 2018-102212 A1 | 07 June 2018 |
| | | WO 2018-156634 A1 | 30 August 2018 |
| | | WO 98-42378 A1 | 01 October 1998 |
| | | ZA 982438 B | 04 November 1998 |
| WO 2022-170971 A1 | 18 August 2022 | CN 115279417 A | 01 November 2022 |
| | | TW 202241521 A | 01 November 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9746260 A
- KR 102087017 B1


### Non-patent literature cited in the description


- **SECCHI, M** ; **LODOLA, C** ; **GARBELLI, A** ; **BIONE, S.** ; **MAGA, G.** DEAD-Box RNA Helicases DDX3X and DDX5 as Oncogenes or Oncosuppressors: A Network Perspective. *Cancers*, 2022, vol. 14, 3820, https://doi.org/10.3390/cancers14153820
- Remington's Pharmaceutical Sciences. 1980